# EUROPEAN PATENT APPLICATION

(11) **EP 4 818 331 A2**
(43) Date of publication of application: **07.10.2026**
(21) Application number: 26199715.9
(22) Date of filing: 09.03.2022
(51) Int. Cl.: C12Q 1/6883

(54) **PREDICTION OF PREECLAMPSIA RISK USING CIRCULATING, CELL-FREE RNA**

(30) Priority: 10.03.2021 US 202163159400 P; 05.11.2021 US 202163276467 P
(62) Document number(s) of the earlier application(s) in accordance with Art. 76 EPC: 22767942.0 / 4 305 204
(71) Applicant: CZ Biohub SF, LLC, San Francisco, CA 94158 (US); The Board of Trustees of the Leland Stanford Junior University, Stanford, CA 94305-2038 (US)
(72) Inventor: MOUFARREJ, Mira N., STANFORD (CA) 94305-2038 (US); VORPERIAN, Sevahn K., STANFORD (CA) 94305-2038 (US); SHAW, Gary M., STANFORD (CA) 94305-2038 (US); STEVENSON, David K., STANFORD (CA) 94305-2038 (US); QUAKE, Stephen R., SAN FRANCISCO (CA) 94158 (US)
(74) Representative: Studio Torta S.p.A.

(57) **Abstract**

The disclosure describes changes in cfRNA gene expression that are associated with risk for preeclampsia. Accordingly, the disclosure provides methods and kits for preeclampsia risk assessment.

## Description

### INCORPORATION BY REFERENCE OF MATERIAL SUBMITTED ELECTRONICALLY

The Sequence Listing, which is a part of the present disclosure, is submitted concurrently with the specification as a text file. The name of the text file containing the Sequence Listing is " 57406_Seqlisting.txt", which was created on March 9, 2022 and is 75,488 bytes in size. The subject matter of the Sequence Listing is incorporated herein in its entirety by reference.

### BACKGROUND OF THE INVENTION

Advances in obstetrics and neonatology have significantly mitigated many of the adverse pregnancy outcomes related to preterm birth (PTB) and preeclampsia (PE) ^{1,2}. Nonetheless, the standards of care implemented today focus on how to treat a mother and child once a complication has been diagnosed, proving both insufficient and costly ³⁻⁶: PE and related hypertensive disorders cause 14% of maternal deaths each year globally, second only to hemorrhage ⁷ and cost $2B in care in the first year following delivery ⁵. Worse, 3 out of 5 maternal deaths in the USA are preventable and often associated with a missed or delayed complication diagnosis ⁸. Such outcomes highlight the need for tools that would aid in identifying which women are at risk for hypertensive diseases, such as PE, before clinical development. Indeed, early prediction of PE, which has not been achieved to date, may prevent or reduce a pregnant mother's risk of developing PE ^{9,10} if coupled with appropriate treatment.

PE globally affects 4-5% of pregnancies ¹¹⁻¹³ and is associated with a significant increase in adverse maternal (*e.g*., maternal death, heart attack, stroke, seizures, and hemorrhage) and perinatal (e.g., fetal growth restriction and PTB coupled with respiratory distress syndrome, intraventricular hemorrhage, cerebral palsy, and bronchopulmonary dysplasia) outcomes ¹⁴⁻¹⁸. Long-term, PE presents an increased maternal risk for cardiovascular ^{19,20} and kidney ^{21,22} diseases. Formally defined as new-onset hypertension coupled with proteinuria or other end-organ damage (e.g., liver, brain) occurring after 20 weeks of gestation ²³, PE can clinically manifest anytime thereafter, including into the post-partum period ²⁴. Detection and diagnosis itself, however, can prove challenging as early signs such as headaches and nausea can be easily confused with general pregnancy discomfort; and because PE shares many signs and symptoms with other common complications like gestational thrombocytopenia and chronic hypertension.

To date, no recommended test exists to predict the future onset of PE early in pregnancy ⁹, and proposed investigational methods that measure diverse biophysical and biochemical signals including the measurement of two angiogenic factors [soluble fms-like tyrosine kinase-1 (sFlt1), placental growth factor (PlGF)] in the second and third trimester ^{25,26} have so far yielded low, uninformative positive predictive values (8-33%) ²⁷. A test with good performance metrics could guide the prophylactic use of low-dose aspirin, which has been shown to reduce the risk of PE if initially administered before 16 weeks of gestation ^{10,28}.

Liquid biopsies that measure plasma cell-free RNA (cfRNA) suggest a means to bridge this gap in clinical care; however, until recently, such work often failed to progress beyond initial discovery ³⁰. Recent efforts have instead either focused on confirmation of PE at clinical diagnosis ³¹ or on limited discovery-stage work (n = 5 PE) earlier in pregnancy with encouraging but unvalidated results ³³. Consequently, the prediction of PE early in gestation (≤ 16 weeks), long before symptoms present when such a test would be most useful to guide the prophylactic use of potential therapeutics (e.g., low-dose aspirin) remains a key objective to improve obstetric care ⁹.

Further, clinical care may also be improved by better understanding the pathogenesis of PE. PE is a disease specific to humans as it does not occur in other species ³⁴. Broadly, it is accepted that PE occurs in two stages - abnormal placentation occurring early in pregnancy followed by systemic endothelial dysfunction ^{15,32,34}. Because PE can clinically present any time after 20 weeks of gestation and with a diversity of symptoms, significant effort has been made to sub-classify the disease based upon the timing of onset (i.e., early-onset at < 34 weeks of gestation vs late-onset thereafter) as a proxy for pathology ^{37,38}; however, debate over the significance of such subtypes is still ongoing ^{34,36,39,40}. Noninvasive methods such as liquid biopsies thus present a means to indirectly observe pathogenesis in real time and identify biological changes associated with PE for all proposed subtypes and both prior to and at diagnosis.

### BRIEF SUMMARY

The present disclosure describes cfRNA transcriptomic changes across gestation and at post-partum that are associated with preeclampsia (PE).

As detailed herein, in one embodiment, evaluation of expression of an 11-gene panel, and subsets thereof, in cfRNA provides a predictive signature of preeclampsia, for example in some embodiments, in cfRNA samples from early time points in pregnancy. In another embodiment evaluation of expression of an 18-gene panel, or subsets thereof, in cfRNA provides a predictive signature of preeclampsia. This summary highlights certain aspects, but not every aspect, of the disclosure
In one aspect, the disclosure provides a method of evaluating risk of preeclampsia in a pregnant subject, or of diagnosing preeclampsia in the pregnant subject, the method comprising quantifying levels of cell-free RNA from a biological sample from the pregnant subject to obtain a risk score, wherein (1) the logarithm of change in expression of each of the quantified genes relative to a reference level obtained from control subjects not at risk of developing preeclampsia is at least ± 0.2 ( | log(FC) | ≥ 0.2); (2) the coefficient of variation of each of the quantified genes relative to the reference level is at most 2; (3) the median expression across all samples is at least 5 counts per million reads (CPM); or (4) a combination of one or more of (1), (2), and (3); wherein an increased risk of preeclampsia is assigned to the pregnant subject when the risk score exceeds a threshold value. In some embodiments, at least two of the two or more genes are selected from the genes listed in Table 4 and Table 12. In some embodiments, the panel comprises at least three, four, five, six, seven, eight, nine, ten, eleven, twelve, thirteen, fourteen, fifteen, sixteen, seventeen, eighteen, nineteen, or at least twenty, thirty, forty, or at least fifty genes selected from the genes listed in Tables 4 and Table 12. In some embodiments, at least one gene of the panel is selected from the genes listed in Table 4. In some embodiments, the control subjects are pregnant normotensive subjects. In some embodiments, the biological sample from the pregnant subject is serum or plasma. In some embodiments, change in expression of each of the quantified genes relative to the reference level is at least 1.5-fold. In some embodiments, the cfRNA sample is from a cell-free blood sample obtained at 5 weeks or later gestation. In some embodiments, the cfRNA sample is from a cell-free blood sample obtained at 5-12 weeks of gestation. In some embodiments, the cfRNA sample is from a cell-free blood sample obtained at 13-18 weeks of gestation. In some embodiments, the cfRNA sample is from a cell-free blood sample obtained at 23-33 weeks of gestation. In some embodiments, the cfRNA sample is from a cell-free blood sample obtained after 33 weeks of gestation. In some embodiments, the step of quantifying the level of cfRNA comprises performing an amplification reaction. In some embodiments, is an RT-PCR reaction. In some embodiments, the step of quantifying the level of cfRNA comprises massively parallel sequencing.

In an additional aspect, the disclosure provides a method of evaluating risk of preeclampsia in a pregnant subject, or of diagnosing preeclampsia in the pregnant subject, the method comprising:
quantifying, in a biological sample obtained from the pregnant subject, levels of cell-free RNA (cfRNA) expressed by two or more genes, or three or more genes, selected from the group consisting of BNIP3L, FECH, HEMGN, SNCA, OAZ1, GSPT1, AKNA, CSF3R, IGF2, RPS15, and MARCH2 in cfRNA from the pregnant subject compared to reference levels of RNA in cfRNA in control subjects;
identifying an increased risk of preeclampsia when the level of cfRNA expressed by each of the two or more genes, or each of the three or more genes, exhibits a change in expression associated with preeclampsia relative to reference levels. In some embodiments, the method comprises quantifying RNA expressed by four or more genes selected from the group consisting of BNIP3L, FECH, HEMGN, SNCA, OAZ1, GSPT1, AKNA, CSF3R, IGF2, RPS15, and MARCH2 compared to reference levels of RNA in control subjects; and identifying an increased risk of preeclampsia when the level of cfRNA expressed by each of the four or more genes exhibits a change in expression associated with preeclampsia relative to reference levels. In some embodiments, the method comprises quantifying RNA expressed by five, six, seven, eight, nine, ten, or all of genes BNIP3L, FECH, HEMGN, SNCA, OAZ1, GSPT1, AKNA, CSF3R, IGF2, RPS15, and MARCH2 compared to reference levels of RNA in control subjects; and
identifying an increased risk of preeclampsia when the level of cfRNA expressed by each of the five, six, seven, eight, nine, ten, or all of genes exhibits a change in expression associated with preeclampsia relative to reference levels. In some embodiments, the method further comprises quantifying cfRNA expressed by one or more genes listed in Table 9. In some embodiments, the method further comprises quantifying cfRNA expressed by one or more genes listed in Table 12. In some embodiments, comparison of expression levels in cfRNA from the pregnant subject to reference levels is performed by applying a classifier. In some embodiments, the classifier is a regression model. In some embodiments, the control subjects are pregnant normotensive subjects. In some embodiments, the biological sample from the pregnant subject is serum or plasma. In some embodiments, change in expression of each of the quantified genes relative to the reference level is at least 1.5-fold. In some embodiments, the cfRNA sample is from a cell-free blood sample obtained at 5 weeks or later gestation. In some embodiments, the cfRNA sample is from a cell-free blood sample obtained at 5-12 weeks of gestation. In some embodiments, the cfRNA sample is from a cell-free blood sample obtained at 13-18 weeks of gestation. In some embodiments, the cfRNA sample is from a cell-free blood sample obtained at 23-33 weeks of gestation. In some embodiments, the cfRNA sample is from a cell-free blood sample obtained after 33 weeks of gestation. In some embodiments, the step of quantifying the level of cfRNA comprises performing an amplification reaction. In some embodiments, is an RT-PCR reaction. In some embodiments, the step of quantifying the level of cfRNA comprises massively parallel sequencing.

In a further aspect, the disclosure provides a method of processing a sample to evaluate risk of preeclampsia in a pregnant subject, the method comprising:
providing cell-free RNA (cfRNA) sample from a biological sample from the pregnant subject; and
quantifying levels of cfRNA expressed by two or more genes, or three or more genes, selected from the group consisting of BNIP3L, FECH, HEMGN, SNCA, OAZ1, GSPT1, AKNA, CSF3R, IGF2, RPS15, and MARCH2 in cfRNA from the pregnant subject compared to reference levels of RNA in cfRNA in control subjects. In some embodiments, the biological sample is serum or plasma. In some embodiments, change in expression of each of the quantified genes is at least 1.5-fold compared to the level in normotensive human females. In some embodiments, the cfRNA sample is from a cell-free blood sample obtained at 5 weeks or later of gestation. In some embodiments, the cfRNA sample is from a cell-free blood sample obtained at 5-12 weeks of gestation. In some embodiments, the cfRNA sample is from a cell-free blood sample obtained at 13-18 weeks of gestation. In some embodiments, the cfRNA sample is from a cell-free blood sample obtained at 23-33 weeks of gestation. In some mbodiments, the cfRNA sample is from a cell-free blood sample obtained later than 33 weeks of gestation. In some embodiments, the step of quantifying the level of RNA comprises performing an amplification reaction. In some embodiments, the amplification reaction is an RT-PCR reaction. In some embodiments, the step of quantifying the level of RNA comprises massively parallel sequencing.

In another aspect, the disclosure provides a kit comprising primers for multiplex amplification for two, three, four, five, six, seven, eight, nine, ten, or all of genes BNIP3L, FECH, HEMGN, SNCA, OAZ1, GSPT1, AKNA, CSF3R, IGF2, RPS15, and MARCH2. In some embodiments, the kit does not comprise primers for amplification of more than 20 gene, more than 50 genes, more than 100 genes, more than 500 genes, or more than 1,000 genes.

In a further aspect, described herein is a method of evaluating risk of preeclampsia in a pregnant subject, or of diagnosing preeclampsia in the pregnant subject, the method comprising quantifying levels of cell-free RNA from a biological sample from the pregnant subject to obtain a risk score, wherein (1) the logarithm of change in expression of each of the quantified genes relative to a reference level obtained from control subjects not at risk of developing preeclampsia is at least ± 0.2 ( | log(FC) | ≥ 0.2); (2) the coefficient of variation of each of the quantified genes relative to the reference level is at most 6; (3) the median expression across all samples is at least 5 counts per million reads (CPM); or (4) a combination of one or more of (1), (2), and (3); wherein an increased risk of preeclampsia is assigned to the pregnant subject when the risk score exceeds a threshold value. In some embodiments, the logarithm of change in expression of each of the quantified genes relative to a reference level obtained from control subjects not at risk of developing preeclampsia is at least ± 0.2. In some embodiments, at least two of the two or more genes are selected from the genes listed in Table 17. In some embodiments, cfRNA is quantified for at least two, three, four, five, six, seven, eight, nine, or ten genes listed in Table 21A and/or Table 21B, and/or Table 21C. In some embodiments cfRNA is quantified for at least two, three, four, five, six, seven, eight, nine, or ten genes genes listed in Table 23. In some embodiments, cfRNA is quantified for at least two, or at least three, four, five, six, seven, eight, nine, or ten genes listed in Table 22. In some embodiments, cfRNA is quantified for at least eleven, twelve, thirteen, fourteen, fifteen, sixteen, seventeen, eighteen, nineteen, or at least twenty, thirty, forty, or at least fifty genes selected from the genes listed in Table 22. In some embodiments, cfRNA is quantified for at least eleven, twelve, thirteen, fourteen, fifteen, sixteen, seventeen, eighteen, nineteen, or at least twenty, thirty, forty, or at least fifty genes selected from the genes listed in Table 23.

In a further aspect, the disclosure describes a method of evaluating risk of preeclampsia in a pregnant subject, or of diagnosing preeclampsia in the pregnant subject, the method comprising:
quantifying, in a biological sample obtained from the pregnant subject, levels of cell-free RNA (cfRNA) for one or more of, two or more of, or three or more of CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564) compared to reference levels of RNA in cfRNA in control subjects; and
identifying an increased risk of preeclampsia when the level of cfRNA expressed by the one or more of, each of the two or more of, or each of the three or more of, exhibits a change in expression associated with preeclampsia relative to reference levels. In some embdoiments, . the levels of cfRNA for a combination of genes set forth in Table 20 is determined. In some embodiments, the methods further comprise evaluating the level of cfRNA for a gene set forth in Table 23. In some embodiments, the methods further comprises evaluating the level of cfRNA for a sequence set forth in Table 22.

In a further aspect, the disclosure describes a method of evaluating risk of severe preeclampsia in a pregnant subject, the method comprising:
quantifying, in a biological sample obtained from the pregnant subject, levels of cell-free RNA (cfRNA) for two or more genes, or three or more genes, selected from the genes listed in Table 24; and
identifying an increased risk of severe preeclampsia when the level of cfRNA expressed by each of the two or more genes, or each of the three or more genes, exhibits a change in expression associated with preeclampsia relative to reference levels. In some embodiments, the method comprises quantifying cfRNA for two or more genes selected from the genes listed in Table 25A, quantifying cfRNA for two or more genes selected from the genes listed in Table 25B, and/or quantifying cfRNA for two or more genes selected from the genes listed in Table 25C.

In another aspect, the disclosure provides a method of monitoring tissue or cell-type health in a pregnant subject, the method comprising: quantifying, in a biological sample obtained from the pregnant subject, levels of cell-free RNA (cfRNA) expressed by two, three, four, five, six, seven, eight, nine, or ten or more genes selected from the genes listed in Table 26; and identifying declining health of the tissue or cell-type when the level of cfRNA expressed by each of the two, three, four, five, six, seven, eight, nine, or ten or more genes, exhibits a change in expression associated with declining health of the tissue or cell-type compared to reference levels. In some embodiments, brain, liver, kidney, heart, bone marrow, placenta, skeletal muscle, and/or smooth muscle is monitored. In some embodiments, astrocytes, excitatory neurons, inhibitory neurons, oligodendrocytes, oligodendrocyte progenitor cells, B-cells, T-cells, NK-cells, granulocytes, extravillous trophoblasts, syncytiotrophoblasts, proximal tubule cells, platelet, endothelial cells, hepatocytes, liver sinusoidal endothelial cells, atrial cardiomyocytes, and/or ventricular cardiomyocytes are monitored.

In some embodiments of the aspects decribed in the foregoing paragrpahs, comparison of expression levels in cfRNA from the pregnant subject to reference levels is performed by applying a classifier. In some embodiments, the classifier is a regression model. In some embodiments, the control subjects are pregnant normotensive subjects. In some embodiments the biological sample from the pregnant subject is serum or plasma. In some embodiments change in expression of each of the quantified genes relative to the reference level is at least 1.5-fold. In some embodiments, the cfRNA sample is from a cell-free blood sample obtained at 5 weeks or later gestation. In some embodiments, the cfRNA sample is from a cell-free blood sample obtained at 5-12 weeks of gestation. In some embodiments, the cfRNA sample is from a cell-free blood sample obtained at 13-18 weeks of gestation. In some embodiments, the cfRNA sample is from a cell-free blood sample obtained at 23-33 weeks of gestation. In some embodiments the cfRNA sample is from a cell-free blood sample obtained after 33 weeks of gestation. In some embodiments, the step of quantifying the level of cfRNA comprises performing an amplification reaction. In some embodiments, the amplification reaction is an RT-PCR reaction. In some embodiments, the step of quantifying the level of cfRNA comprises massively parallel sequencing.

In a further aspect, the disclosure describes a method of processing a sample to evaluate risk of preeclampsia in a pregnant subject, the method comprising: providing cell-free RNA (cfRNA) sample from a biological sample from the pregnant subject; and quantifying levels of cfRNA expressed by two or more genes, or three or more genes selected from the group consisting of CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564) in cfRNA from the pregnant subject compared to reference levels of RNA in cfRNA in control subjects. In some embodiments, the biological sample is serum or plasma. In some embodiments, change in expression of each of the quantified genes is at least 1.5-fold compared to the level in normotensive human females. In some embodiments, the cfRNA sample is from a cell-free blood sample obtained at 5 weeks or later of gestation. In some embodiments, the cfRNA sample is from a cell-free blood sample obtained at 5-12 weeks of gestation. In some embodiments, the cfRNA sample is from a cell-free blood sample obtained at 13-18 weeks of gestation. In some embodiments, the cfRNA sample is from a cell-free blood sample obtained at 23-33 weeks of gestation. In some embodiments, the cfRNA sample is from a cell-free blood sample obtained later than 33 weeks of gestation. In some embodiments, the step of quantifying the level of RNA comprises performing an amplification reaction. In some embodiments, the amplification reaction is an RT-PCR reaction. In some embodiments, the step of quantifying the level of RNA comprises massively parallel sequencing.

In a further aspect, the disclosure provides a kit comprising primers for multiplex amplification for two, three, four, five, six, seven, eight, nine, ten, or all of genes CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564); wherein the kit does not comprise primers for amplification of more than 100 genes. In some embodiments, the kit does not comprise primers for amplification of more than 20 gene, more than 50 genes, more than 500 genes, or more than 1,000 genes.

### BRIEF DESCRIPTION OF THE DRAWINGS

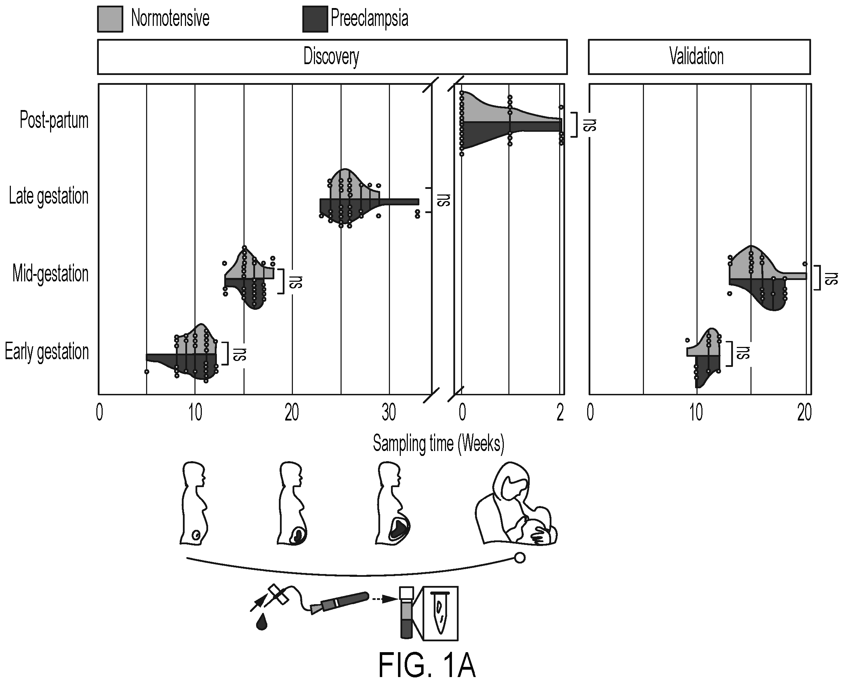

**FIG. 1A-D** **Sample, maternal, and pregnancy characteristics with the exception of gestational age at delivery are matched across NT and PE groups.** Panels illustrate matched sample collection time (weeks) in (A), matched maternal characteristics (Left to right: BMI, age, and previous pregnancies) in (B), matched gestational age at PE onset regardless of PE symptom severity in (C), and gestational age at delivery in (D). For (A), schematic depicts blood sampling across gestation and plasma isolation. X-axis represents gestational age during pregnancy and time post-delivery (weeks) thereafter (ns = not significant, ** = 10⁻⁵).

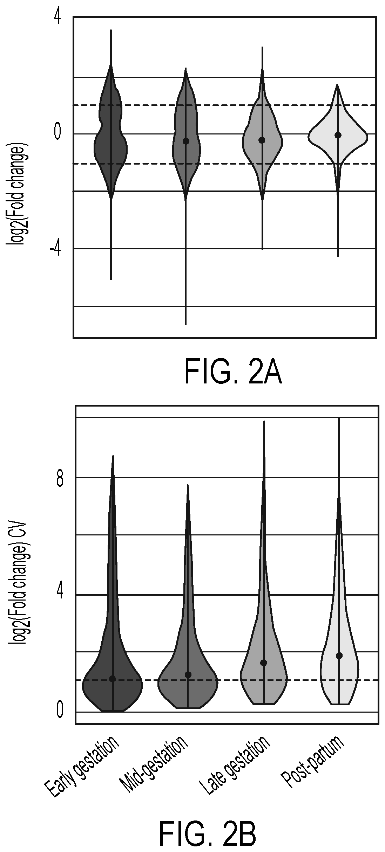

**FIG. 2A-E** **Across gestation and prior to diagnosis, changes in the cfRNA transcriptome segregate PE and NT samples and agree with known PE biology**. (A) Distribution of log₂(Fold change) 2ith dashed lines at log(Fold change) = ±1 and (B) coefficients of variation (CV) with dashed line at CV = 1 for all differentially expressed genes between PE and NT samples across gestation. In (B), 2, 9, 5, and 10 genes with CV > 10 across early, mid-, late gestation and post-partum respectively are omitted. (C) Across gestation, in each sample collection period, a subset of differentially expressed genes can separate PE and NT samples despite differences in symptom severity, PE onset subtype, and gestational age (GA) at delivery. (D) Across gestation, differentially expressed genes can be described by 3 longitudinal trends as revealed by k-means clustering. (E) The genes in each longitudinal trend group reflect known PE biology as highlighted across biological processes and the reactome. Some PE associated terms are emphasized in bold, colored text that corresponds to group color. Heatmap only includes parent terms.

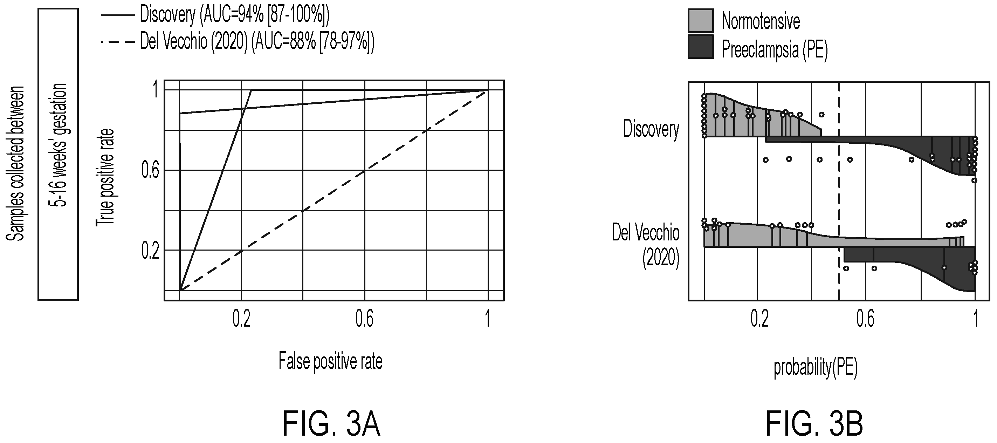

**FIG. 3A-E** **Subset of cfRNA changes can predict risk of preeclampsia early in gestation** Classifier performance as quantified by a receiver operator curve for samples collected in early gestation between 5-16 weeks in (A) and samples collected later in gestation between 17-38 weeks in (C). For each cohort including 3 independent previously published cohorts, legend states the area under the curve (AUC) and the corresponding 95% confidence interval in square brackets. (B, D) Estimated probability of PE as outputted by logistic regression for both PE and NT samples in each cohort shows that the model is well-calibrated and fairly confident (probability(PE) = 0 or 1) across most predictions. Dashed line at 0.5 indicates classifier cutoff where probability(PE) > 0.5 constitutes a sample predicted as PE. (E) Prediction of PE incorporates cfRNA levels for 11 genes for which centered log₂(Fold change) trends hold across discovery and independent validation (Del Vecchio (2020)) cohors.

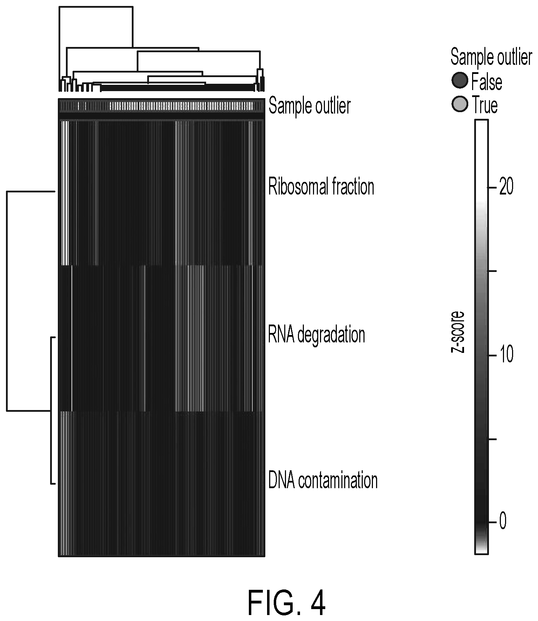

**FIG. 4** This figure depicts that sample with outlier values for at least one of QC metric cluster separately from most non-outlier samples.

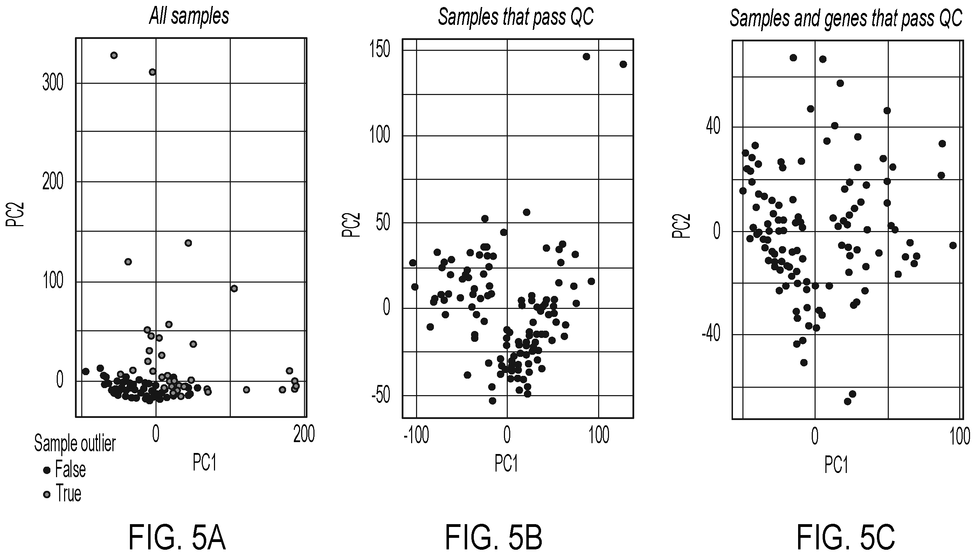

**FIG. 5A-C** **Sample outliers and poorly detected genes drive principal component analysis (PCA)and serve as leverage points.** Visualization of the top two principal components when performed using all samples and all genes (A) or only samples that pass QC metrics (B) reveals that certain samples can act as leverage points. Once sample outliers and lowly detected genes are removed from the cfRNA gene matrix (C), the top two principal components reflect natural variance in the data and are no longer driven by a few leverage points.

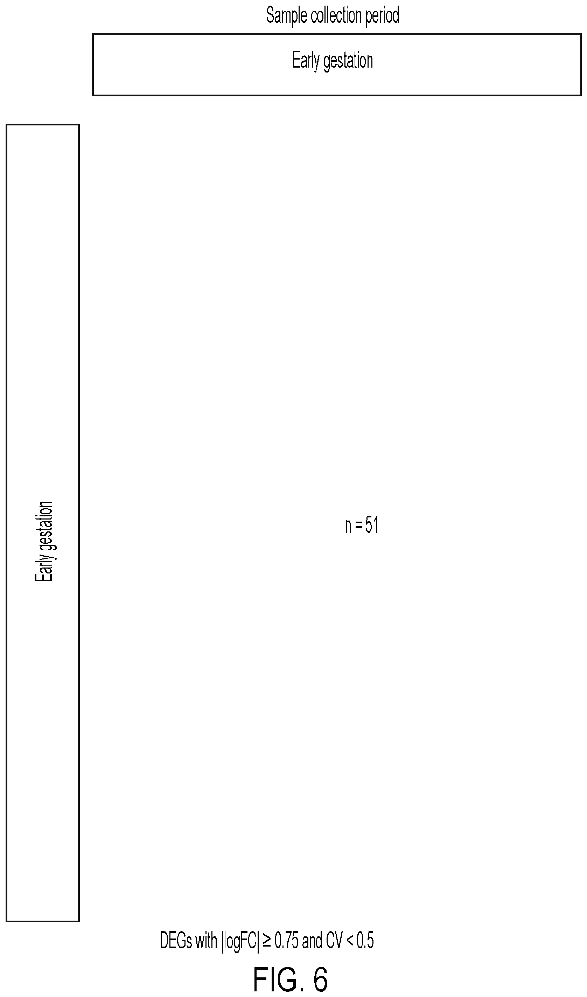

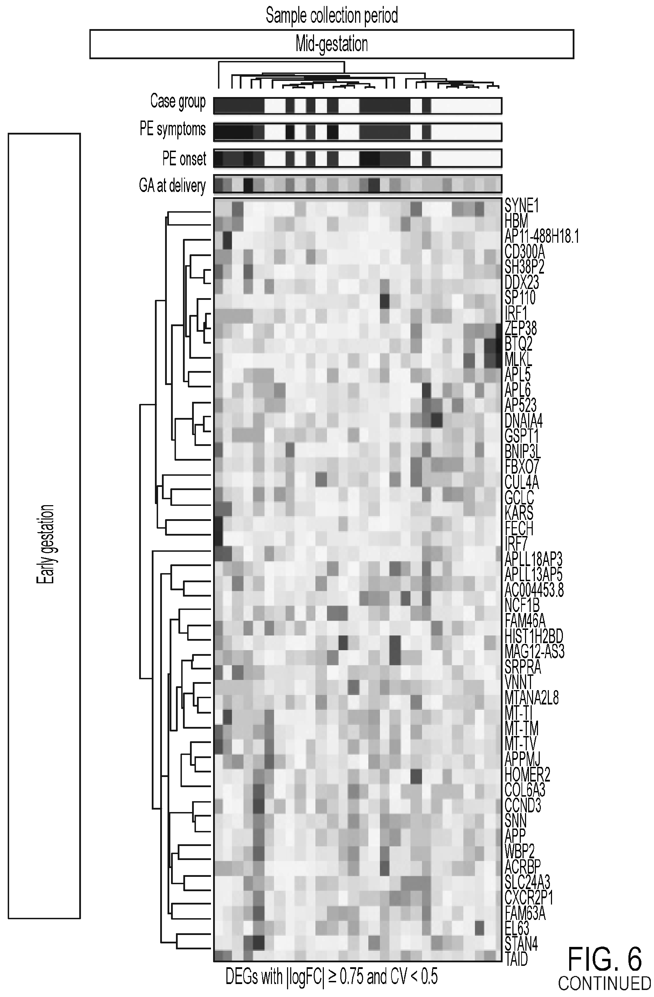

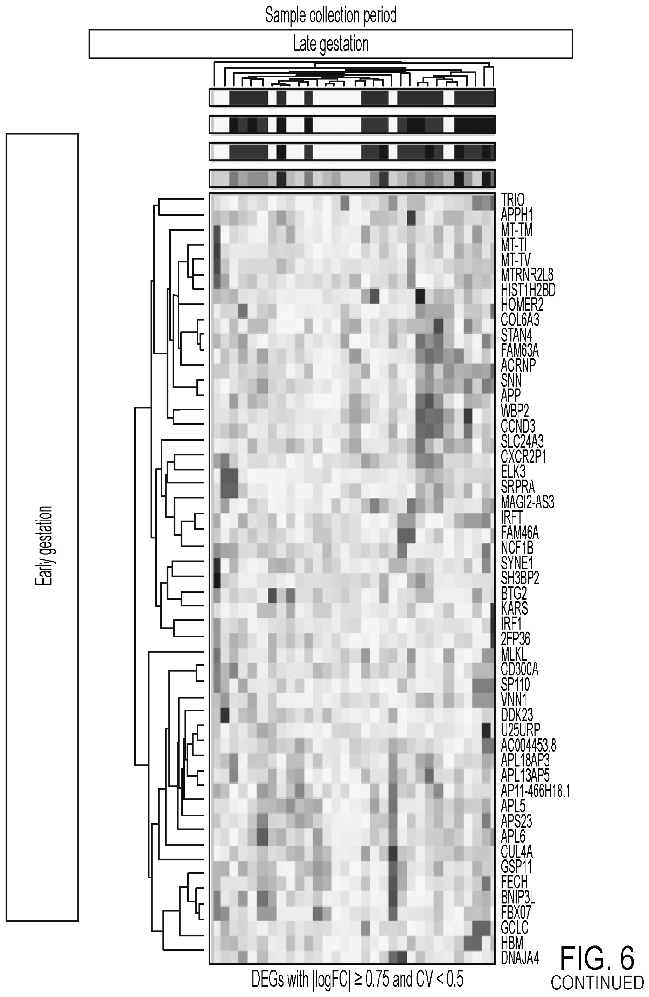

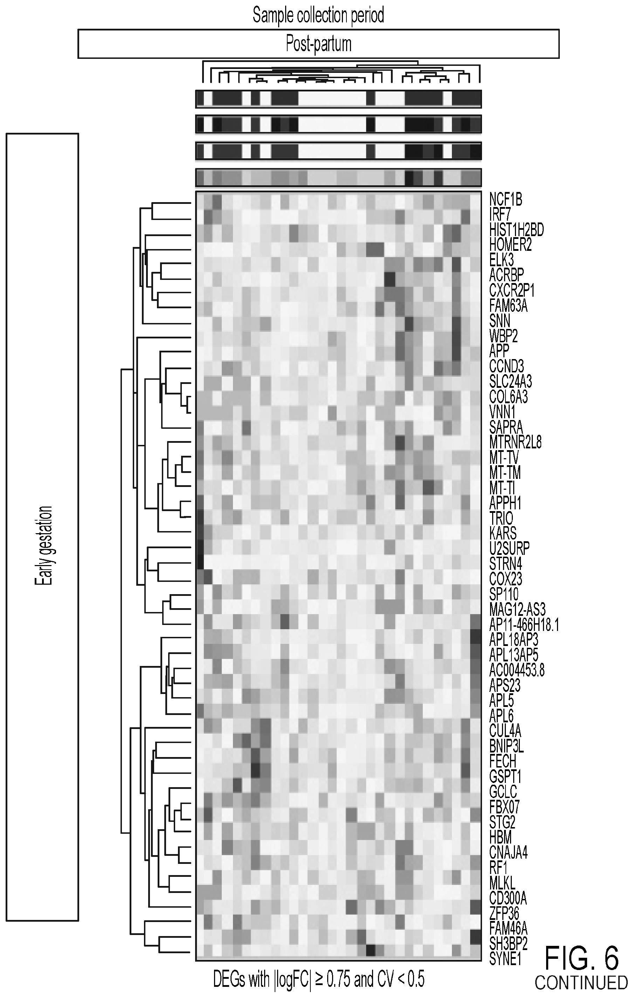

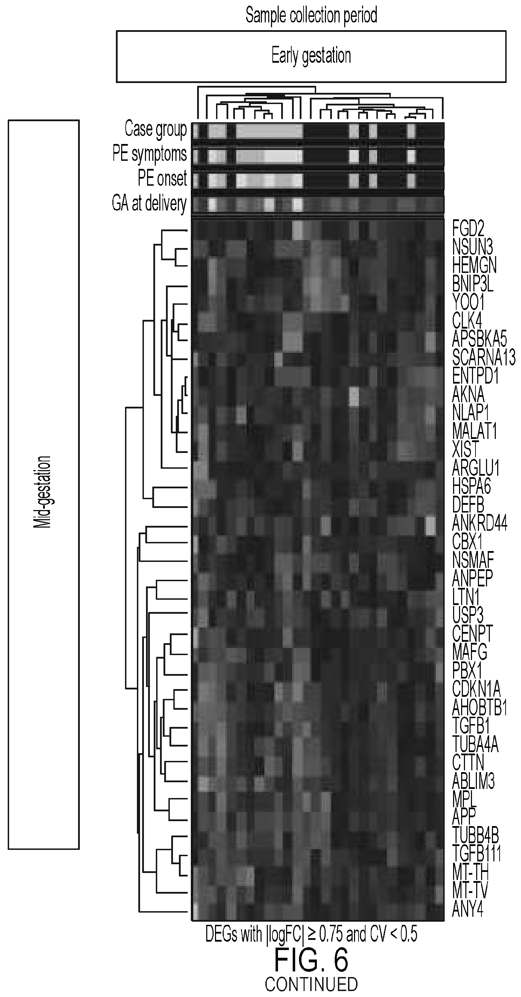

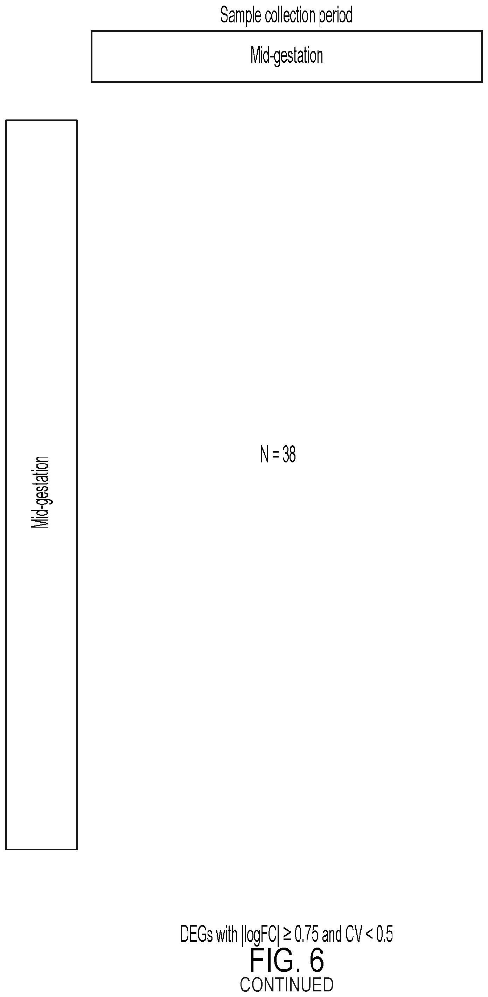

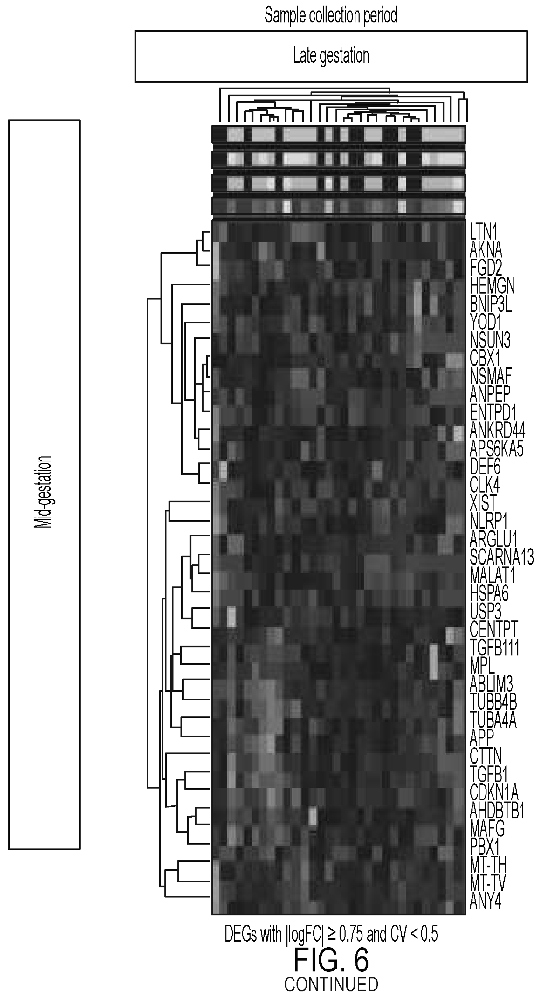

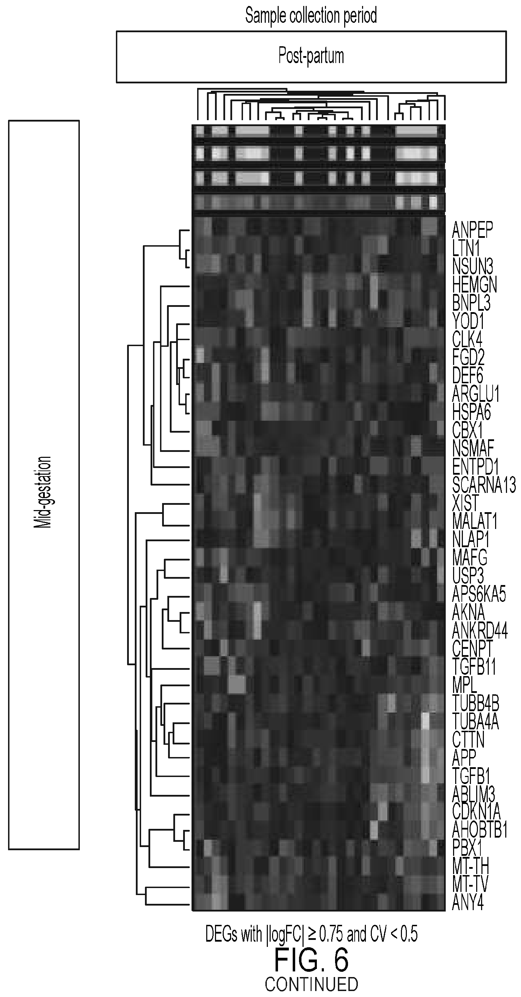

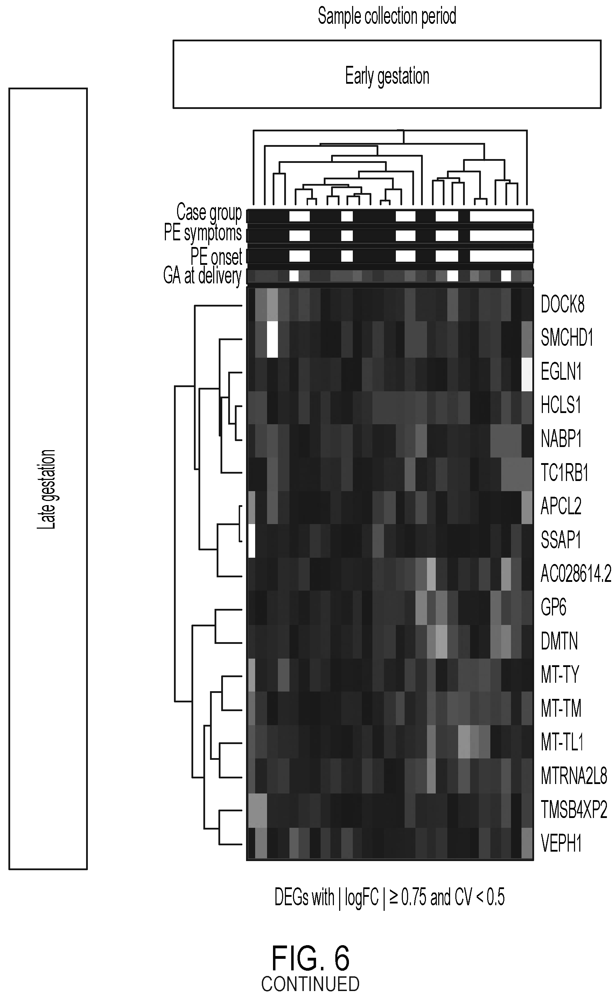

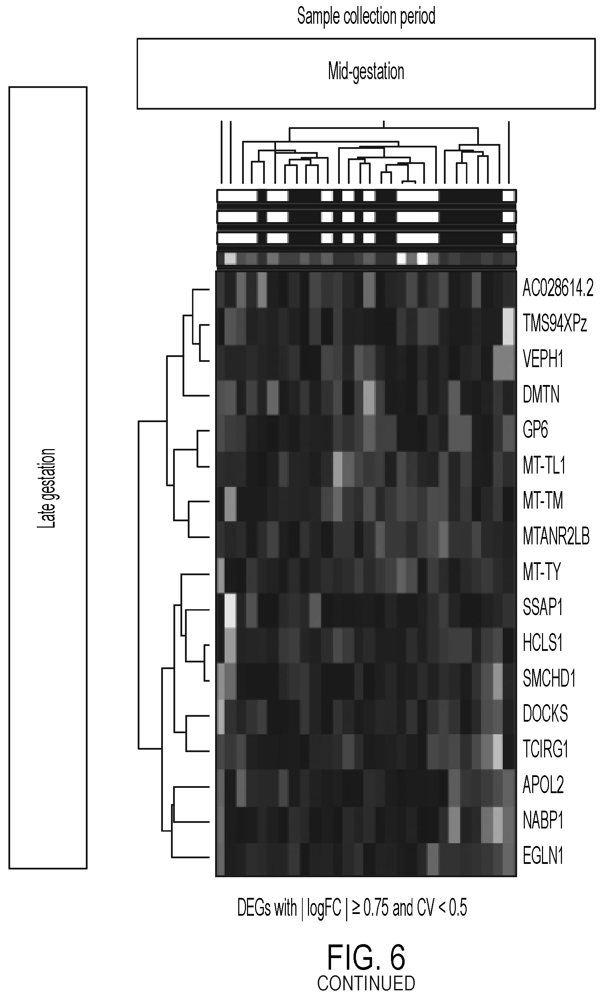

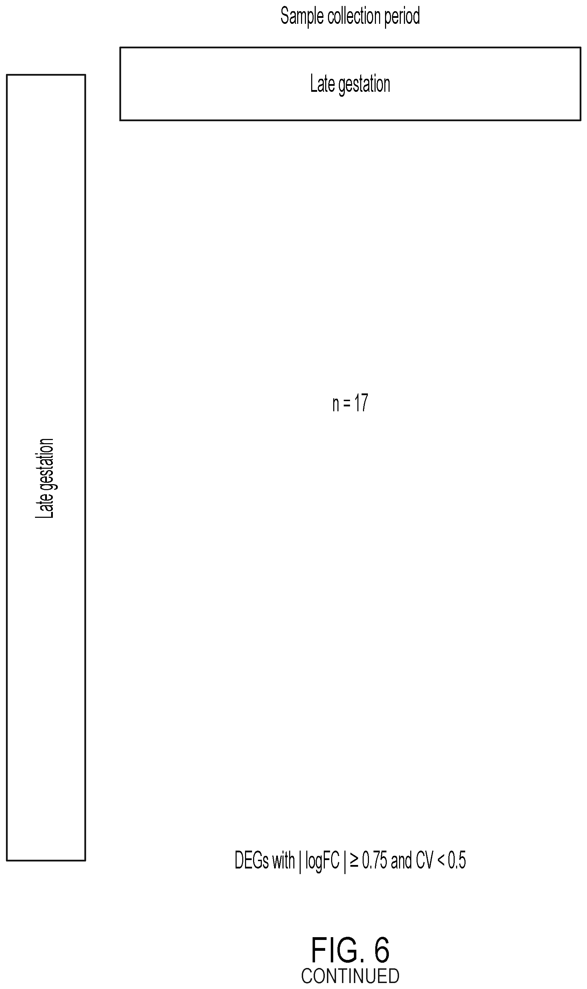

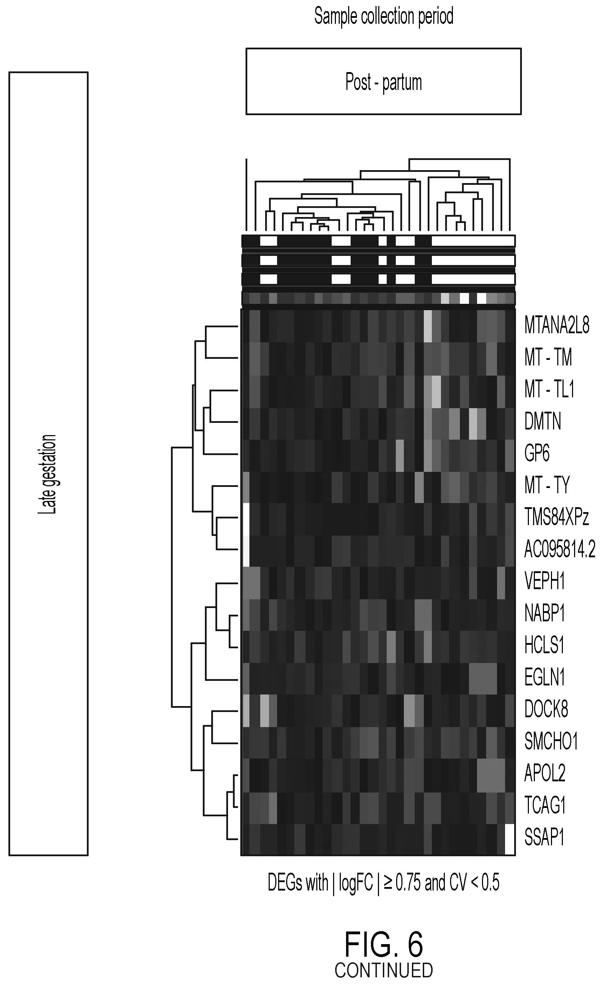

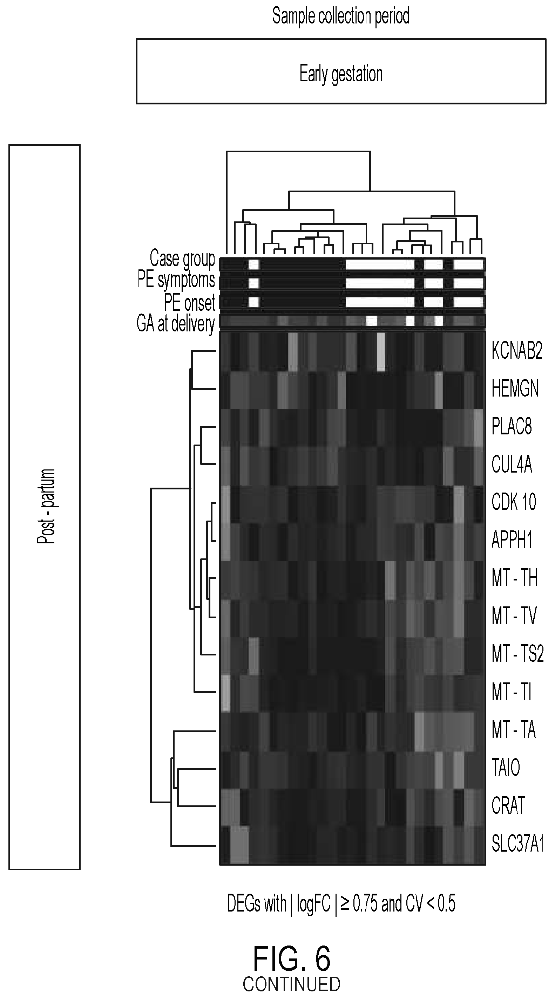

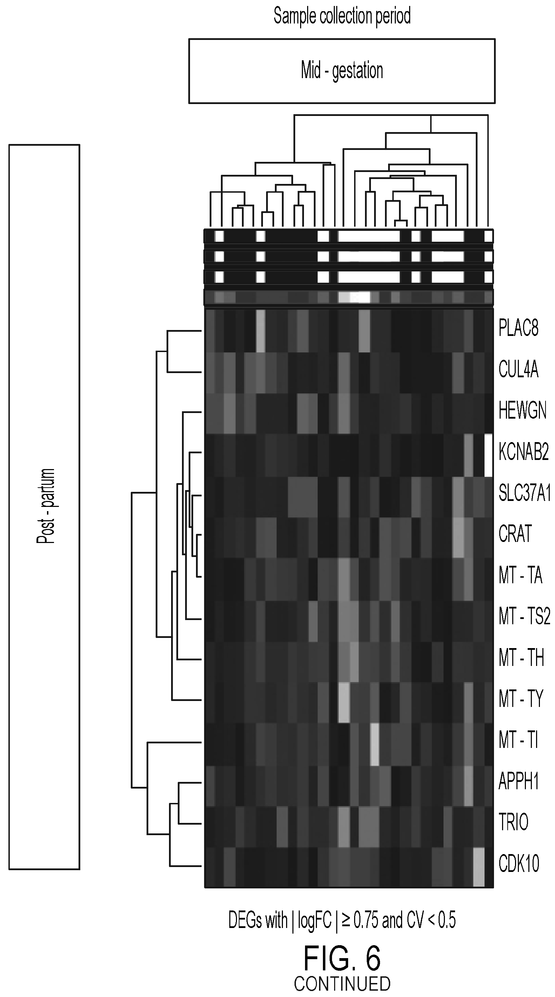

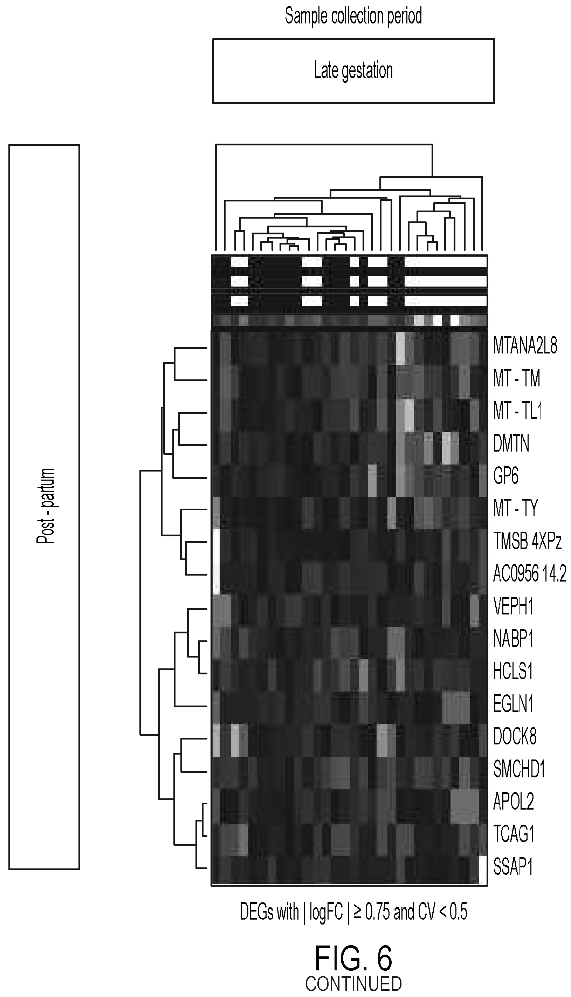

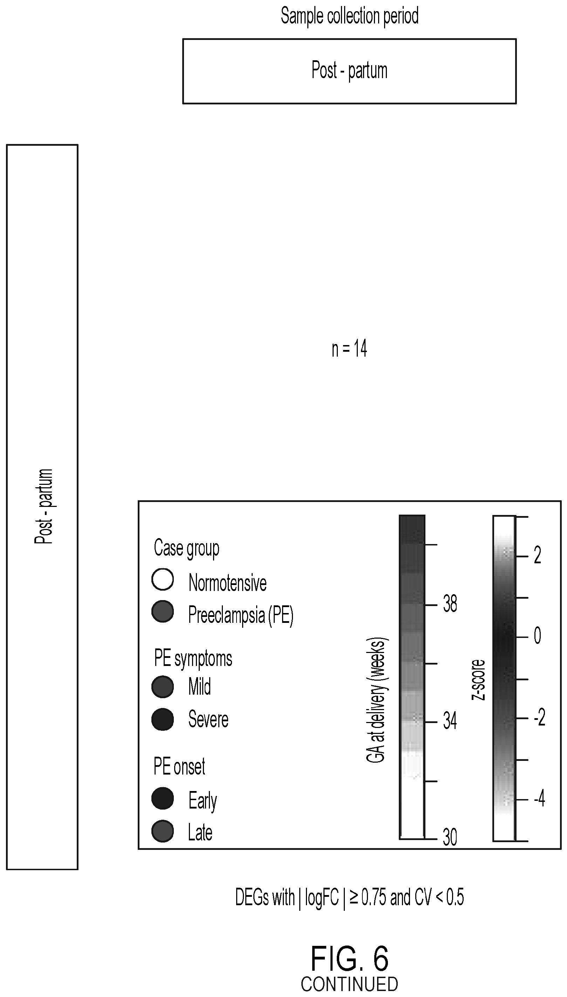

**FIG. 6** **Across gestation and prior to diagnosis, changes in the cfRNA transcriptome identified at one time point can moderately segregate PE and NT samples at other time points.** Differentially expressed genes (DEGs) with |logFC| ≥ 0.75 and CV < 0.5 were identified at each time point across gestation. Each row visualizes how well a specific DEG subset from a given sample collection period can separate PE and NT samples in all other collection periods (columns). The number of genes identified per sample collection period is highlighted along the main diagonal.

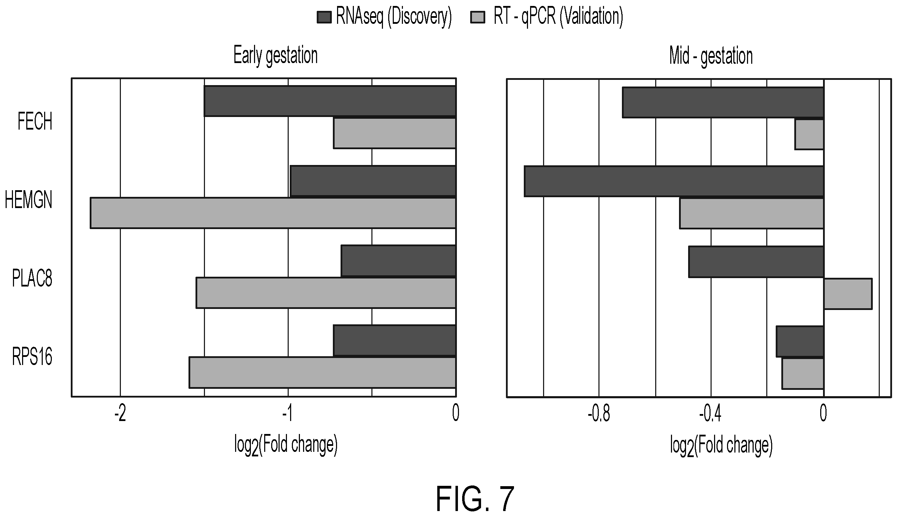

**FIG. 7** This figure depicts that log(Fold change) as estimated by RNAseq and RT-qPCR across two cohorts (discovery and validation) broadly agree with the exception of PLAC8 at mid-gestation.

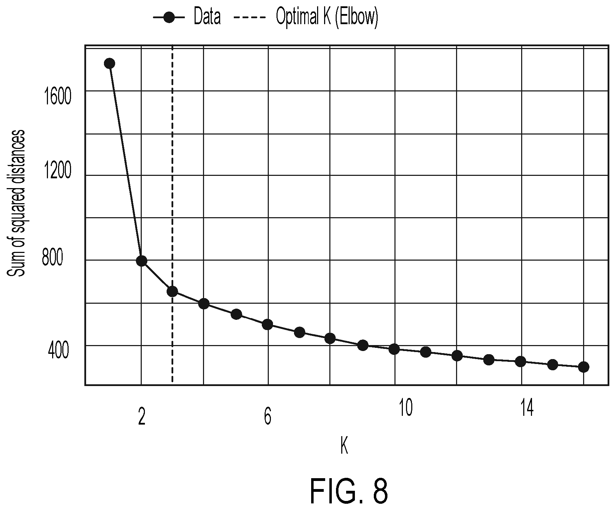

**FIG. 8** **Longitudinal dynamics across gestation can be best described in 3 clusters.** The optimal k clusters (dashed line) or elbow of this convex plot comparing a performance metric, the sum of squared distances, and values of k occurs at k = 3,

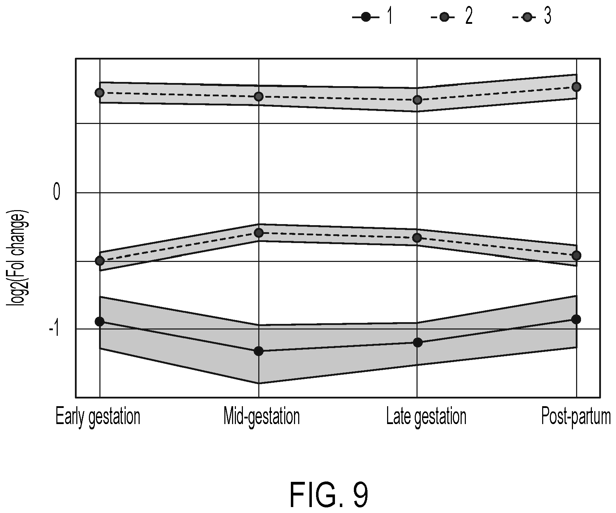

**FIG. 9****K-means clustering reveals meaningful longitudinal patterns**. Following permutation of the data matrix prior to k-means clustering, longitudinal changes over gestation are replaced by 3 flat lines.

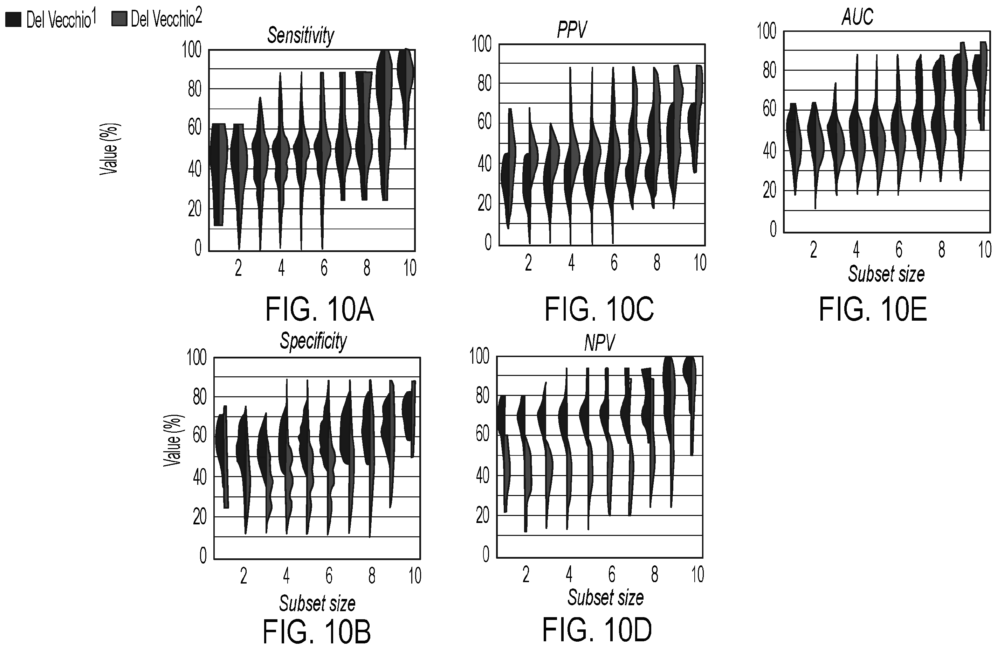

**Fig. 10A-E** **Logistic regression models trained on subsets of 1-10 genes of the initial 11 genes can moderately predict future PE onset with improving performance as subset size increases and as characterized by sensitivity (A), specificity (B), PPV (C), NPV (D), and ROC AUC (E).** Control group 1 (Del Vecchio ^{control 1}) is defined as samples from any pregnant mother who did not develop PE including those with other underlying or pregnancy-related complications like chronic hypertension and gestational diabetes respectively. Del Control 2 (Del Vecchio ^{control 2}) is defined as samples strictly from NT pregnant mothers who did not experience complications.

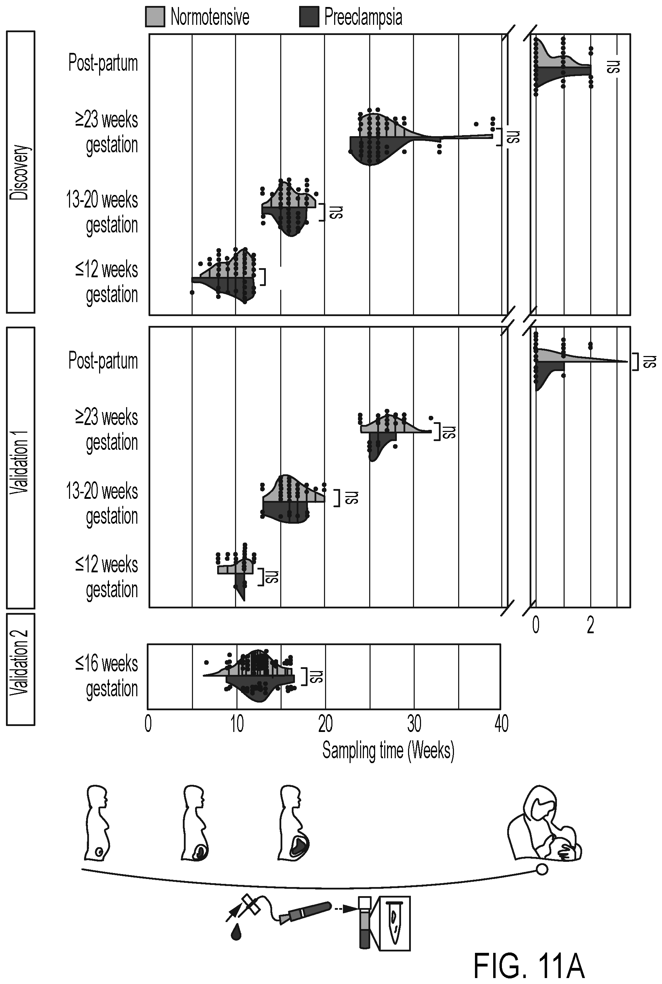

**FIG. 11A-D.** **Comparing sample, maternal, and pregnancy characteristics for NT and PE groups across all cohorts.** Panels illustrate matched sample collection time (weeks) in (A), maternal characteristics (Top to bottom: BMI, age, and gravidity) in (B), matched gestational age at PE onset regardless of PE symptom severity in (C), and gestational age at delivery in (D) for Discovery, Validation 1, and Validation 2 cohorts. For (A), schematic depicts blood sampling across gestation and plasma isolation. X-axis represents gestational age during pregnancy and time post-delivery (weeks) thereafter (ns = not significant, * p < 0.05 ** ≤ 10⁻⁷). BMI data is not available for Validation 2.

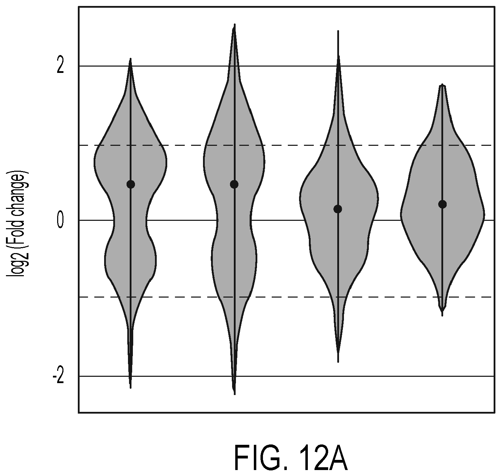

**FIG. 12A-E.** Before 20 weeks of gestation, changes in the cfRNA transcriptome segregate PE and NT samples and are enriched for neuromuscular, endothelial and immune cell types and tissues. (A) Distribution of log(Fold change) with dashed lines at log(Fold change) = ± 1 (B) At ≤12 and between 13-20 weeks of gestation, a subset of differentially expressed genes can separate PE and NT samples despite differences in symptom severity, PE onset subtype, and gestational age (GA) at delivery. (C) Comparison of log(Fold change) for DEGs between Discovery (x-axis) and Validation 2 (y-axis) reveals excellent agreement: 92%, and 94% of genes had the same logFC sign with a spearman correlation of 0.71 and 0.72 (p < 10-15) at ≤12 and 13-20 weeks of gestation, respectively. (D) Across gestation, differentially expressed genes for PE as compared to NT can be described by 2 longitudinal trends: Increased in PE over gestation in orange and Decreased in PE over gestation in dark blue as revealed by k-means clustering. (E) Approximately 13% of DEGs are tissue- or cell-type specific when compared with the Human Protein Atlas (HPA) and an augmented Tabula Sapiens (TSP+) atlas.

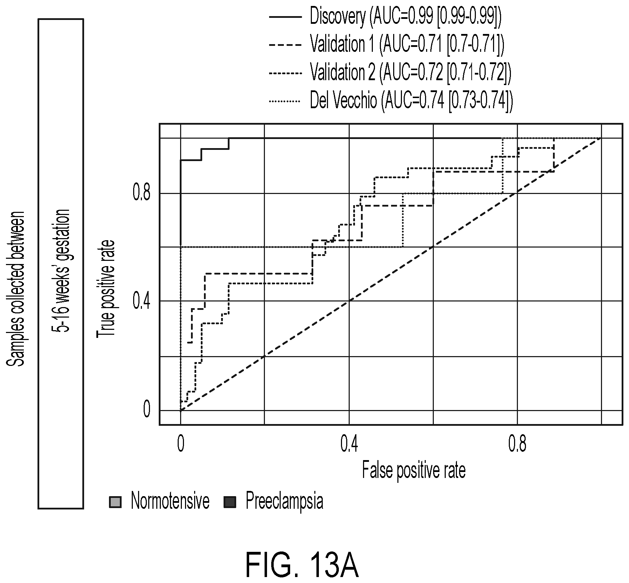

**FIG. 13A-B** A subset of cfRNA changes can predict risk of PE early in gestation (A) Classifier performance as quantified by ROC for samples collected in early gestation between 5-16 weeks. For each cohort, including 3 validation cohorts of which Validation 2 and Del Vecchio are independent, the legend states the AUROC and the corresponding 90% CI in square brackets. (B) Prediction of PE incorporates cfRNA levels for 18 genes for which normalized centered log₂(Fold change) trends hold across discovery, Validation 1, Validation 2, and Del Vecchio cohorts as confirmed using univariate analysis (*p ≤ 0.05, **p ≤ 0.01, ***p ≤ 0.005; one-sided Mann-Whitney U-Test with Benjamini-Hochberg correction). For box-plots, center line, box limits, whiskers, and outliers represent the median, upper and lower quartiles, 1.5x interquartile range, and any outliers outside that distribution respectively. Plot limits are -8 to 4 to better visualize the main distribution.

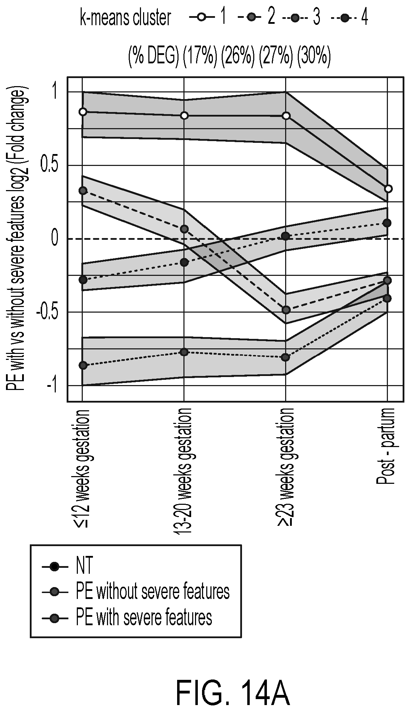

**FIG. 14A-B.** **Changes in the cfRNA transcriptome reflect PE's multifactorial nature and pathogenesis over pregnancy prior to diagnosis**. (A) Across gestation, differentially expressed genes for PE with as compared to without severe features (503 DEGs) can be described by 4 longitudinal trends as revealed by k-means clustering. Points indicate median per DEG cluster and shaded region indicates 95% CI. (B) Comparison of organ and cell-type changes over gestation for eight organ systems reflect the multifactorial nature of PE and provide a possible means to monitor maternal organ health (Top to bottom: Brain, immune, placenta and kidney, heart and endothelial-linked, liver and muscle). Points indicate median per sample group (NT in black, PE without severe features in yellow, PE with severe features in red) and shaded region indicates 75% CI.

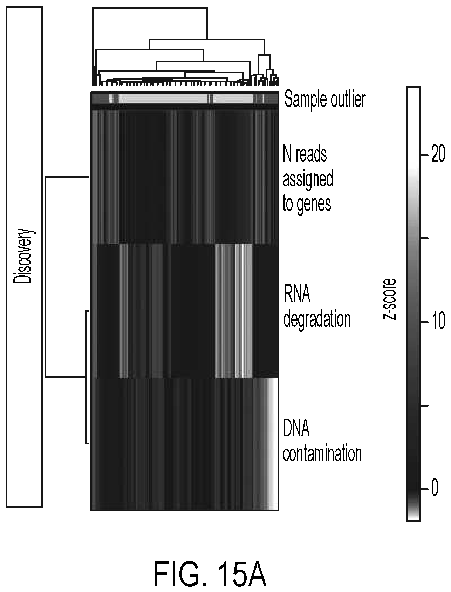

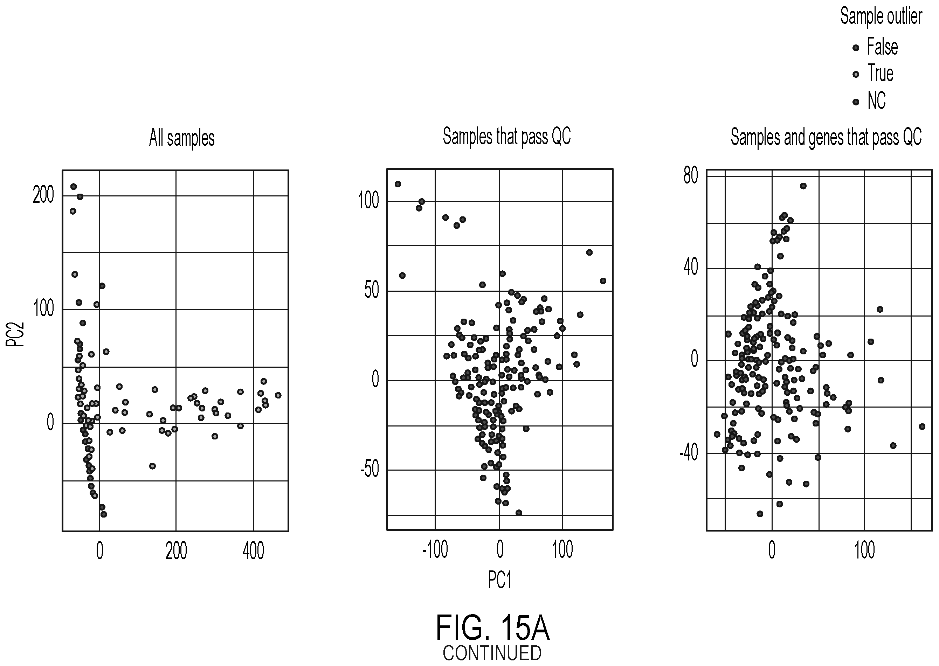

**FIG. 15A-E** Samples with outlier values for at least one of QC metric cluster separately from most non-outlier samples. For Discovery (A), Validation 1 (B), and Validation 2 (C), hierarchical clustering (left) and PCA reveals that most outlier samples cluster with negative control (NC) samples (H2O) and separately from non-outlier samples. (D, E) Visualization of other QC metrics like the amount of cfRNA extracted (D) and the percent of reads that align uniquely to the human genome (E). For PCA in (A-C), sample outliers and poorly detected genes drive PCA and serve as leverage points. The top two principal components are visualized when performed using all samples and all genes (leftmost PCA) or only samples that pass QC metrics (middle PCA) reveals that certain samples can act as leverage points. Once sample outliers and lowly detected genes are removed from the cfRNA gene matrix (rightmost PCA), the top two principal components reflect natural variance in the data and are no longer driven by a few leverage points.

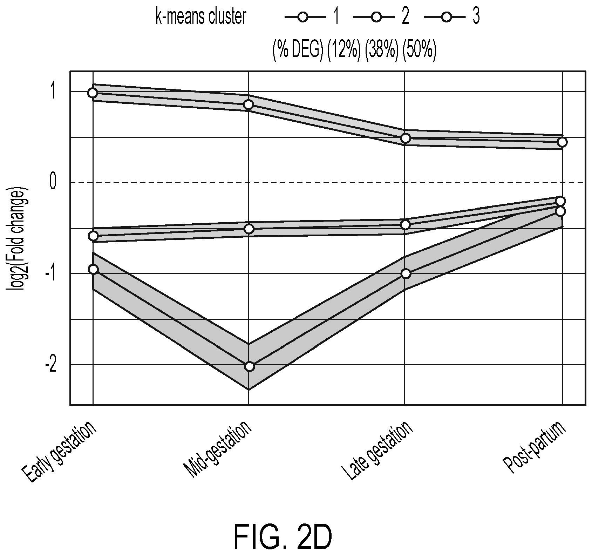

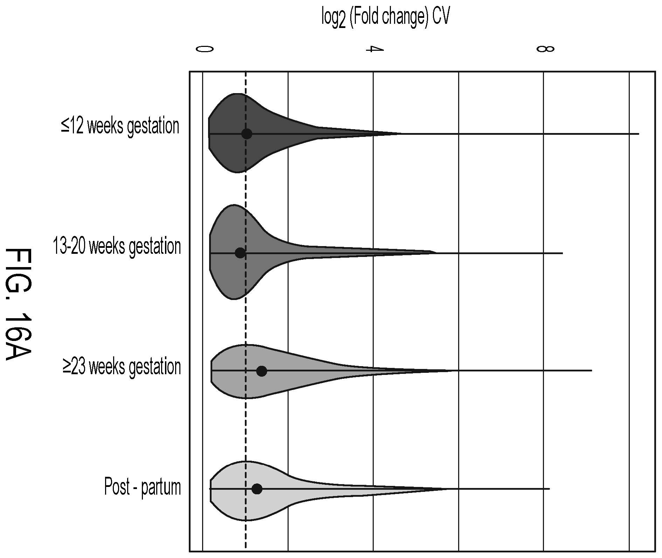

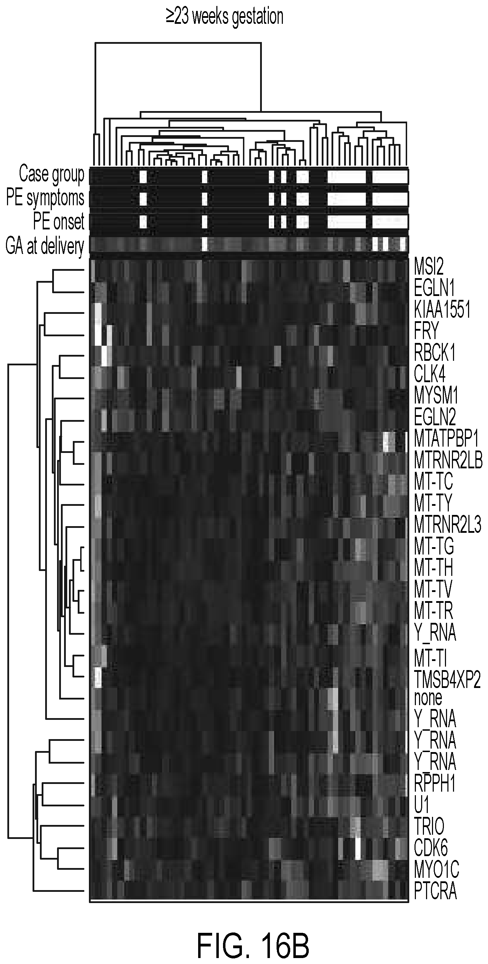

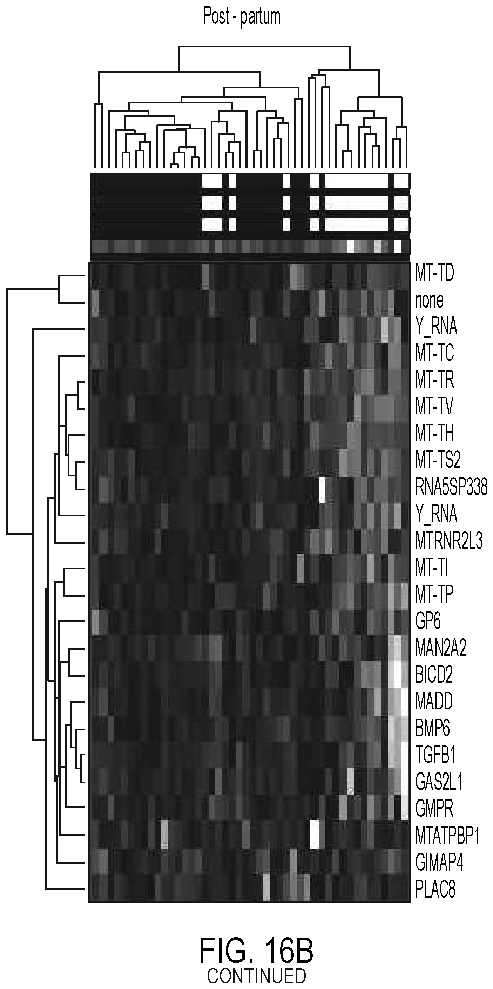

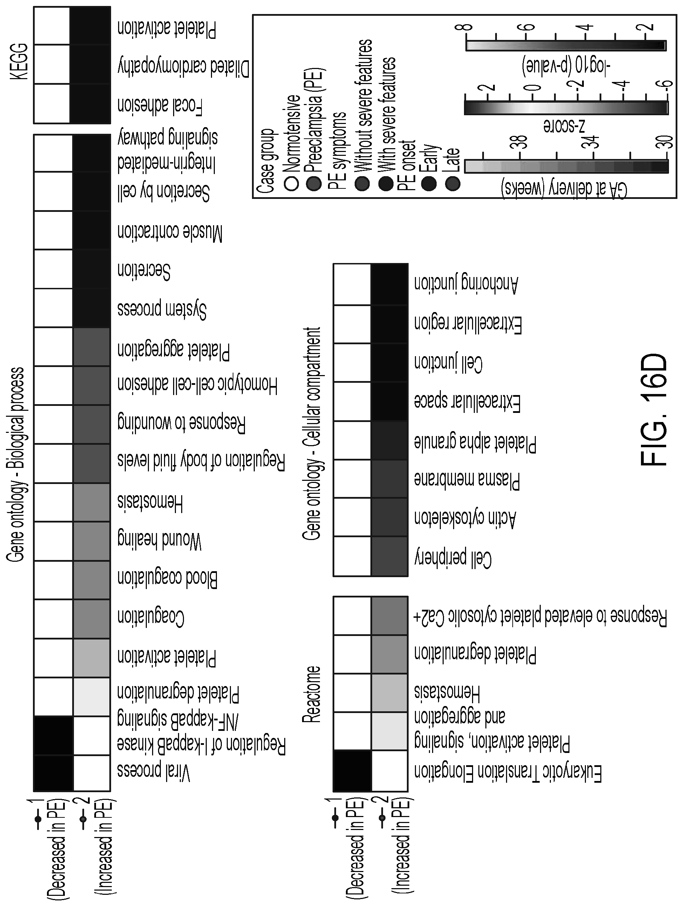

**FIG. 16A-E**Across gestation prior to diagnosis, changes in the cfRNA transcriptome segregate PE and NT samples and reflect known PE biology. (A) Distribution of CVs with dashed line at CV = 1 for all DEGs between PE as compared to NT samples across gestation. (B) At ≥23 weeks of gestation and post-partum, in each sample collection period, a subset of DEGs can separate PE and NT samples despite differences in symptom severity, PE onset subtype, and gestational age (GA) at delivery. (C) Comparison of log(Fold change) for DEGs for PE as compared to NT between Discovery (x-axis) and Validation 1 (y-axis) reveals good agreement across gestation but not post-partum: 82%, 83%, and 60% of genes had the same logFC sign with a Spearman correlation of 0.67, 0.69, and 0.35 (p < 10⁻¹⁵) at 13-20, ≥23 weeks, and post-partum, respectively. (D) The genes in each longitudinal trend group reflect known PE etiology as highlighted across four databases (GO biological processes, KEGG, the reactome, and GO cellular compartment). Some PE associated terms are emphasized in bold, colored text that corresponds to group color from Fig 2D (Dark blue and orange indicate decreased and increased in PE vs NT, respectively). (E) Comparison of log(Fold change) for DEGs for PE without severe features vs NT (x-axis) and PE with severe features vs NT in the Discovery cohort (y-axis) reveals good agreement along the y=x axis with a slope of 0.93, 1.03, 0.77, and 0.86 at ≤12 weeks, 13-20, ≥23 weeks, and post-partum, respectively.

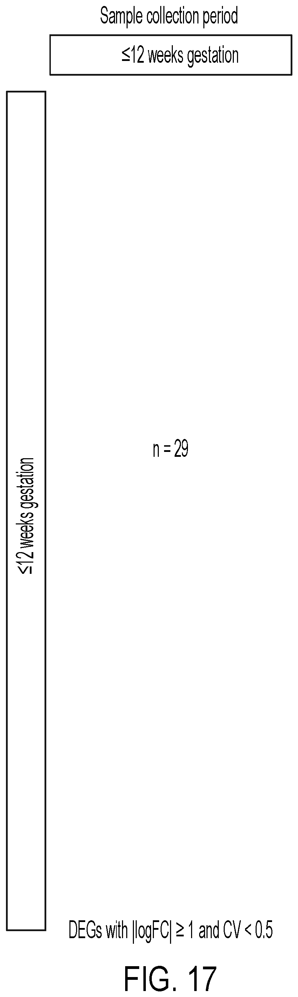

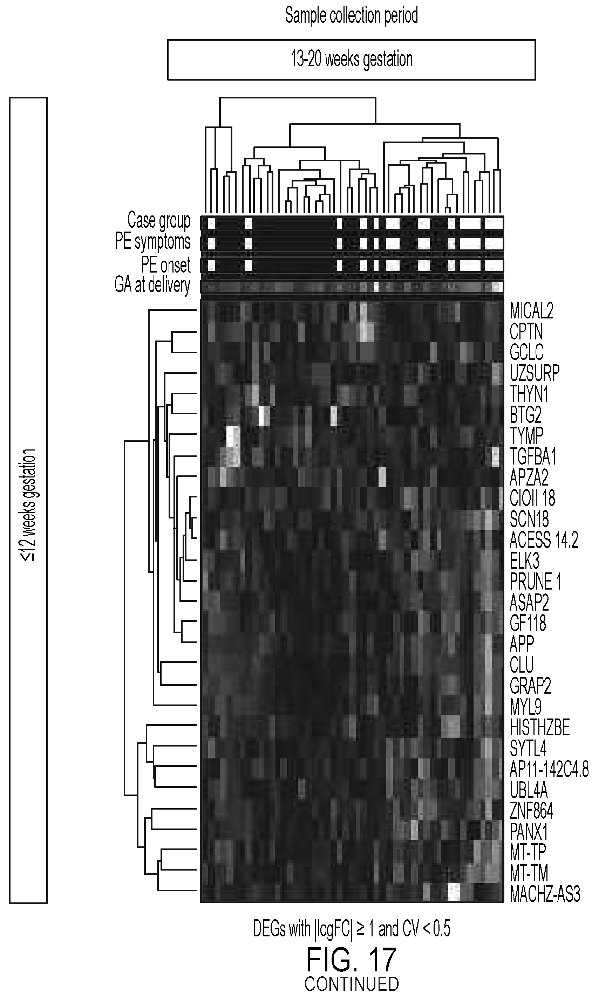

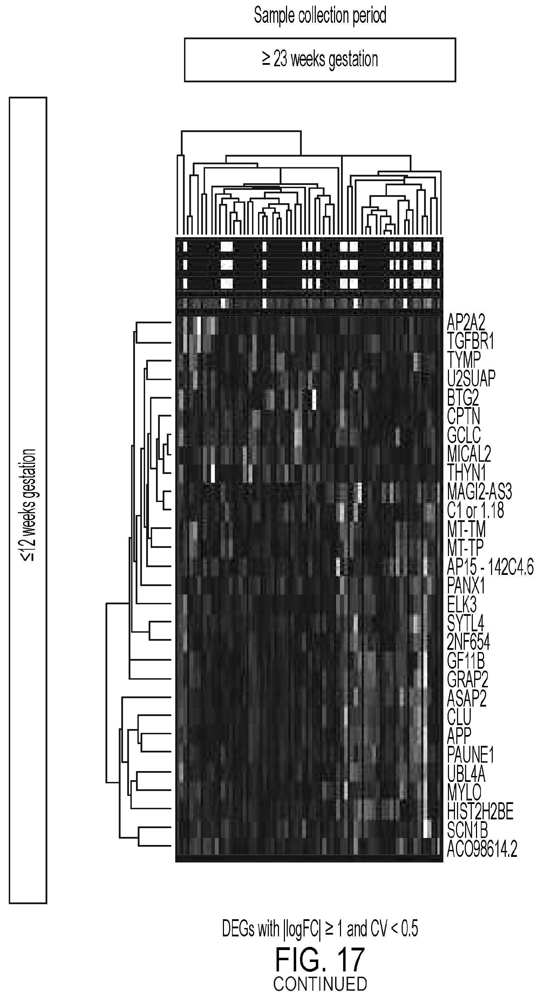

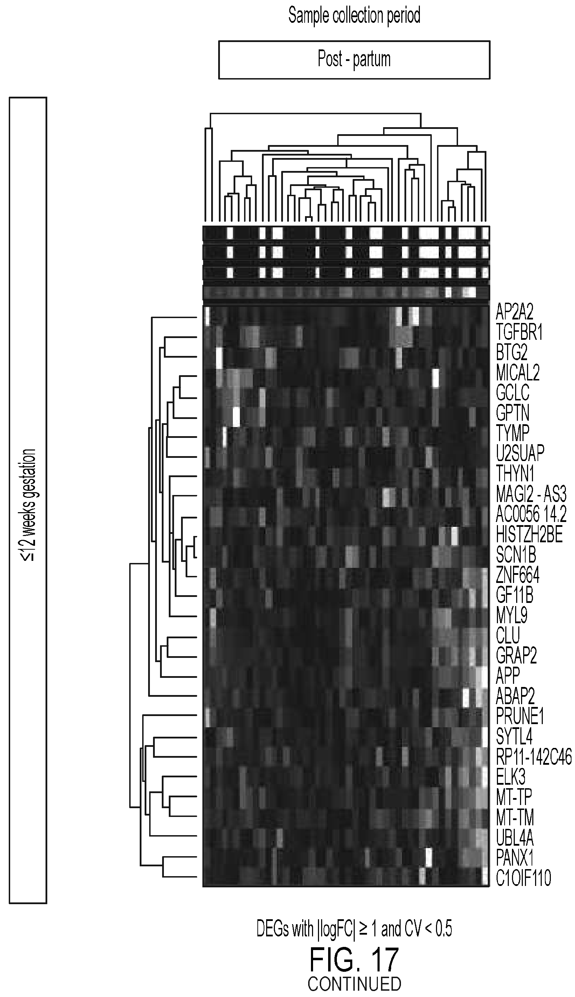

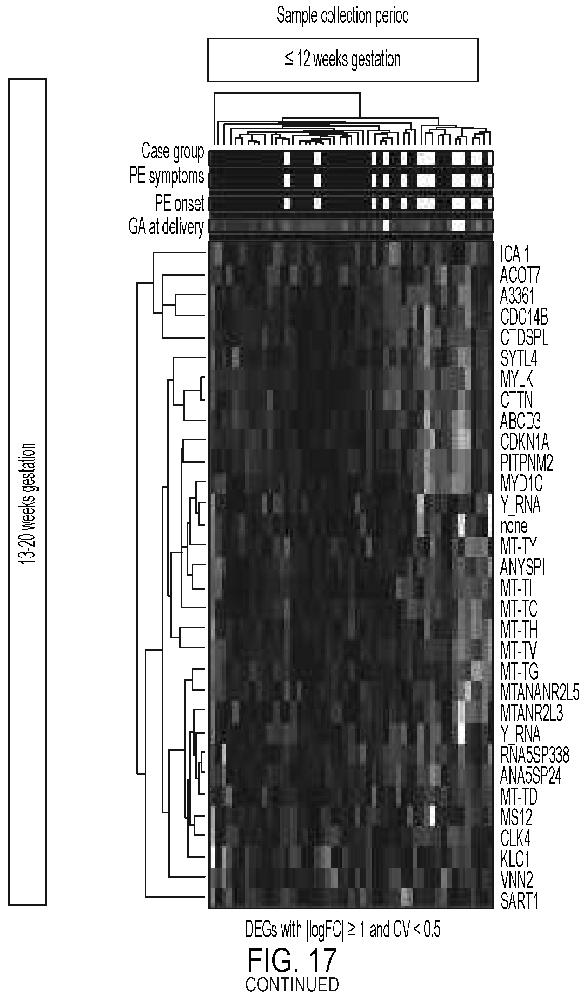

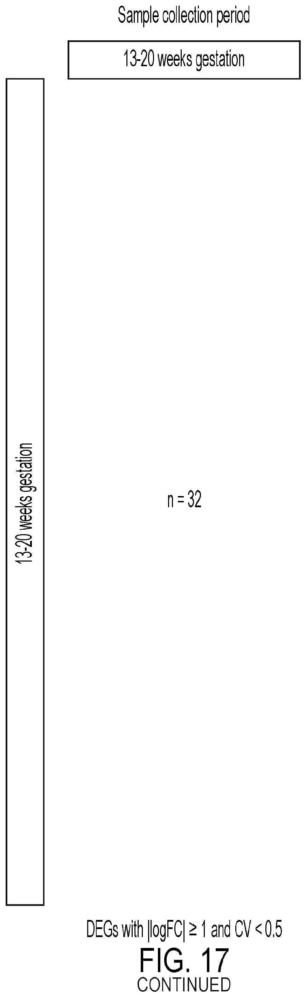

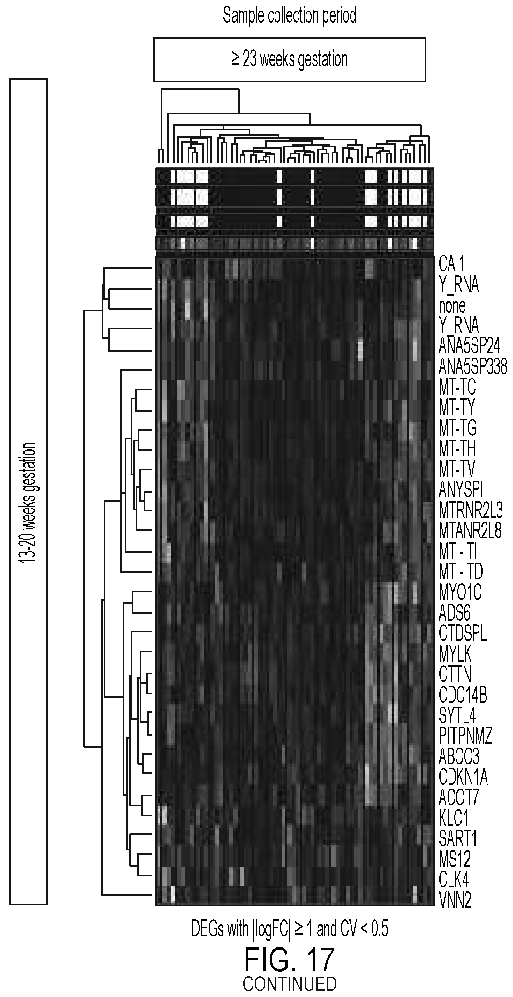

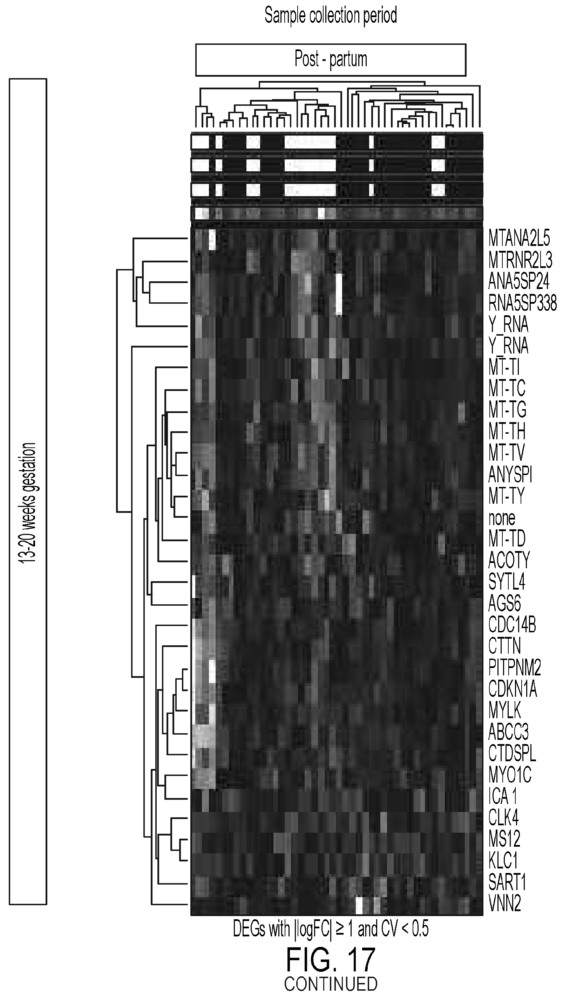

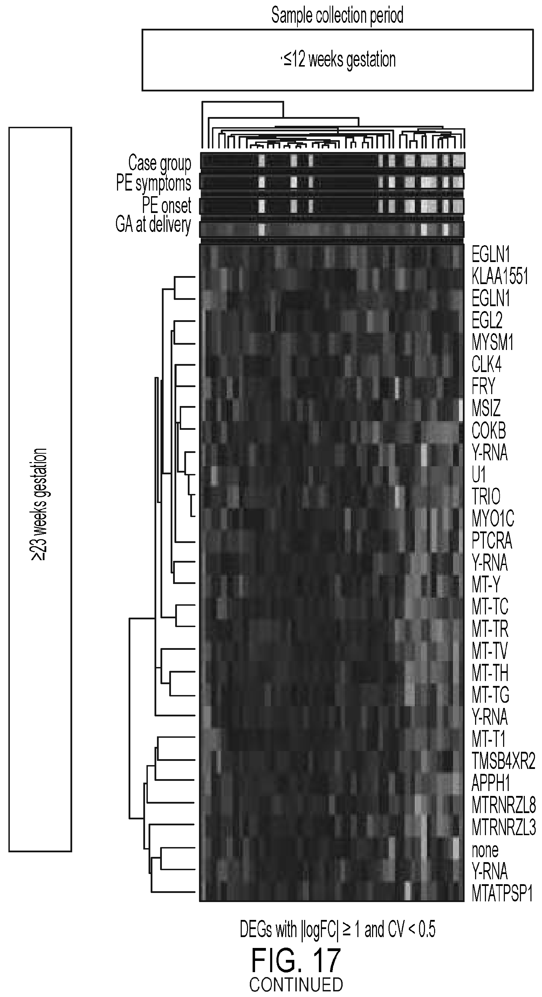

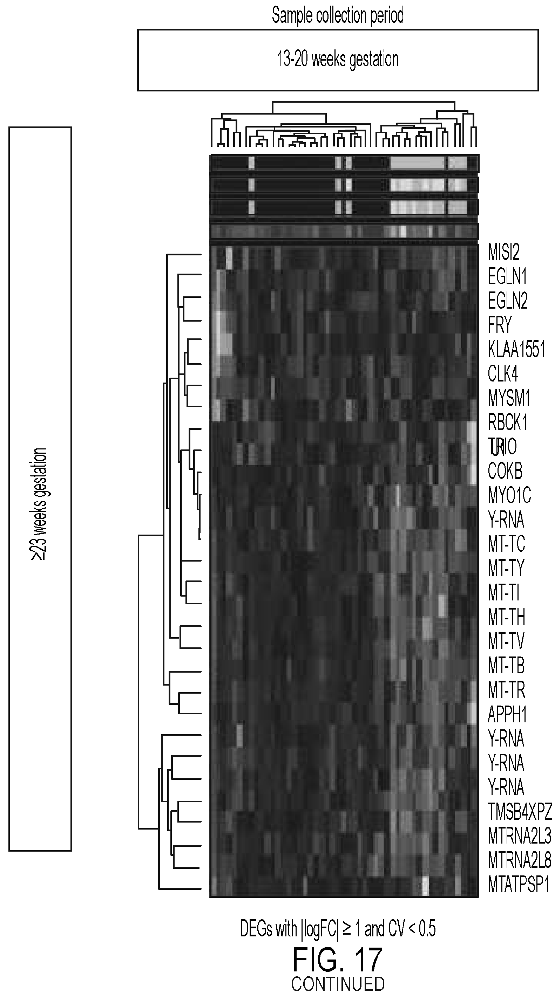

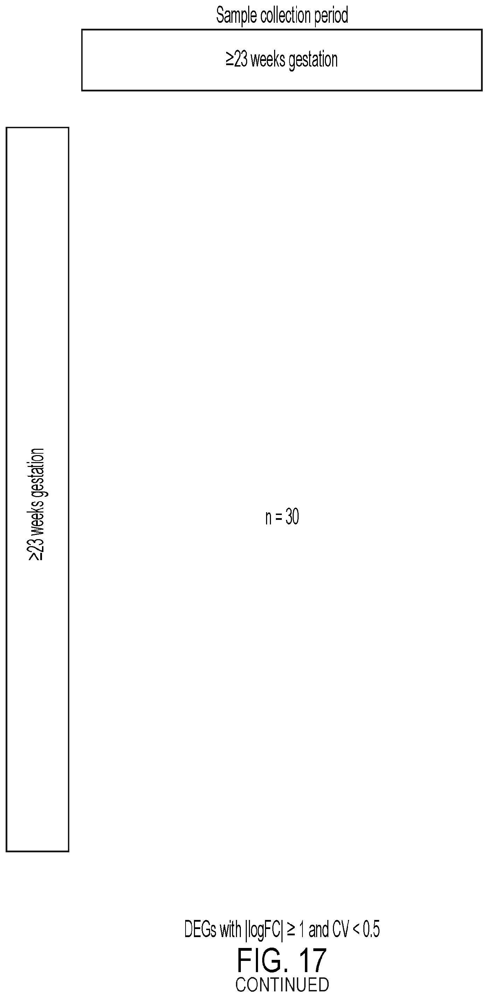

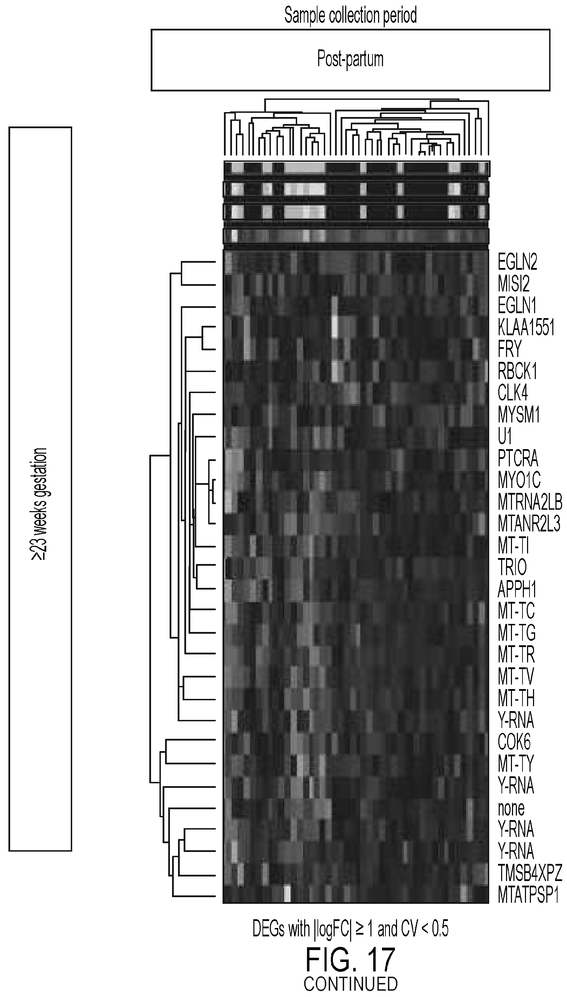

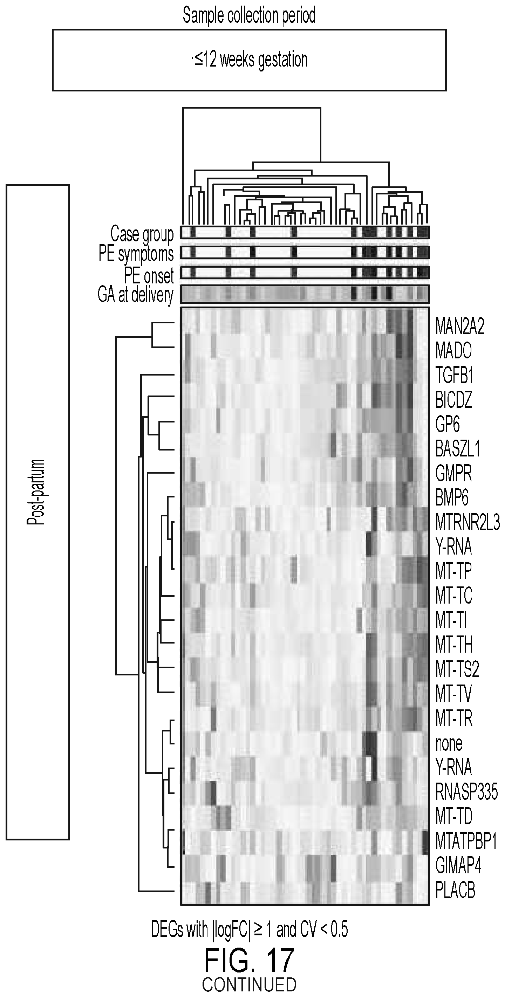

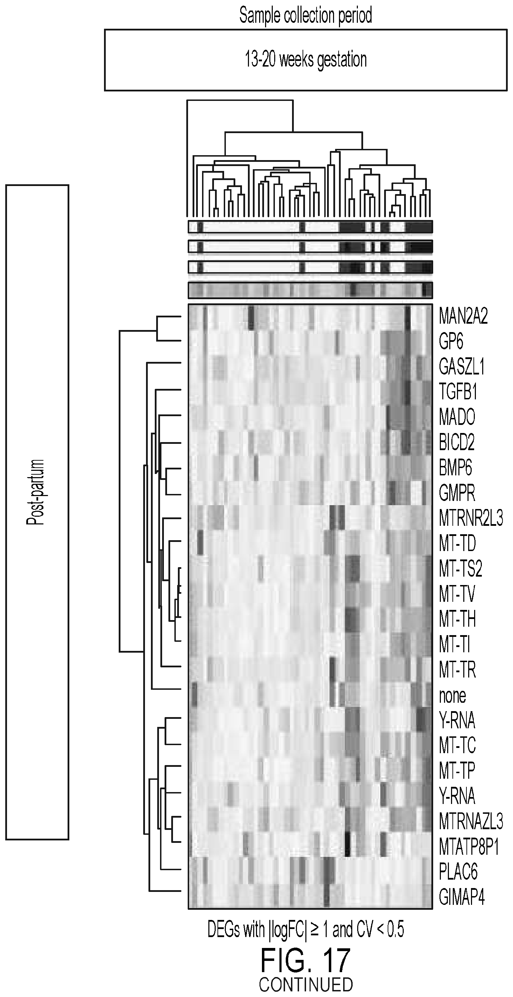

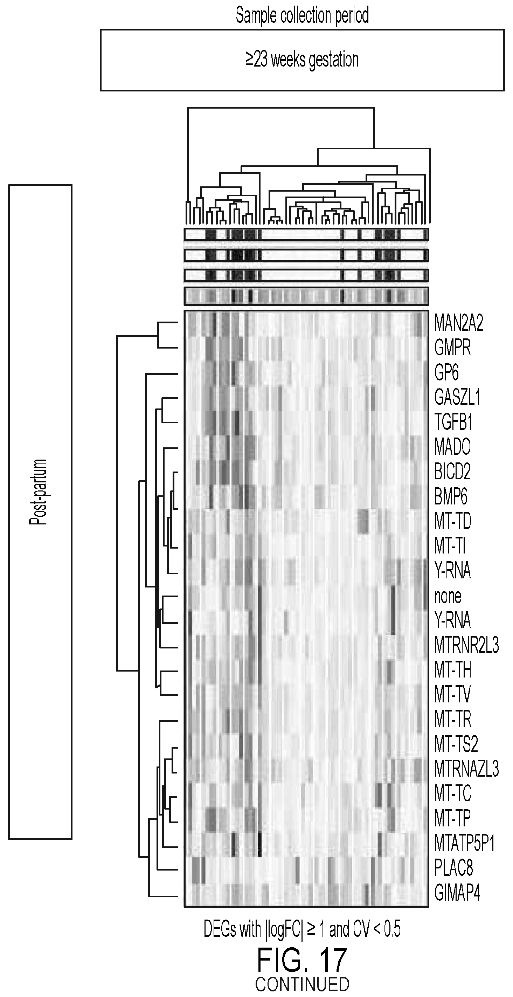

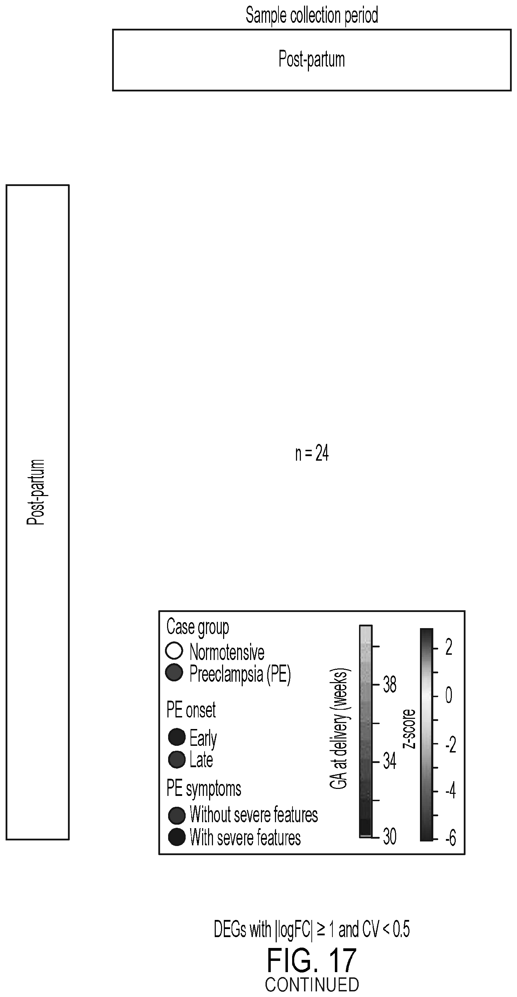

**FIG. 17** **Across gestation and prior to diagnosis, changes in the cfRNA transcriptome identified at one timepoint can moderately segregate PE and NT samples at other timepoints.** DEGs with |logFC| ≥ 1 and CV < 0.5 or 0.4 for 13-20 weeks timepoint were identified at each timepoint across gestation. Each row visualizes how well a specific DEG subset from a given sample collection period can separate PE and NT samples in all other collection periods (columns). The number of genes identified per sample collection period is highlighted along the main diagonal.

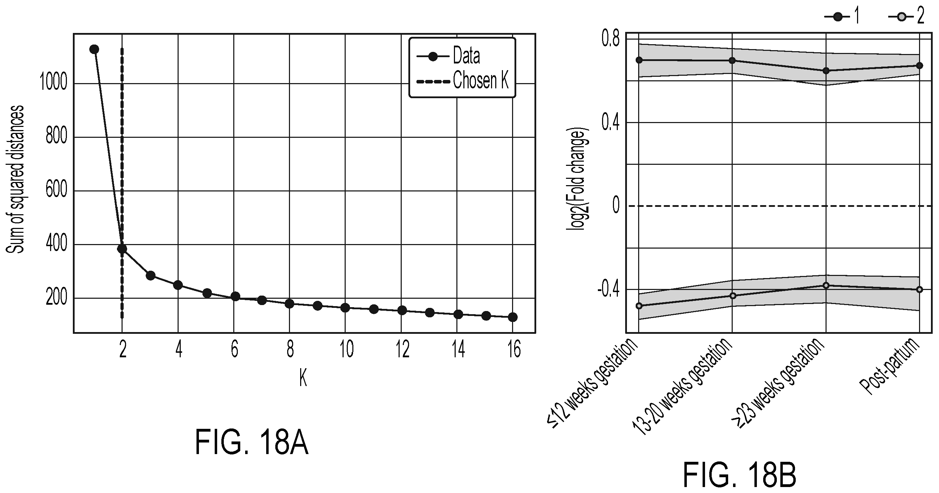

**FIG. 18A-D.** **K-means clustering reveals meaningful longitudinal patterns.** The chosen k clusters (dashed line) comparing a performance metric, the sum of squared distances, and values of k for clustering of DEGs for PE vs NT related to Fig 2D in (A) and DEGsfor PE with vs without severe features related to Fig 4A in (C). Following permutation of the data matrix prior to k-means clustering, longitudinal changes over gestation are replaced by (B) 2 flat lines for clustering of logFC for PE vs NT and (D) 4 uninformative lines for clustering of logFC for PE with vs without severe features.

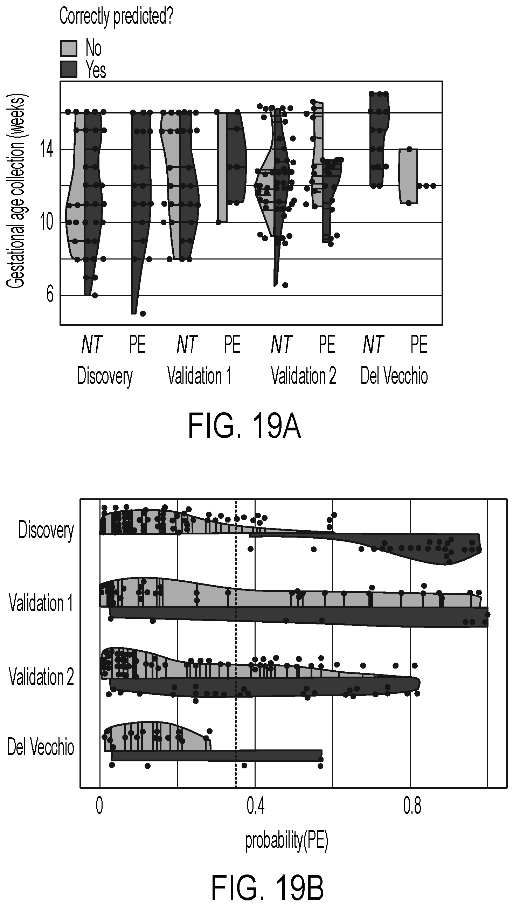

**FIG. 19A-C.** **Examining the logisitic regression model used to predict risk of PE early in gestation** (A) Comparison of gestational age at sample collection (weeks) for incorrectly predicted (yellow) or correctly predicted (green) samples across NT and PE groups in Discovery, Validation 1, Validation 2, and Del Vecchio shows that incorrectly predicted PE samples (false negatives) are collected at later gestational ages. (B) Estimated probability of PE as outputted by logistic regression for both PE and NT samples shows that the model is well-calibrated across most predictions. Dashed line at 0.35 indicates classifier cutoff where probability(PE) ≥ 0.35 constitutes a sample predicted as PE. (C) Logistic regression models trained on subsets of 1-18 genes of the initial 18 genes can moderately predict future PE onset in Validation 2 cohort with improving performance as subset size increases and as characterized by PPV, NPV, sensitivity, specificity, and AUROC (left to right).

### DETAILED DESCRIPTION

In one aspect, described herein are methods for predicting the risk or existence of preeclampsia; and or risk of pregnancy complications related to preeclampsia, such as gestational diabetes and/or gestational-onset hypertension. Such methods comprise quantifying the RNA expression level in a cfRNA sample obtained from a pregnant human subject, *e.g.,* at five weeks or longer gestation, of at least one gene of a panel of genes comprising BNIP3L, FECH, HEMGN, SNCA, OAZ1, GSPT1, AKNA, CSF3R, IGF2, RPS15, and MARCH2. In some embodiments, the expression level is determined for a subset of the panel of genes that comprises at least two genes selected from BNIP3L, FECH, HEMGN, SNCA, OAZ1, GSPT1, AKNA, CSF3R, IGF2, RPS15, and MARCH2. In some embodiments, the expression level is determined for a subset of the panel of genes that comprises at least three genes selected from BNIP3L, FECH, HEMGN, SNCA, OAZ1, GSPT1, AKNA, CSF3R, IGF2, RPS15, and MARCH2. In some embodiments, the expression level is determined for a subset of the panel of genes that comprises at least four genes selected from BNIP3L, FECH, HEMGN, SNCA, OAZ1, GSPT1, AKNA, CSF3R, IGF2, RPS15, and MARCH2. In some embodiments, the expression level is determined for a subset of the panel of genes comprises at least five or six genes selected from BNIP3L, FECH, HEMGN, SNCA, OAZ1, GSPT1, AKNA, CSF3R, IGF2, RPS15, and MARCH2, In some embodiments, the expression level is determined for a subset of the panel of genes that comprises at least seven, eight, nine or ten genes selected from BNIP3L, FECH, HEMGN, SNCA, OAZ1, GSPT1, AKNA, CSF3R, IGF2, RPS15, and MARCH2. In some embodiments, the expression level is determined for each of the eleven genes BNIP3L, FECH, HEMGN, SNCA, OAZ1, GSPT1, AKNA, CSF3R, IGF2, RPS15, and MARCH2. In some embodiments, the method further comprises applying a classifier to assess the risk of the patient for preeclampsia relative to a control population, *e.g*., normotensive human females.

In another aspect, described herein are methods for predicting the risk or existence of preeclampsia. Such methods comprise quantifying the RNA expression level in a cfRNA sample obtained from a pregnant human subject, *e.g.,* at five weeks or longer gestation, of at least one gene of a panel of genes comprising CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564). In some embodiments, the expression level is determined for a subset of the panel of genes that comprises at least two genes selected from CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564). In some embodiments, the expression level is determined for a subset of the panel of genes that comprises at least three genes selected from CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564). In some embodiments, the expression level is determined for a subset of the panel of genes that comprises at least four genes selected from CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564). In some embodiments, the expression level is determined for a subset of the panel of genes comprises at least five or six genes selected from CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564). In some embodiments, the expression level is determined for a subset of the panel of genes that comprises at least seven, eight, nine or ten genes selected from CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564). In some embodiments, the expression level is determined for a subset of the panel of genes that comprises at least twelve, thirteen, fourteen or fifteen genes selected from CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564). In some embodiments, the expression level is determined for a subset of the panel of genes that comprises sixteen or seventeen genes selected from CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564). In some embodiments, the expression level is determined for each of the eighteen genes CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564). In some embodiments, the method further comprises applying a classifier to assess the risk of the patient for preeclampsia relative to a control population, *e.g*., normotensive human females.

In another aspect, the disclosure provides methods for predicting the risk of severe preeclampsia. Such methods comprise quantifying cfRNA levels in a cfRNA sample obtained from a pregnant human subject, *e.g.,* at five weeks or longer gestation, of genes set forth in in Table 24, Table 25A, Table 25B, or Table 25C.

In a further aspect, the disclosure provides methods for monitoring maternal organ health by quantifying cfRNA levels in a patient sample during gestation, *e.g.,* at five weeks or longer gestation, of multiple genes set forth in Table 26.

### Terminology

As used herein, the following terms have the meanings ascribed to them unless specified otherwise.

The terms "a," "an," or "the" as used herein not only include aspects with one member, but also include aspects with more than one member. For instance, the singular forms "a," "an," and "the" include plural referents unless the context clearly dictates otherwise. Thus, for example, reference to "an agent" includes reference to one or more agents known to those skilled in the art, and so forth.

As used herein, "preeclampsia" is as defined in accordance with the American College of Obstetricians and Gynecologists (ACOG) guidelines ²⁷ based on two diagnostic criteria: 1) new-onset hypertension developing on or after 20 weeks of gestation and 2) new-onset proteinuria, or in the absence of proteinuria, thrombocytopenia, impaired liver function, renal insufficiency, pulmonary edema, or cerebral or visual disturbances. New-onset hypertension is defined when systolic and/or diastolic blood pressure is at least 140 or 90 mm Hg, respectively, as measured on at least 2 separate occasions between 4 hours and 1 week apart. Proteinuria is defined when 300 mg protein is present within a 24-hour urine collection, or when an individual urine sample contains a protein/creatinine ratio of 0.3 mg/dL, or when a random urine specimen has more than 1 mg protein (*e.g.,* as measured by dipstick). Thrombocytopenia, impaired liver function, and renal insufficiency are defined as a platelet count of less than 100,000/µL, liver transaminases ≥ 2-times normal, and serum creatinine > 1.1 mg/dL, respectively. Symptoms are defined as severe in accordance with ACOG guidelines. Specifically, PE is defined as "severe" if any of the following symptoms are present and diagnosed as described above: new-onset hypertension with systolic and/or diastolic blood pressure of at least 160 or 110 mm Hg respectively, thrombocytopenia, impaired liver function, renal insufficiency, pulmonary edema, new-onset headache unresponsive to medication and unaccounted for otherwise, or visual disturbances. Although diagnostic criteria for PE may further evolve, the findings described herein remain applicable.

Preeclampsia is a human disease that does not occur naturally in animals. As used herein, a "pregnant subject" or "pregnant patient" refers to a human.

The term "cell-free RNA sample" or "cfRNA sample" refers to a nucleic acid sample comprising extracellular RNA, which nucleic acid sample is obtained from any cell-free biological fluid, for example, whole blood processed to remove cells, urine, saliva, or amniotic fluid. In some embodiments, cfRNA for analysis is obtained from whole blood processed to remove cells, *e.g.,* a plasma or serum sample. As used herein, the terms "cell-free RNA" or "cfRNA" refer to RNA recoverable from the non-cellular fraction of a bodily fluid, such as blood, and includes fragments of full-length RNA transcripts.

The terms "determining," "assessing," "assaying," "measuring" and "detecting" as used herein are used interchangeably and refer to quantitative determinations.

The term "amount" or "level" refers to the quantity of copies of an RNA transcript being assayed, including fragments of full-length transcripts that can be unambiguously identified as fragments of the transcript being assayed. Such quantity may be expressed as the total quantity of the RNA, in relative terms, *e.g.,* compared to the level present in a control cfRNA sample, or as a concentration *e.g.,* copy number per milliliter, of the RNA in the sample.

As used herein, the term "expression level" of a gene as described herein refers to the level of expression of an RNA transcript of the gene.

Genes are typically referred to herein using the official symbol and official nomenclature for the human gene as assigned by the HUGO Gene Nomenclature Committee, when HUGO nomenclature is available. In some embodiments, *e.g.,* for certain genes listed in Table 12 or Table 22, only the ENSEMBL designation is provided. In the present disclosure, an individual gene as designated herein may also have alternative designations, *e.g.*, as indicated in the HGNC database. As used herein, the term "signature gene" refers to a gene whose expression is correlated, either positively or negatively, with risk for preeclampsia. A "gene panel" or "signature gene panel" is a collection of such signature genes for which gene expression scores are generated and used to provide a risk score for preeclampsia and/or a pregnancy complication such as gestational-onset hypertension or gestational diabetes. Thus, for example, an eleven-gene panel, or a subset thereof as described herein, includes the following genes, as designated in the HGNC database: BNIP3L, FECH, HEMGN, SNCA, OAZ1, GSPT1, AKNA, CSF3R, IGF2, RPS15, and MARCH2. An illustrative eighteen-gene panel, or a subset thereof as described herein, includes the following genes, as designated in the HGNC database: CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564). Reference to the gene by name includes any human allelic variant or splice variants, that are encoded by the gene.

The term "nucleic acid" or "polynucleotide" as used herein refers to a deoxyribonucleotide or ribonucleotide in either single- or double-stranded form. In the context of primers or probes, the term encompasses nucleic acids containing known analogues of natural nucleotides which have similar or improved binding properties, for the purposes desired, as the reference nucleic acid; and nucleic-acid-like structures with synthetic backbones.

The term "treatment," "treat," or "treating" typically refers to a clinical intervention, including multiple interventions over a period of time, to ameliorate at least one symptom of preeclampsia or otherwise slow progression. This includes alleviation of symptoms, diminishment of any direct or indirect pathological consequences of preeclampsia, amelioration of preeclampsia, and improved prognosis. It is understood that treatment does not necessarily refer to prevention of preeclampsia.

The term "risk score" refers to a statistically derived value that can provide physicians and caregivers valuable diagnostic and prognostic insight into whether or not the subject is likely to develop preeclampsia. An individual's score can be compared to a reference score or a reference score scale to determine risk of disease recurrence/relapse or to assist in the selection of therapeutic intervention or disease management approaches.

### Gene Expression Panels

The methods described herein are based, in part, on the identification of a panel of eleven genes, and subsets of the eleven genes, that provide a risk score for preeclampsia in pregnant subjects. Such a panel may also be used to predict preeclampsia in the pregnant subject, *e.g.,* before clinical diagnosis. In some embodiments, the pregnant subject is normotensive. As used in this context, or with reference to a control population or reference population of normotensive human females, "normotensive" refers to systolic blood pressure less than 140 mmHg and diastolic blood press less than 90 mmHg. In alternative embodiments, the pregnant subject may have a pregnancy complication that is often observed with preeclampsia, *e.g.,* gestational-onset or chronic hypertension and/or gestational diabetes. The method of assessing risk comprises quantifying cfRNA expression levels for a panel of genes, or a subset of the genes, in cfRNA from a pregnant subject. Genes evaluated for risk of preeclampsia as described herein include the following genes, or subsets thereof.

### 11-gene panel and expanded panels and subsets

The "ENSG" designation is shown based on ENSEMBL version 82.

| | | |
|---|---|---|
| GSPT1 | ENSG00000103342; gene name: | G1 to S phase transition 1; |
| BNIP3L | ENSG00000104765; gene name: | BCL2 interacting protein 3 like |
| MARCH2 | ENSG00000099785; gene name: | membrane associated ring-CH-type finger 2 |
| IGF2 | ENSG0000016724; gene name: | insulin like growth factor 2 |
| HEMGN | ENSG00000136929; gene name: | hemogen |
| OAZ1 | ENSG00000104904; gene name: | ornithine decarboxylase antizyme 1 |
| CSF3R | ENSG00000119535; gene name: | colony stimulating factor 3 receptor |
| RPS15 | ENSG00000115268; gene name: | ribosomal protein S15 |
| AKNA | ENSG00000106948; gene name: | AT-hook transcription factor |
| SNCA | ENSG00000145335; gene name: | synuclein alpha |
| FECH | ENSG00000066926; gene name: | ferrochelatase |

Additional gene information, including chromosome location and an illustrative protein sequence accession number (corresponding to the longest transcript encoded by the gene) is included in Table 10. Reference to the gene by name includes variants, such as allelic variants, including SNP variants, splice variants, and the like. The genome build used for Table 10 is genome build GRCh38.p3 released in Dec 2013 and associated with genome build accession is NCBI:GCA_000001405.18. This corresponds to the Ensembl Version 82. An illustrative human cDNA sequence for each of genes CSF3R, SNCA, BNIP3L, HEMGN, AKNA, IGF2, GSPT1, FECH, RPS15, OAZ1, and MARCH2 is provided in the listing of examples of sequences provided after the EXAMPLES section. The polypeptide sequence is designated using an ENSEMBL designation number. This listing provides examples of cDNA sequences only. Expression of cfRNA for preeclampsia expression is not limited to the particular RNA transcript corresponding to the illustrative cDNA sequence. For example, sequences having at least 90% identity to the illustrative sequence provided in the listing, or that may have 90% identity to a region of the illustrative sequence, *e.g.,* a region of at least 100 or 200 nucleotides in length, or 300 nucleotides in length, may also be encoded by the designated gene.

In some embodiments, detection of preeclampsia risk comprises assessing expression levels in cfRNA of two of the eleven genes, three of the eleven genes, four of eleven genes, five of the eleven genes, six of the eleven genes, seven of the eleven genes, eight of the eleven genes, nine of the eleven gene, or ten of the eleven genes. In some embodiments, detection of preeclampsia risk comprises assessing RNA expression levels of all of the eleven genes in a cfRNA sample. In some embodiments, detection of preeclampsia risk comprises assessing cfRNA levels of a combination of genes. Illustrative subsets of informative genes and combinations of genes for predicting risk of preeclampsia are shown in Table 5. In some embodiments, risk determination comprises quantifying cfRNA for a subset of four, or at least five, of the genes of the 11-gene panel with reference to control levels in cfRNA from normotensive pregnant subjects. Illustrative subsets are shown in Table 6. In some embodiments, risk determination comprises quantifying cfRNA for a subset of four, or at least five, of the genes of the 11-gene panel with reference to control levels in cfRNA that include normotensive subjects as well as subjects who have a complication such as gestational diabetes and/or chronic or gestational-onset of hypertension. Illustrative subsets are shown in Table 7.

In some embodiments, risk for preeclampsia comprises quantifying cfRNA for a subset of one, two, or three members of the 11-gene panel. Illustrative subsets are shown in Table 8.

One of skill understands that many other subsets of the 11-gene panel can be informative in determining preeclampsia risk, *e.g.,* depending on the sensitivity and specificity desired for the assay. The illustrative subsets described in the Tables are examples and are not limiting.

In some embodiments, assessment of risk comprises assessing cfRNA expression level of at least one gene selected from BNIP3L, FECH, HEMGN, SNCA, OAZ1, GSPT1, AKNA, CSF3R, IGF2, RPS15, or MARCH2; or two, three, four, five , six, seven, eight, nine, or ten genes selected from BNIP3L, FECH, HEMGN, SNCA, OAZ1, GSPT1, AKNA, CSF3R, IGF2, RPS15, or MARCH2; or cfRNA expression levels of all 11 genes; and at least one more gene, *e.g.,*, two or more gene, three or more genes, four or more genes, five or more genes, six or more genes, seven or more genes, eight or more genes, nine or more genes, or ten or more genes selected from the genes listed in Table 12. In some embodiments, such a panel does not include a gene encoding a protein listed in WO2019/227015.

In some embodiments, cfRNA expression level can be determined to assess risk of preeclampsia, or a pregnancy complication such as gestational diabetes or gestational-onset hypertension, for a panel of genes comprising at least two genes listed in Table 12. In some embodiments, the panel comprises at least 3, 4, 5, 6, 7, 8, 9, 10, 11, 12, 13, 14, 15, 16, 17, 18, 19, 20, or at least 25, 30, or 35 or more genes selected from the genes listed in Table 12. In some embodiments, such a panel does not include a gene encoding a protein listed in WO2019/227015.

In some embodiments, risk for preeclampsia is determined by quantifying cfRNA for a subset of genes comprising two or more genes selected from those listed in Table 9. In some embodiment, the subset comprises at least one gene selected from BNIP3L, FECH, HEMGN, SNCA, OAZ1, GSPT1, AKNA, CSF3R, IGF2, RPS15, or MARCH2 and a second gene listed in Table 9 that is not BNIP3L, FECH, HEMGN, SNCA, OAZ1, GSPT1, AKNA, CSF3R, IGF2, RPS15, or MARCH2. In alternative embodiments, a subset of genes comprising two or more genes listed in Table 9 used for assessing preeclampsia risk using cfRNA, *e.g.,* from a serum or plasma sample, does not include analysis of expression levels of BNIP3L, FECH, HEMGN, SNCA, OAZ1, GSPT1, AKNA, CSF3R, IGF2, RPS15, or MARCH2.

In some embodiments, cfRNA expressed by a panel comprising the eleven genes BNIP3L, FECH, HEMGN, SNCA, OAZ1, GSPT1, AKNA, CSF3R, IGF2, RPS15, or MARCH2, or comprising subsets of the eleven genes, can be evaluated to provide a risk score for a pregnancy complication such as gestational-onset hypertension or gestational diabetes. In some embodiments, the method of assessing risk comprises evaluating cfRNA levels of one, two, three, four, five, six, seven, eight, nine, ten, or all eleven genes of the panel.

Changes in the direction and magnitude of expression and stability of each of the 11 genes BNIP3L, FECH, HEMGN, SNCA, OAZ1, GSPT1, AKNA, CSF3R, IGF2, RPS15, or MARCH2, shown as log fold-change (logFC), and coefficients of variation (CV), shown across times of gestation, are illustrated in Table 11.

In some embodiments, risk of preeclampsia in a pregnant subject, or of diagnosing preeclampsia in the pregnant subject, comprises quantifying levels of cell-free RNA for a panel of genes, *e.g.,* as described herein in Table 12, from a biological sample from the pregnant subject to obtain a risk score where genes are selected for which 1) the logarithm of change in expression of each of the quantified genes relative to a reference level obtained from control subjects not at risk of developing preeclampsia is at least ± 0.2 ( | log(FC) | ≥ 0.2); (2) the coefficient of variation of each of the quantified genes relative to the reference level is at most 2; (3) the median expression across all samples is at least 5 counts per million reads (CPM); or (4) a combination of one or more of (1), (2), and (3); wherein an increased risk of preeclampsia is assigned to the pregnant subject when the risk score exceeds a threshold value.

### 18-gene panel and expanded panels and subsets

The methods described herein are additionally based, in part, on the identification of a panel of eighteen genes, and subsets of the eighteen genes, that provide a risk score for preeclampsia in pregnant subjects. Such a panel may be used to predict preeclampsia in the pregnant subject, *e.g.,* before clinical diagnosis. In some embodiments, the pregnant subject is normotensive. As used in this context, or with reference to a control population or reference population of normotensive human females, "normotensive" refers to systolic blood pressure less than 140 mmHg and diastolic blood press less than 90 mmHg. As detailed herein, the method of assessing risk comprises quantifying cfRNA expression levels for the panel of genes, or a subset of the genes, in cfRNA from a pregnant subject. Genes of an 18-gene panel that are evaluated for risk of preeclampsia as described herein include the following genes, or subsets thereof. The "ENSG" designation is shown based on ENSEMBL version 82.

| | | |
|---|---|---|
| CAMK2G | ENSG00000148660 | calcium/calmodulin dependent protein kinase II gamma; |
| DERA | ENSG00000023697 | deoxyribose-phosphate aldolase |
| FAM46A | ENSG00000112773 | terminal nucleotidyltransferase 5A |
| KIAA1109 | ENSG00000138688 | KIAA1109 |
| LRRC58 | ENSG00000163428 | leucine rich repeat containing 58 |
| MYLIP | ENSG00000007944 | myosin regulatory light chain interacting protein |
| NDUFV3 | ENSG00000160194 | NADH:ubiquinone oxidoreductase subunit V3 |
| NMRK1 | ENSG00000106733 | nicotinamide riboside kinase 1 |
| PI4KA | ENSG00000241973 | phosphatidylinositol 4-kinase alpha |
| PRTFDC1 | ENSG00000099256 | phosphoribosyl transferase domain containing 1 |
| PYGO2 | ENSG00000163348 | pygopus family PHD finger 2 |
| RNF149 | ENSG00000163162 | ring finger protein 149 |
| TFIP11 | ENSG00000100109 | tuftelin interacting protein 11 |
| TRIM21 | ENSG00000132109 | tripartite motif containing 21 |
| USB1 | ENSG00000103005 | U6 snRNA biogenesis phosphodiesterase 1 |
| Y_RNA | ENSG00000201412 | Y RNA |
| Y_RNA | ENSG00000238912 | Y RNA; and |
| YWHAQP5 | ENSG00000236564 | YWHAQ pseudogene 5 |

Additional gene information, including chromosome location, is provided below:

| Gene | ENSEMBL | Chromosome | Start position | End position | Strand |
|---|---|---|---|---|---|
| PYGO2 | ENSG00000163348 | 1 | 1.55E+08 | 1.55E+08 | - |
| YWHAQP5 | ENSG00000236564 | 2 | 98694109 | 98695490 | + |
| RNF149 | ENSG00000163162 | 2 | 1.01E+08 | 1.01E+08 | - |
| Y_RNA | ENSG00000238912 | 3 | 27547757 | 27547858 | - |
| LRRC58 | ENSG00000163428 | 3 | 1.2E+08 | 1.2E+08 | - |
| KIAA1109 | ENSG00000138688 | 4 | 1.22E+08 | 1.22E+08 | + |
| MYLIP | ENSG00000007944 | 6 | 16129125 | 16148248 | + |
| FAM46A | ENSG00000112773 | 6 | 81491439 | 81752774 | - |
| NMRK1 | ENSG00000106733 | 9 | 75060573 | 75088217 | - |
| TRIM21 | ENSG00000132109 | 11 | 4384897 | 4393696 | - |
| PRTFDC1 | ENSG00000099256 | 10 | 24848607 | 24952604 | - |
| CAMK2G | ENSG00000148660 | 10 | 73812501 | 73874591 | - |
| Y_RNA | ENSG00000201412 | 10 | 92710499 | 92710608 | - |
| DERA | ENSG00000023697 | 12 | 15911172 | 16037282 | + |
| USB1 | ENSG00000103005 | 16 | 57999546 | 58021618 | + |
| PI4KA | ENSG00000241973 | 22 | 20707691 | 20859417 | - |
| TFIP11 | ENSG00000100109 | 22 | 26491225 | 26512505 | - |
| NDUFV3 | ENSG00000160194 | 21 | 42879644 | 42913304 | + |

| Gene | ENSEMBL | Example protein ENSEMBL ID | | | Example transcript ID |
|---|---|---|---|---|---|
| PYGO2 | ENSG00000163348 | ENSP00000357442.2 | | | |
| YWHAQP5 | ENSG00000236564 | NA | | | ENST00000415616.1 |
| RNF149 | ENSG00000163162 | ENSP00000295317.3 | | | |
| Y_RNA | ENSG00000238912 | NA | | | ENST00000391229.2 |
| LRRC58 | ENSG00000163428 | ENSP00000295628.3 | | | |
| KIAA1109 | ENSG00000138688 | ENSP00000505357.1 | | | |
| MYLIP | ENSG00000007944 | ENSP00000349298.3 | | | |
| FAM46A | ENSG00000112773 | ENSP00000318298.6 | | | |
| NMRK1 | ENSG00000106733 | ENSP00000354387.4 | | | |
| TRIM21 | ENSG00000132109 | ENSP00000254436.7 | | | |
| PRTFDC1 | ENSG00000099256 | ENSP00000318602.5 | | | |
| CAMK2G | ENSG00000148660 | ENSP00000410298.3 | | | |
| Y_RNA | ENSG00000201412 | NA | ENST00000364542.1 | | |
| DERA | ENSG00000023697 | ENSP00000416583.2 | | | |
| USB1 | ENSG00000103005 | ENSP00000219281.3 | | | |
| PI4KA | ENSG00000241973 | ENSP00000255882.6 | | | |
| TFIP11 | ENSG00000100109 | ENSP00000384421.1 | | | |
| NDUFV3 | ENSG00000160194 | ENSP00000346196.2 | | | |

Reference to the gene by name includes variants, such as allelic variants, including SNP variants, splice variants, and the like. The genome build used for the above listing of chromosomal positions is genome build GRCh38.p3 released in Dec 2013 and associated with genome build accession is NCBI:GCA_000001405.18. This corresponds to the Ensembl Version 82. An illustrative human cDNA sequence for each of genes CAMK2G, DERA, FAM46A, KIAA1109, LRRC58, MYLIP, NDUFV3, NMRK1, PI4KA, PRTFDC1,PYGO2, RNF149, TFIP11, TRIM21, and USB1; illustrative RNA sequence for Y_RNA (ENSG00000201412) and Y_RNA (ENSG00000238912), and illustrative pseudogene sequence for YWHAQP5 (ENSG00000236564) is provided in the listing of examples of sequences provided after the EXAMPLES section. The listing of genes with annotations relating to biological and molecular functions is provided in Table 17. The sequence corresponds to the protein-encoding cDNA sequence or transcript indicated on the database as "Ensembl Canonical". This listing provides only examples of sequences. Expression of cfRNA for preeclampsia expression is not limited to a particular RNA transcript (for protein-encoding gene, the particular RNA transcript corresponding to the illustrative cDNA sequence). For example, sequences having at least 90% identity to the illustrative sequence provided in the listing, or that may have 90% identity to a region of the illustrative sequence, *e.g.,* a region of at least 100 or 200 nucleotides in length, or 300 nucleotides in length, may also be encoded by the designated gene. In some embodiments, the RNA transcript is not a protein-encoding RNA.

In some embodiments, detection of preeclampsia risk comprises assessing expression levels in cfRNA of two of the eighteen genes, three of the eighteen genes, four of eighteen genes, five of the eighteen genes, six of the eighteen genes, seven of the eighteen genes, eight of the eighteen genes, nine of the eighteen gene, ten of the eighteen genes, eleven of the eighteen genes, twelve of the eighteen genes, thirteen of the eighteen genes, fourteen of the eighteen genes, fifteen of the eighteen genes, sixteen of the eighteen genes, or seventeen of the eighteen genes. In some embodiments, detection of preeclampsia risk comprises assessing RNA expression levels of all of the eighteen genes in a cfRNA sample. In some embodiments, detection of preeclampsia risk comprises assessing cfRNA levels of a combination of genes. In some embodiments, detection of preeclampsia risk comprises assessing cfRNA levels of a combination of genes. Illustrative subsets of informative genes and combinations of genes for predicting risk of preeclampsia are shown in Table 20 (Subsets are defined as predictive if they had at least 50% specificity and 50% sensitivity on Validation 2-see Example 2). In some embodiments, risk determination comprises quantifying cfRNA for a subset of fifteen genes of the 18-gene panel with reference to control levels in cfRNA from normotensive pregnant subjects. In some embodiments, risk determination comprises quantifying cfRNA for a subset of twelve or thirteen genes of the 18-gene panel with reference to control levels in cfRNA from normotensive pregnant subjects. In some embodiments, risk determination comprises quantifying cfRNA for a subset of nine, ten, or eleven genes of the 18-gene panel with reference to control levels in cfRNA from normotensive pregnant subjects. In some embodiments, risk determination comprises quantifying cfRNA for a subset of seven, eight, or nine genes of the 18-gene panel with reference to control levels in cfRNA from normotensive pregnant subjects. In some embodiments, risk determination comprises quantifying cfRNA for a subset of four, five, or six genes of the 18-gene panel with reference to control levels in cfRNA from normotensive pregnant subjects. In some embodiments, risk for preeclampsia comprises quantifying cfRNA for a subset of one, two, or three members of the 18-gene panel. Illustrative subsets of combinations of informative genes are shown in Table 20. Additional informative genes or gene combinations are shown in Table 18. In some embodiments, risk determination comprises quantifying TRIM21, Y_RNA (ENSG00000238912), PI4KA, PYGO2, FAM45A, TFIP11, USB1, MYLIP, DERA, and/or LRRC58 RNA levels.

One of skill understands that many other subsets of the 18-gene panel can be informative in determining preeclampsia risk, *e.g.,* depending on the sensitivity and specificity desired for the assay. The illustrative subsets described in Table 20 are examples and are not limiting.

In some embodiments, the subset of genes comprises one or more genes listed in Table 21A, 21B, or 21C**.** In some embodiments, cfRNA levels are evaluated for a subset comprising at least 10, at least 15, or at least 20 genes listed in Table 21A. In some embodiments, cfRNA levels are evaluated for a subset comprising 24-29 of the genes listed in Table 21A. In some embodiments, cfRNA levels are evaluated for a subset comprising at least 10, at least 15, or at least 20 genes listed in Table 21B. In some embodiments, cfRNA levels are evaluated for a subset comprising 24-32 of the genes listed in Table 21B. In some embodiments, cfRNA levels are evaluated for a subset comprising at least 10, at least 15, or at least 20 genes listed in Table 21C. In some embodiments, cfRNA levels are evaluated for a subset comprising 24-30 of the genes listed in Table 21C. In some embodiments, cfRNA levels of a sample obtained at 12 or fewer weeks of gestation are evaluated for a subset comprising multiple genes from Table 21A; cfRNA levels of a sample obtained at 13-20 weeks of gestation are evaluated for a subset comprising multiple genes from Table 21B, and cfRNA levels of a sample obtained 23 or greater weeks of gestation are evaluated for a subset comprising multiple genes from Table 21C.

In some embodiments, assessment of risk comprises assessing cfRNA expression level of at least one gene from the 18-gene panel; or two, three, four, five , six, seven, eight, nine, ten, eleven, twelve, thirteen, fourteen, fiften, sixteen, or seventen genes of the 18-gene panel; or cfRNA expression levels of all 18 genes; and at least one more gene, *e.g.*, two or more genes, three or more genes, four or more genes, five or more genes, six or more genes, seven or more genes, eight or more genes, nine or more genes, or ten or more genes selected from the genes listed in Table 22 or the genes listed in Table 23. In some embodiments, such a panel does not include a gene encoding a protein listed in WO2019/227015.

In some embodiments, cfRNA expression level can be evaluated to assess risk of preeclampsia for a panel of genes comprising multiple genes listed in Table 22. For all 544 genes shown in Table 22 that were identified as distinct between PE with or without severe features and NT pregnancies (see, Example 2), their corresponding logFC (how striking the difference is) and CV (how stable the difference is across all samples) is provided. In some embodiments, the panel comprises at least 3, 4, 5, 6, 7, 8, 9, 10, 11, 12, 13, 14, 15, 16, 17, 18, 19, 20, or at least 25, 30, or 35 or more genes selected from the genes listed in Table 22. In some embodiments, such a panel does not include a gene encoding a protein listed in WO2019/227015.

In some embodiments, risk for preeclampsia is determined by quantifying cfRNA for a subset of genes comprising two or more genes selected from those listed in Table 23. For every gene in Table 23, the symbol, ENSEMBL ID, sample collection groups for which the gene passed cutoff thresholds, full name, ENSEMBL gene type, and a subset of GO biological processes and molecular functions are reported. In some embodiment, the subset comprises at least one gene selected from CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), and YWHAQP5 (ENSG00000236564); and a second gene listed in Table 23 that is not CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), or YWHAQP5 (ENSG00000236564). In alternative embodiments, a subset of genes comprising two or more genes listed in Table 23 used for assessing preeclampsia risk using cfRNA, *e.g.,* from a serum or plasma sample, does not include analysis of expression levels of CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), or YWHAQP5 (ENSG00000236564).

Changes in the direction and magnitude of expression and stability of each of the 18 genes CAMK2G, DERA, FAM46A, KIAA1109, LRRC58, MYLIP, NDUFV3, NMRK1, PI4KA, PRTFDC1,PYGO2, RNF149, TFIP11, TRIM21, USB1, Y_RNA (ENSG00000201412), Y_RNA (ENSG00000238912), or YWHAQP5 (ENSG00000236564) is shown as log fold-change (logFC), and coefficients of variation (CV), shown across times of gestation, are illustrated in Table 22**.**

In some embodiments, risk of preeclampsia in a pregnant subject, or of diagnosing preeclampsia in the pregnant subject, comprises quantifying levels of cell-free RNA for a panel of genes, *e.g.,* from a biological sample from the pregnant subject to obtain a risk score where genes are selected for which (1) the logarithm of change in expression of each of the quantified genes relative to a reference level obtained from control subjects not at risk of developing preeclampsia is at least ± 0.2 ( | log(FC) | ≥ 0.2); (2) the coefficient of variation of each of the quantified genes relative to the reference level is at most 6; (3) the median expression across all samples is at least 5 counts per million reads (CPM); or (4) a combination of one or more of (1), (2), and (3); wherein an increased risk of preeclampsia is assigned to the pregnant subject when the risk score exceeds a threshold value. In some embodiments, (2) and/or (3) are not employed in the selection. In some embodiments, (2) employs a coefficient of variation for each of the quantified genes relative to the reference level is at most 12.

### Genes for determining severe PE risk

In a further aspect, the disclosure also describes a method of determining risk of severe PE, the method comprising determining cfRNA levels of one or more genes selected from the genes listed in Table 24, which are able to separate severe PE from PE without severe features. Table 24 shows the 503 genes identified as distinct between PE with as compared to without severe features in Example 2, their corresponding logFC (how striking the difference is) and CV (how stable the difference is across all samples) is provided. Changes in the direction and magnitude of expression and stability of genes is shown in Table 24 is shown as log fold-change (logFC), and coefficients of variation (CV), shown across times of gestation.

In some embodiments, risk for severe preeclampsia compared to eclampsia without severe features is determined by quantifying cfRNA for a subset of genes comprising at least one gene selected from those listed in Table 24. In some embodiments, cfRNA levels are evaluated for a subset comprising at least 10, 20, 30, 40, 50, 60, 70, 80, 90, or at least 100 genes listed in Table 24. In some embodiments, cfRNA levels are evaluated for a subset comprising at least one of the genes listed in Table 25A. In some embodiments, cfRNA levels are evaluated for a subset comprising at least two, three, four, or five genes listed in Table 25A. In some embodiments, cfRNA levels are evaluated for a subset comprising 10 or more genes, or all of the genes, listed in Table 25A. In some embodiments, cfRNA levels are evaluated for a subset comprising at least one of the genes listed in Table 25B. In some embodiments, cfRNA levels are evaluated for a subset comprising at least two, three, four, or five genes listed in Table 25B. In some embodiments, cfRNA levels are evaluated for a subset comprising 10 or more genes, or all of the genes, listed in Table 25B. In some embodiments, cfRNA levels are evaluated for a subset comprising at least one of the genes listed in Table 25C. In some embodiments, cfRNA levels are evaluated for a subset comprising at least two, three, four, or five genes listed in Table 25C. In some embodiments, cf RNA levels are evaluated for a subset comprising 10 or more genes, or all of the genes, listed in Table 25C. In some embodiments, cfRNA levels are evaluated for a subset comprising one or more genes from each of Tables 25, 26, and 27. In some embodiments, cfRNA levels of a sample obtained at 12 or fewer weeks of gestation are evaluated for a subset comprising multiple genes from Table 25A; and cfRNA levels of a sample obtained at 13-20 weeks of gestation are evaluated for a subset comprising multiple genes from Table 25B.

In some embodiments, risk for severe preeclampsia in a pregnant subject comprises quantifying levels of cell-free RNA for a panel of genes, *e.g.,* from a biological sample from the pregnant subject to obtain a risk score where genes are selected for which 1) the logarithm of change in expression of each of the quantified genes relative to a reference level obtained from control subjects not at risk of developing severe preeclampsia is at least ± 0.2 ( | log(FC) | ≥ 0.2); (2) the coefficient of variation of each of the quantified genes relative to the reference level is at most 2; (3) the median expression across all samples is at least 5 counts per million reads (CPM); or (4) a combination of one or more of (1), (2), and (3); wherein an increased risk of preeclampsia is assigned to the pregnant subject when the risk score exceeds a threshold value. In some embodiments, (2) and/or (3) are not employed in the selection. In some embodiments, (2) employs a coefficient of variation for each of the quantified genes relative to the reference level is at most 6 or is at most 12.

### Genes for monitoring maternal organ health

In some embodiments, cfRNA levels of one or more genes set forth in Table 26 can be evaluated to monitor maternal organ health (health of a maternal tissue or cell type), in a pregnant subject. In the context of the present invention, the health of a tissue or cell type is reflected by the level of cfRNA for genes selected from those listed in Table 26 in a pregnant subject compared to cfRNA levels in normal controls, *e.g.,* normotensive pregnant control. Tissue and cell-type cfRNA scores are typically normalized using another blood sample from the same person for comparison to normotensive and preeclampsia values.

In some embodiments, monitoring organ health comprises evaluating cfRNA levels for at least 5, 10, 15, 20, 25, 30, or 35 or more genes listed in Table 26. In some embodiments cfRNA levels for at least 50 genes listed in Table 26 are evaluated. In some embodiments, cfRNA is monitored to assess brain, liver, kidney, heart, bone marrow, placenta, skeletal muscle, and/or or smooth muscle health. In some embodiments, cfRNA is monitored to assess health of astrocytes, excitatory neurons, inhibitory neurons, oligodendrocytes, oligodendrocyte progenitor cells, B-cells, T-cells, NK-cells, granulocytes, extravillous trophoblasts, syncytiotrophoblasts, proximal tubule cells, platelet, endothelial cells, hepatocytes, liver sinusoidal endothelial cells, atrial cardiomyocytes, and/or ventricular cardiomyocytes.

In some embodiments, genes for monitoring tissue/cell type health by monitoring cfRNA levels include those meeting one or a combination of two or more of the following criteria:
The Gini index for each of the genes as quantified using a reference atlas such as the Human Protein Atlas (HPA) or Tabula Sapiens (TSP) is at least 0.5 (Gini ≥ 0.5);
The average (mean or median) expression of a gene in a given cell-type or tissue is maximum or within a reasonable margin of the maximum (e.g., within the 80th percentile) as compared to all other quantified tissues or cell-types in the reference atlas; or
The gene is annotated as specific to a given cell-type or tissue in a given reference (e.g., For the HPA, these labels would be Tissue enriched, Tissue enhanced, or Group enriched).

### Methods of quantifying RNA expression

In order to evaluate preeclampsia risk, a cfRNA is isolated from a sample of a bodily fluid that does not contain cells, *e.g.,* a blood sample lacking platelets and other blood cells, *e.g.,* a serum or plasma sample, obtained from a pregnant subject. The cfRNA is processed to evaluate levels of cfRNA of one or more genes as described herein. In some embodiments, the blood sample is obtained from the pregnant subject at 5 weeks of gestation or later. In some embodiments, the blood sample is obtained in a time frame of 5-12 weeks of gestation. In some embodiments, the blood sample is obtained at 13-18 weeks of gestation. In some embodiments, the blood sample is obtained at 23-33 weeks of gestation. In other embodiments, the blood sample may be obtained after 33 weeks of gestation.

The level of RNA in a cfRNA sample obtained from a subject, *e.g.,* a plasma or serum sample, expressed by the genes of a panel as described above, or a subset thereof, can be detected or measured by a variety of methods including, but not limited to, an amplification assay, sequencing assay, or a microarray chip (hybridization) assay. As used herein, "amplification" of a nucleic acid sequence has its usual meaning, and refers to *in vitro* techniques for enzymatically increasing the number of copies of a target sequence. Amplification methods include both asymmetric methods in which the predominant product is single-stranded and conventional methods in which the predominant product is double-stranded. The term "microarray" refers to an ordered arrangement of hybridizable elements, *e.g.,* gene-specific oligonucleotides, attached to a substrate. Hybridization of nucleic acids from the sample to be evaluated is determined and converted to a quantitative value representing relative gene expression levels.

Non-limiting examples of methods to evaluate levels of cfRNA include amplification assays such as quantitative RT-PCR, digital PCR, massively parallel sequencing, microarray analysis; ligation chain reaction, oligonucleotide elongation assays, multiplexed assays, such as multiplexed amplification assays. In some embodiments, expression level is determined by sequencing, *e.g.,* using massively parallel sequencing methodologies. For example, RNA-Seq can be employed to determine RNA expression levels. Illustrative methods for cfRNA analysis are described, for example, in WO2019/084033.

Typically measured cfRNA values are normalized to account for sample-to-sample variations in RNA isolation and the like. Methods for normalization are well known in the art. In some embodiments, normalization of values is performed using trimmed mean of M values (TMM) normalization, *e.g.,* when using RNA-Seq to evaluate cfRNA expression levels. In some embodiments, normalized values may be obtained using a reference level for one or more of control gene; or exogenous RNA oligonucleotides such as those provided by the External RNA Controls Consortium, or all of the assayed RNA transcripts, or a subset thereof, may also serve as reference. A control value for normalization of RNA values can be predetermined, determined concurrently, or determined after a sample is obtained from the subject. Thus, for example, the reference control level for normalization can be evaluated in the same assay or can be a known control from one or more previous assays.

### Establishing preeclampsia risk scores

After quantifying expression of the genes of a gene signature panel for predicting preeclampsia risk or monitoring organ/cell type health as described herein a risk score can be calculated based on the level of RNA expression of each member of a gene panel as described herein, or a subset thereof. In some embodiments, the level of expression of each gene is weighted with a predefined coefficient. The predefined coefficient can be the same or different for the genes and can be determined by statistical or machine learning regression or classification such as, but not limited to, linear regression, including least squares regression, ridge or LASSO regression, elastic net regression, regularized Cox regression, logistic regression, orthogonal matching pursuit models, a Bayesian regression model, or deep learning methods, such as convolutional neural networks, recurrent neural networks and generative adversarial networks (see, *e.g.,* LeCun et al., .Nature 521: 436-444, 2015). Preeclampsia risk can be determined using any number of models. Machine-learning algorithms include quadratic discriminate analysis, support vector machines, including without limitation support vector classification-based regression processes, stochastic gradient descent algorithms, nearest neighbors algorithms, Gaussian processes such as Gaussian process regression, cross-decomposition algorithms, including partial least squares and/or canonical correlation analysis; probabilistic graphical models including naive Bayes methods; models based on decision trees, such as decision tree classification algorithms. Additional machine-learning algorithms include ensemble methods such as bagging meta-estimator, randomized forest algorithms, AdaBoost, gradient tree boosting, and/or voting classifier methods. Details relating to various statistical methods are found in the following references: Ruczinski et al., 12 J. OF COMPUTATIONAL AND GRAPHICAL STATISTICS 475-511 (2003); Friedman, J. H., 84 J. OF THE AMERICAN STATISTICAL ASSOCIATION 165-75 (1989); Hastie, Trevor, Tibshirani, Robert, Friedman, Jerome, The Elements of Statistical Learning, Springer Series in Statistics (2001); Breiman, L., Friedman, J. H., Olshen, R. A., Stone, C. J. Classification and regression trees, California: Wadsworth (1984); Breiman, L., 45 MACHINE LEARNING 5-32 (2001); Pepe, M. S., The Statistical Evaluation of Medical Tests for Classification and Prediction, Oxford Statistical Science Series, 28 (2003); and Duda, R. O., Hart, P. E., Stork, D. G., Pattern Classification, Wiley Interscience, 2nd Edition (2001), each of which is incorporated by reference.

In some embodiments, risk of preeclampsia (or a complication of pregnancy such as gestational-onset hypertension or gestation diabetes; or risk of severe preeclampsia) may be assigned based on a cutoff value using a reference scale, *e.g.,* from 0 to 1.0. In some embodiments, a cutoff value of 0.5 or greater may be employed to define risk. In some embodiments, a cutoff value of 0.35 or greater may be employed to define risk. In some embodiments, a patient's risk score is categorized as "high," "intermediate," or "low", *e.g.,* based on the highest tertile, intermediate tertile and bottom tertile. In some embodiments, risk may be further stratified.

In some embodiments, organ or cell-type health may be assigned, *e.g.,* employing least squares regression analysis, based on a cutoff value between the minimum and maximum normalized organ or cell-type signature score. Typically, another blood sample obtained from the patient is used for normalization for quantification. Samples are further scaled for normalization to a range of negative and positive values around 0 (*e.g*., a z-score).

### Control Subjects, Reference Levels

In one aspect the method disclosed herein comprises detecting or measuring a difference (or change) in expression of a gene (e.g., one or more of the 11 genes of the 11-gene panel; or one or more of the 18 genes of the 18-gene panel described herein), relative to a reference level of expression of the gene, or control level wherein the change is associated with preeclampsia or risk of preeclampsia and the reference or control level indicative or low risk is determined from a control population.

In order to establish a reference or control risk scale or a threshold value for practicing the methods of this invention, a control population of subjects can be used. Illustrative control populations include, but are not limited to, normotensive human females, normotensive human females of reproductive age, pregnant normotensive human females, or normotensive pregnant women who do not develop preeclampsia. In some embodiments, the control population may include pregnant subjects who have a pregnancy complication such gestational diabetes and/or chronic or gestational-onset hypertension, but do not develop preeclampsia. In some embodiments, *e.g.,* for determining risk of severe PE, a control population may be pregnant subjects who do not develop severe PE.

The expression profile of a gene panel for assessing preeclampsia risk, or risk of a pregnancy complication associated with preeclampsia risk, such as gestational diabetes or gestational-onset hypertension is compared to a reference profile to determine a risk score. As used herein, the term "expression profile," refers to the cfRNA expression level from a maternal sample of one or a plurality of genes. An expression profile may be determined using any suitable method, as described above.

As used herein, a "reference profile" is an expression profile derived from a reference population, such as those listed above. In some embodiments, the reference population is a subpopulation of pregnant women, *e.g.,* characterized by maternal age, race, ethnicity, body mass index (BMI), and/or number of pregnancies. In some embodiments, a reference population of pregnant mothers can be one in which the pregnancy is not only normotensive, but absent other complications such as preterm birth, small for gestational age deliveries and where features such as multi-gestation, fetal sex and history of PE or other pregnancy complications are controlled for. A reference profile is generated by combining expression profiles of a statistically significant number of women in the population and, for a specified gene product, may reflect the mean transcript level in the population, the median transcript level in the population, or may be determined using any of a number of methods known in the fields of epidemiology and medicine. A reference population will typically comprise at least 10 subjects (e.g., 10-200 subjects), sometimes 50 or more subjects, and sometimes 1000 or more subjects.

### Treatment

Subjects who are determined to have an increased risk of preeclampsia can be treated with low-dose aspirin. Patients with increased risk of preeclampsia will also typically be monitored more frequently for increases in blood pressure or other symptoms of preeclampsia.

In some embodiments, preeclampsia may be diagnosed via the cfRNA expression profile comprising a gene panel described herein. In instances where preeclampsia is diagnosed using such a cfRNA panel, the patient may be treated for preeclampsia, for example, using antihypertensive medications; or in instances of severe preeclampsia, with corticosteroids or anticonvulsants.

It will be appreciated that, although certain features of panels are discussed in this section, the invention is not limited to these particular described embodiments.

In some approaches, multiple different profile panels are used during the course of a subject's pregnancy. For example, a first profile panel may be used in the first trimester and a different profile panel may be used in the second trimester. In some embodiments, the same expression panel may be used to monitor preeclampsia risk throughout pregnancy, *e.g.,* at any time 5 weeks or later of gestation.

### Kits and Compositions

The present disclosure also provides kits for practicing the methods described herein. The kits may comprise any or all of the reagents to perform the methods described herein. In some embodiments, a kit may include any or all of the following: assay reagents, buffers, nucleic acids that bind to at least one of the members of the eleven-gene panel, or subset as described herein, and hybridization probes and/or primers*.* In addition, the kit may include reagents such as nucleic acids, hybridization probes, or primers and the like that specifically bind to a reference gene or a reference polypeptide.

The term "kit" as used herein in the context of detection reagents, are intended to refer to such things as combinations of multiple gene expression product detection reagents, or one or more gene expression product detection reagents in combination with one or more other types of elements or components (*e.g.*, other types of biochemical reagents, containers, packages such as packaging intended for commercial sale, substrates to which gene expression detection product reagents are attached, electronic hardware components, *etc.*).

In some embodiments, a kit comprises primers and probes that specifically hybridize to an RNA, or amplify cDNA, of a gene panel, or subset thereof, as described herein. It is well within the ability of persons of ordinary skill in the art to design probes and primers for their intended uses, taking into account methods of amplification (e.g., addition of adaptors or universal primers), target sequence composition, base composition, avoiding artifacts such as primer dimer formation, as well as the fragmented nature of cfRNA. In some embodiments, the kit comprises primers for amplification of no more than 50 genes or no more than 100 genes. In some embodiments, the kit comprises primers for amplification of no more than 500 genes or for amplification of no more than 1,000 genes.

In some embodiments, provided herein are array compositions for determining cfRNA expression levels. In some embodiments, the microarray comprises probes for hybridization to detect expression of no more than 50 genes or no more than 100 genes. In some embodiments, the microarray comprises probes for hybridization to detect expression of no more than 500 genes or no more than 1000 genes.

### Computer-Implemented Methods

In some embodiments, a database comprising reference values for cfRNA levels of the 11-gene panel, or subset thereof. In some embodiments, a database comprising expression data from a plurality of human females *e.g.* normotensive human females, and optionally different subpopulations, is provided. Accordingly, aspects of the invention provide systems and methods for the use and development of a database. In some approaches, the database is used in combination with an algorithm that enables generation of new reference profiles selected based on characteristics of an individual subject.

Methods of the invention may be implemented using a computer-based system. As used herein, "a computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

In some embodiments, a database comprising reference profiles is used in methods of the invention. In some embodiments, a database comprising expression data from a plurality of women, and optionally different subpopulations of women, is provided. Accordingly, aspects of the invention provide systems and methods for the use and development of a database. In some approaches the database is used in combination with an algorithm that enables generation of new reference profiles selected based on characteristics of an individual woman.

Any of the computer systems mentioned herein may utilize any suitable number of subsystems. In some embodiments, a computer system includes a single computer apparatus, where the subsystems can be the components of the computer apparatus. In other embodiments, a computer system can include multiple computer apparatuses, each being a subsystem, with internal components. A computer system can include desktop and laptop computers, tablets, mobile phones and other mobile devices.

A computer system can include a plurality of the same components or subsystems, e.g., connected together by external interface, by an internal interface, or via removable storage devices that can be connected and removed from one component to another component. In some embodiments, computer systems, subsystem, or apparatuses can communicate over a network. In such instances, one computer can be considered a client and another computer a server, where each can be part of a same computer system. A client and a server can each include multiple systems, subsystems, or components.

Aspects of embodiments can be implemented in the form of control logic using hardware circuitry (e.g. an application specific integrated circuit or field programmable gate array) and/or using computer software with a generally programmable processor in a modular or integrated manner. As used herein, a processor can include a single-core processor, multi-core processor on a same integrated chip, or multiple processing units on a single circuit board or networked, as well as dedicated hardware. Based on the disclosure and teachings provided herein, a person of ordinary skill in the art will know and appreciate other ways and/or methods to implement embodiments of the present invention using hardware and a combination of hardware and software.

Any of the software components or functions described in this application may be implemented as software code to be executed by a processor using any suitable computer language such as, for example, Java, C, C++, C#, Objective-C, Swift, or scripting language such as Perl or Python using, for example, conventional or object-oriented techniques. The software code may be stored as a series of instructions or commands on a computer readable medium for storage and/or transmission. A suitable non-transitory computer readable medium can include random access memory (RAM), a read only memory (ROM), a magnetic medium such as a hard-drive or a floppy disk, or an optical medium such as a compact disk (CD) or DVD (digital versatile disk), flash memory, and the like. The computer readable medium may be any combination of such storage or transmission devices.

The databases may be provided in a variety of forms or media to facilitate their use. "Media" refers to a manufacture that contains the expression information of the present invention. The databases of the present invention can be recorded on computer readable media, e.g. any medium that can be read and accessed directly by a computer (e.g., an internet database). Such media include, but are not limited to: magnetic storage media, such as floppy discs, hard disc storage medium, and magnetic tape; optical storage media such as CD-ROM; electrical storage media such as RAM and ROM; and hybrids of these categories such as magnetic/optical storage media. One of skill in the art can readily appreciate how any of the presently known computer readable media can be used to create a manufacture comprising a recording of the present database information. "Recorded" refers to a process for storing information on computer readable medium, using any such methods as known in the art. Any convenient data storage structure may be chosen, based on the means used to access the stored information. A variety of data processor programs and formats can be used for storage, e.g. word processing text file, database format, etc.

Such programs may also be encoded and transmitted using carrier signals adapted for transmission via wired, optical, and/or wireless networks conforming to a variety of protocols, including the Internet. As such, a computer readable medium may be created using a data signal encoded with such programs. Computer readable media encoded with the program code may be packaged with a compatible device or provided separately from other devices (e.g., via Internet download). Any such computer readable medium may reside on or within a single computer product (e.g. a hard drive, a CD, or an entire computer system), and may be present on or within different computer products within a system or network. A computer system may include a monitor, printer, or other suitable display for providing any of the results mentioned herein to a user.

Any of the methods described herein may be totally or partially performed with a computer system including one or more processors, which can be configured to perform the steps. Thus, embodiments can be directed to computer systems configured to perform the steps of any of the methods described herein, potentially with different components performing a respective step or a respective group of steps. Although presented as numbered steps, steps of methods herein can be performed at a same time or at different times or in a different order. Additionally, portions of these steps may be used with portions of other steps from other methods. Also, all or portions of a step may be optional. Additionally, any of the steps of any of the methods can be performed with modules, units, circuits, or other means of a system for performing these steps.

Although the foregoing invention has been described in some detail by way of illustration and example for purposes of clarity of understanding, one of skill in the art will appreciate that certain changes and modifications may be practiced within the scope of the appended claims. In addition, each reference provided herein is incorporated by reference in its entirety to the same extent as if each reference was individually incorporated by reference.

### Examples

The following examples are offered to illustrate, but not to limit, the claimed invention. The Examples describe the identification and validation of a panel of genes for assessing risk of preeclampsia.

### Example 1. Identification of cfRNA changes across gestation in mothers who developed PE

This example demonstrates that cfRNA transcriptomic changes can distinguish between normotensive (NT) and PE pregnancies before clinical diagnosis across gestation: early (5-12 weeks), mid (13-18 weeks), and late (23-33 weeks), and even into the post-partum period (0-2 weeks after delivery) regardless of PE subtype. The majority of these cfRNA changes are most pronounced early in pregnancy suggesting that the identified cfRNA signal may correlate with PE pathogenesis, which is thought to also occur at this time. Indeed, gene ontology (GO) analysis identified pathways that reflect known PE biology. These examples detail identification and validation (n = 8 NT, 8 PE/gestational hypertension) that 11 genes, which measured between 5-16 weeks of gestation, can form a predictive PE signature having a specificity of 88% [55-99%] and sensitivity of 100% [74-100%] in validation (AUC=94%, [79-100%]) (All reported as value, [95% confidence interval]). These results showed that cfRNA can form the basis for a robust predictor of PE well before its clinical presentation and that such measurements may provide a means by which to characterize the pathogenesis of PE in real time.

### Clinical study design

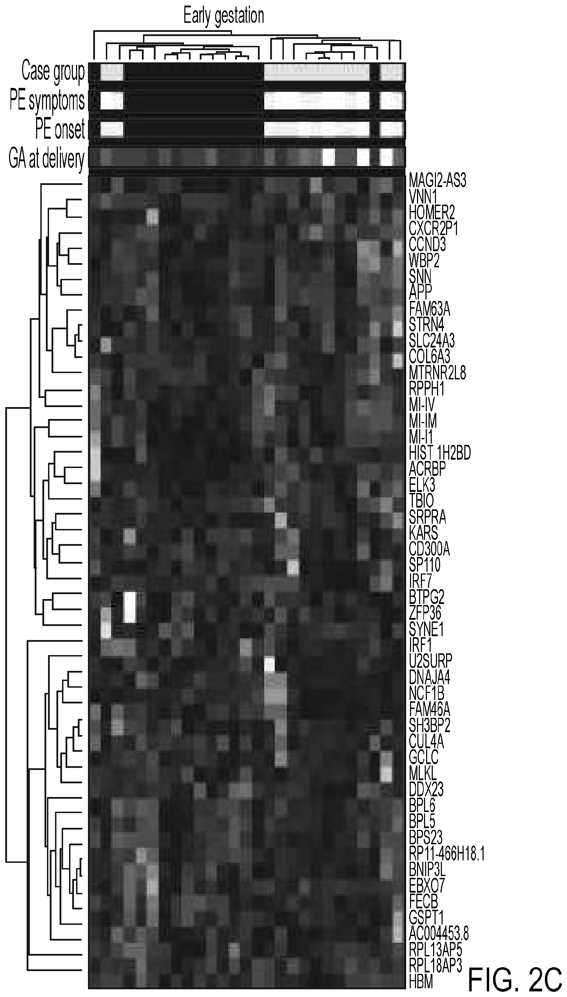

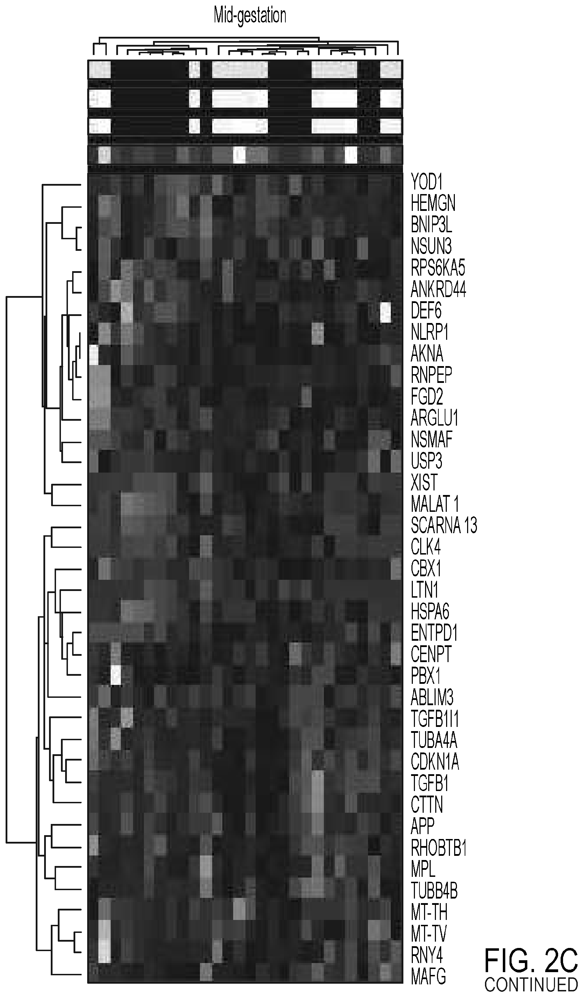

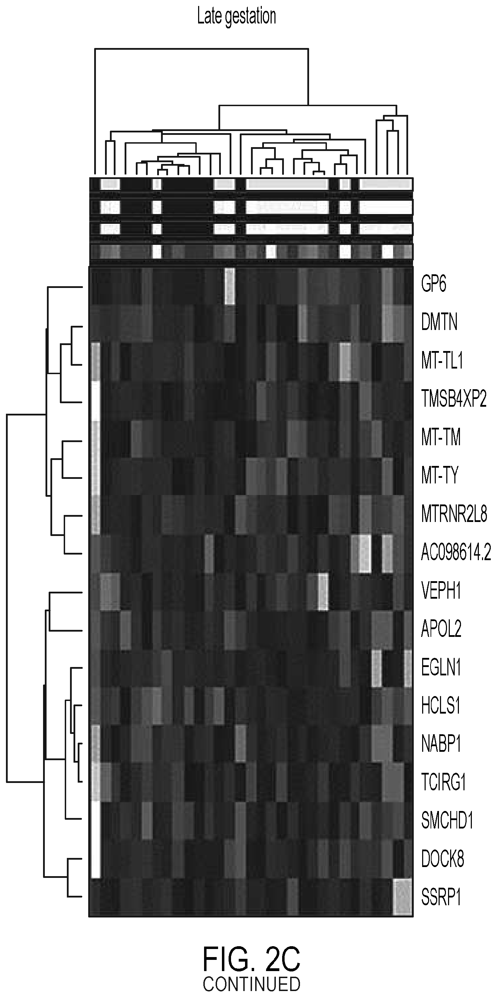

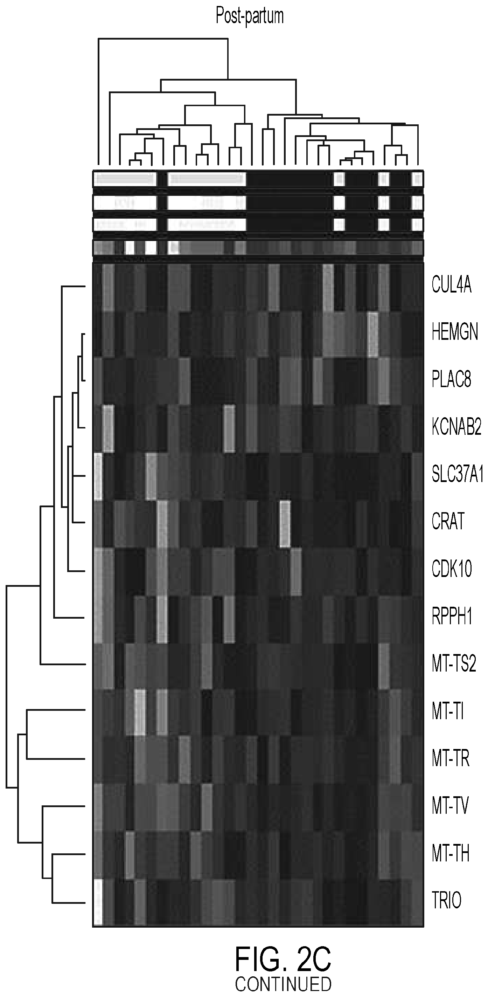

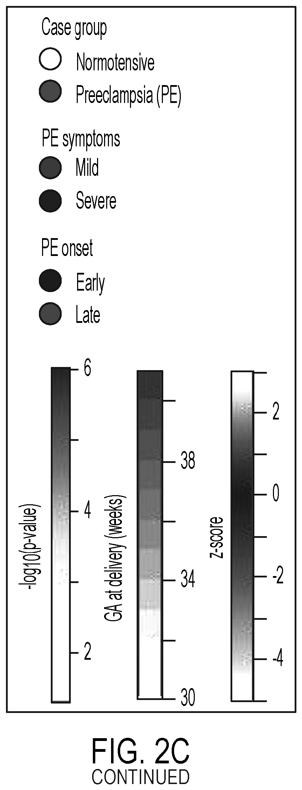

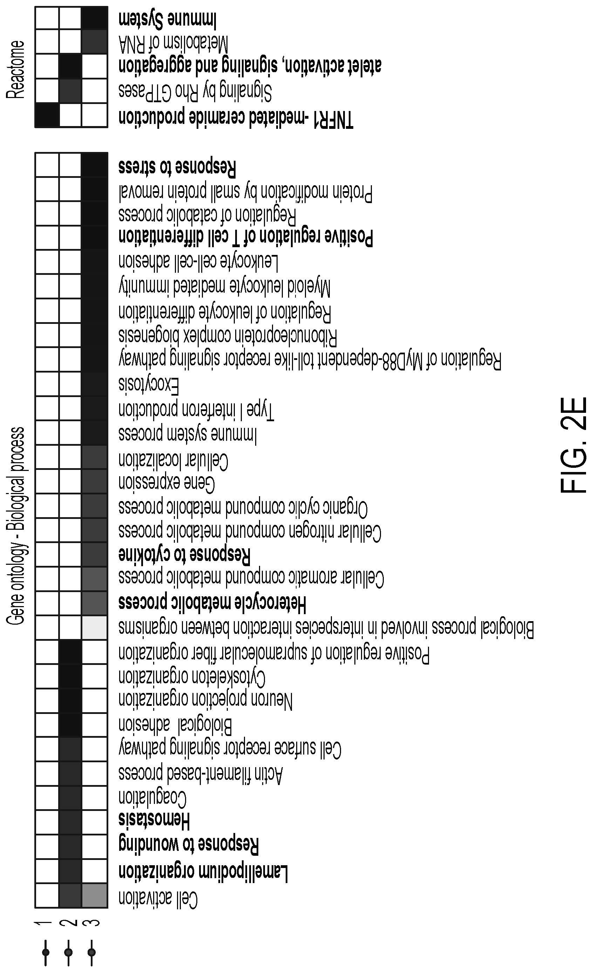

To identify changes associated with PE well before traditional diagnoses, we designed a prospective study and recruited pregnant mothers at their first clinical visit, between 5-12 weeks of gestation, of which 66 were included in this work (28 normotensive (NT), 38 preeclampsia (PE)). For each participant, we then collected 4 blood plasma samples corresponding to early (5 -12 weeks), mid- (13-18 weeks), and late (23-33 weeks) gestation, and post-partum (0-2 weeks after delivery) (Fig 1A). We also recorded maternal pre-pregnancy and pregnancy characteristics (Table 1). We defined a pregnancy as NT if it was both uncomplicated and went to full-term (≥ 37 weeks) and as PE based on current guidelines (see Methods). For mothers who developed PE, all antenatal blood samples were collected prior to diagnosis. To verify our results, we split this larger group into discovery (n = 49, [20 NT, 29 PE]) and validation (n = 17, [8 NT, 9 PE]) cohorts that were processed at separate times. Our final analysis included a subset of these samples that passed pre-defined quality metrics (Methods, Figs 4,2).

**Table 1. Participant, pregnancy, and PE characteristics across both discovery and validation cohorts. Maternal age and BMI, gestational age (GA) at delivery, fetal weight, and GA at PE onset are reported mean ± standard deviation. All other values are reported as percentages. Small for GA (SGA) was defined as an infant with a birthweight below the 10^{th} centile for their GA at delivery.**

| **Maternal pre-pregnancy characteristics** | | | | |
|---|---|---|---|---|
| | | Age (years) | BMI (kg/m²) | Nulliparous (%) |
| Discovery | NT | 32 ± 5 | 23 ± 3 | 44 |
| | PE | 31 ± 6 | 30 ± 8 | 38 |
| Validation | NT | 32 ± 3 | 25 ± 6 | 12 |
| | PE | 32 ± 4 | 26 ± 4 | 29 |

### Maternal ethnicity/race

| | | Ethnicity | Race | | | |
|---|---|---|---|---|---|---|
| | | Hispanic (%) | White (%) | Black (%) | Asian (%) | Unknown (%) |
| Discovery | NT | 0 | 100 | 0 | 0 | 0 |
| | PE | 42 | 50 | 4 | 25 | 21 |
| Validation | NT | 38 | 100 | 0 | 0 | 0 |
| | PE | 57 | 86 | 0 | 14 | 0 |

### Pregnancy characteristics

| | GA at delivery (weeks) | Mode of delivery (% vaginal) | Multi-gestation (%) | Preterm delivery (%) | Fetal sex (% male) | Fetal weight (kg) | SGA (%) | |
|---|---|---|---|---|---|---|---|---|
| Discovery | NT | 40 ± 1 | 61 | 0 | 0 | 56 | 3.5 ± 0.4 | 0 |
| | PE | 36 ± 3 | 46 | 12 | 38 | 54 | 2.8 ± 0.7 | 4 |
| Validation | NT | 39 ± 1 | 62 | 0 | 0 | 25 | 3.6 ± 0.6 | 0 |
| | PE | 38 ± 1 | 57 | 0 | 14 | 57 | 3.4 ± 0.3 | 0 |

### Preeclampsia characteristics

| | | GA at onset (weeks) | Onset type (% early) | Symptom severity (% severe) | History of PE (%) |
|---|---|---|---|---|---|
| Discovery | PE | 36 ± 4 | 25 | 54 | 17 |
| Validation | PE | 38 ± 2 | 0 | 43 | 14 |

After confirming sample quality, 147 samples from 57 mothers (26 NT, 31 PE) were included in the final analysis. Specifically, 118 samples from 42 participants (18 NT, 24 PE) were sequenced (RNAseq) in discovery and 29 samples from 15 participants (8 NT, 7 PE) were processed using reverse-transcription quantitative polymerase chain reaction (RT-qPCR) in validation.

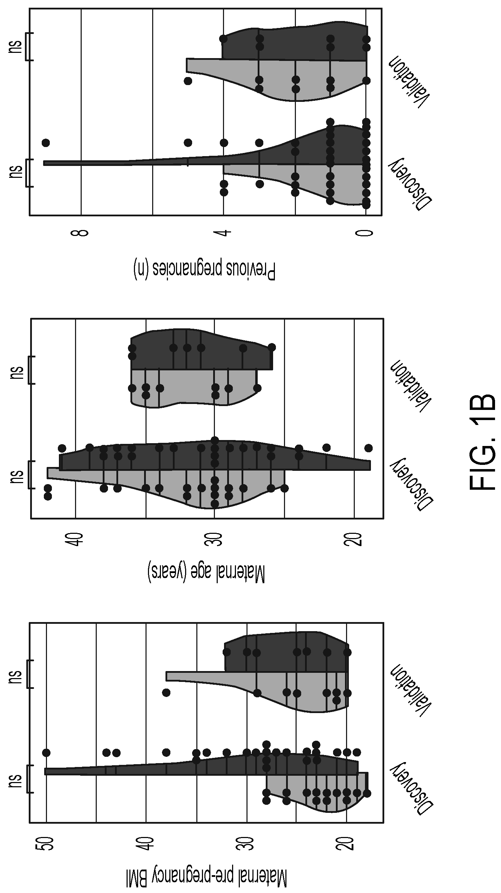

In the discovery cohort, we analyzed cfRNA from samples from four timepoints (early, mid-, and late gestation, and post-partum) whereas in validation, we focused on samples collected at early and mid-gestation because of the aforementioned clinical need for high quality predictive tests for PE early in gestation (Fig 1A). Across gestational time points in both discovery and validation cohorts, we found no significant difference in sampling time between PE and NT groups (p ≥ 0.7, 0.06). Known risk factors for PE, such as pre-pregnancy maternal body mass index (BMI), maternal age, and the number of prior pregnancies, followed expected trends, but were not significantly different between PE and NT groups (p ≥ 0.19, 0.52) (Fig 1B).

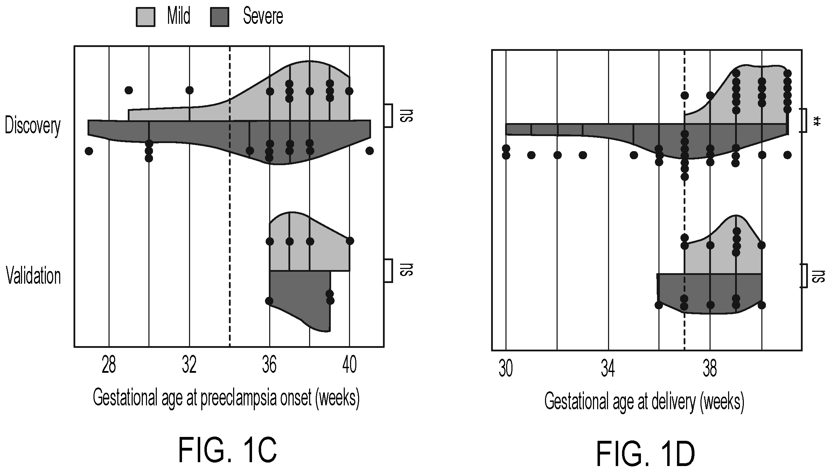

In mothers who later developed PE, a small fraction, 4 (17%) and 1 (14%) had a history of PE in the discovery and validation cohorts, respectively. We also observed no significant difference (p = 0.14, 1.0) in gestational age at onset between those who experienced mild (n = 11, 4) as compared with severe (n = 13, 3) symptoms (Fig 1C). Furthermore, 10 mothers who developed PE also delivered preterm (n = 9, 1) as compared with no mothers in the NT group as reflected by significantly different gestational ages at delivery (p = 10⁻⁵, 0.25, one-sided test) (Fig 1D), which was consistent with our NT group inclusion criteria and epidemiological evidence that PE increases the risk of spontaneous or indicated preterm delivery ^{5, 27} (values reported as discovery, validation, Mann-Whitney rank test).

### cfRNA changes across gestation

To identify gene changes associated with PE across pregnancy, we performed differential expression profiling and excluded post-partum samples, which instead served as a hold-out set, since they presented a clear break in gestational time and a marked change in physiology, specifically the absence of contributions from the fetus and placenta. This analysis identified 556 differentially expressed genes (DEGs) that differed across gestation between mothers who later developed PE and those who did not experience complications (adjusted p ≤ 0.05).

To further understand when these changes occur during gestation, we estimated the log fold-change (logFC) for each gene by early, mid-, and late gestation as well as at post-partum. e observed that these gene changes occurred most strikingly in early and mid-gestation as indicated by a clear bimodal distribution with two peaks centered around logFC of ±0.75 and broadly showed no difference at post-partum (mean logFC = 0.02) (Fig 2A). We then quantified he relative dispersion around the estimated logFC for each gene using an approximation for the coefficient of variation (CV) or the ratio between an error bound, ∂, and the estimated logFC (Fig 2B). After bootstrapping ∂, defined here as the error bound associated with the lower (or upper in the case of negative logFC values) 95% confidence interval (CI), we found that gene changes were most stable during early and mid-gestation where over 40% of genes had a CV < 1 as compared to 27% at late gestation and 21% at post-partum.

We then asked whether a subset of gene changes approximately proportional in number to total sample number (n = 27, 28, 31, 30) was sufficient to segregate PE (n = 13, 14, 19, 16) and NT (n = 14, 14, 12, 14) samples across gestation. We performed hierarchical clustering on a subset of all DEGs for each sampled time group based on two criteria designed to identify gene changes that were stable (CV < 0.5) and striking (|logFC| ≥ 0.75). In total, 51, 38, 17, and 14 genes met these criteria in early, mid-, late gestation, and post-partum, respectively, of which 11 enes met the specific cutoff criteria at 2 or more time points. This method separated PE and NT samples across gestation and at post-partum with good specificity (93 [71-99], 56 [32-80], 75 [47-92], and 86% [62-97%]) and sensitivity (85 [59-97], 79 [53-94], 74 [52-89], and 81% [57-4%]) (Fig 2C, values reported for early, mid-, and late gestation and post-partum, [95% CI]).

Noting that 11 genes were shared across time point specific DEG subsets after applying hard cutoffs, we then evaluated whether changes associated with each subset persisted across time points but at reduced levels. Further analysis indicated that genes identified at one time point (i.e., early gestation) can moderately separate PE and NT samples from other time points (i.e., mid- and late gestation and post-partum), and that typically such separation is most pronounced at a time point closest to that in which the gene changes were identified (Fig 6). Finally, we validated 4 DEG changes in early and mid-gestation using RT-qPCR and validation cohort samples. For all but 1 gene at 1 time point (PLAC8, mid-gestation), which had a CV close to 1 indicating reduced confidence in the estimate, we found that the log₂FC estimated using this method were consistent with those estimated in discovery using RNAseq, an orthogonal method (Fig 7).

Next, we sought to understand these changes' longitudinal dynamics over gestation using k-means clustering. We observed that the 556 identified DEGs could be well categorized into 3 distinct trends (Fig 2D, Fig. 8). Resembling a valley or V-shape, the first trend (Group 1) described the longitudinal behavior of 66 genes (12%), for which measured levels were significantly reduced in PE samples (-2x to -4x) across gestation with a minimum in mid-gestation. Peaking in early gestation (2x), the second trend (Group 2) described the behavior of 212 genes (38%) that had significantly elevated levels in PE samples across early and mid-gestation and to a lesser extent in late gestation (1.4x). Finally, the third trend (Group 3) described the behavior of 278 (50%) genes with consistently lower levels (-1.4x) across gestation. In all cases, gene changes were far less evident post-partum and trended toward no difference between PE and NT, which may reflect a placental contribution to DEG levels. Separately, we confirmed that these patterns were not spurious (i.e., an artifact of the k-means clustering algorithm) by permuting the data thereby scrambling any time-related structure while preserving its overall distribution. Following permutation, we observed no longitudinal patterns, which were instead replaced 3 nearly flat, uninformative trends (Fig 9).

We also asked what pathways these genes participated in. Grouping genes by longitudinal behavior, GO analysis revealed clear associations with PE pathogenesis and progression (Fig 2E). Broadly, genes with decreased levels across gestation in groups 1 and 3 corresponded to the immune response, cellular apoptosis (i.e., TNFR1-mediated ceramide production), and cellular metabolism and catabolism (i.e., heterocycle metabolic process, regulation of catabolic process). In contrast, genes in group 2 that had increased levels across gestation were involved in endothelial function (i.e., platelet activation, signaling, and aggregation), cellular activation (i.e., signaling by Rho GTPases), cellular invasion (i.e., lamellipodium organization), and wound healing (i.e., hemostasis).

### Development of a machine learning classifier to predict future PE onset before 17 weeks of gestation

Having established that we can observe gene changes associated with the development of PE across gestation, we sought to build a robust classifier that can identify mothers at risk of PE at or before 16 weeks of gestation. After training, the final model performed well on the discovery cohort with a near perfect AUC (94%, [87-100%]), perfect specificity (100%, [91-100%]) and positive predictive value (PPV) (100%, [89-100%]), and high sensitivity (88%, [70-96%]) and negative predictive value (NPV) (89%, [74-97%]) (Fig 3A, Table 2).

**Table 2. PE prediction performance metrics for samples collected early in gestation (Between 5-16 weeks). Control and PE case (as defined by the cited study) sample numbers are reported as the total sample number and in parentheses, the number of samples misclassified. Case column also include the sample number split between PE and gestational hypertension (GH) cases in square brackets. All other statistics including sensitivity specificity, positive predictive value (PPV), negative predictive value (NPV) and area under the curve (AUC) are reported as the estimated percentage followed by the 95% confidence interval in square brackets. In Del Vecchio^{control 1}, the control group is defined as samples from any pregnant mother who did not develop PE or GH including those with other underlying or pregnancy-related complications like chronic hypertension and gestational diabetes respectively. In Del Vecchio ^{control 2}, the control group is defined as samples strictly from NT pregnant mothers who did not experience complications. The associated performance metrics for each data split is reported.**

| | **Control (n)** | **Case (n)** | **Sensitivity (%)** | **Specificity (%)** | **PPV (%)** | **NPV (%)** | **AUC (%)** |
|---|---|---|---|---|---|---|---|
| Discovery | 25 (0) | 24 (3) [All PE] | 88 [70-96] | 100 [91-100] | 100 [89-100] | 89 [74-97] | 94 [87-100] |
| Del Vecchio ^{control1} (2020) | 17 (4) | 8 (0) [5 PE, 3 GH] | 100 [74-100] | 76 [53-91] | 67 [39-88] | 100 [83-100] | 88 [78-97] |
| Del Vecchio ^{control2} (2020) | 8 (1) | 8 (0) [5 PE, 3 GH] | 100 [74-100] | 88 [55-99] | 89 [59-99] | 100 [71-100] | 94 [79-100] |

We then tested this model on a completely independent cohort recently reported on by Del Vecchio and colleagues (Del Vecchio, et al. Epigenetics 1-20 "Cell-free DNA Methylation and Transcriptomic Signature Prediction of Pregnancies with Adverse Outcomes"; E-published Oct 13, 2020; doi:10.1080/15592294.2020.1816774), who isolated and sequenced cfRNA from 25 samples collected between 12-17 weeks of gestation from mothers who experienced diverse complications (n = 8 NT, 8 PE/gestational hypertension, 7 gestational diabetes, 2 chronic hypertension). Across this entire cohort, the final model once again performed well with high AUC (88%. [78-97%]), perfect sensitivity (100%, [74-100%]) and NPV (100%, [83-100%]), and good specificity (76%, [53-91%]) and PPV (67%, [39-88%]) (Fig 3A, Table 2).

Upon further inspection of the 4 erroneously classified samples (false positives), 1 and 2 were from mothers who had chronic hypertension or later developed gestational diabetes, respectively - known risk factors for PE ³⁵⁻⁴¹. To match our discovery cohort's control group, we then focused on comparing samples from PE or gestational hypertension to samples from NT participants alone and observed an AUC (94%, [79-100%]) identical to that measured in the discovery cohort, and equally high values for sensitivity (100%, [74-100%]), specificity (88%, [55-99%]), PPV (89%, [59-99%]), and NPV (100%, [71-100%]).

The final model also proved well-calibrated across both cohorts with a calibration curve slope of about 1 (1.27, 1.06) and intercept of close to 0 (-0.06, -0.05) (values reported as Discovery, Del Vecchio *et al*.). The probability of PE was also estimated as nearly 0% for most NT or otherwise complicated pregnancy samples and as almost 100% for PE or gestational hypertension samples. Far fewer samples were estimated to have a 50% probability of PE, the cutoff for classification (Fig 3B).

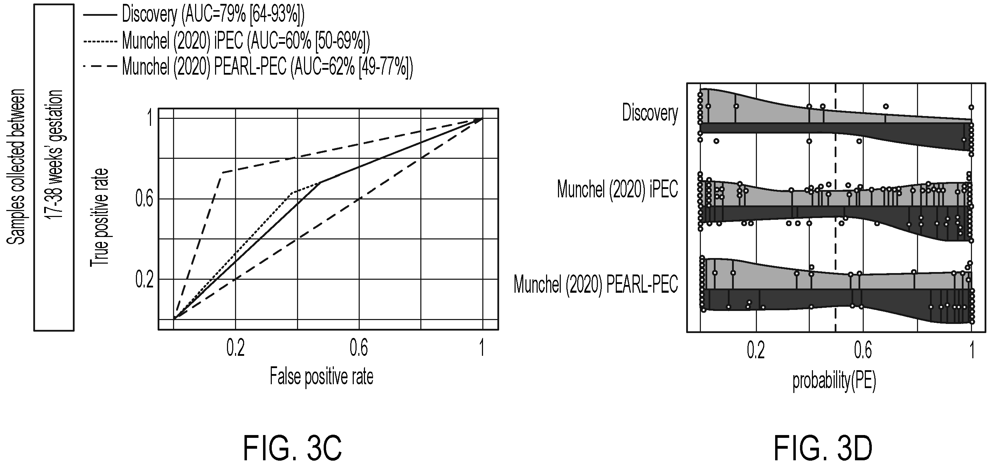

We then evaluated whether this model provided any predictive power for samples collected later in gestation or at diagnosis between 17-38 weeks' gestation. We measured model performance across three cohorts: the discovery cohort (samples collected ≥ 16 weeks of gestation but not post-partum) and two independently collected cohorts (Munchel, et al. "Circulating transcripts in maternal blood reflect a molecular signature of early-onset preeclampsia" Sci. Transl. Med. 2020 Jul 1;12(550):eaaz0131 doi: 10.1126/scitranslmed.aaz0131). For both independent cohorts (iPEC, PEARL-PEC), Munchel and colleagues isolated and sequenced cfRNA from 97 NT (n = 73, 24) and PE (n = 40, 24) samples collected at time of PE diagnosis or a matched timepoint for NT samples. As expected, we find a reduced, but still significant, signal to predict PE with moderate AUC (79% [64-93%], 60% [50-69%], 62% [49-77%]), sensitivity (73 [48-90], 68 [52-80], 62% [43-80%]), and specificity (85 [59-97], 52 [41-63], 62% [43-80%]) (Values reported as discovery, iPEC, PEARL-PEC) (Table 3, Fig 3C). At these later gestational timepoints shortly prior to or at diagnosis, the model remains moderately calibrated in discovery and poorly calibrated in testing with a calibration curve slope of less than 1 (0.7, 0.07, 0.12) and intercept of close to 0 (0.06, 0.28, 0.43) (values reported as discovery, iPEC, PEARL-PEC). Nonetheless, the model still discriminated between PE and NT pregnancies with many samples still receiving a probability estimate at either extreme (0 or 100%) but an increased sample number with poor scores around 50% (Fig 3D) (All reported as value, [95% CI]).

**Table 3. PE prediction performance metrics for samples collected late in gestation or at PE diagnosis (between 17-38 weeks). Control and PE sample numbers are reported as the total sample number and in parentheses, the number of samples misclassified. All other statistics including sensitivity specificity, positive predictive value (PPV), negative predictive value (NPV) and area under the curve (AUC) are reported as the estimated percentage followed by the 95% confidence interval in square brackets. The associated performance metrics for each data split is reported.**

| | **NT (n)** | **PE (n)** | **Sensitivity (%)** | **Specificity (%)** | **PPV (%)** | **NPV (%)** | **AUC (%)** |
|---|---|---|---|---|---|---|---|
| Discovery | 13 (2) | 15 (4) | 73 [48-90] | 85 [59-97] | 85 [59-97] | 73 [48-90] | 79 [64-93] |
| Munchel (2020) iPEC | 73 (35) | 40 (13) | 68 [52-80] | 52 [41-63] | 44 [32-56] | 75 [61-85] | 60 [50-69] |
| Munchel (2020) PEARL-PEC | 24 (9) | 24 (9) | 62 [43-80] | 62 [43-80] | 62 [43-80] | 62 [43-80] | 62 [49-77] |

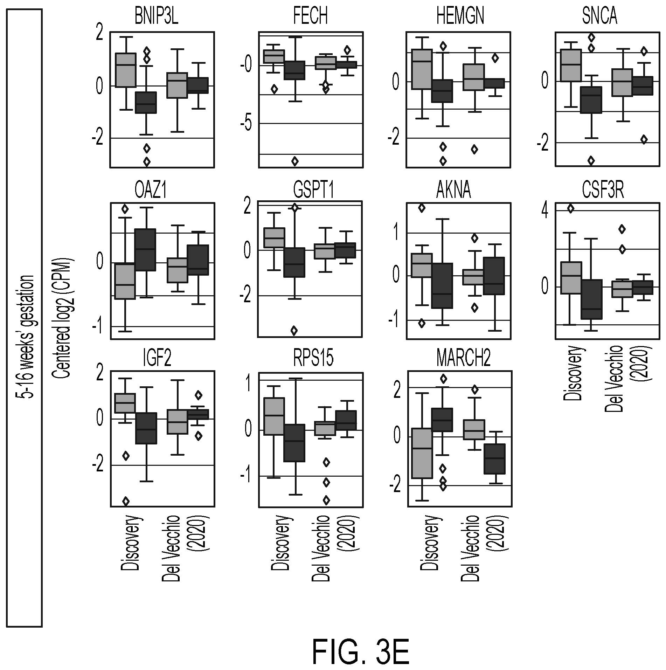

We then inspected the 11 genes (Table 4) used by the model to yield probability estimates. We found that many of the trends (i.e., decreased or increased gene levels in PE) observed in the discovery cohort are upheld in Del Vecchio and colleagues' cohort, although not to the level of statistical significance after multiple hypothesis correction (adjusted p ≥ 0.40, one-sided Mann-Whitney rank test) (Fig 3E). We also found that some models trained using a subset of the 11 initial genes can predict future PE onset with varying performance. Notably, performance improved across all metrics (sensitivity, specificity, PPV, NPV, AUC) as we increased the number of genes included for model training with some subsets of sizes 4-10 achieving nearly equivalent performance to the full model, which further emphasizes the importance of the gene signature in aggregate as opposed to individually (Table 5, Fig. 10A-E).

**Table 4. PE prediction relies on the cfRNA levels of 11 genes. For every gene, symbol, ENSEMBL ID, full name, odds ratio based on the logistic regression coefficient, and a subset of GO biological processes and molecular functions are reported from left to right.**

| **Gene** | **ENSEMBL** | **Name** | **Odds ratio** | **Biological process [GO]** | **Molecular function [GO]** |
|---|---|---|---|---|---|
| GSPT1 | ENSG00000103342 | G1 to S phase transition 1 | 1.69 | regulation of translational termination, protein methylation, G1/S transition of mitotic cell cycle | GTPase activity, RNA binding, GTP binding, translation release factor activity, protein binding |
| BNIP3L | ENSG00000104765 | BCL2 interacting protein 3 like | 0 | regulation of protein targeting to mitochondrion, defense response to virus, regulation of apoptotic process | lamin binding, protein homodimerization activity, identical protein binding, protein binding |
| MARCH2 | ENSG00000099785 | membrane associated ring-CH-type finger 2 | 0.02 | protein ubiquitination, endocytosis | protein binding, ubiquitin protein ligase activity |
| IGF2 | ENSG00000167244 | insulin like growth factor 2 | 0.12 | in utero embryonic development, platelet degranulation, regulation of gene expression by genetic imprinting | growth factor activity, integrin binding, insulin receptor binding, protein serine/threonine kinase activator activity |
| HEMGN | ENSG00000136929 | hemogen | 15.83 | multicellular organism development, regulation of osteoblast | protein binding |
| | | | | differentiation, cell differentiation | |
| OAZ1 | ENSG00000104904 | ornithine decarboxylase antizyme 1 | 51.6 | positive regulation of intracellular protein transport, negative regulation of polyamine transmembrane transport | ornithine decarboxylase inhibitor activity, protein binding |
| CSF3R | ENSG00000119535 | colony stimulating factor 3 receptor | 0.19 | regulation of myeloid cell differentiation, cytokine-mediated signaling pathway, cell adhesion, defense response | signaling receptor activity, cytokine receptor activity |
| RPS15 | ENSG00000115268 | ribosomal protein S15 | 0.76 | osteoblast differentiation, translation, rRNA processing | RNA binding, structural constituent of ribosome, protein binding |
| AKNA | ENSG00000106948 | AT-hook transcription factor | 0.08 | neuroblast division in subventricular zone, regulation of inflammatory response, epithelial to mesenchymal transition | DNA binding, protein binding |
| SNCA | ENSG00000145335 | synuclein alpha | 2.09 | calcium ion homeostasis, synaptic vesicle exocytosis, receptor internalization, regulation of norepinephrine uptake | actin binding, tau protein binding, SNARE binding, phosphoprotein binding, protein kinase inhibitor activity |
| FECH | ENSG00000066926 | ferrochelatase | 1.98 | response to drug, heme biosynthetic process | ferrochelatase activity, 2 iron |

**Table 5. Logistic regression models trained on some subsets of 4-10 genes of the initial 11 genes can predict future PE onset with nearly equivalent performance metrics. The associated performance metrics for each data split and some high-performing gene subsets is reported including sensitivity (Sens), specificity (Spec), positive predictive value (PPV), negative predictive value (NPV) and area under the curve (AUC), which are reported as the estimated percentage. In Del Vecchio^{control 1}, the control group is defined as samples from any pregnant mother who did not develop PE or gestational hypertension including those with other underlying or pregnancy-related complications like chronic hypertension and gestational diabetes respectively. In Del Vecchio^{control 2}, the control group is defined as samples strictly from NT pregnant mothers who did not experience complications.**

| *Subset* | *Size* | **Del Vecchio et al (control 1)** | | | | | **Del Vecchio et al (control 2)** | | | | |
|---|---|---|---|---|---|---|---|---|---|---|---|
| | | *PPV* | *NPV* | *Sens* | *Spec* | *AUC* | *PPV* | *NPV* | *Sens* | *Spec* | *AUC* |
| MARCH2, OAZ1, CSF3R, SNCA | 4 | 64 | 93 | 88 | 76 | 82 | 88 | 88 | 88 | 88 | 88 |
| GSPT1, MARCH2, OAZ1, CSF3R, SNCA | 5 | 70 | 93 | 88 | 82 | 85 | 88 | 88 | 88 | 88 | 88 |
| MARCH2, OAZ1, CSF3R, RPS15, AKNA, SNCA | 6 | 70 | 93 | 88 | 82 | 85 | 88 | 88 | 88 | 88 | 88 |
| MARCH2, HEMGN, OAZ1, CSF3R, RPS15, SNCA, FECH | 7 | 70 | 93 | 88 | 82 | 85 | 88 | 88 | 88 | 88 | 88 |
| GSPT1, MARCH2, HEMGN, OAZ1, CSF3R, RPS15, SNCA, FECH | 8 | 70 | 93 | 88 | 82 | 85 | 88 | 88 | 88 | 88 | 88 |
| BNIP3L, MARCH2, IGF2, HEMGN, OAZ1, CSF3R, RPS15, AKNA, FECH | 9 | 67 | 100 | 100 | 76 | 88 | 89 | 100 | 100 | 88 | 94 |
| BNIP3L, MARCH2, IGF2, HEMGN, OAZ1, CSF3R, RPS15, AKNA, SNCA, FECH | 10 | 67 | 100 | 100 | 76 | 88 | 89 | 100 | 100 | 88 | 94 |

### Summary

The studies in Example 1 described above demonstrated that cfRNA measurements taken during pregnancy could clearly distinguish between PE and NT pregnancies with the most pronounced differences occurring early on, and broadly showed no difference at post-partum, consistent with known PE biology, namely that the placenta drives the disease process ^{34,36}. These findings were supported by orthogonal analyses including clustering and machine learning. Specifically, we found that the most pronounced and stable changes as defined by high |logFC| and low CV occurred more frequently in early or mid-gestation as compared to later on and as broadly supported by longitudinal dynamics analysis. Notably, small subsets of the DEGs identified here (n = 11-51) can also be used to separate PE and NT pregnancies with the best sensitivity and specificity observed early in pregnancy as confirmed by both hierarchical clustering and machine learning.

The genes identified and their longitudinal dynamics are also consistent with what is known about PE, namely that it likely develops very early in pregnancy with the resultant maternal syndrome manifesting later ³⁴. Following GO analysis, we found that the DEGs identified here both agree with prior evidence regarding PE's pathology and clinical presentation and provide new hypotheses to explore. Specifically, GO terms that relate to cellular invasion and organ perfusion (i.e., lamellipodium organization and hemostasis, Group 2), cellular apoptosis, catabolism and response to stress (i.e., TNFR1-mediated ceramide production, regulation of catabolic process, Group 1,3), and endothelial dysfunction (i.e., platelet activation, signaling, and aggregation, Group 2) closely reflect what is known about the stages of the pathogenesis of PE: abnormal placentation as characterized by insufficient placental invasion of the uterine arteries and subsequent reduced placental perfusion, inadequate nutrient exchange, and systemic endothelial dysfunction ³⁴.

Early in pregnancy during normal placentation, cytotrophoblasts invade the spiral arteries, which then undergo a transformation to accommodate larger blood volumes for increased fetal and placental nutrient requirements later in pregnancy, and peripheral villi regress via a mechanism involving oxidative stress. PE is thought to be a uniquely human disease because of the large nutritional exchange requirement imposed on the mother (3x higher than that for other mammals in the third trimester) ³⁴. Later on, PE presents in the mother as systemic endothelial dysfunction, where endothelial cells are closely implicated in platelet aggregation.

Other terms such as innate immune system process, type I interferon production, and positive regulation of T cell differentiation (Group 3) highlight the involvement of the innate and adaptive immune system, which is both consistent with previous work on immune system dynamics during early gestation in PE ⁵⁶ and with known pregnancy biology about sustaining a successful pregnancy ⁵⁷ and establishing maternal tolerance, the lack of which has been previously associated with PE ⁵⁸.

PE is also a broad and complex syndrome. Because the complication can present clinically across more than 20 weeks with a diversity of symptoms, significant effort has been made to subclassify the disease based on timing of onset. Used as a proxy for hypothesized pathogenesis, the timing of PE onset subdivides the disease into 'placental' early-onset PE (occurring before 34 weeks of gestation) and 'maternal' late-onset PE (occurring on or after 34 weeks) ^{34,37,59}. However, early- and late-onset PE may represent a spectrum of disease severity that corresponds with timing of onset and may lead to additional pregnancy complications, such as intrauterine growth restriction (IUGR) ^{34,36,38,39}. Indeed, some evidence like transcriptional profiling of whole blood drawn at diagnosis suggests a common gene signature for both subtypes⁴⁰. Our findings further corroborate that timing of onset may lie on a spectrum, and a common signature can identify both early- and late-onset PE of varied severity very early in gestation including as early as 5 weeks.

Finally, prediction of PE early in pregnancy has remained an unrealized key objective for the field of obstetrics with important health consequences. The results presented here provide evidence that it is possible both to predict PE risk before 16 weeks of gestation with high specificity and sensitivity, and to differentiate PE from other pregnancy-related complications such as gestational diabetes or underlying conditions such as chronic hypertension. Of the 7 gestational diabetes samples included the independent cohort of Del Vecchio *et al.*, only 2 were misclassified as PE. We note that identifying other adverse outcomes that increase one's risk of PE may be an acceptable error mode whereas classifying an uncomplicated, NT pregnancy as PE is not and would result in unnecessary, added stress. In our analysis, we found that only 1 NT pregnancy was misclassified across 25 discovery and 8 test samples. Given the small sample size and limited ethnic and racial diversity in these cohorts, the performance metrics, although consistent and striking, will also benefit from validation in a larger and more diverse follow-up study.

These predictions also relied on only 11 gene measurements - none of which overlapped with previously reported genes that are not altered until much closer to diagnosis (sFlt1 and PlGF) ²⁵ or the cfRNA results in recent proof-of-concept work ^{32,33}. We also note that 11 genes can be easily measured using RT-qPCR, highlighting its clinical relevance and presenting an inexpensive alternative to RNAseq in future validation work. Given that both expense and efficacy are important factors in addressing barriers to care, an inexpensive test with striking efficacy may dramatically improve care and prevent missed or delayed diagnoses, which at the moment may account for as many as 3 in 5 maternal deaths related to pregnancy in the US.

Tests such as the evaluation described herein that identify who is at risk of PE early in pregnancy have long been acknowledged as an unrealized key objective in PE care ⁹. They can also importantly be coupled with the administration of low-dose aspirin, which if started early has been shown to reduce risk of developing PE ^{10,28}. Although these results await further confirmation in a blinded, larger validation study, the findings reported here demonstrates that an 11 gene cfRNA-based classifier can predict risk of PE with consistent, clinically relevant high sensitivity and specificity (88-100% for both metrics) across 2 independent cohorts that were collected and processed by completely separate teams.

Taken together, we have shown that cfRNA measurement can form the basis for a robust liquid biopsy test that predicts PE very early in gestation, to date an unrealized key objective for obstetric care, and can be used to help characterize the pathogenesis of PE in real time.

### Example 2-Identification of signature panel-- expanded population, panels for assessing risk of severe PE, and panels for monitoring tissue/cell-type health

### Results

### Clinical study design

To identify changes associated with PE well before traditional diagnosis, we designed a prospective study and recruited pregnant mothers at their first clinical visit to Stanford's Lucile Packard Children's Hospital, between 5-12 weeks of gestation, of which 131 were included in this study (94 NT, 37 with PE). For each participant, we analyzed cfRNA for samples collected before or at 12 weeks, between 13-20 weeks, and at or after 23 weeks of gestation, and post-partum (0-4 weeks after delivery). We then split this larger group into Discovery (n = 88, [60 NT, 28 with PE]) and Validation 1 (n = 43, [34 NT, 9 with PE]) cohorts. We also obtained samples from an independent cohort collected at several separate institutions (Validation 2), which consisted of 89 samples collected prior to 16 weeks of gestation from 87 mothers (61 NT, 26 with PE) (Fig 11A).

For all cohorts, we included individuals of diverse racial and ethnic backgrounds in approximately matched proportions across NT and PE groups (Table 14). We recorded maternal pre-pregnancy and pregnancy characteristics, and defined a pregnancy as NT if it was both uncomplicated and went to full-term (≥ 37 weeks) or as PE with or without severe features based on current guidelines (see Methods). For mothers who developed PE, all antenatal blood samples were collected prior to diagnosis.

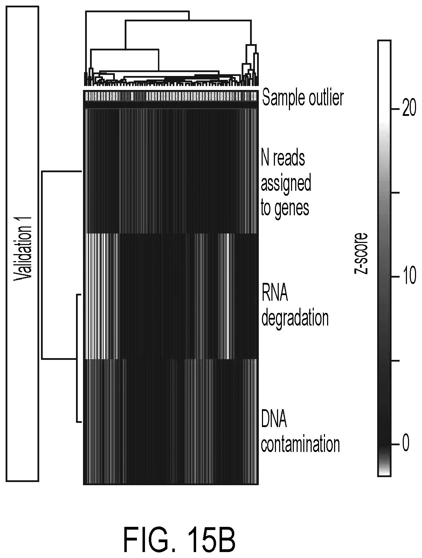

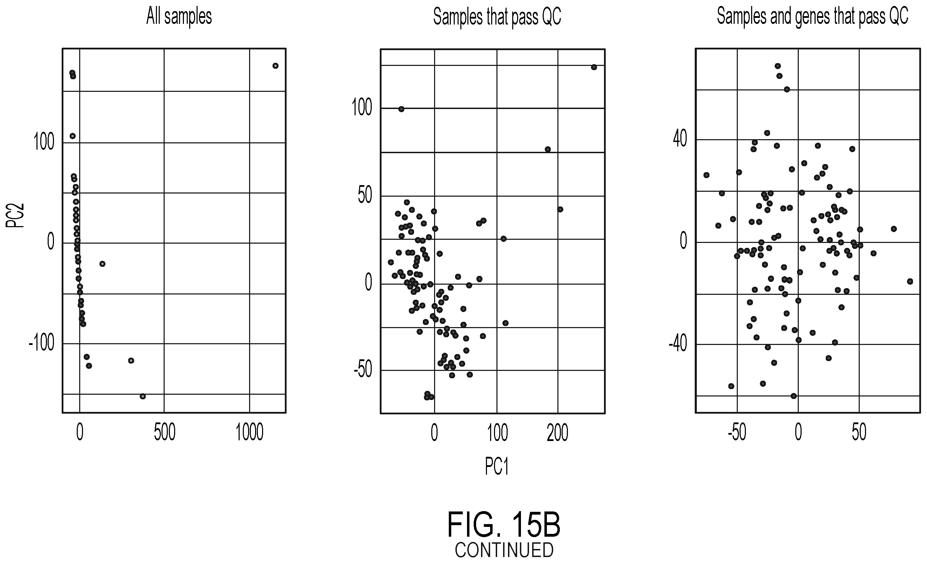

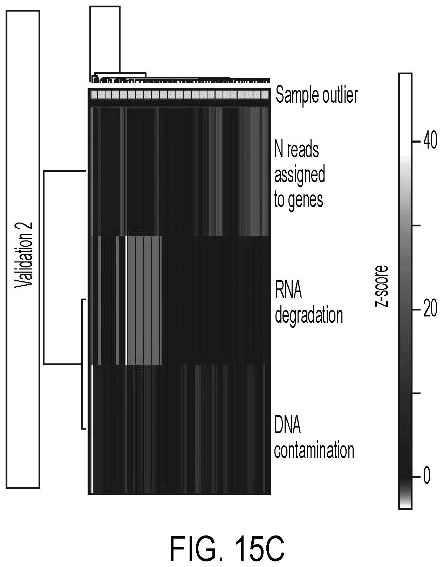

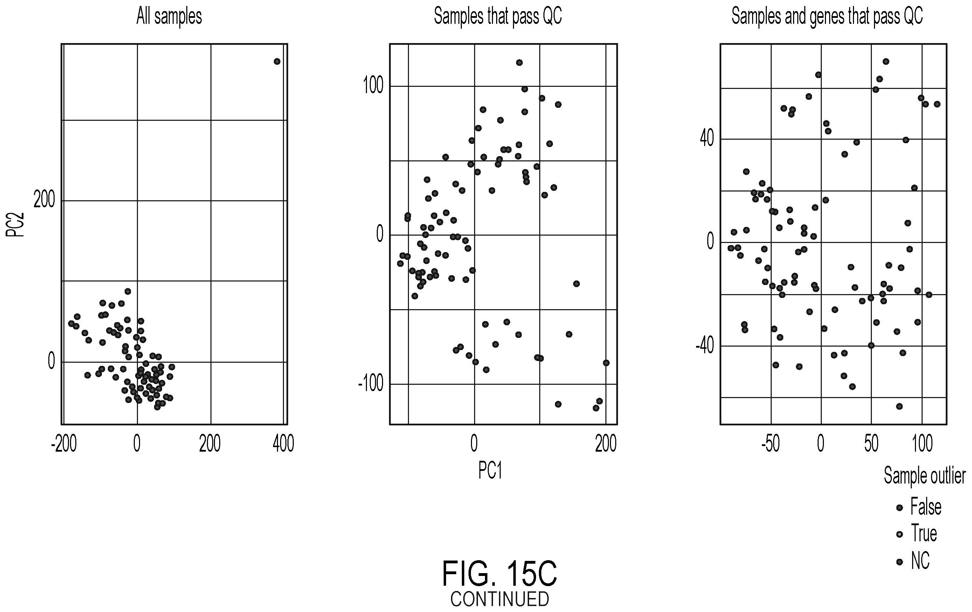

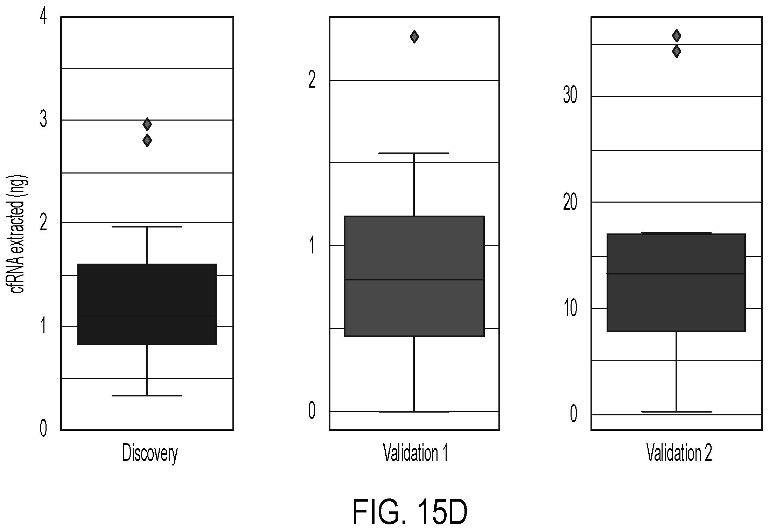

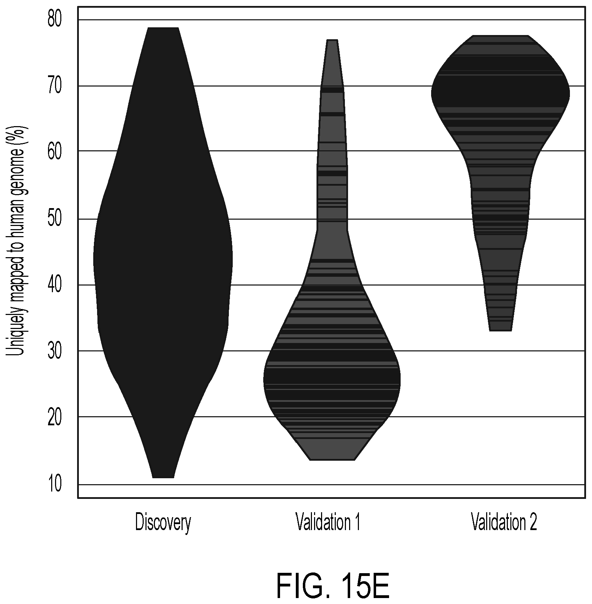

Our final analysis included a subset of those samples which passed pre-defined quality metrics (Supp. note 1, Methods, Fig 15). After confirming sample quality, 404 samples from 199 mothers (142 NT, 57 with PE) were included in the final analysis (Table 14). Specifically, 209, 106, and 89 samples from 73, 39, and 87 participants (49, 32, 61 NT; 24, 7, 26 with PE) were included in Discovery, Validation 1, and Validation 2, respectively (RNAseq).

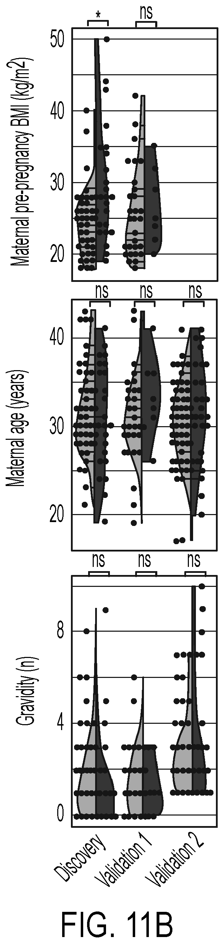

Across gestational time points in all cohorts, we found no significant difference in sampling time between PE and NT groups (p ≥ 0.26, 0.11, 0.46). Known risk factors for PE, such as pre-pregnancy maternal body mass index (BMI), maternal age, and gravidity followed expected trends. BMI specifically was significantly different between PE and NT groups in Discovery cohort alone (p = 0.02, 0.45, NA) while maternal age and gravidity were not (p ≥ 0.29, 0.16, 0.2) (Fig 11B, Table 14). History of PTB and mode of delivery were significantly different between NT and PE groups only for Validation 2. Other demographic factors like race, ethnicity, and nulliparity differed across cohorts but not between case groups within each cohort (adjusted p ≤ 0.05, chi-squared test for categorical or ANOVA for continuous variables with Bonferroni correction, Table 14).

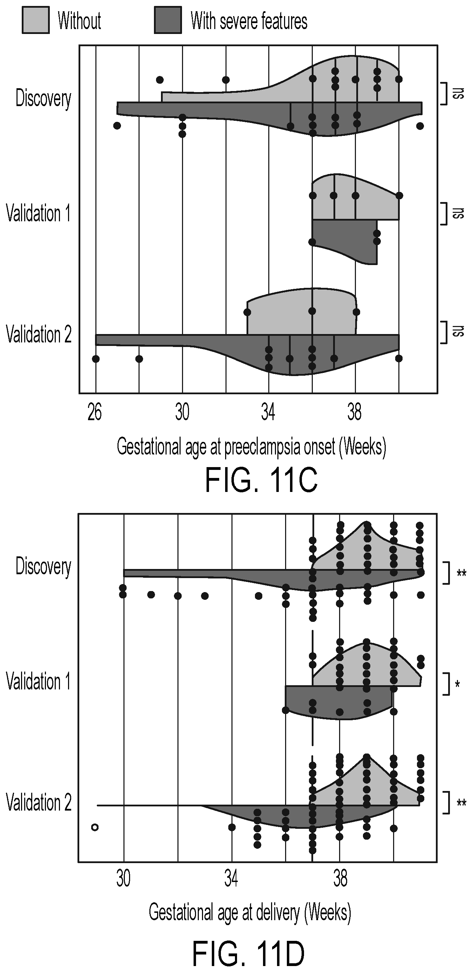

In mothers who later developed PE, we observed no significant difference (p = 0.14, 1.0, 0.4) in gestational age at onset between those who did not experience severe symptoms (n = 11, 4, 3*) as compared with those with did experience severe (n = 13, 3, 13*) symptoms (Fig 11C). Furthermore, 21 mothers who developed PE also delivered preterm (n = 9, 1, 11) as compared with no mothers in the NT group as reflected by significantly different gestational ages at delivery (p = 10⁻⁷, 0.04, 10⁻⁹, one-sided test) (Fig 11D) and lower fetal weight at delivery (Table 14), which was consistent with epidemiological evidence that PE increases the risk of spontaneous or indicated preterm delivery ^{5,27} (values reported as Discovery, Validation 1, Validation 2, Mann-Whitney rank test unless otherwise specified, * denotes data were incomplete for specified cohort).

### Identification of cfRNA changes across gestation in mothers who developed PE

There were 544 differentially expressed genes (DEGs) that differed across gestation and post-partum between mothers who later developed PE with or without severe features as compared with NT mothers who did not experience complications (adjusted p ≤ 0.05, see Methods and Supplementary Note 2). Most DEGs (498) were annotated as protein-coding, and a small fraction (43, 8%) were other types including 11 mitochondrial t-RNAs, 6 long non-coding RNAs, 8 pseudogenes, and 1 small nucleolar RNA (snoRNA).

To further understand when these changes occur during gestation, we estimated the log fold-change (logFC) for each gene by each gestational time point as well as post-partum. We observed that these gene changes occurred most strikingly before 20 weeks of gestation as indicated by a clear bimodal distribution with two peaks centered around logFC of +0.8 and -0.6 (Fig 12A). We also found that gene changes were also most stable before 20 weeks of gestation where over 50% of genes had a coefficient of variation (CV) < 1 as compared to 31% at or after 23 weeks of gestation and 36% at post-partum (Fig 16A).

We then asked whether a subset of gene changes approximately proportional in number to total sample number (n = 49, 49, 57, 46 for each time point) was sufficient to segregate PE (n = 13, 16, 20, 17) and NT (n = 36, 33, 37, 29) samples across gestation. We found that 24-32 genes were sufficient to separated PE and NT samples across gestation and at post-partum with good specificity (86% [75-93%], 79% [66-88%], 97% [90-100%], and 90% [78-96%]) and sensitivity (85% [64-95%], 88% [69-96%], 65% [47-80%], and 71% [51-86%]) (Fig 2B, S2B, All values reported for ≤12 weeks, 13-20 weeks, ≥23 weeks of gestation and post-partum, [90% CI]) (See also Fig 17).

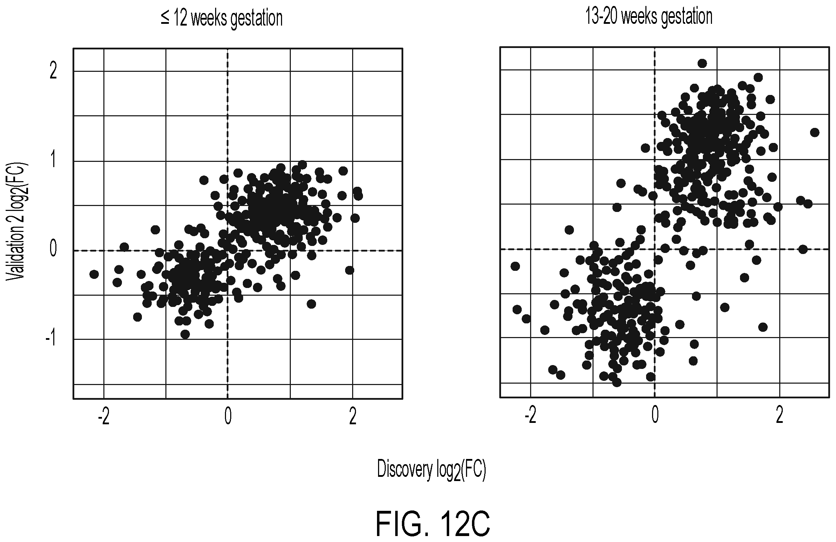

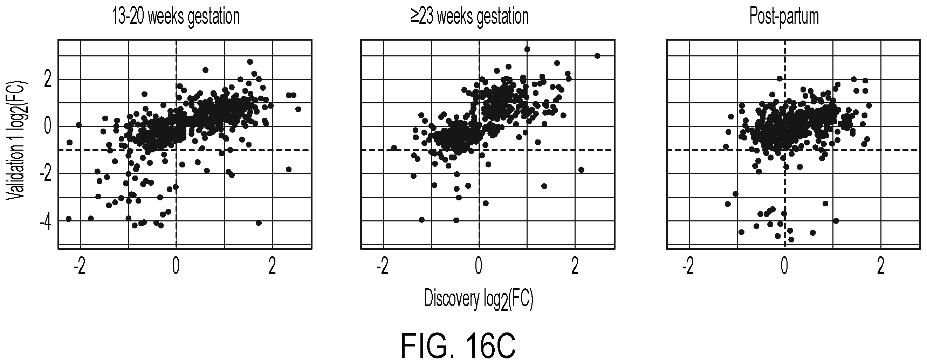

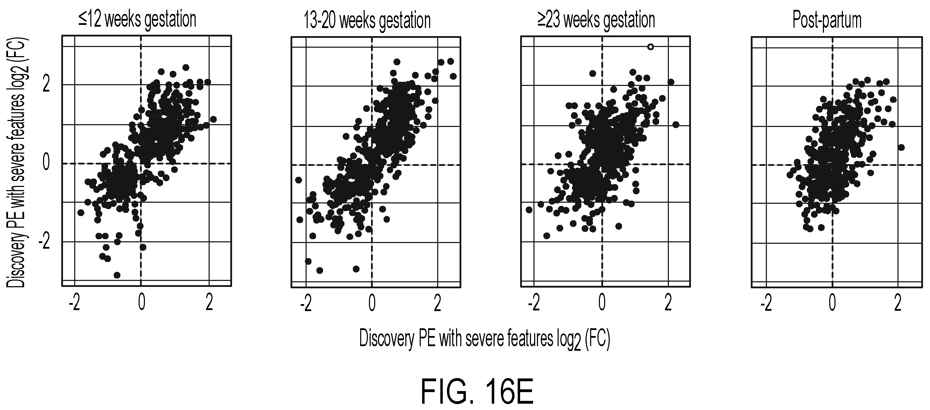

Nearly all 544 DEG changes showed excellent agreement in both Validation cohorts as compared to Discovery across gestation but not post-partum. Specifically, more than 82% and 92% of genes across gestation had the same logFC sign with a spearman correlation of at least 0.67 and 0.71 for Validation 1 and 2 respectively (p<10⁻¹⁵) as compared with 60% and 0.35 post-partum (Fig 12C, 16C). Finally, we asked whether symptom severity (without or with severe features) correlated with logFC magnitude for these 544 DEGs common to both PE subtypes. We found that on average, symptom severity did not influence logFC magnitude as reflected by a slope of nearly 1 across gestation and post-partum (Fig 16E).

### cfRNA changes reflect disease pathophysiology in mothers who developed PE

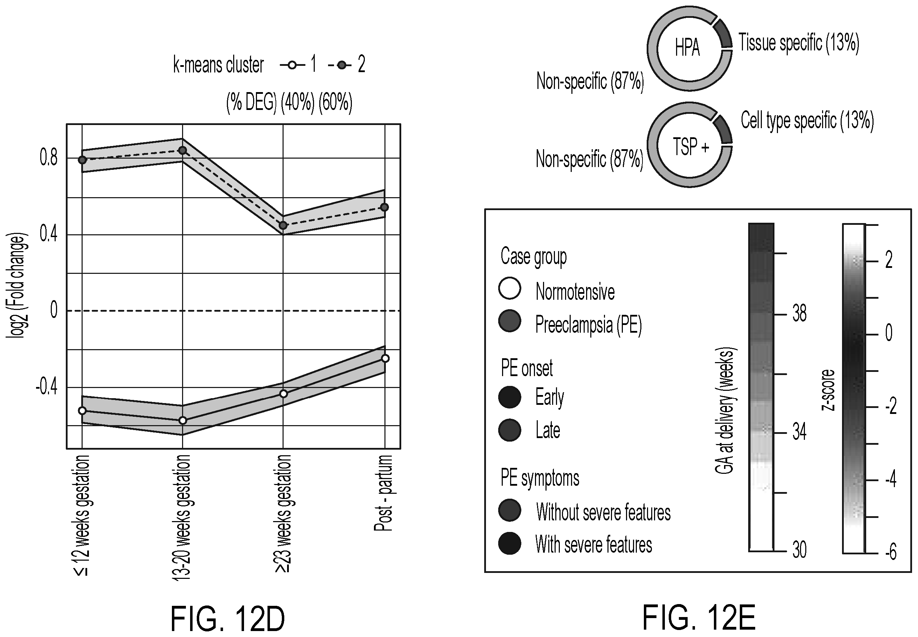

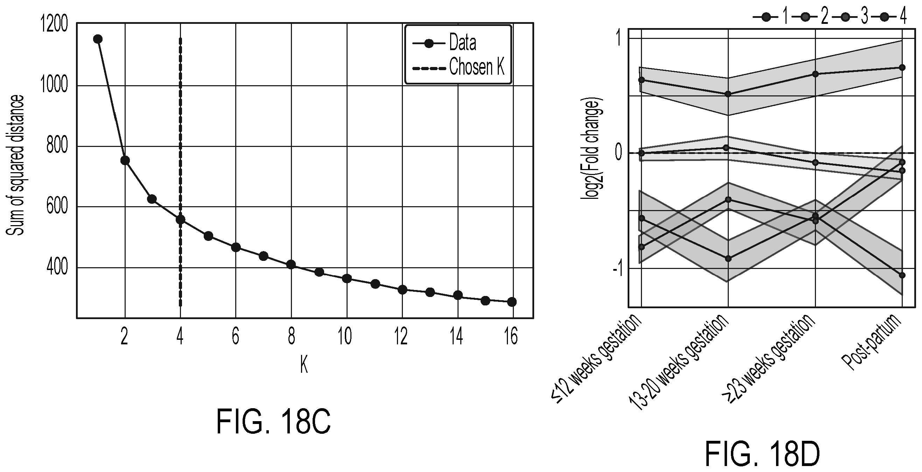

The 544 identified DEGs could be well categorized into two longitudinal trends (Fig 12D,18A,C). Resembling a valley or V-shape, the first trend (Group 1) described the longitudinal behavior of 216 genes (40%), for which measured levels were reduced in PE samples (-1.3x to -1.5x) across gestation with a minimum between 13-20 weeks. Peaking in early gestation before 20 weeks (1.75x), the second trend (Group 2) described the behavior of 328 genes (60%) that had significantly elevated levels in PE samples before 20 weeks and to a lesser extent after 23 weeks of gestation (1.3x). For Group 1 but not 2, gene changes were far less evident post-partum and trended toward no difference between PE and NT, which may reflect a placental contribution to DEG levels.

Approximately 13% of DEGs were tissue or cell-type specific (Fig 12E). Genes that were decreased in PE across gestation (Group 1) were broadly enriched for the immune system, whereas, those genes increased in PE across gestation (Group 2) were enriched for nervous, muscular, endothelial, and immune contributions as reflected by cell-type (adjusted p ≤ 0.05, hypergeometric test) and pathway enrichment (adjusted p ≤ 0.05, hypergeometric test) (Fig 16E, Table 16). Consistent with current understanding of the pathogenesis of PE, we identified a strong endothelial-linked signal underscored by contributions from capillary aerocytes (p = 0.03), an endothelial cell-type specific to the lungs ⁴⁵, platelets (p = 10⁻³³), and several platelet related pathways like platelet degranulation and platelet activation, signaling, and aggregation (p ≤ 10⁻⁸) among others.

Surprisingly, we also found significant, elevated nervous and muscular contributions for PE as emphasized by contributions from excitatory neurons (p = 0.02), oligodendrocytes (p = 0.005), and smooth muscle (p = 0.0003) and terms like muscle contraction and dilated cardiomyopathy (p ≤ 0.02). The immune system also contributes to both elevated (e.g., mesenchymal stem cells, total PBMCs) and decreased (granulocytes, T-cells) changes across gestation. Genes in both groups were enriched for signaling pathways (i.e., secretion by cell, integrin-mediated signaling pathway, regulation of I-kappaB kinase, NF-kappaB signaling). Group 2 was also enriched for cellular compartments such as the cell periphery, cell junctions, and extracellular space, consistent with reports that PE may be associated with signaling from the fetoplacental complex ⁴⁶. Finally, DEGs were broached enriched for genes previously implicated in PE ⁴⁷ (30 gene overlap, p = 0.006, hypergeometric test) (Table 16).

### A machine-learning classifier predicts risk of PE on or before 16 weeks of gestation

Since gene changes associated with PE pathogenesis across gestation are readily detected irrespective of symptom severity, we sought to build a classifier that could identify mothers at risk of PE at or before 16 weeks of gestation (Supplementary Note 2, Fig 16E). We trained a logistic regression model on the Discovery cohort (n = 61 NT, 24 PE samples). After training, the final model performed well with a near perfect AUROC (0.99 [0.99-0.99]), good specificity (85% [77-91%]), and perfect sensitivity (100% [92-100%]) (Fig 13A, Table 13). We then tested this model on Validation 1 (n = 35 NT, 8 PE) and two other independent cohorts, which were collected at separate institutions: Validation 2 (n = 61 NT, 28 PE samples) and Del Vecchio and colleagues ³³ cohort (n = 8 NT, 5 with PE, 7 with gestational diabetes, 2 with chronic hypertension). Across these cohorts, the final model once again performed well with consistent AUROC (0.71 [0.70-0.71], 0.72 [0.71-0.72], 0.74 [0.73-0.74]), sensitivity (75% [46-92%], 56% [42-72%], 60% [26-87%]) and specificity (56% [43-70%], 69% [59-78%], 100% [89-100%]) (All reported as Validation 1, Validation 2, Del Vecchio) (Fig 13A, Table 13).

Next, we inspected erroneously classified samples from the Validation 2 and Del Vecchio cohorts. For false negatives in Validation 2 and Del Vecchio, we find a shift to later gestational ages at collection (13.5 ± 2, 12.5 ± 2 weeks) as compared to PE samples that were correctly classified (12 ± 2, 12 ± 0 weeks; mean ± SD for Validation 2, Del Vecchio) (Fig 19A). This suggests that in practice, there may an optimal collection window to reduce false negatives. Indeed, if we only consider samples before 14 weeks of gestation, we observe a 9% and 15% increase in sensitivity with corresponding AUROC values of 0.73 and 0.90 for Validation 2 and Del Vecchio respectively. There were no false positives in the Del Vecchio cohort, suggesting that the model can distinguish between PE and other risks like chronic hypertension or gestational diabetes. The model also proved well-calibrated estimating a slightly elevated probability of PE for gestational diabetes (0.15 ± 0.08) and chronic hypertension (0.18 ± 0.13) - known PE risk factors ⁴⁸⁻⁵⁴ - as compared to the estimate for NT samples (0.09 ± 0.08) (mean ± SD, Fig S5B). These elevated probabilities for other risk factors impacted the test's AUROC (0.74 [0.73-0.74] as compared to 0.8 [0.79-0.8] for only PE vs NT samples, Table 1) (all reported as value, [90% CI]).

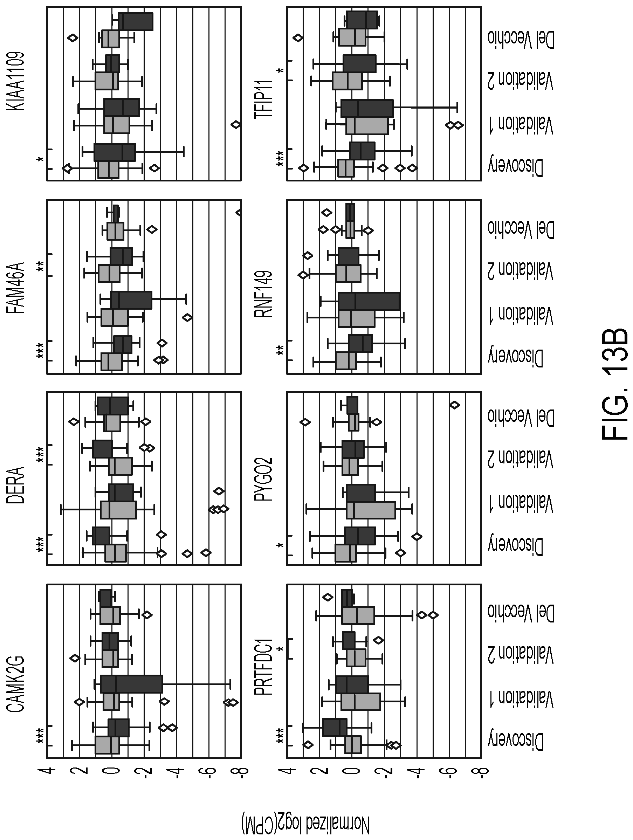

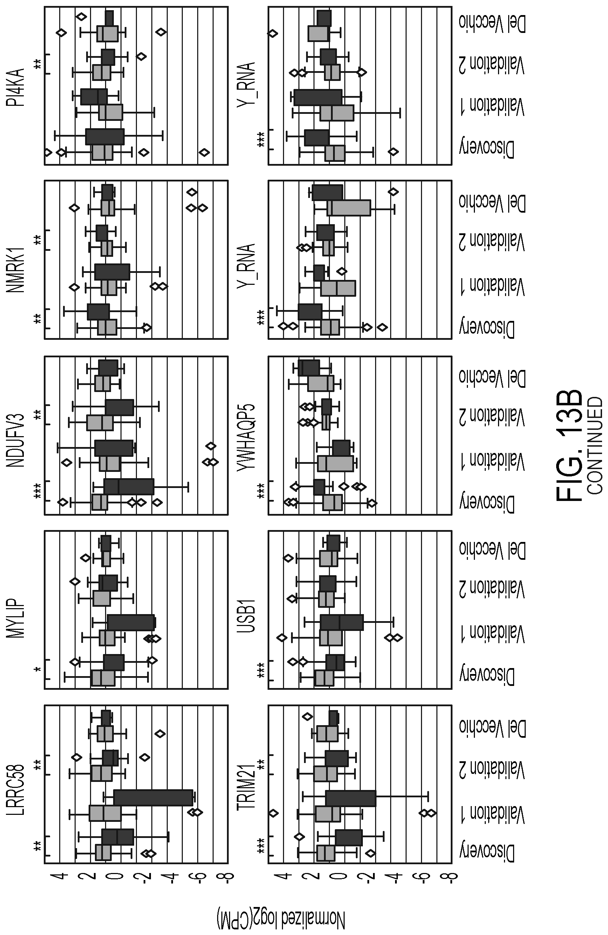

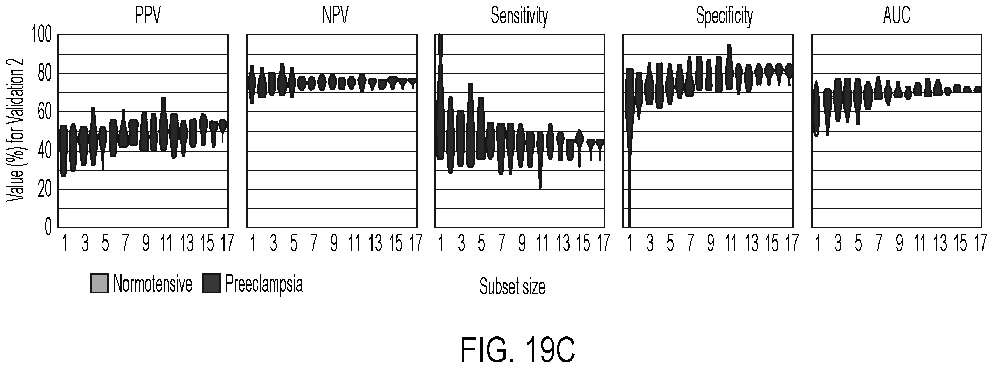

Finally, we inspected the 18 genes (Fig 13B, Table 17) used by the model to yield probability estimates. Eight genes were annotated in the Human Protein Atlas (HPA)⁵⁵ as enhanced or enriched in the placenta (FAM46A, MYLIP), neuromuscular (CAMK2G, NDUFV3, PI4KA, PRTFDC1) and immune systems (RNF149, TRIM21). Univariate analysis further confirmed that 9 of the gene trends (i.e., decreased or increased gene levels in PE) observed in the Discovery dataset are upheld in Validation 2 (adjusted p ≤ 0.05, one-sided Mann-Whitney rank test) (Fig 3B). We also found that the majority of models trained using a subset of the 18 initial genes can predict future PE onset with varying performance. Notably, performance improved across all metrics (sensitivity, specificity, and AUROC) as we increased the number of genes included for model training (Table 18, Fig 19C).

### The multifactorial nature of PE and maternal organ health is reflected in cfRNA

We next wondered whether cfRNA measurement reflects the multifactorial nature of PE. We identified 503 DEGs (adjusted p ≤ 0.05) that differed across gestation between mothers who later developed PE with as compared to without severe symptoms. Since there were no significant differences in symptom severity as related to timing of PE onset (Fig 11C), we believe that our observations contrasting PE with and without severe symptoms are not obscured by differences in PE-onset type. As before, most DEGs (484) were annotated as protein-coding, and a small fraction (18, 4%) were other types including 12 pseudogenes and 4 long non-coding RNAs.

We observed that DEGs could be well categorized into 4 longitudinal trends (Fig 14A, 18B,D). Two groups (Group 1, 3) described the temporal behavior of 217 genes (44%), for which measured levels were either consistently increased (Group 1) or reduced (Group 3) in PE with as compared to without severe symptoms (±1.8x) across gestation and trended towards no change post-partum. In contrast, Groups 2 and 4 (286 genes, 56%) changed signs in mid-gestation beginning as slightly elevated (Group 2, 1.2x) or decreased (Group 4, -1.2x) in severe PE and then moving to decreased (Group 2, -1.4x) or unchanged (Group 4, 1x) at ≥23 weeks of gestation.

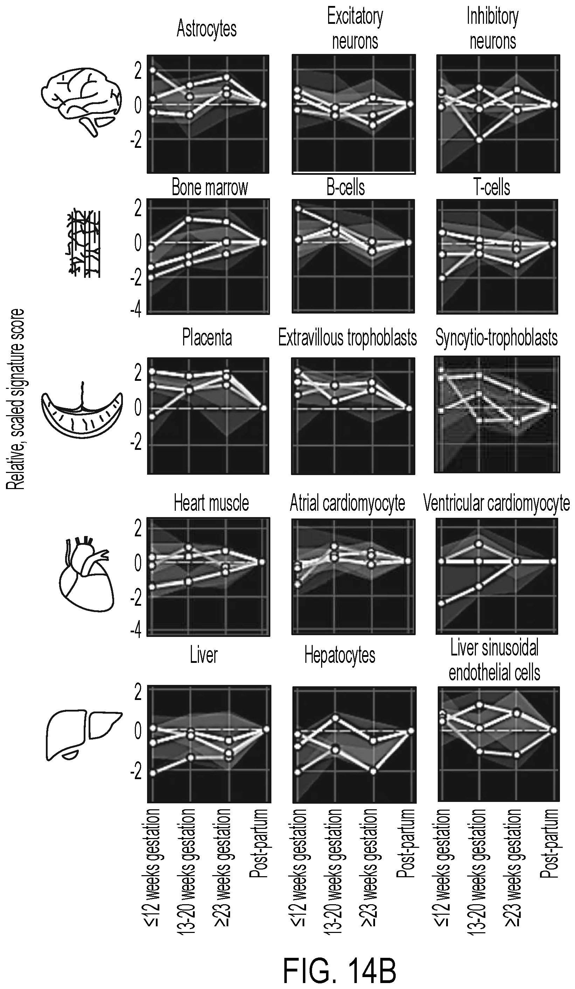

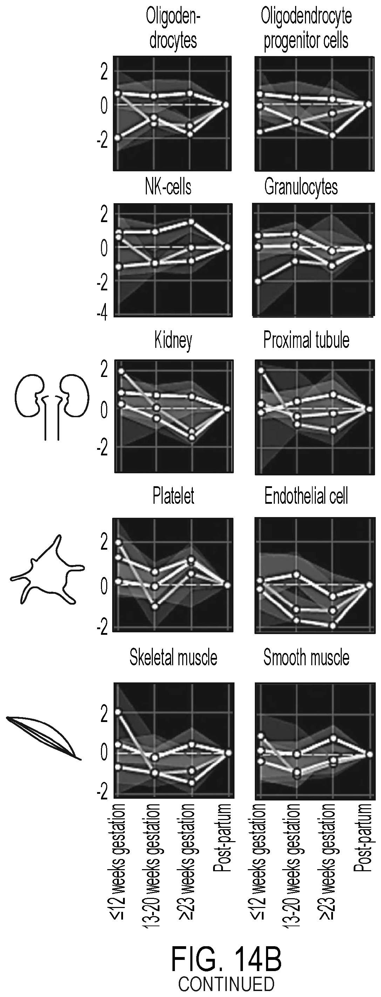

Analysis of the enriched cell types and tissues of origin for each of these groups revealed that elevated gene differences in severe PE were driven by contributions from endothelial cells and the adaptive immune system (bone marrow). In contrast, genes that changed signs over gestation were enriched for innate immune cell types (e.g., granulocytes and neutrophils for Group 2, thymus for Group 4) (Table 19). Quantifying total cfRNA signal confirmed an increased bone marrow signal for only severe PE across gestation and a decreased granulocyte signal for only PE without severe features at ≤12 weeks of gestation (Fig 14B). Gene ontology analysis further revealed pathways specific to genes that were only decreased for severe PE in early gestation (Group 4) like axon guidance, nervous development, and metabolism of RNA (adjusted p < 0.05).

We next investigated whether it might be possible to monitor organ health noninvasively focusing on eight organ systems (Fig 14B) relevant to PE presentation with consequences such as proteinuria, impaired liver function, renal insufficiency, and epilepsy. We found striking shifts in total contributions for all systems. We observed an increased astrocyte signal before 20 weeks of gestation and decreased oligodendrocytes and excitatory neurons at ≥23 weeks of gestation for all PE relative to NT (Fig 14B, 1^{st} row). Although placental contributions increased over pregnancy with a peak in late gestation as expected, placental tissue and syncytiotrophoblast contributions were reduced for PE pregnancies before 20 weeks of gestation. Finally, we observed a decreased signal in hepatocyte, kidney, endothelial cell, and smooth muscle signatures across gestation and increased platelet signal before 12 weeks of gestation for PE. These tissue and cell type specific changes are both consistent with common PE pathogenesis and the specific, prominent diagnoses in our cohort (e.g., thrombocytopenia, proteinuria, impaired liver function, renal insufficiency).

### Summary

Noninvasive measurements of the cf-transcriptome present an opportunity to study human development and disease from any organ at a molecular scale. Here, we showed that circulating cfRNA measurements taken during pregnancy can clearly distinguish between PE and NT pregnancies. We found that the most striking differences occur early on and broadly showed no reproducible difference at post-partum, consistent with known PE etiology, namely that the placenta drives the disease process ^{34,36}. We next validated these changes using two separate cohorts (Validation 1 and 2) and explored their physiological relevance.

Our findings provide molecular evidence supporting generally accepted physiological understanding of PE pathogenesis: early abnormal placentation and systemic endothelial dysfunction ³⁴. Early in gestation, we observe a reduced placental signal for PE regardless of onset type or symptom severity. Concurrently, platelets and endothelial cells drive cfRNA changes in all PE as compared to NT and between PE with or without severe symptoms especially before 20 weeks of gestation. Increases in cell-type specific cfRNA may occur through signaling and secretion by cells, as underscored by functional enrichment analysis. The innate and adaptive immune system also heavily contribute to cfRNA changes in PE with clear, marked shifts related to bone marrow, T-cells, B-cells, granulocytes, and neutrophils, consistent with previous studies on the maternal-placental interface and PE ^{34,56-58}.

PE is a broad and complex syndrome. Because the complication can present clinically across more than 20 weeks with a diversity of symptoms, significant effort has been made to subclassify the disease based on timing of onset. Used as a proxy for hypothesized pathogenesis, the timing of PE onset subdivides the disease into 'placental' early-onset PE (occurring before 34 weeks of gestation) and 'maternal' late-onset PE (occurring on or after 34 weeks) ^{34,37,59}. However, early- and late-onset PE may represent a spectrum of disease severity that corresponds with timing of onset and may lead to additional pregnancy complications, such as intrauterine growth restriction (IUGR) ^{34,36,38,39}. Indeed, some evidence such as transcriptional profiling of whole blood drawn at diagnosis suggests a common gene signature for both subtypes ⁴⁰. Our findings further corroborate a common signature across onset types and suggest that PE may be better stratified based on symptom severity.

Indeed, PE may be best subtyped molecularly. Given the diversity of clinical presentations, we provide a noninvasive means of monitoring a mother's risk of specific organ damage, common in PE. The cfRNA changes we characterized here reflect dysfunction in at least five organ systems (brain, liver, kidney, muscle, bone marrow), and can in some cases further distinguish between PE with or without severe symptoms. As a molecular lens into maternal health, liquid biopsies present an opportunity as both a research and clinical tool to learn about the pathogenesis of a human disease in humans and as a predictor of maternal health.

Here, we have shown proof of principle that cfRNA measurements can form the basis for a robust liquid biopsy test, which predicts PE very early in gestation and if validated in controlled clinical studies, could help discover and manage those at risk for PE. Such a test could serve as a complement to recent efforts based on clinical and laboratory data⁶⁰, and even be coupled with tests taken later during gestation. We further demonstrated that cfRNA measurements reflect who is at risk for specific organ damage. Together, these results form the basis for a series of clinical tests that can be used to help characterize and stratify the pathogenesis of PE in real time, to date unrealized key objectives for obstetric care.

### Materials & Methods Employed in Studies Described in Example 1

### Clinical study design

For this longitudinal, prospective study, we enrolled pregnant mothers (aged 18 years or older) receiving routine antenatal care on or prior to 12 weeks of gestation at Lucile Packard Children's Hospital at Stanford University, following study review and approval by the Institutional Review Board at Stanford University (#21956). All signed informed consent prior to enrollment. Whole blood samples for plasma isolation were then collected at three distinct timepoints during their pregnancy course and once post-partum. Mothers were defined as having PE (cases, n = 38) based upon current American College of Obstetrics and Gynecology (ACOG) guidelines (see below). Our control (NT) cohort (n = 28) consisted of women who had uncomplicated term pregnancies and either normal spontaneous vaginal or caesarean deliveries. For mothers who developed PE, all antenatal samples included in this study were collected prior to clinical diagnosis.

### Definition of preeclampsia

Preeclampsia was defined per the ACOG guidelines based on two diagnostic criteria: 1) new-onset hypertension developing on or after 20 weeks of gestation and 2) new-onset proteinuria or in its absence, thrombocytopenia, impaired liver function, renal insufficiency, pulmonary edema, or cerebral or visual disturbances.

New-onset hypertension was defined when the systolic and/or diastolic blood were at least 140 or 90 mm Hg, respectively, on at least 2 separate occasions between 4 hours and 1 week apart. Proteinuria was defined when either 300 mg protein was present within a 24-hour urine collection or an individual urine sample contained a protein/creatinine ratio of 0.3 mg/dL, or if these were not available, a random urine specimen had more than 1 mg protein as measured by dipstick. Thrombocytopenia, impaired liver function, and renal insufficiency were defined as a platelet count of less than 10,000/µL, liver transaminases ≥ 2x of normal, and serum creatinine > 1.1 mg/dL, respectively.

Symptoms were defined as severe per the ACOG guidelines. Specifically, PE is defined as severe if any of the following symptoms were present and diagnosed as described above: new-onset hypertension with systolic and/or diastolic blood pressure of at least 160 or 110 mm Hg respectively, thrombocytopenia, impaired liver function, renal insufficiency, pulmonary edema, new-onset headache unresponsive to medication and unaccounted for otherwise, or visual disturbances.

Finally, a pregnant mother was considered to have early-onset PE if onset occurred before 34 weeks of gestation and late onset thereafter.

### Sample preparation

### Plasma processing

Blood samples were collected in either EDTA-coated (Cat No 368661, Becton-Dickinson) or Streck cfRNA BCT (Cat No 218976, Streck) tubes at early, mid, and late gestation, and post-partum for each participant. Within 30 minutes, tubes were then centrifuged at 1600g for 30 minutes at room temperature. Plasma was transferred to 2-mL microfuge tubes and centrifuged at 13000g for another 10 minutes in a microfuge. One milliliter aliquots were then transferred to 2-mL Sarsted screw cap microtubes (Cat No 50809242, Fisher Scientific) and stored at -80°C until analysis.

### cfRNA isolation

In 96-sample batches, cfRNA from 1 mL plasma samples was extracted in a semi-automated fashion using the Opentrons 1.0 system and Plasma/Serum Circulating and Exosomal RNA Purification 96-Well Kit (Slurry Format) (Cat No 29500, Norgen). Samples were subsequently treated with Baseline-ZERO DNAse (Cat No DB0715K, Lucigen) for 20 minutes at 37°C. DNAse-treated cfRNA was then cleaned and concentrated into 12 µL using RNA Clean and Concentrator-96 kit (Cat No R1080, Zym).

Following cfRNA extraction from plasma samples, isolated RNA concentrations were estimated for a randomly selected 11 samples per batch using Bioanalyzer RNA 6000 Pico Kit (Cat No 5067-1513, Agilent) per manufacturer instructions.

### Sequencing library preparation

cfRNA sequencing libraries were prepared with Takara's SMARTer Stranded Total RNAseq Kit v2 - Pico Input Mammalian Components (Cat No 634419) from 4 µL of eluted cfRNA according to the manufacturer's instructions. Samples were barcoded using Takara's SMARTer RNA Unique Dual Index Kit - 96U Set A (Cat No 634452), and then pooled in an equimolar fashion and sequenced on Illumina's NovaSeq platform (2x75 bp) to an average depth of 50 million reads per sample.

### RT-qPCR assay

We performed RT-qPCR in two stages: reverse transcription and preamplification followed by Taqman probe-based qPCR per manufacturer instructions. All primers and controls were commercially designed and validated (Bio-Rad). Specifically, we prepared cDNA using random hexamers from an initial volume of 4 µL cfRNA per sample (Bio-Rad Reliance Select cDNA Synthesis Kit, Cat No 12012801). Prior to reverse transcription (RT), we spiked in 1 µL RT control probe into each sample to measure the latter step's efficiency. After RT, we combined 5 µL of prepared cDNA with a primer pool, which was then preamplified for 12 cycles and subsequently diluted 1:5 (Tris EDTA) (Bio-Rad SsoAdvanced PreAmp Supermix, Cat No 1725160). Finally, we performed each qPCR assay in triplicate on the Bio-Rad CFX384 touch machine. Each replicate contained 1 µL of pre-amplified and diluted cDNA sample, 0.5 µL of the corresponding primers, and super-mix at the appropriate concentration (Bio-Rad SsoAdvanced Universal Probes Supermix, Cat No 1725285). Following qPCR, cycle threshold (Cₜ) values were extracted using CFX software.

### Sequencing Data Analysis

### Bioinformatic processing

For each sample, raw sequencing reads were trimmed using trimmomatic (v 0.36) and then mapped to the human reference genome (hg38) with STAR (v 2.7.3a). Duplicate reads were then removed by GATK's (v 4.1.1) MarkDuplicates tool. Finally, mapped reads were sorted and quantified using htseq-count (v 0.11.1). Read statistics were estimated using FastQC (v 0.11.8).

Across samples, the bioinformatic pipeline was managed using snakemake (v 5.8.1). Read and tool performance statistics were aggregated across samples and steps using MultiQC (v 1.7). Following sample quality filtering, all gene counts were adjusted to log₂-transformed counts per million reads (CPM) with trimmed mean of M values (TMM) normalization ⁴⁹.

### Sample quality filtering

For every sequenced sample, we estimated three quality parameters as previously described. To estimate RNA degradation in each sample, we first counted the number of reads per exon and then annotated each exon with its corresponding gene ID and exon number using htseq-count. Using these annotations, we measured the frequency of genes for which all reads mapped exclusively to the 3' most exon as compared to the total number of genes detected. RNA degradation for a given sample can then be approximated as the fraction of genes where all reads mapped to the 3' most exon. To estimate ribosomal read fraction, we compared the number of reads that mapped to the ribosome (Region GL00220.1:105424-118780, hg38) relative to the total number of reads (Samtools view). To estimate DNA contamination, we quantified the ratio of reads that mapped to intronic as compared to exonic regions of the genome.

After measuring these three metrics across nearly 700 samples, we empirically estimated each one's 95^{th} percentile bound. We considered any given sample an outlier, low quality sample if its value for at least one of these metrics was greater than or equal to the 95^{th} percentile bound.

Once values for each metric was estimated across the entire cohort, we visualized: (1) whether low-quality samples clustered separately using hierarchical clustering (average linkage, Euclidean distance metric) and (2) whether sample quality drove variance in gene measurements using principal component analysis (PCA). These analyses were performed in python (v 3.6) using scikit-learn for PCA (v 0.23.2), scipy for hierarchical clustering (v 1.5.1), and nheatmap for heatmap and clustering visualization (v 0.1.4).

### Differential expression analysis

Differential expression analysis was performed in R using limma (v 3.38.3). To identify gene changes associated with PE across gestation, we used a mixed effects model and included gestational age (continuous variable), case (binary variable, NT = 0 vs PE = 1), and the interaction between the two in the design matrix. We chose to model gestational age as a continuous variable, specifically a natural cubic spline with 4 degrees of freedom to account for the range across which samples were collected (1-3 months per collection period). We also blocked for participant identity (categorical variable), modeling it as a random effect to account for auto-correlation between samples from the same person. Post-partum samples were excluded from differential expression, instead serving as a hold-out dataset since they presented a clear break in gestational time.

Per the limma-voom guide, to account for sample auto-correlation over time, we ran the function voomWithQualityWeights twice. We first ran it without any blocking on participant identity, and used this base estimation to approximate sample auto-correlation based on participant identity using the function duplicateCorrelation. After estimating correlation, voomWithQualityWeights was run again, this time blocking for participant identity and including the estimated auto-correlation level. A linear model was then fit for each gene using lmFit and differential expression statistics were approximated using Empirical Bayes (eBayes) methods. Finally, differentially expressed genes were identified using the relevant design matrix coefficients and the function, topTable, with Benjamini-Hochberg multiple hypothesis correction.

### log(Fold change) and coefficient of variation (CV) estimation

We define log₂-transformed fold-change (logFC) as the difference between the median gene level (logCPM, see Bioinformatic processing section above) between PE and NT samples for a given sample collection period (i.e., early, mid, and late gestation, or post-partum). In the case where a given person had multiple samples included into a specific collection period, we only used the values associated with the first collected sample to avoid artificially reducing within-group (PE or NT) variance due to auto-correlation among samples from the same person.

We define the coefficient of variation (CV) using an approximation. Specifically, we consider CV to be the ratio between an error bound, ∂, and the estimated logFC. We defined the error bound, ∂, as the one-sided error bound associated with the lower (or upper in the case of negative logFC values) 95% confidence interval (CI) as estimated by bootstrapping. This definition of ∂ as a one-sided bound that approaches 0 (equivalent to no FC) allowed us explore how confident we could be in an estimated logFC. For instance, a CV value of 1 would indicate that at the boundary of proposed values, the logFC for a given gene becomes effectively 0. Similarly, a CV of greater than 1 would indicate even less confidence in a proposed average logFC and indicate that at the boundary, the estimated logFC changes signs (i.e., a negative logFC becomes a positive one or vice versa).

### Hierarchical clustering analysis

For each sample collection period, hierarchical clustering was performed using differentially expressed genes with an |logFC| ≥ 0.75 and CV < 0.5. For each gene that passed these thresholds, we calculated a z-score across all samples (at most 1 per subject, the earliest collected sample in a given group) in each sample collection period using the function StandardScaler in scikit-learn (v 0.23.2), Average linkage hierarchical clustering with a Euclidean distance metric was then performed for both rows (gene z-scores) and columns (samples in same collection group) for a given matrix in python using scipy (v 1.5.1). Clustering and corresponding heatmaps were visualized using nheatmap (v 0.1.4).

### Longitudinal dynamics analysis

To group gene changes by longitudinal behavior, we performed k-means clustering on a matrix where each row was a differentially expressed gene and each column was the estimated logFC for a given sample collection period (556 genes x 4 time points). We measured the sum of squared distances for every 'k' between 1 and 16 (4²) where 16 represents the maximum possible k (4 time points with 2 possibilities each, logFC > 0 or logFC < 0). We then identified the optimal k clusters using the well-established elbow method to identify the smallest 'k' that can best explain the data, visually described as the elbow (or knee) of a convex plot like that for the sum of squared distances vs k (Fig 8). To do so, we used the function KneeLocator as implemented in the package kneed (v 0.7.0). Having identified the optimal number of clusters, 'k', we labeled every differentially expressed gene with its assigned cluster and visualized average behavior (mean) and the 95% CI per cluster using Seaborn line plot (v 0.10.0).

To confirm that the identified patterns were not spurious (i.e., an artifact of the k-means clustering algorithm), we permuted the data columns (logFC per time point) for each gene thereby scrambling any time-related structure while preserving its overall distribution. We then visualized the result using Seaborn line plot as described above.

### Gene ontology analysis

Gene ontology (GO) analysis was performed using the tool, GProfiler (v 1.0.0), for the following data sources, Gene ontology: biological processes (GO:BP) and Reactome (REAC). In identifying GO terms, electronic GO annotations (IEA) were included in the analysis. DEGs related to the ribosome were excluded from GO analysis given their extensive annotation that can lead to spurious term associations. To narrow this initial list (77 GO terms) to a smaller number for plotting purposes, the initial GO table was then pruned to only include parent terms (as filtered by the column, parents).

### Logistic regression feature selection and training

To build a robust classifier that can identify mothers at risk of PE at or before 16 weeks of gestation, we first pre-selected features using the differential expression analysis (see section with same name above) as a starting point. Next, to correct for batch effect, where we define batch as a set of samples processed at the same time by a distinct group (e.g., Discovery cohort = batch, Del Vecchio and colleagues' cohort = batch), we subtracted the mean logCPM per gene from all cfRNA measurements in the batch (zero-centering). We note that all cohorts used in this work had balanced batch-group design and therefore met the requirements for zero-centering to work as expected. Model training then used the entire discovery cohort and consisted of two stages - further feature pre-selection based on three metrics followed by the construction of a logistic regression model with an elastic net penalty.

For feature pre-selection, we focused on three practical metrics measured across all samples collected on or before 16 weeks of gestation: gene change size (|logFC|), gene change stability (CV), and median gene expression (median CPM). Filtering on median gene expression helped ensure that any genes selected could be robustly detected in smaller initial plasma volumes like those that might be drawn in the clinic. All model hyperparameters and feature pre-selection cutoffs were tuned using accuracy as the outcome metric and leave-one-out cross validation.

### Calibration analysis

We assessed model calibration using the calibration curve's slope and intercept. Specifically, we estimated the calibration curve using the function, calibration_curve, from sklearn.calibration and 10 bins. We then estimated slope and intercept using the function polyfit with 1 degree from numpy. A well-calibrated model is considered to have a slope of approximately 1 and an intercept of approximately 0. Curve slopes greater than 1 suggested moderate, underestimated risk scores whereas slopes less than 1 suggested the opposite. Additionally, negative calibration curve intercept values suggested underestimation whereas positive values suggested overestimation.

### Performance metric analysis

Model performance was assessed using several statistics including sensitivity, specificity, PPV, NPV, and ROC AUC. Given a 2x2 confusion matrix where rows 1 and 2 represent true negatives and positives and columns 1 and 2 represent negative and positive predictions respectively, we can define the value in row 1, column 1 as true negatives (TN), row 1, column 2 as false positives (FP), row 2, column 1 as false negatives (FN), and row 2, column 2 as true positives (TP). We can then define the following proportions: (1) Sensitivity = TP / (TP + FN) (2) Specificity = TN / (TN + FP) (3) PPV = TP / (TP + FP) (4) NPV = TN / (TN + FN). For each proportion, we calculated 95% confidence intervals using Jeffrey's interval ⁵² and the function, proportion_confint, from statsmodels.stats.proportion. We also approximated AUC and its corresponding 95% confidence interval using the scikit-learn function, roc_auc_score, and bootstrapping respectively.

### RT-qPCR Data Analysis

### Sample quality filtering

RT-qPCR and sample quality were assessed using two controls. First, we confirmed the absence of genomic DNA contamination using a commercially available control. Any sample where genomic DNA was detected as defined by the manufacturer would have been excluded from further analysis; however, in this work, no sample contained DNA contamination at a detectable level. Next, we measured reverse-transcription efficiency (RT) using a control probe spiked in at a known concentration prior to RT and subsequently measuring the probe's Cₜ following preamplification (no primers included for RT in preamplification) and qPCR as detailed above (see RT-qPCR assay). For inclusion in any further analysis, we required that the RT probe (at levels below the corresponding non-template control) be detected in a given sample well.

### Fold-change estimation

We estimated fold-change from RT-qPCR data using the ΔΔCt method. Specifically, for every gene of interest (Gᵢ), we first estimated ΔCₜ = Cₜ (Gᵢ) - Cₜ (RT control) to account for different reverse-transcription efficiencies across samples. For more details about the RT control, refer to the sections titled "Sample quality filtering" and "RT-qPCR assay". Having defined ΔCt for every gene measured in each sample, we can then define ΔΔCt for a given gene as the difference between the median ΔCt in the PE group and in the NT group. Fold-change can then be defined as 2^{-ΔΔC}*^{t}.*

### Statistical analysis

All p-values reported herein were calculated using the non-parametric Mann-Whitney rank test unless otherwise stated. One-sided tests were performed where required based on the hypothesis tested.

### Code/data availability

All computational analyses were performed using Python 3.6 or R 3.5, and will be available on Github.

### Supplementary text

### Supplementary note 1: Establishing quality metrics to identify sample outliers

Because cfRNA measurements can be noisy, we have previously developed and reported on three quality metrics that can flag sequenced cfRNA samples with poor quality Specifically, these metrics aim to quantify unusually high levels of RNA degradation, DNA contamination, and/or ribosomal RNA by comparing a given sample's value for any of these metrics with what we expect empirically. We defined reasonable expected values for each metric based on the 95^{th} percentile for ~700 previously sequenced samples across 3 cohorts.

We found that samples with outlier values for at least one of these three metrics both clustered separately (Fig. 4) and served as leverage points in principal component analysis (PCA) (Fig. 5A-C). To avoid introducing unwanted bias, we removed these low-quality samples from any further analysis. After removing outlier samples, we reran PCA and noticed that two samples continued to serve as leverage points (Fig5B). We suspected that this may be due to genes that were poorly detected and consequently performed further filtering to identify well-detected genes across the entire cohort. Specifically, we used a basic cutoff that required a given gene be detected at a level of at least 0.5 counts per million reads (CPM) in at least 75% of samples after removing outlier samples. Following this step, we retain 7,041 genes for analysis. Upon inspection, we find that visualization using PCA is no longer driven by leverage points (Fig 5C).

### Materials & Methods Employed in Studies Described in Example 2

### Clinical study design

Discovery and Validation 1 were collected as part of a longitudinal, prospective study. We enrolled pregnant mothers (aged 18 years or older) receiving routine antenatal care on or prior to 12 weeks of gestation at Lucile Packard Children's Hospital at Stanford University, following study review and approval by the Institutional Review Board (IRB) at Stanford University (#21956). All signed informed consent prior to enrollment. Whole blood samples for plasma isolation were then collected at three distinct time points during their pregnancy course and once (or twice for 2 individuals) post-partum.

Validation 2 was collected as part of the Global Alliance to Prevent Preterm and Stillbirth (GAPPS) at several, independent sites. Samples were processed and sequenced at Stanford under the same IRB as above (#21956). All signed informed consent prior to enrollment. Whole blood samples for plasma isolation were collected at a single time point (or 2 timepoints in the case of 2 individuals with PE) prior to or at 16 weeks of gestation.

For all three cohorts, we ensured that all included individuals did not have chronic hypertension or gestational diabetes. Mothers were defined as having PE based upon current American College of Obstetrics and Gynecology (ACOG) guidelines (see below). Mothers were defined as controls if they had uncomplicated term pregnancies and either normal spontaneous vaginal or caesarean deliveries. For mothers who developed PE, all antenatal samples included in this study were collected prior to clinical diagnosis.

We tested for within cohort (NT vs PE) and across cohort differences in demographic variables using a chi-squared test and ANOVA for categorical and continuous variables respectively. We then applied Bonferroni correction and reported any differences as significant if adjusted p ≤ 0.05.

### Definition of PE

PE was defined as described in Example 1.

### Plasma processing

At Lucile Packard Children's Hospital, blood samples were collected in either EDTA-coated (Cat No 368661, Becton-Dickinson) or Streck cfRNA BCT (Cat No 218976, Streck) tubes at ≤12, 13-20, and ≥23 weeks of gestation, and post-partum for each participant. Within 30 minutes, tubes were then centrifuged at 1600g for 30 minutes at room temperature. Plasma was transferred to 2-mL microfuge tubes and centrifuged at 13000g for another 10 minutes in a microfuge. One milliliter aliquots were then transferred to 2-mL Sarstedt screw cap microtubes (Cat No 50809242, Fisher Scientific) and stored at -80°C until analysis.

At GAPPS, blood samples were collected in EDTA-coated tubes at ≤16 weeks of gestation from a network of collection sites including Yakima Valley Memorial Hospital, Swedish Medical Center, and the University of Washington Medical Center. Per Standard Operating Procedure (SOP), tubes were then centrifuged within 2 hours of collection at 2500RPM for 10 minutes at room temperature in a swinging bucket rotor. Plasma was transferred to 2-mL cryovials in at most 1 mL aliquots and stored at -80°C until analysis. Sample volume was also recorded.

### cfRNA isolation

cfRNA was isolated as described in Example 1.

### Sequencing library preparation

cfRNA sequencing libraries were prepared with SMARTer Stranded Total RNAseq Kit v2 - Pico Input Mammalian Components (Cat No 634419, Takara) from 4 µL of eluted cfRNA according to the manufacturer's instructions. Samples were barcoded using SMARTer RNA Unique Dual Index Kit - 96U Set A (Cat No 634452, Takara), and then pooled in an equimolar fashion and sequenced on Illumina's NovaSeq platform (2x75 bp) to a mean depth of 54, 33, and 38 million reads per sample for Discovery, Validation 1, and Validation 2 cohorts, respectively. Some samples (12, 61, 0 for Discovery, Validation 1, and Validation 2 cohorts) were not sequenced due to failed library preparation.

### Bioinformatic processing

For each sample, raw sequencing reads were trimmed using Trimmomatic (v 0.36) and then mapped to the human reference genome (hg38) with STAR (v 2.7.3a). Duplicate reads were then removed by GATK's (v 4.1.1) MarkDuplicates tool. Finally, mapped reads were sorted and quantified using htseq-count (v 0.11.1) generating a counts table (genes x samples). Read statistics were estimated using FastQC (v 0.11.8).

Across samples, the bioinformatic pipeline was managed using Snakemake (v 5.8.1). Read and tool performance statistics were aggregated across samples and steps using MultiQC (v 1.7). Following sample quality and gene filtering, all gene counts were adjusted to log₂-transformed counts per million reads (CPM) with trimmed mean of M values (TMM) normalization ⁶¹.

### Sample quality filtering

For every sequenced sample, we estimated three quality parameters as previously described ^{62,63}. To estimate RNA degradation in each sample, we first counted the number of reads per exon and then annotated each exon with its corresponding gene ID and exon number using htseq-count. Using these annotations, we measured the frequency of genes for which all reads mapped exclusively to the 3' most exon as compared to the total number of genes detected. RNA degradation for a given sample can then be approximated as the fraction of genes where all reads mapped to the 3' most exon. To estimate the number of reads that mapped to genes, we summed counts for all genes per sample using the counts table generated from bioinformatic processing above. To estimate DNA contamination, we quantified the ratio of reads that mapped to intronic as compared to exonic regions of the genome.

After measuring these three metrics across nearly 700 samples, we empirically estimated RNA degradation and DNA contamination's 95^{th} percentile bound. We considered any given sample an outlier, low quality sample if its value for at least one of these metrics was greater than or equal to the 95^{th} percentile bound or if no reads were assigned to genes.

Once values for each metric were estimated across the entire dataset, we visualized: (1) whether low-quality samples clustered separately using hierarchical clustering (average linkage, Euclidean distance metric) and (2) whether sample quality drove variance in gene measurements using principal component analysis (PCA). These analyses were performed in Python (v 3.6) using Scikit-learn for PCA (v 0.23.2), Scipy for hierarchical clustering (v 1.5.1), and nheatmap for heatmap and clustering visualization (v 0.1.4).

### Gene filtering

We performed filtering to identify well-detected genes across the entire cohort. Specifically, we used a basic cutoff that required a given gene be detected at a level of at least 0.5 CPM in at least 75% of discovery samples after removing outlier samples. Following this step, we retain 7,160 genes for DE analysis.

### Differential expression analysis

Differential expression analysis was performed in R using Limma (v 3.38.3). To identify gene changes associated with PE across gestation and post-partum, we used a mixed-effects model. We performed DE using two design matrices: (1) Examine the interaction between time to PE onset or delivery for NT and PE symptoms (i.e., PE with or without severe symptoms) and (2) Examine the interaction between time to PE onset or delivery for NT and PE broadly. In both design matrices, we included time to PE onset or delivery for NT (continuous variable), whether a sample was collected post-partum (binary variable), the interaction between time and PE symptoms for (1) or PE for (2), the interaction between whether a sample is post-partum and PE symptoms for (1) and PE for (2), and 7-8 confounding factors.

In (1), we defined PE symptoms categorially using 3 levels - NT, PE without severe symptoms, PE with severe symptoms). In (2), we defined whether a sample was PE using a binary, indicator variable (0 = NT, 1 = PE). The 7-8 confounding variables included were maternal race (categorial variable), maternal ethnicity (binary variable), fetal sex (binary variable), maternal pre-pregnancy BMI group (categorical variable), maternal age (continuous variable, only included in design 1), and sequencing batch (categorical variable). We defined time to PE onset or delivery as the difference between gestational age at onset or delivery and gestational age at sample collection. We defined BMI group as follows: Obese (BMI ≥ 30), Overweight (25 ≤ BMI < 30), Healthy (18.5 ≤ BMI < 25), Underweight (BMI < 18.5). We chose to model time to PE onset or delivery as a continuous variable, specifically a natural cubic spline with 4 degrees of freedom to account for the range across which samples were collected (1-3 months per collection period). We also blocked for participant identity (categorical variable), modeling it as a random effect to account for auto-correlation between samples from the same person.

Per the Limma-Voom guide, to account for sample auto-correlation over time, we ran the function voomWithQualityWeights twice. We first ran it without any blocking on participant identity, and used this base estimation to approximate sample auto-correlation based on participant identity using the function duplicateCorrelation. After estimating correlation, voomWithQualityWeights was run again, this time blocking for participant identity and including the estimated auto-correlation level. A linear model was then fit for each gene using lmFit and differential expression statistics were approximated using Empirical Bayes (eBayes) methods. For comparing PE with vs without severe symptoms, we contrasted the relevant coefficients (makeContrasts) and then applied Empirical Bayes as opposed to directly after lmFit.

DEGs were then identified using the relevant design matrix coefficients and the function, topTable, with Benjamini-Hochberg multiple hypothesis correction at a significance level of 0.05. For design 1, we identified DEGs related to 3 comparisons: PE without severe symptoms vs NT (1759 DEGs), severe PE vs NT (1198 DEGs), and PE with vs without severe symptoms (503 DEGs). We find 544 genes in common for PE without and with severe symptoms vs NT. These 544 DEGs were explored in Figure 2 and the related main text. For design 2, we identified DEGs related to PE vs NT alone (330 DEGs), which we used as the initial gene set for building a logistic regression model (see Supplementary Note 2). Finally, we removed the effect of sequencing batch alone on estimated logCPM values with TMM normalization for the Discovery cohort using the limma-voom function, removeBatchEffect.

### log(Fold change) and CV estimation

We define log₂-transformed fold-change (logFC) as the difference between the median gene level (logCPM, see Bioinformatic processing section above) between PE and NT samples for a given sample collection period (i.e., ≤12, 13-20, and ≥23 weeks of gestation, or post-partum). In the case where a given person had multiple samples included into a specific collection period, we only used the values associated with the first collected sample to avoid artificially reducing within-group (PE or NT) variance due to auto-correlation among samples from the same person.

We then quantified the relative dispersion around the estimated logFC for each gene using an approximation for CV. Specifically, we consider CV to be the ratio between an error bound, ∂, and the estimated logFC. We defined the error bound, ∂, as the one-sided error bound associated with the lower (or upper in the case of negative logFC values) 95% CI as estimated by bootstrapping. This definition of ∂ as a one-sided bound that approaches 0 (equivalent to no FC) allowed us to explore how confident we could be in an estimated logFC. For instance, a CV = 1 would indicate that at the boundary of proposed values, the logFC for a given gene becomes effectively 0. Similarly, a CV > 1 would indicate even less confidence in a proposed average logFC and indicate that at the boundary, the estimated logFC changes signs (i.e., a negative logFC becomes a positive one or vice versa).

### Hierarchical clustering analysis

For each sample collection period, hierarchical clustering was performed using differentially expressed genes with an |logFC| ≥ 1 and CV < 0.5 or 0.4 in the case of the 13-20 weeks of gestation time point so that the number of genes used did not exceed the number of samples. For each gene that passed these thresholds, we calculated a z-score across all samples (at most 1 per subject, the earliest collected sample in a given group) in each sample collection period using the function StandardScaler in Scikit-learn (v 0.23.2), Average linkage hierarchical clustering with a Euclidean distance metric was then performed for both rows (gene z-scores) and columns (samples in same collection group) for a given matrix in Python using Scipy (v 1.5.1). Clustering and corresponding heatmaps were visualized using nheatmap (v 0.1.4).

### Longitudinal dynamics analysis

To group gene changes by longitudinal behavior, we performed k-means clustering on a matrix where each row was a differentially expressed gene and each column was the estimated logFC for a given sample collection period (N genes x 4 timepoints). We measured the sum of squared distances for every 'k' between 1 and 16 (4²) where 16 represents the maximum possible k (4 timepoints with 2 possibilities each, logFC > 0 or logFC < 0). We then identified the optimal k clusters by using the well-established elbow method to identify the smallest 'k' that best explained the data, visually described as the elbow (or knee) of a convex plot like that for the sum of squared distances vs k (Fig S4A,C). To do so, we either visually inspected and identified the elbow (Fig 2D) or used the function KneeLocator as implemented in the package Kneed (v 0.7.0) (Fig 4A). We used visual inspection for Fig 2D as we observed that given two k-values (e.g., k = 2,3) with similar sum of squared distances, KneeLocator would choose the larger value whereas we preferred a simpler model. Having identified the optimal number of clusters, 'k', we labeled every differentially expressed gene with its assigned cluster and visualized average behavior (median) and the 95% CI (bootstrapped using 1000 iterations) per cluster using Seaborn line plot (v 0.10.0).

To confirm that the identified patterns were not spurious (i.e., an artifact of the k-means clustering algorithm), we permuted the data columns (logFC per timepoint) for each gene thereby scrambling any time-related structure while preserving its overall distribution. We then visualized the result using Seaborn line plot as described above. Following permutation, we observed no longitudinal patterns, which were instead replaced by nearly flat, uninformative trends (Fig S4B,D).

### Correlation analysis

To verify DEGs identified in the Discovery cohort, we compared logFCs for the Discovery as compared to both Validation 1 and Validation 2 cohorts. We calculated the percentage of genes for which the logFC had the same sign across cohorts (i.e., both positive or both negative) and the spearman correlation using the function scipy.stats.spearmanr. We did not calculate logFCs for DEGs at ≤12 weeks of gestation in Validation 1 because of small sample numbers (3 PE samples prior to 12 weeks).

We also sought to compare whether symptom severity (without or with severe) correlated with logFC magnitude for 544 DEGs identified as common to all PE in design 1. To do so, we calculated the slope of a best-fit line where x and y were defined as logFCs for PE without (x) and with (y) severe features vs NT. We would expect a slope > 1 and < 1 if logFC magnitudes for PE with as compared to without severe symptoms were larger or smaller on average respectively. Similarly, a slope = 1 would reflect that symptom severity did not correlate with logFC magnitude for the 544 DEGs tested.

Finally, to confirm that the identified correlations were significant, we permuted the data columns (logFC per cohort) for each gene thereby scrambling any structure while preserving its overall distribution. We then calculated the same statistics. Following permutation, we observe about 50-55% logFC agreement, as expected at random, a spearman correlation of 0, and slope of 0.

### Defining cell-type and tissue-specific gene profiles

Cell-type and tissue specific gene profiles were all identified as previously described ⁴⁴. We also briefly summarize this method below.

On the tissue level, for genes and tissues (and some blood and immune cell types) measured in the Human Protein Atlas (HPA, v19)⁵⁵, we calculated a Gini index per gene as a measure of tissue specificity. As a measure of inequality, Gini index values closer to 1 represent genes that are tissue specific. We defined a given gene Y as specific to tissue X if Gini(Y) ≥ 0.6 and max expression for Y is in tissue X. Although the aforementioned method identifies fairly tissue specific genes, it is possible to have a gene Y where Gini(Y) ≥ 0.6 and the gene is expressed in more than 1 tissue (e.g., enrichment in placenta and muscle). To this end, when tracking cell-type and tissue trajectories over gestation (e.g., Fig 4B), where the specificity of a given gene profile is especially important, we imposed a further constraint to ensure that any gene signal only reflects the named tissue (e.g., any gene named placenta specific is specific to the placenta alone). Specifically, we required that genes be annotated by HPA as 'Tissue enriched' or 'Tissue enhanced' and term this reference, HPA strict.

On the cell-type level, we augmented Tabula Sapiens v1.0 (TSP) with cell types from missing (e.g., placenta, brain), incompletely described tissues (e.g., kidney), or additional annotations (e.g., liver) known to be important in PE. We term this augmented reference TSP+. For genes and cell types measured in Tabula Sapiens v1.0 (TSP), we defined a given gene Y as specific to cell-type X if Gini(Y) ≥ 0.8 and max mean expression for Y is in cell-type X. We combined annotations for all neutrophil and endothelial subtypes as these were based on manifold clustering and it was unclear if the subtypes were truly distinct enough to be distinguished at a whole-body level for our purposes. For genes and cell types described in individual tissue single-cell atlases, we required that a gene be differentially expressed in the specific single cell atlases and tissue specific per the HPA (Gini ≥ 0.6). The following single cell atlases were used for each organ: 1) Placenta ^{64,65} 2) Liver ⁶⁶ 3) Kidney ⁶⁷ 4) Heart ⁶⁸ 5) Brain ⁶⁹.

For TSP+, we then took the union of TSP and individual atlas gene annotations. A small number of genes had conflicting double annotations in TSP as compared to at most one individual tissue single cell atlas. In these rare instances, which most often occurred for genes related to cell-types missing in TSP (e.g., placental or brain cell-types), we preferred the individual single-cell atlas label to TSP.

### Determining cell type and tissue of origin

We determined whether a given cell-type or tissue was enriched in PE by comparing PE DEGs with cell-type and tissue gene profiles using a hypergeometric test. For every tissue (HPA) or cell-type (TSP+) with at least 2 DEGs specific to it, we performed the following. First, we defined a hypergeometric distribution (scipy.stats.hypergeom, (v 1.5.1)) with the following parameters where category refers to tissue when using HPA and cell-type when using TSP+: M = Number of genes specific to any category, n = Number of genes specific to this category, N = number of DEGs in this k-means longitudinal cluster specific to this category. Next, we estimated a p-value using the survival function (1-CDF) for the specified distribution. Specifically, a p-value is defined as the cumulative probability, prob(X>(n_DEGs_specific_to_this_category-1)), that the distribution takes a value greater than the number of DEGS specific to this category - 1. Finally, once we estimated a p-value for every cell-type (TSP+) or tissue (HPA) identified in each DEG k-means longitudinal cluster, we adjusted for multiple hypotheses using Benjamini-Hochberg and a significance threshold of 0.05.

### Defining relative signature score per cell type or tissue

We define a signature score as the sum of logCPM values over all genes in a given tissue or cell-type gene profile. We required that a cell type or tissue gene profile have at least 5 specified genes to be considered for signature scoring in cfRNA. Genes were defined as specific to a given tissue based on the reference, HPA strict, and to a given cell type, based on the reference TSP+ (see "Defining cell-type and tissue-specific genes" for details).

To account for our prior observation that baseline cfRNA levels vary per subject - the consequence of biological and technical (e.g., sample processing) factors, we chose to calculate relative as opposed to absolute signature scores. For each subject for which the post-partum sample passed sample QC (see "Sample quality filtering" for details), we estimated a relative signature score defined as the difference between the signature score at a given gestational time point and the post-partum sample. For both Discovery and Validation 1, 49 NT and 24 PE subjects had a post-partum sample that survived sample QC. After normalization, all samples at post-partum had a similar baseline (0). We note that one can define a relative signature score based on any sampled time point for a given person. We chose the post-partum sample because we were interested in tracking maternal organ health over gestation.

Finally, we scaled (i.e., z-score) the relative signature scores for a given cell type or tissue by dividing by the interquartile range, a robust alternative to standard deviation, using the sklearn.preprocessing class, RobustScaler. This accounted for differing gene profile lengths and gene expression levels, and allowed us to compare both different cell-type and tissue contributions and case groups per cell-type or tissue.

Having defined a relative signature score per cell-type and tissue, we visualized average behavior (median) and the 75% CI, a non-parametric estimation of standard deviation, (bootstrapped relative signature score per case group and timepoint using 1000 iterations) using Seaborn line plot (v 0.10.0).

### Functional enrichment analysis

Functional enrichment analysis was performed using the tool, GProfiler (v1.0.0) for the following data sources, Gene ontology: biological processes and cellular compartments (GO:BP, GO:CC, released 2021-05-01), Reactome (REAC, released 2021-05-07), and Kyoto Encyclopedia of Genes and Genomes (KEGG, released 2021-05-03). To identify GO terms, we excluded electronic GO annotations (IEA) and used a custom background of only the 7160 genes that were included in DE. We then performed the recommended multiple hypothesis correction (g:SCS) with an experiment wide significance threshold of a = 0.05 ⁷⁰.

### Logistic regression feature selection and training

To build a robust classifier that can identify mothers at risk of PE at or before 16 weeks of gestation, we first pre-selected features using the set of 330 DEGs when contrasting PE vs NT (see design 2 in "Differential expression analysis" and Supplementary Note 2) as a starting point.

We normalized gene measurements using a series of steps. First, to correct for batch effect, where we define batch as a set of samples processed at the same time by a distinct group (e.g., Discovery Table S4cohort = batch, Del Vecchio and colleagues' cohort = batch), we centered the data by subtracting the median logCPM per gene for a given cohort. Next, we scaled gene values for each cohort using its corresponding interquartile range in the Discovery cohort. Finally, to account for sampling differences across sample, we used an approach similar to when analyzing RT-qPCR data, and normalized data using multiple internal control (i.e., housekeeping) genes. On a per sample basis, we subtracted the median, normalized logCPM value (centered and scaled) for all internal control genes, which we define as 66 genes for which the measured value did not change across PE vs NT comparisons (All genes with adjusted p-value > 0.99 for PE vs NT, Design 2). When calculating the median value for all internal control genes, we excluded any 0 logCPM values as these were likely the consequence of technical dropout.

Model training then used the Discovery cohort alone split into 80% for hyperparameter tuning and 20% for model selection and consisted of two stages - further feature pre-selection based on two metrics followed by the construction of a logistic regression model with an elastic net penalty. Using a split Discovery cohort for training mitigated overfitting even though all Discovery samples were used for differential expression, which defined the initial feature set.

For feature pre-selection, we calculated logFC values using the 80% Discovery split for all 330 genes for PE vs NT. We focused on two practical metrics measured across the 80% split of Discovery samples collected on or before 16 weeks of gestation: gene change size (|logFC|) and gene change stability (CV). All model hyperparameters were then tuned using AUROC as the outcome metric and 5-fold cross validation. Next, we selected the best model including tuned feature pre-selection cutoffs again using AUROC. Specifically, we calculated an AUROC score for both the 80% and 20% Discovery splits separately, and the selected model achieved the best score on both splits

Finally, we tuned the probability threshold, P, at which a sample is labeled as at risk of PE if prob(PE) ≥ P using the entire Discovery cohort. To do so, we constructed a receiver operator characteristic curve (ROC) and calculated the false positive rate (FPR) and true positive rate (TPR) at different thresholds, P_i,. We identified the threshold, P_i, at which FPR=10%, and round to the nearest 5 (e.g., 0.37 would become 0.35). This yielded a tuned threshold of P = 0.35. All classifications as negative or positive were then made based on this threshold.

To understand the importance of each gene feature, we trained a separate logistic regression model for a subset of all possible feature subset (307 combinations out of a total of 262143 for 1-17 genes). No feature pre-selection was performed for this sub-analysis. All model hyperparameters were tuned as previously described. We defined a gene subset as weakly predictive if the model yielded an AUROC > 0.5 on the test set (Validation 2).

In all cases, performance metrics were assessed as described below (see next section) and used Scikit-learn (v 0.23.2),

### Performance metric analysis

Model performance was assessed using several statistics including sensitivity, specificity, PPV, NPV, and AUROC. Given a 2x2 confusion matrix where rows 1and 2 represent true negatives and positives and columns 1 and 2 represent negative and positive predictions respectively, we can define the value in row 1, column 1 as true negatives (TN), row 1, column 2 as false positives (FP), row 2, column 1 as false negatives (FN), and row 2, column 2 as true positives (TP). We can then define the following proportions: (1) Sensitivity = TP / (TP + FN) (2) Specificity = TN / (TN + FP) (3) PPV = TP / (TP + FP) (4) NPV = TN / (TN + FN). For each proportion, we calculated 90% CIs using Jeffrey's interval ⁷¹ and the function, proportion_confint, from statsmodels.stats.proportion. We also approximated AUC and its corresponding 90% CI using the Scikit-learn function, roc_auc_score, and the binormal approximation respectively.

### Statistical analyses and code/data availability

All p-values reported herein were calculated using the non-parametric Mann-Whitney rank test unless otherwise stated. One-sided tests were performed where required based on the hypothesis tested.

All computational analyses were performed using Python 3.6 or R 3.5, and will be available on Github. Raw and processed sequencing data will be deposited with the SRA and GEO, respectively.

### Supplementary note 1: Establishing quality metrics to identify sample outliers

Because cfRNA measurements can be noisy ^{31,72}, we have previously developed and reported on three quality metrics that can flag sequenced cfRNA samples with poor quality ^{62,63}. Specifically, these metrics aim to quantify unusually high levels of RNA degradation and/or DNA contamination by comparing a given sample's value for any of these metrics with what we expect empirically. We defined reasonable expected values for each metric based on the 95th percentile for ~700 previously sequenced samples across 3 cohorts.

We found that samples with outlier values for at least one of these metrics both clustered separately and served as leverage points in PCA (Fig 15A-C). To avoid introducing unwanted bias, we removed these low-quality samples from any further analysis. After removing outlier samples, we reran PCA and noticed that some samples continued to serve as leverage points. We suspected that this may be due to genes that were poorly detected and consequently performed further filtering to identify well-detected genes across the entire cohort. Specifically, we used a basic cutoff that required a given gene be detected at a level of at least 0.5 CPM reads in at least 75% of samples after removing outlier samples. Following this step, we retain 7,160 genes for analysis. Upon inspection, we find that visualization using PCA is no longer driven by leverage points.

### Supplementary note 2: Selecting an initial feature set for machine learning

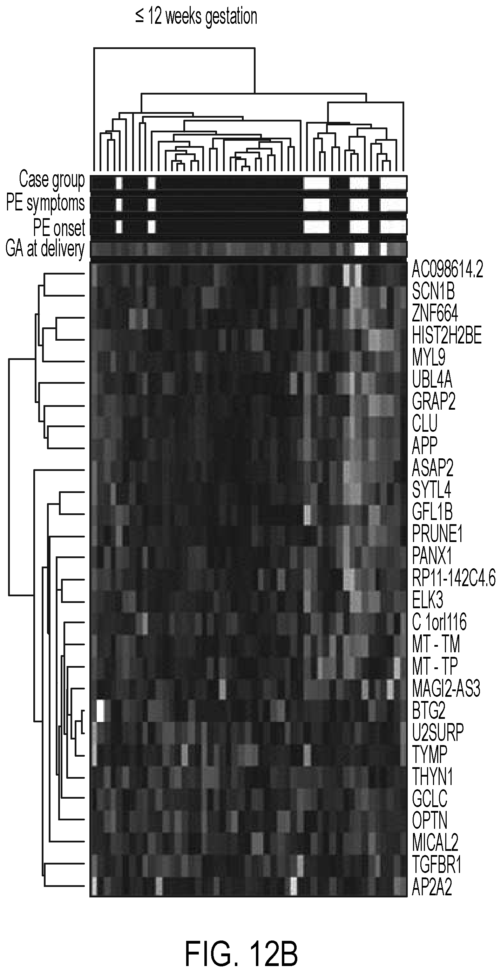

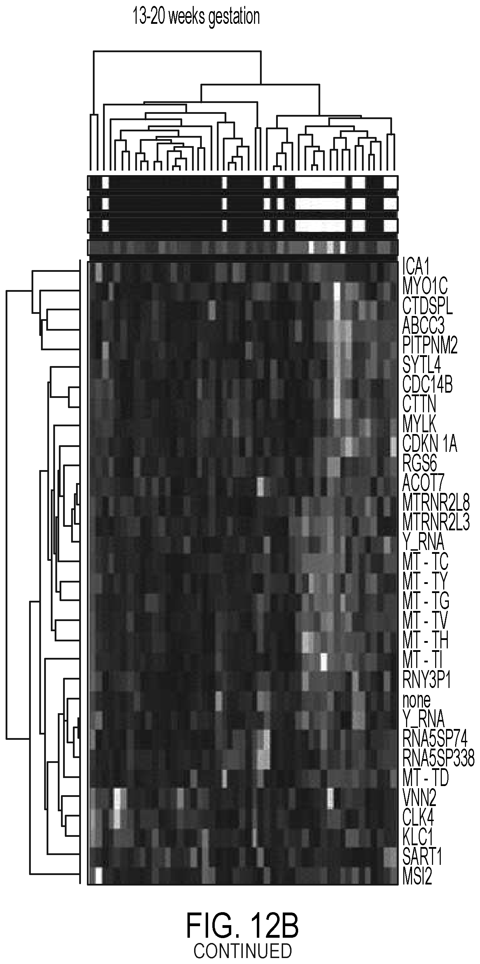

We first explored whether a common gene set could describe PE with or without severe features. We observed that we could separate PE from NT samples (Fig 12) irrespective of symptom severity and that PE with or without severe features as compared to NT had on average the same log FC (Fig 16E). With this in mind, we reran differential expression to identify a core set of genes that can distinguish PE (as a binary case group) from NT (See Design 2 in methods section "Differential expression analysis" for more details). This identified 330 genes that we used as an initial feature set for machine learning.

### References cited in application using number citation:

1. Centers for Disease Control and Prevention (CDC). Healthier mothers and babies. MMWR Morb. Mortal. Wkly. Rep. 48, 849-858 (1999).
2. Basso, O. et al. Trends in fetal and infant survival following preeclampsia. JAMA 296, 1357-1362 (2006).
3. Behrman, R. E., Butler, A. S. & Institute of Medicine (US) Committee on Understanding Premature Birth and Assuring Healthy Outcomes. Societal Costs of Preterm Birth. (2007).
4. Blencowe, H. et al. Born too soon: the global epidemiology of 15 million preterm births. Reprod Health 10 Suppl 1, S2 (2013).
5. Stevens, W. et al. Short-term costs of preeclampsia to the United States health care system. Am. J. Obstet. Gynecol. 217, 237-248.e16 (2017).
6. Beam, A. L. et al. Estimates of healthcare spending for preterm and low-birthweight infants in a commercially insured population: 2008-2016. J. Perinatol. 40, 1091-1099 (2020).
7. Say, L. et al. Global causes of maternal death: a WHO systematic analysis. Lancet Glob. Health 2, e323-33 (2014).
8. Petersen, E. E. et al. Vital Signs: Pregnancy-Related Deaths, United States, 2011-2015, and Strategies for Prevention, 13 States, 2013-2017. MMWR Morb. Mortal. Wkly. Rep. 68, 423-429 (2019).
9. McCarthy, F. P., Ryan, R. M. & Chappell, L. C. Prospective biomarkers in preterm preeclampsia: A review. Pregnancy hypertension 14, 72-78 (2018).
10. Roberge, S. et al. The role of aspirin dose on the prevention of preeclampsia and fetal growth restriction: systematic review and meta-analysis. Am. J. Obstet. Gynecol. 216, 110-120.e6 (2017).
11. Duley, L. The global impact of pre-eclampsia and eclampsia. Semin Perinatol 33, 130-137 (2009).
12. Hutcheon, J. A., Lisonkova, S. & Joseph, K. S. Epidemiology of pre-eclampsia and the other hypertensive disorders of pregnancy. Best Pract Res Clin Obstet Gynaecol 25, 391-403 (2011).
13. Abalos, E., Cuesta, C., Grosso, A. L., Chou, D. & Say, L. Global and regional estimates of preeclampsia and eclampsia: a systematic review. Eur. J. Obstet. Gynecol. Reprod. Biol. 170, 1-7 (2013).
14. Zhang, J., Meikle, S. & Trumble, A. Severe maternal morbidity associated with hypertensive disorders in pregnancy in the United States. Hypertens Pregnancy 22, 203-212 (2003).
15. Steegers, E. A. P., von Dadelszen, P., Duvekot, J. J. & Pijnenborg, R. Pre-eclampsia. Lancet 376, 631-644 (2010).
16. Li, X. et al. Hypertensive disorders of pregnancy and risks of adverse pregnancy outcomes: a retrospective cohort study of 2368 patients. J Hum Hypertens (2020). doi:10.1038/s41371-020-0312-x
17. Hansen, A. R., Barnés, C. M., Folkman, J. & McElrath, T. F. Maternal preeclampsia predicts the development of bronchopulmonary dysplasia. J. Pediatr. 156, 532-536 (2010).
18. Wang, A. et al. Circulating anti-angiogenic factors during hypertensive pregnancy and increased risk of respiratory distress syndrome in preterm neonates. J. Matern. Fetal Neonatal Med. 25, 1447-1452 (2012).
19. Bellamy, L., Casas, J.-P., Hingorani, A. D. & Williams, D. J. Pre-eclampsia and risk of cardiovascular disease and cancer in later life: systematic review and meta-analysis. BMJ 335, 974 (2007).
20. Ahmed, R., Dunford, J., Mehran, R., Robson, S. & Kunadian, V. Pre-eclampsia and future cardiovascular risk among women: a review. J. Am. Coll. Cardiol. 63, 1815-1822 (2014).
21. Vikse, B. E., Irgens, L. M., Leivestad, T., Skjaerven, R. & Iversen, B. M. Preeclampsia and the risk of end-stage renal disease. N. Engl. J. Med. 359, 800-809 (2008).
22. McDonald, S. D., Han, Z., Walsh, M. W., Gerstein, H. C. & Devereaux, P. J. Kidney disease after preeclampsia: a systematic review and meta-analysis. Am. J. Kidney Dis. 55, 1026-1039 (2010).
23. Hypertension in pregnancy. Report of the American College of Obstetricians and Gynecologists' Task Force on Hypertension in Pregnancy. Obstet. Gynecol. 122, 1122-1131 (2013).
24. Goel, A. et al. Epidemiology and mechanisms of de novo and persistent hypertension in the postpartum period. Circulation 132, 1726-1733 (2015).
25. Zeisler, H. et al. Predictive Value of the sFlt-1:PlGF Ratio in Women with Suspected Preeclampsia. N. Engl. J. Med. 374, 13-22 (2016).
26. Sovio, U. et al. Prediction of Preeclampsia Using the Soluble fms-Like Tyrosine Kinase 1 to Placental Growth Factor Ratio: A Prospective Cohort Study of Unselected Nulliparous Women. Hypertension 69, 731-738 (2017).
27. Gestational hypertension and preeclampsia: ACOG practice bulletin, number 222. Obstet. Gynecol. 135, e237-e260 (2020).
28. Roberge, S. et al. Early administration of low-dose aspirin for the prevention of preterm and term preeclampsia: a systematic review and meta-analysis. Fetal Diagn Ther 31, 141-146 (2012).
29. Henderson, J. T., Vesco, K. K., Senger, C. A., Thomas, R. G. & Redmond, N. Aspirin use to prevent preeclampsia and related morbidity and mortality: updated evidence report and systematic review for the US preventive services task force. JAMA 326, 1192-1206 (2021).
30. Whitehead, C. L., Walker, S. P. & Tong, S. Measuring circulating placental RNAs to noninvasively assess the placental transcriptome and to predict pregnancy complications. Prenat. Diagn. 36, 997-1008 (2016).
31. Munchel, S. et al. Circulating transcripts in maternal blood reflect a molecular signature of early-onset preeclampsia. Sci. Transl. Med. 12, (2020).
32. Tsang, J. C. H. et al. Integrative single-cell and cell-free plasma RNA transcriptomics elucidates placental cellular dynamics. Proc. Natl. Acad. Sci. USA 114, E7786-E7795 (2017).
33. Del Vecchio, G. et al. Cell-free DNA Methylation and Transcriptomic Signature Prediction of Pregnancies with Adverse Outcomes. Epigenetics 16, 642-661 (2021).
34. Phipps, E. A., Thadhani, R., Benzing, T. & Karumanchi, S. A. Pre-eclampsia: pathogenesis, novel diagnostics and therapies. Nat. Rev. Nephrol. 15, 275-289 (2019).
35. Pennington, K. A., Schlitt, J. M., Jackson, D. L., Schulz, L. C. & Schust, D. J. Preeclampsia: multiple approaches for a multifactorial disease. Dis. Model. Mech. 5, 9-18 (2012).
36. Burton, G. J., Redman, C. W., Roberts, J. M. & Moffett, A. Pre-eclampsia: pathophysiology and clinical implications. BMJ 366, 12381 (2019).
37. von Dadelszen, P., Magee, L. A. & Roberts, J. M. Subclassification of preeclampsia. Hypertens Pregnancy 22, 143-148 (2003).
38. Huppertz, B. Placental origins of preeclampsia: challenging the current hypothesis. Hypertension 51, 970-975 (2008).
39. Raymond, D. & Peterson, E. A critical review of early-onset and late-onset preeclampsia. Obstet Gynecol Surv 66, 497-506 (2011).
40. Chaiworapongsa, T. et al. Differences and similarities in the transcriptional profile of peripheral whole blood in early and late-onset preeclampsia: insights into the molecular basis of the phenotype of preeclampsiaa. J Perinat Med 41, 485-504 (2013).
41. Leavey, K. et al. Unsupervised placental gene expression profiling identifies clinically relevant subclasses of human preeclampsia. Hypertension 68, 137-147 (2016).
42. Benton, S. J., Leavey, K., Grynspan, D., Cox, B. J. & Bainbridge, S. A. The clinical heterogeneity of preeclampsia is related to both placental gene expression and placental histopathology. Am. J. Obstet. Gynecol. 219, 604.e1-604.e25 (2018).
43. Koh, W. et al. Noninvasive in vivo monitoring of tissue-specific global gene expression in humans. Proc. Natl. Acad. Sci. USA 111, 7361-7366 (2014).
44. Vorperian, S. K., Moufarrej, M. N., Consortium, T. S. & Quake, S. R. Cell types of origin in the cell free transcriptome in human health and disease. BioRxiv (2021). doi:10.1101/2021.05.05.441859
45. Gillich, A. et al. Capillary cell-type specialization in the alveolus. Nature 586, 785-789 (2020).
46. Arck, P. C. & Hecher, K. Fetomaternal immune cross-talk and its consequences for maternal and offspring's health. Nat. Med. 19, 548-556 (2013).
47. Uzun, A., Triche, E. W., Schuster, J., Dewan, A. T. & Padbury, J. F. dbPEC: a comprehensive literature-based database for preeclampsia related genes and phenotypes. Database (Oxford) 2016, (2016).
48. Ostlund, I., Haglund, B. & Hanson, U. Gestational diabetes and preeclampsia. Eur. J. Obstet. Gynecol. Reprod. Biol. 113, 12-16 (2004).
49. Schneider, S., Freerksen, N., Röhrig, S., Hoeft, B. & Maul, H. Gestational diabetes and preeclampsia--similar risk factor profiles? Early Hum. Dev. 88, 179-184 (2012).
50. Nerenberg, K. A. et al. Risks of gestational diabetes and preeclampsia over the last decade in a cohort of Alberta women. J Obstet Gynaecol Can 35, 986-994 (2013).
51. Weissgerber, T. L. & Mudd, L. M. Preeclampsia and diabetes. Curr Diab Rep 15, 9 (2015).
52. Rey, E. & Couturier, A. The prognosis of pregnancy in women with chronic hypertension. Am. J. Obstet. Gynecol. 171, 410-416 (1994).
53. McCowan, L. M., Buist, R. G., North, R. A. & Gamble, G. Perinatal morbidity in chronic hypertension. Br J Obstet Gynaecol 103, 123-129 (1996).
54. Sibai, B. M. et al. The impact of prior preeclampsia on the risk of superimposed preeclampsia and other adverse pregnancy outcomes in patients with chronic hypertension. Am. J. Obstet. Gynecol. 204, 345.e1-6 (2011).
55. Uhlen, M. et al. A genome-wide transcriptomic analysis of protein-coding genes in human blood cells. Science 366, (2019).
56. Han, X. et al. Differential dynamics of the maternal immune system in healthy pregnancy and preeclampsia. Front. Immunol. 10, 1305 (2019).
57. Ander, S. E., Diamond, M. S. & Coyne, C. B. Immune responses at the maternal-fetal interface. Sci. Immunol. 4, (2019).
58. Szarka, A., Rigó, J., Lázár, L., Beko, G. & Molvarec, A. Circulating cytokines, chemokines and adhesion molecules in normal pregnancy and preeclampsia determined by multiplex suspension array. BMC Immunol. 11, 59 (2010).
59. Lisonkova, S. & Joseph, K. S. Incidence of preeclampsia: risk factors and outcomes associated with early- versus late-onset disease. Am. J. Obstet. Gynecol. 209, 544.e1-544.e12 (2013).
60. Marić, I. et al. Early prediction of preeclampsia via machine learning. American Journal of Obstetrics & Gynecology MFM 2, 100100 (2020).
61. Robinson, M. D. & Oshlack, A. A scaling normalization method for differential expression analysis of RNA-seq data. Genome Biol. 11, R25 (2010).
62. Pan, W. Development of diagnostic methods using cell-free nucleic acids. (Stanford University, 2016).
63. Moufarrej, M. N., Wong, R. J., Shaw, G. M., Stevenson, D. K. & Quake, S. R. Investigating Pregnancy and Its Complications Using Circulating Cell-Free RNA in Women's Blood During Gestation. Front. Pediatr. 8, 605219 (2020).
64. Vento-Tormo, R. et al. Single-cell reconstruction of the early maternal-fetal interface in humans. Nature 563, 347-353 (2018).
65. Suryawanshi, H. et al. A single-cell survey of the human first-trimester placenta and decidua. Sci. Adv. 4, eaau4788 (2018).
66. Aizarani, N. et al. A human liver cell atlas reveals heterogeneity and epithelial progenitors. Nature 572, 199-204 (2019).
67. Stewart, B. J. et al. Spatiotemporal immune zonation of the human kidney. Science 365, 1461-1466 (2019).
68. Litviňuková, M. et al. Cells of the adult human heart. Nature 588, 466-472 (2020).
69. Mathys, H. et al. Single-cell transcriptomic analysis of Alzheimer's disease. Nature 570, 332-337 (2019)*.*
70. Raudvere, U. et al. g:Profiler: a web server for functional enrichment analysis and conversions of gene lists (2019 update). Nucleic Acids Res. 47, W191-W198 (2019).
71. Brown, L. D., Cai, T. T. & DasGupta, A. Interval Estimation for a Binomial Proportion. Stat Sci 16, 101-133 (2001).
72. Ibarra, A. et al. Non-invasive characterization of human bone marrow stimulation and reconstitution by cell-free messenger RNA sequencing. Nat. Commun. 11, 400 (2020).

The following is a listing of an illustrative human cDNA sequence for each of genes CSF3R, SNCA, BNIP3L, HEMGN, AKNA, IGF2, GSPT1, FECH, RPS15, OAZ1, and MARCH2. The polypeptide sequence is designated using an ENSEMBL designation number. This listing provides examples of cDNA sequences only. Other RNA and protein expression products of human CSF3R, SNCA, BNIP3L, HEMGN, AKNA, IGF2, GSPT1, FECH, RPS15, OAZ1, and MARCH2 genes are known and not limited to these specific examples.
**Nucleotide sequence encoding CSF3R illustrative polypeptide sequence ENSP00000362195**
   (SEQ IDN NO: 1) 
ATGGCAAGGCTGGGAAACTGCAGCCTGACTTGGGCTGCCCTGATCATCCTGCTGCTCCCCGGAAGTCTGG
AGGAGTGCGGGCACATCAGTGTCTCAGCCCCCATCGTCCACCTGGGGGATCCCATCACAGCCTCCTGCAT
CATCAAGCAGAACTGCAGCCATCTGGACCCGGAGCCACAGATTCTGTGGAGACTGGGAGCAGAGCTTCAG
CCCGGGGGCAGGCAGCAGCGTCTGTCTGATGGGACCCAGGAATCTATCATCACCCTGCCCCACCTCAACC
ACACTCAGGCCTTTCTCTCCTGCTGCCTGAACTGGGGCAACAGCCTGCAGATCCTGGACCAGGTTGAGCT
GCGCGCAGGCTACCCTCCAGCCATACCCCACAACCTCTCCTGCCTCATGAACCTCACAACCAGCAGCCTC
ATCTGCCAGTGGGAGCCAGGACCTGAGACCCACCTACCCACCAGCTTCACTCTGAAGAGTTTCAAGAGCC
GGGGCAACTGTCAGACCCAAGGGGACTCCATCCTGGACTGCGTGCCCAAGGACGGGCAGAGCCACTGCTG
CATCCCACGCAAACACCTGCTGTTGTACCAGAATATGGGCATCTGGGTGCAGGCAGAGAATGCGCTGGGG
ACCAGCATGTCCCCACAACTGTGTCTTGATCCCATGGATGTTGTGAAACTGGAGCCCCCCATGCTGCGGA
CCATGGACCCCAGCCCTGAAGCGGCCCCTCCCCAGGCAGGCTGCCTACAGCTGTGCTGGGAGCCATGGCA
GCCAGGCCTGCACATAAATCAGAAGTGTGAGCTGCGCCACAAGCCGCAGCGTGGAGAAGCCAGCTGGGCA
 
CTGGTGGCCCCCTCCCCTTGGAGGCCCTTCAGTATGAGCTCTGCGGCTCCTCCCAGCCACGGCCTACA
CCCTGCAGATACGCTGCATCCGCTGGCCCCTGCCTGGCCACTGGAGCGACTGGAGCCCCAGCCTGGAGCT
GAGAACTACCGAACGGCCCCCACTGTCAGACTGGACACATGGTGGCGGCAGAGGCAGCTGGACCCCAGG
ACAGTGCAGCTGTTCTGGAAGCCAGTGCCCCTGGAGGAAGACAGCGGACGGATCCAAGGTTATGTGGTTT
CTTGGAGACCCTCAGGCCAGGCTGGGCCATCCTGCCCCTCTGCAACACCACAGAGCTCAGCTGCACCTT
CCACCTGCCTTCAGAAGCCCAGGAGGTGGCCCTTGTGGCCTATAACTCAGCCGGGACCTCTCGTCCCACT
CCGGTGGTCTTCTCAGAAAGCAGAGGCCCAGCTCTGACCAGACTCCATGCCATGGCCCGAGACCCTCACA
GCCTCTGGGTAGGCTGGGAGCCCCCCAATCCATGGCCTCAGGGCTATGTGATTGAGTGGGGCCTGGGCCC
CCCCAGCGCGAGCAATAGCAACAAGACCTGGAGGATGGAACAGAATGGGAGAGCCACGGGGTTTCTGCTG
AAGGAGAACATCAGGCCCTTTCAGCTCTATGAGATCATCGTGACTCCCTTGTACCAGGACACCATGGGAC
CCTCCCAGCATGTCTATGCCTACTCTCAAGAAATGGCTCCCTCCCATGCCCCAGAGCTGCATCTAAAGCA
CATTGGCAAGACCTGGGCACAGCTGGAGTGGGTGCCTGAGCCCCCTGAGCTGGGGAAGAGCCCCCTTACC
CACTACACCATCTTCTGGACCAACGCTCAGAACCAGTCCTTCTCCGCCATCCTGAATGCCTCCTCCCGTG
GCTTTGTCCTCCATGGCCTGGAGCCCGCCAGTCTGTATCACATCCACCTCATGGCTGCCAGCCAGGCTGG
GGCCACCAACAGTACAGTCCTCACCCTGATGACCTTGACCCCAGAGGGGTCGGAGCTACACATCATCCTG
GGCCTGTTCGGCCTCCTGCTGTTGCTCACCTGCCTCTGTGGAACTGCCTGGCTCTGTTGCAGCCCCAACA
GGAAGAATCCCCTCTGGCCAAGTGTCCCAGACCCAGCTCACAGCAGCCTGGGCTCCTGGGTGCCCACAAT
CATGGAGGAGCTGCCCGGACCCAGACAGGGACAGTGGCTGGGGCAGACATCTGAAATGAGCCGTGCTCTC
ACCCCACATCCTTGTGTGCAGGATGCCTTCCAGCTGCCCGGCCTTGGCACGCCACCCATCACCAAGCTCA
CAGTGCTGGAGGAGGATGAAAAGAAGCCGGTGCCCTGGGAGTCCCATAACAGCTCAGAGACCTGTGGCCT
CCCCACTCTGGTCCAGACCTATGTGCTCCAGGGGGACCCAAGAGCAGTTTCCACCCAGCCCCAATCCCAG
TCTGGCACCAGCGATCAGGTCCTTTATGGGCAGCTGCTGGGCAGCCCCACAAGCCCAGGGCCAGGGCACT
ATCTCCGCTGTGACTCCACTCAGCCCCTCTTGGCGGGCCTCACCCCCAGCCCCAAGTCCTATGAGAACCT
CTGGTTCCAGGCCAGCCCCTTGGGGACCCTGGTAACCCCAGCCCCAAGCCAGGAGGACGACTGTGTCTTT
GGGCCACTGCTCAACTTCCCCCTCCTGCAGGGGATCCGGGTCCATGGGATGGAGGCGCTGGGGAGCTTCT
AG
**Nucleotide sequence encoding SNCA illustrative polypeptide sequence ENSP00000378442**
   (SEQ ID NO: 2) 
ATGGATGTATTCATGAAAGGACTTTCAAAGGCCAAGGAGGGAGTTGTGGCTGCTGCTGAGAAAACCAAAC
AGGGTGTGGCAGAAGCAGCAGGAAAGACAAAAGAGGGTGTTCTCTATGTAGGCTCCAAAACCAAGGAGGG
AGTGGTGCATGGTGTGGCAACAGTGGCTGAGAAGACCAAAGAGCAAGTGACAAATGTTGGAGGAGCAGTG
GTGACGGGTGTGACAGCAGTAGCCCAGAAGACAGTGGAGGGAGCAGGGAGCATTGCAGCAGCCACTGGCT
TTGTCAAAAAGGACCAGTTGGGCAAGAATGAAGAAGGAGCCCCACAGGAAGGAATTCTGGAAGATATGCC
TGTGGATCCTGACAATGAGGCTTATGAAATGCCTTCTGAGGAAGGGTATCAAGACTACGAACCTGAAGCC
TAA
**Nucleotide sequence encoding BNIP3L illustrative polypeptide sequence**
   **ENSP00000370003**
   (SEQ ID NO: 3) 
ATGTCGTCCCACCTAGTCGAGCCGCCGCCGCCCCTGCACAACAACAACAACAACTGCGAGGAAAATGAGC
AGTCTCTGCCCCCGCCGGCCGGCCTCAACAGTTCCTGGGTGGAGCTACCCATGAACAGCAGCAATGGCAA
TGATAATGGCAATGGGAAAAATGGGGGGCTGGAACACGTACCATCCTCATCCTCCATCCACAATGGAGAC
ATGGAGAAGATTCTTTTGGATGCACAACATGAATCAGGACAGAGTAGTTCCAGAGGCAGTTCTCACTGTG
ACAGCCCTTCGCCACAAGAAGATGGGCAGATCATGTTTGATGTGGAAATGCACACCAGCAGGGACCATAG
CTCTCAGTCAGAAGAAGAAGTTGTAGAAGGAGAGAAGGAAGTCGAGGCTTTGAAGAAAAGTGCGGACTGG
GTATCAGACTGGTCCAGTAGACCCGAAAACATTCCACCCAAGGAGTTCCACTTCAGACACCCTAAACGTT
CTGTGTCTTTAAGCATGAGGAAAAGTGGAGCCATGAAGAAAGGGGGTATTTTCTCCGCAGAATTTCTGAA
 
GGTGTTCATTCCATCTCTCTTCCTTTCTCATGTTTTGGCTTTGGGGCTAGGCATCTATATTGGAAAGCGA
CTGAGCACACCCTCTGCCAGCACCTACTGA
**Nucleotide sequence encoding HEMGN illustrative polypeptide sequence**
   **ENSP00000259456**
   SEQ ID NO: 4 
ATGGATTTGGGAAAGGACCAATCTCATTTGAAGCACCATCAGACACCTGACCCTCATCAAGAAGAGAACC
ATTCTCCAGAAGTCATTGGAACCTGGAGTTTGAGAAACAGAGAACTACTTAGAAAAAGAAAAGCTGAAGT
GCATGAAAAGGAAACATCACAATGGCTATTTGGAGAACAGAAAAAACGCAAGCAGCAGAGAACAGGAAAA
GGAAATCGAAGAGGCAGAAAGAGACAACAAAACACAGAATTGAAGGTGGAGCCTCAGCCACAGATAGAAA
AGGAAATAGTGGAGAAAGCACTGGCACCTATAGAGAAAAAAACTGAGCCACCTGGGAGCATAACCAAAGT
ATTTCCTTCAGTAGCCTCCCCGCAAAAAGTTGTGCCTGAGGAACACTTTTCTGAAATATGTCAAGAAAGT
AACATATATCAGGAGAATTTTTCTGAGTACCAAGAAATAGCAGTACAAAACCATTCTTCTGAAACATGCC
AACATGTGTCTGAACCTGAAGACCTCTCTCCTAAAATGTACCAAGAAATATCTGTACTTCAAGACAATTC
TTCCAAAATATGCCAAGACATGAAGGAACCTGAAGACAACTCTCCTAACACATGCCAAGTAATATCTGTA
ATTCAAGACCATCCTTTCAAAATGTACCAAGATATGGCTAAACGAGAAGATCTGGCTCCTAAAATGTGCC
AAGAAGCTGCTGTACCCAAAATCCTTCCTTGTCCAACATCTGAAGACACAGCTGATCTGGCAGGATGCTC
TCTTCAAGCATATCCAAAACCAGATGTGCCTAAAGGCTATATTCTTGACACAGACCAAAATCCAGCAGAA
CCAGAGGAATACAATGAAACAGATCAAGGAATAGCTGAGACAGAAGGCCTTTTTCCTAAAATACAAGAAA
TAGCTGAGCCTAAAGACCTTTCTACAAAAACACACCAAGAATCAGCTGAACCTAAATACCTTCCTCATAA
AACATGTAACGAAATTATTGTGCCTAAAGCCCCCTCTCATAAAACAATCCAAGAAACACCTCATTCTGAA
GACTATTCAATTGAAATAAACCAAGAAACTCCTGGGTCTGAAAAATATTCACCTGAAACGTATCAAGAAA
TACCTGGGCTTGAAGAATATTCACCTGAAATATACCAAGAAACATCCCAGCTTGAAGAATATTCACCTGA
AATATACCAAGAAACACCGGGGCCTGAAGACCTCTCTACTGAGACATATAAAAATAAGGATGTGCCTAAA
GAATGCTTTCCAGAACCACACCAAGAAACAGGTGGGCCCCAAGGCCAGGATCCTAAAGCACACCAGGAAG
ATGCTAAAGATGCTTATACTTTTCCTCAAGAAATGAAAGAAAAACCCAAAGAAGAGCCAGGAATACCAGC
AATTCTGAATGAGAGTCATCCAGAAAATGATGTCTATAGTTATGTTTTGTTTTAA
**Nucleotide sequence encoding AKNA illustrative polypeptide sequence ENSP00000363201**
   (SEQ ID NO: 5) 
ATGGCCAGCTCGGAGACTGAGATCCGCTGGGCTGAGCCTGGCCTGGGGAAGGGCCCCCAGCGGCGGCGCT
GGGCCTGGGCCGAGGACAAGAGGGATGTGGATAGAAGTAGTTCACAAAGCTGGGAAGAAGAGAGACTCTT
TCCCAATGCCACCAGCCCCGAGCTCCTAGAGGACTTCCGCCTGGCCCAGCAGCACCTGCCGCCCCTGGAG
TGGGACCCACACCCGCAGCCCGATGGCATCAGGATTCCGAGTCAGGAGAGACTTCGGGAGAAGAGGCTG
AAGCAGAGGATGTGGACAGCCCAGCAAGTTCCCATGAGCCTCTTGCCTGGCTCCCCCAGCAGGGCCGTCA
GCTGGACATGACTGAAGAGGAGCCAGATGGGACCCTCGGAAGTCTGGAGGTTGAGGAGGCTGGAGAGAGC
TCCTCAAGGTTGGGGTATGAGGCTGGTCTCAGCTTGGAAGGCCATGGAAACACCAGCCCCATGGCTCTTG
GGCATGGTCAGGCCAGGGGCTGGGTGGCTTCTGGCGAACAAGCCAGTGGGGACAAACTTTCTGAACATTC
CGAGGTCAACCCATCCGTTGAACTCAGCCCGGCAAGGTCCTGGAGCAGTGGGACAGTGAGCCTCGACCAC
CCTAGTGACAGCCTTGATTCTACCTGGGAAGGAGAGACCGATGGCCCCCAGCCCACTGCCCTGGCAGAAA
CCTTGCCAGAGGGCCCCAGCCACCACCTCCTAAGCCCAGATGGCAGAACTGGAGGCAGTGTTGCTCGGGC
AACCCCCATGGAATTCCAGGACTCCTCAGCTCCCCCAGCCCAGAGTCCGCAGCATGCCACAGATAGATGG
AGGAGAGAAACGACCAGATTCTTCTGCCCTCAGCCCAAGGAACACATCTGGAAGCAGACAAAGACGTCAC
CTAAGCCACTCCCTTCCCGATTCATTGGCTCCATCAGCCCCCTGAATCCCCAGCCCAGGCCAACGCGGCA
GGGCAGGCCGCTGCCCAGACAGGGAGCCACTCTGGCTGGCCGCTCCTCTTCTAATGCCCCCAAGTATGGC
CGGGGGCAGTTGAACTACCCACTCCCTGATTTCTCCAAGGTAGGGCCCCGGGTGAGATTCCCCAAAGATG
AGAGCTACCGTCCCCCCAAGTCCAGAAGCCACAACAGGAAGCCTCAGGCCCCTGCCAGGCCCCTCATCTT
CAAGTCTCCAGCTGAGATTGTGCAGGAGGTGCTGTTGAGCAGTGGAGAAGCAGCCCTGGCAAAGGACACG
 
CCTCCTGCCCACCCTATCACCAGGGTACCCCAAGAATTTCAGACGCCTGAGCAAGCCACTGAGCTGGTCC
ATCAGCTCCAGGAAGACTACCACAGGCTCCTCACCAAGTACGCTGAGGCCGAGAACACCATTGACCAGCT
ACGCCTCGGGGCCAAGGTGAACCTGTTCTCTGACCCACCCCAGCCCAACCACAGCATCCACACGGGAATG
GTGCCCCAGGGGACCAAGGTCTTGTCCTTCACCATCCCACAGCCCCGCTCTGCAGAGTGGTGGCCGGGCC
CGGCCGAGGACCCCCAGGCCTCTGCGGCCTCAGGGTGGCCATCAGCTCGAGGAGACTTGAGCCCCTCCTC
GCTTACCAGCATGCCCACCCTGGGGTGGCTTCCGGAGAACCGGGACATCTCTGAGGACCAGTCCTCAGCA
GAGCAGACCCAGGCACTGGCTTCTCAGGCCAGCCAGTTCCTGGCCAAGGTGGAGTCCTTTGAAAGACTGA
TACAGGCAGGACGTCTCATGCCCCAGGACCAAGTCAAGGGCTTCCAGCGGCTGAAGGCTGCCCACGCGGC
CCTAGAGGAGGAGTACCTGAAGGCTTGTCGGGAGCAACACCCTGCCCAGCCGCTTGCCGGCTCCAAGGGG
ACGCCTGGAAGATTTGATCCTCGCAGGGAGCTGGAGGCAGAGATATACCGTCTGGGAAGCTGCCTGGAAG
AGCTGAAGGAACACATAGACCAGACCCAGCAAGAGCCTGAGCCGCCCGGGTCAGACTCAGCTCTGGACAG
CACCCCAGCCCTGCCCTGCCTCCATCAGCCAACGCACCTGCCTGCTCCTTCTGGACAAGCCCCCATGCCA
GCCATCAAGACCTCCTGCCCTGAGCCTGCTACCACCACTGCCGCCGCCAGCACTGGCCCCTGCCCATTGC
ACGTAAATGTGGAGGTGAGCTCTGGCAACAGTGAGGTGGAGGACAGGCCACAGGACCCCCTGGCCCGACT
CAGGCACAAGGAGCTGCAGATGGAGCAAGTTTACCATGGCCTCATGGAGCGGTACCTCAGTGTGAAGTCT
CTCCCAGAAGCCATGAGAATGGAGGAGGAGGAAGAAGGAGAGGAGGAGGAGGAAGAGGGGGGAGGTG
ACTCCCTGGAAGTTGATGGGGTGGCTGCAACTCCAGGGAAAGCAGAGGCCACCAGGGTCCTCCCAAGGCA
GTGCCCGGTGCAGGCTGAGAAAAGTCATGGGGCTCCCCTGGAGGAGGCCACGGAGAAGATGGTATCTATG
AAGCCACCAGGTTTCCAGGCATCCCTGGCTAGAGACGGGCACATGTCAGGCCTGGGCAAGGCTGAGGCAG
CCCCTCCAGGCCCTGGCGTGCCACCCACCCTCCAGGCACCAAGTCCGCAGCATCCCACCAAAGTAGTAT
GACCAGCCTGGAGGGAAGCGGCATCTCTGAGCGCCTTCCACAGAAGCCTTTGCACCGAGGCGGTGGGCCC
CACCTGGAGGAGACCTGGATGGCGTCCCCAGAGACAGACAGTGGCTTTGTGGGCTCAGAAACAAGCAGAG
TTTCACCCCTCACCCAGACTCCAGAGCACCGGCTCTCCCACATCAGCACAGCAGGAACATTAGCCCAGCC
CTTTGCTGCATCTGTGCCCAGGGATGGAGCTTCCTACCCCAAGGCCAGGGGTTCTCTGATTCCCAGAAGA
GCCACAGAGCCCAGCACACCCCGGAGCCAAGCACAGAGGTACCTCTCCAGCCCAAGTGGGCCTCTCCGGC
AGAGGGCACCCAACTTCAGCCTGGAGCGGACACTGGCAGCCGAGATGGCGGTTCCTGGCTCAGAGTTTGA
GGGGCACAAACGGATTTCTGAACAGCCCCTTCCCAACAAGACAATCAGCCCACCCCCAGCCCCCGCCCCT
GCCGCTGCGCCTCTACCCTGTGGACCAACAGAGACCATCCCCAGCTTCCTGCTCACCAGGGCAGGGCGAG
ACCAGGCCATCTGTGAGCTGCAAGAAGAGGTGTCCCGGCTTCGTCTGCGGCTGGAAGACAGCCTGCACCA
GCCACTCCAGGGCAGCCCGACACGCCCAGCATCTGCCTTTGACCGCCCCGCCCGGACCCGCGGCCGGCCA
GCAGACTCCCCAGCCACCTGGGGCTCCCATTATGGCAGTAAATCCACAGAGAGATTGCCTGGTGAGCCTA
GAGGTGAAGAGCAGATTGTCCCTCCAGGAAGGCAGCGAGCCAGGTCTTCCTCAGTGCCTCGGGAGGTGCT
CCGACTGTCCCTGAGTTCAGAATCTGAGCTGCCCTCCCTACCACTGTTCTCTGAGAAGAGCAAGACCACC
AAGGACAGTCCACAGGCAGCTCGGGATGGAAAGAGAGGGGTGGGCAGTGCTGGATGGCCAGACAGGGTCA
CCTTCCGGGGCCAATACACAGGCCACGAATACCATGTTCTGTCCCCTAAGGCGGTCCCAAAAGGCAATGG
CACAGTCTCCTGTCCCCACTGCCGGCCCATTAGGACCCAGGATGCGGGTGGTGCTGTCACAGGGGACCCA
CTGGGACCGCCTCCCGCTGATACCCTTCAGTGTCCCCTGTGTGGTCAAGTTGGGTCTCCCCCAGAGGCAG
ATGGTCCAGGCTCAGCCACCTCTGGGGCAGAGAAGGCCACCACGAGGAGAAAAGCATCTTCAACTCCCAG
CCCCAAGCAGAGGAGCAAGCAGGCGGGGTCGTCGCCACGCCCACCCCCCGGACTGTGGTATCTGGCAACA
GCGCCCCCAGCACCAGCCCCTCCAGCCTTTGCCTACATCTCCTCGGTTCCCATCATGCCTTATCCACCTG
CCGCTGTGTACTATGCGCCTGCAGGACCTACCTCAGCCCAACCAGCTGCCAAGTGGCCGCCCACAGCCTC
TCCCCCACCAGCCCGGAGACACCGGCACTCCATCCAGCTCGACCTGGGCGACCTAGAGGAGCTCAACAAG
GCCCTGAGCCGGGCCGTGCAGGCTGCCGAGAGCGTCCGCTCTACCACCAGGCAGATGAGAAGCTCGCTGT
CAGCCGACCTGCGCCAGGCTCACAGCCTGCGGGGCTCCTGCCTCTTCTGA
**Nucleotide sequence encoding IGF2 illustrative polypeptide sequence ENSP00000414497**
   (SEQ ID NO: 6) 
ATGGAATCCCAATGGGAAGTCGATGCTGGTGCTTCTCACCTTCTTGGCCTTCGCCTCGTGCTGCATTG
CTGCTTACCGCCCCAGTGAGACCCTGTGCGGCGGGGAGCTGGTGGACACCCTCCAGTTCGTCTGTGGGGA
CCGCGGCTTCTACTTCAGCAGGCCCGCAAGCCGTGTGAGCCGTCGCAGCCGTGGCATCGTTGAGGAGTGC
 
TGTTTCCGCAGCTGTGACCTGGCCCTCCTGGAGACGTACTGTGCTACCCCCGCCAAGTCCGAGAGGACG
TGTCGACCCCTCCGACCGTGCTTCCGGACAACTTCCCCAGATACCCCGTGGGCAAGTTCTTCCAATATGA
CACCTGGAAGCAGTCCACCCAGCGCCTGCGCAGGGGCCTGCCTGCCCTCCTGCGTGCCCGCCGGGGTCAC
GTGCTCGCCAAGGAGCTCGAGGCGTTCAGGGAGGCCAAACGTCACCGTCCCCTGATTGCTCTACCCACCC
AAGACCCCGCCCACGGGGGCGCCCCCCCAGAGATGGCCAGCAATCGGAAGTGA
**Nucleotide sequence encoding GSPT1 illustrative polypeptide sequence ENSP00000398131**
   (SEQ ID NO: 7) 
ATGGATCCGGGCAGTGGCGGCGGCGGCGGCGGCGGCGGCGGCGGAGCAGCAGCGGCAGCAGCAGCA
GCGACTCGGCGCCTGACTGCTGGGACCAGGCGGACATGGAAGCCCCGGGCCGGGCCCTTGCGGCGGCGG
CGGCTCCCTGGCGGCGGCGGCCGAGGCCCAGCGGGAGAACCTCAGCGCGGCCTTCAGCCGGCAACTCAAC
GTCAACGCCAAGCCCTTCGTGCCCAACGTCCACGCCGCCGAGTTCGTGCCGTCCTTCCTGCGGGGCCCGG
CAGCGCCGCCACCCCCAGTTGGCGGCGCCGCCAATAACCACGGAGCCGGCAGCGGCGCGGAGGCCGTGC
GGCACCTGTGGAATCCTCTCAAGAGGAACAGTCATTGTGTGAAGGTTCAAATTCAGCTGTTAGCATGGAA
CTTTCAGAACCTATTGTAGAAAATGGAGAGACAGAAATGTCTCCAGAAGAATCATGGGAGCACAAAGAAG
AAATAAGTGAAGCAGAGCCAGGGGGTGGTTCCTTGGGAGATGGAAGGCCGCCAGAGGAAAGTGCCCATGA
AATGATGGAGGAGGAAGAGGAAATCCCAAAACCTAAGTCTGTGGTTGCACCGCCAGGTGCTCCTAAGAAA
GAGCATGTAAATGTAGTATTCATTGGGCACGTAGATGCTGGCAAGTCAACCATTGGAGGACAAATAATGT
ATTTGACTGGAATGGTTGACAAAAGGACGCTTGAAAAGTATGAAAGAGAAGCTAAAGAGAAAAACAGAGA
AACTTGGTACTTGTCTTGGGCCTTAGACACAAATCAGGAAGAACGAGACAAGGGTAAAACAGTAGAAGTG
GGTCGTGCCTATTTTGAAACCGAAAAGAAGCATTTCACAATTCTAGATGCCCCTGGCCACAAGAGTTTTG
TCCCAAATATGATTGGTGGTGCCTCTCAAGCTGATTTGGCTGTGCTGGTAATCTCAGCCAGGAAAGGAGA
GTTTGAAACTGGATTTGAAAAAGGAGGACAGACAAGAGAACATGCAATGTTGGCAAAGACAGCAGGTGTA
AAACACCTAATTGTGCTAATTAATAAGATGGATGATCCAACAGTAAATTGGAGCAATGAGAGATATGAAG
AATGTAAGGAGAAACTAGTGCCATTTTTGAAAAAAGTTGGCTTCAATCCCAAAAAGGACATTCACTTTAT
GCCCTGCTCAGGACTTACTGGAGCAAATCTCAAAGAGCAGTCGGATTTCTGTCCTTGGTACATTGGATTA
CCGTTTATTCCATATCTGGATAATTTGCCGAACTTCAATAGATCAGTTGATGGACCAATCAGGCTGCCAA
TTGTGGATAAGTACAAGGATATGGGCACTGTGGTCCTGGGAAAGCTGGAATCAGGATCTATTTGTAAAGG
CCAGCAGCTTGTGATGATGCCAAACAAGCACAACGTGGAAGTTCTTGGAATACTTTCCGATGATGTAGAG
ACTGATACCGTAGCCCCAGGTGAAAACCTCAAAATCAGACTGAAAGGAATTGAAGAAGAGGAGATTCTTC
CAGGGTTTATACTTTGTGATCCTAATAATCTTTGTCATTCTGGACGCACATTTGATGCCCAGATAGTGAT
TATAGAGCACAAATCCATCATCTGCCCAGGCTATAATGCGGTGCTGCATATTCATACCTGTATTGAGGAG
GTGGAAATAACAGCCTTAATCTGCTTGGTAGACAAAAAATCAGGAGAAAAAAGTAAGACCCGACCCCGTT
TTGTCAAACAAGATCAAGTATGCATTGCTCGCTTAAGGACAGCAGGAACCATCTGCCTTGAGACCTTTAA
AGACTTCCCTCAGATGGGTCGTTTCACCTTAAGAGATGAGGGTAAGACCATTGCAATTGGAAAAGTTCTG
AAACTGGTTCCAGAGAAAGACTAA
**Nucleotide sequence encoding FECH illustrative polypeptide sequence ENSP00000498358**
   (SEQ ID NO: 8) 
ATGCGTTCACTCGGCGCAAACATGGCTGCGGCCCTGCGCGCCGCGGGCGTCCTGCTCCGCGATCCGCTGG
CATCCAGCAGCTGGAGGGTCTGTCAGCCATGGAGGTGGAAGTCAGGTGCAGCTGCAGCGGCCGTCACCAC
AGAAACAGCCCAGCATGCCCAGGGTGCAAAACCTCAAGTTCAACCGCAGAAGAGGTATGAGTCTAACATC
AGGAAGCCGAAAACTGGAATATTAATGCTAAACATGGGAGGCCCTGAAACTCTTGGAGATGTTCACGACT
TCCTTCTGAGACTCTTCTTGGACCGAGACCTCATGACACTTCCTATTCAGAATAAGCTGGCACCATTCAT
CGCCAAACGCCGAACCCCCAAGATTCAAGAGCAGTACCGCAGGATTGGAGGCGGATCCCCCATCAAGATA
TGGACTTCCAAGCAGGGAGAGGGCATGGTGAAGCTGCTGGATGAATTGTCCCCCAACACAGCCCCTCACA
AATACTATATTGGATTTCGGTACGTCCATCCTTTAACAGAAGAAGCAATTGAAGAGATGGAGAGAGATGG
CCTAGAAAGGGCTATTGCTTTCACACAGTATCCACAGTACAGCTGCTCCACCACAGGCAGCAGCTTAAAT
GCCATTTACAGATACTATAATCAAGTGGGACGGAAGCCCACGATGAAGTGGAGCACTATTGACAGGTGGC
 
CCACACATCACCTCCTCATCCAGTGCTTTGCAGATCATATTCTAAAGGAACTGGACCATTTTCCACTTGA
GAAGAGAAGCGAGGTGGTCATTCTGTTTTCTGCTCACTCACTGCCCATGTCTGTGGTCAACAGAGGCGAC
CCATATCCTCAGGAGGTAAGCGCCACTGTCCAAAAAGTCATGGAAAGGCTGGAGTACTGCAACCCCTACC
GACTGGTGTGGCAATCCAAGGTTGGTCCAATGCCCTGGTTGGGTCCTCAAACAGACGAATCTATCAAAGG
GCTTTGTGAGAGGGGGAGGAAGAATATCCTCTTGGTTCCGATAGCATTTACCAGTGACCATATTGAAACG
CTGTATGAGCTGGACATCGAGTACTCTCAAGTTTTAGCCAAGGAGTGTGGAGTTGAAAACATCAGAAGAG
CTGAGTCTCTTAATGGAAATCCATTGTTCTCTAAGGCCCTGGCCGACTTGGTGCATTCACACATCCAGTC
AAACGAGCTGTGTTCCAAGCAGCTGACCCTGAGCTGTCCGCTCTGTGTCAATCCTGTCTGCAGGGAGACT
AAATCCTTCTTCACCAGCCAGCAGCTGTGA
**Nucleotide sequence encoding RPS15 illustrative polypeptide sequence ENSP00000466010**
   (SEQ ID NO: 9) 
ATGTTGGGGCGAGGGCGGACTTGGCAGAAGTAGAGCAGAAGAAGCGGACCTTCCGCAAGTTCACCT
ACCGCGGCGTGGACCTCGACCAGCTGCTGGACATGTCCTACGAGCAGCTGATGCAGCTGTACAGTGCGCG
CCAGCGGCGGCGGCTGAACCGGGGCCTGCGGCGGAAGCAGCACTCCCTGCTGAAGCGCCTGCGCAAGGCC
AAGAAGGAGGCGCCGCCCATGGAGAAGCCGGAAGTGGTGAAGACGCACCTGCGGGACATGATCATCCTAC
CCGAGATGGTGGGCAGCATGGTGGGCGTCTACAACGGCAAGACCTTCAACCAGGTGGAGATCAAGCCCGA
GATGATCGGCCACTACCTGGGCGAGTTCTCCATCACCTACAAGCCCGTAAAGCATGGCCGGCCCGGCATC
GGGGCCACCCACTCCTCCCGCTTCATCCCTCTCAAGTAA
**Nucleotide sequence encoding OAZ1 illustrative polypeptide sequence ENSP00000473381**
   (SEQ ID NO: 10) 
ATGGTGAAATCCTCCCTGCAGCGGATCCTCAATAGCCACTGCTTCGCCAGAGAGAAGGAAGGGGATAAAC
CCAGCGCCACCATCCACGCCAGCCGCACCATGCCGCTCCTAAGCCTGCACAGCCGCGGCGGCAGCAGCAG
TGAGAGTTCCAGGGTCTCCCTCCACTGCTGTAGTAACCCGGGTCCGGGGCCTCGGTGGTGCTCCGATGCC
CCTCACCCACCCCTGAAGATCCCAGGTGGGCGAGGGAATAGTCAGAGGGATCACAATCTTTCAGCTAACT
TATTCTACTCCGATGATCGGCTGAATGTAACAGAGGAACTAACGTCCAACGACAAGACGAGGATTCTCAA
CGTCCAGTCCAGGCTCACAGACGCCAAACGCATTAACTGGCGAACAGTGCTGAGTGGCGGCAGCCTCTAC
ATCGAGATCCCGGGCGGCGCGCTGCCCGAGGGGAGCAAGGACAGCTTTGCAGTTCTCCTGGAGTTCGCTG
AGGAGCAGCTGCGAGCCGACCATGTCTTCATTTGCTTCCACAAGAACCGCGAGGACAGAGCCGCCTTGCT
CCGAACCTTCAGCTTTTTGGGCTTTGAGATTGTGAGACCGGGGCATCCCCTTGTCCCCAAGAGACCCGAC
GCTTGCTTCATGGCCTACACGTTCGAGAGAGAGTCTTCGGGAGAGGAGGAGGAGTAG
**Nucleotide sequence encoding MARCH2 illustrative polypeptide sequence**
   **ENSP00000471536**
   (SEQ ID NO: 11) 
ATGACGACGGGTGACTGCTGCCACCTCCCCGGCTCCCTGTGTGACTGCTCCGGCAGCCCTGCCTTCTCCA
AGGTCGTGGAGGCTACGGGCCTCGGACCGCCCCAGTATGTGGCACAGGTGACTTCAAGGGATGGCCGGCT
CCTCTCCACCGTCATCCGTGCCTTGGACACACCGAGTGATGGTCCTTTCTGCCGGATCTGCCATGAGGGA
GCGAACGGGGAGTGCTTGCTGTCCCCGTGTGGCTGCACCGGCACGCTGGGTGCCGTGCATAAGAGCTGTC
TGGAGAAGTGGCTTTCCTCATCTAACACCAGCTACTGCGAGCTGTGCCACACGGAGTTTGCAGTGGAGAA
ACGGCCTCGACCCCTCACAGAGTGGCTGAAGGACCCGGGGCCGCGGACGGAGAAGCGGACACTGTGCTGC
GACATGGTGTGTTTCCTGTTCATCACACCGCTGGCCGCCATCTCAGGCTGGTTGTGCCTGCGCGGGGCCC
AGGACCACCTCCGGCTCCACAGCCAGCTGGAGGCCGTGGGTCTCATTGCCCTCACCATCGCCCTCTTCAC
CATCTATGTCCTCTGGACGCTGGTCTCCTTCCGCTACCACTGCCAGCTGTACTCCGAGTGGAGAAAGACC
AACCAGAAAGTTCGCCTGAAGATCCGGGAGGCGGACAGCCCCGAGGGCCCCCAGCATTCTCCACTGGCAG
 CTGGACTCCTGAAGAAGGTGGCAGAGGAGACACCAGTATGA
   CAMK2G-207 cds:protein_coding (SEQ ID NO: 12) 
ATGGCCACCACCGCCACCTGCACCCGTTTCACCGACGACTACCAGCTCTTCGAGGAGCTT
GGCAAGGGTGCTTTCTCTGTGGTCCGCAGGTGTGTGAAGAAAACCTCCACGCAGGAGTAC
GCAGCAAAAATCATCAATACCAAGAAGTTGTCTGCCCGGGATCACCAGAAACTAGAACGT
GAGGCTCGGATATGTCGACTTCTGAAACATCCAAACATCGTGCGCCTCCATGACAGTATT
TCTGAAGAAGGGTTTCACTACCTCGTGTTTGACCTTGTTACCGGCGGGGAGCTGTTTGAA
GACATTGTGGCCAGAGAGTACTACAGTGAAGCAGATGCCAGCCACTGTATACATCAGATT
CTGGAGAGTGTTAACCACATCCACCAGCATGACATCGTCCACAGGGACCTGAAGCCTGAG
AACCTGCTGCTGGCGAGTAAATGCAAGGGTGCCGCCGTCAAGCTGGCTGATTTTGGCCTA
GCCATCGAAGTACAGGGAGAGCAGCAGGCTTGGTTTGGTTTTGCTGGCACCCCAGGTTAC
TTGTCCCCTGAGGTCTTGAGGAAAGATCCCTATGGAAAACCTGTGGATATCTGGGCCTGC
GGGGTCATCCTGTATATCCTCCTGGTGGGCTATCCTCCCTTCTGGGATGAGGATCAGCAC
AAGCTGTATCAGCAGATCAAGGCTGGAGCCTATGATTTCCCATCACCAGAATGGGACACG
GTAACTCCTGAAGCCAAGAACTTGATCAACCAGATGCTGACCATAAACCCAGCAAAGCGC
ATCACGGCTGACCAGGCTCTCAAGCACCCGTGGGTCTGTCAACGATCCACGGTGGCATCC
ATGATGCATCGTCAGGAGACTGTGGAGTGTTTGCGCAAGTTCAATGCCCGGAGAAAACTG
AAGGGTGCCATCCTCACGACCATGCTTGTCTCCAGGAACTTCTCAGTTGGCAGGCAGAGC
TCCGCCCCCGCCTCGCCTGCCGCGAGCGCCGCCGGCCTGGCCGGGCAAGCTGCCAAAAGC
CTATTGAACAAGAAGTCGGATGGCGGTGTCAAGAAAAGGAAGTCGAGTTCCAGCGTGCAC
CTAATGCCACAGAGCAACAACAAAAACAGTCTCGTAAGCCCAGCCCAAGAGCCCGCGCCC
TTGCAGACGGCCATGGAGCCACAAACCACTGTGGTACACAACGCTACAGATGGGATCAAG
GGCTCCACAGAGAGCTGCAACACCACCACAGAAGATGAGGACCTCAAAGCTGCCCCGCTC
CGCACTGGGAATGGCAGCTCGGTGCCTGAAGGACGGAGCTCCCGGGACAGAACAGCCCCC
TCTGCAGGCATGCAGCCCCAGCCTTCTCTCTGCTCCTCAGCCATGCGAAAACAGGAGATC
ATTAAGATTACAGAACAGCTGATTGAAGCCATCAACAATGGGGACTTTGAGGCCTACACG
AAGATTTGTGATCCAGGCCTCACTTCCTTTGAGCCTGAGGCCCTTGGTAACCTCGTGGAG
GGGATGGATTTCCATAAGTTTTACTTTGAGAATCTCCTGTCCAAGAACAGCAAGCCTATC
CATACCACCATCCTAAACCCACACGTCCACGTGATTGGGGAGGACGCAGCGTGCATCGCC
TACATCCGCCTCACCCAGTACATCGACGGGCAGGGTCGGCCTCGCACCAGCCAGTCAGAA
GAGACCCGGGTCTGGCACCGTCGGGATGGCAAGTGGCTCAATGTCCACTATCACTGCTCA
GGGGCCCCTGCCGCACCGCTGCAGTGA
   DERA-201 cds:protein_coding (SEQ ID NO: 13) 
ATGTCCGCGCACAATCGGGGCACCGAGCTCGACCTTAGCTGGATCTCCAAAATACAAGTG
AATCACCCGGCAGTTCTGAGGCGTGCGGAACAAATCCAGGCTCGCAGAACCGTGAAAAAG
GAGTGGCAGGCTGCTTGGCTCCTGAAAGCTGTTACCTTTATAGATCTTACTACACTTTCA
GGTGATGATACATCTTCCAACATTCAAAGGCTCTGTTATAAAGCCAAATACCCAATCCGG
GAAGATCTCTTAAAAGCTTTAAATATGCATGATAAAGGCATTACTACAGCCGCCGTTTGT
GTTTATCCCGCCCGGGTGTGTGATGCTGTAAAAGCACTCAAGGCTGCAGGCTGTAATATC
CCTGTGGCATCAGTGGCCGCTGGATTTCCAGCTGGACAGACTCATTTGAAGACACGATTA
GAAGAGATCAGATTGGCTGTGGAAGATGGAGCTACAGAAATCGACGTGGTAATTAACAGA
AGCTTGGTGCTGACAGGCCAGTGGGAAGCCCTGTATGATGAGATTCGTCAGTTTCGCAAG
GCCTGTGGGGAGGCTCATCTTAAAACTATATTAGCGACAGGAGAACTTGGAACTCTTACT
AATGTCTATAAAGCCAGTATGATAGCAATGATGGCAGGATCAGATTTTATTAAGACCTCT
ACTGGAAAAGAAACAGTAAATGCCACCTTCCCGGTAGCTATAGTAATGCTGCGGCCATT
AGAGATTTCTTCTGGAAAACTGGAAACAAGATAGGGTTTAAACCAGCAGGAGGCATCCGC
 
AGTGCAAAGGATTCCCTTGCTTGGCTCTCTCTTGTAAAGGAGGAGCTTGGAGATGAGTGG
CTGAAGCCAGAACTCTTTCGAATAGGTGCCAGTACTCTGCTCTCGGACATTGAGAGGCAG
ATTTACCATCATGTGACTGGAAGATATGCAGCTTATCATGATCTTCCAATGTCTTAA
   >KIAA1109-217 cds:protein_coding (SEQ ID NO: 14) 
ATGGATCAAAGGAAGAATGAGAGTATTGTTCCTAGTATAACTCAATTAGAAGATTTTCTT
ACAGAACACAATTCCAATGTTGTTTGGCTCCTTGTTGCTACCATTTTGTCCTGTGGATGG
ATTATTTACCTCACATATTATAATTCCCGGAATGTTGGTCTTATTTAACACTAGTTCTT
AACAGATTATACAAGCATGGCTATATCCATATTGGTTCCTTCTCTTTCTCTGTTCTTTCT
GGAAAAGTCATGGTTCGTGAAATCTATTACATTACAGAAGACATGTCTATTAGGATTCAA
GATGGATTCATCATTTTTCGGTGGTGGAAAATGTATAACCCAAAACAGAAGCAACATGAT
CCAAAGGCAGAAACCAGGTTATACATCACAGTCAATGACTTTGAATTTCATGTCTATAAT
CGCTCGGATCTTTATGGACGCCTTCAAGAGTTGTTTGGTTTGGAGCCAACAATAATTCCA
CCTAAGAAAGATGATGATAAAACACGGAAATTGGAAGAACAAGAACCCAATCGAAAATT
GAAAGAGTTAAAGTAAAAACAGAATCCCAAGACCCCACATCTTCATGGAGATCACTTATT
CCAGTCATAAAGGTCAATGTGAGCACAGGACGTTTGGCCTTTGGAAATCACTACCAGCCG
CAAACTCTGTGCATCAACTTTGATGATGCTTTCTTAACTTATACTACAAAACCACCTTCA
AGTCATCTTGACCAATTCATGCATATTGTGAAAGGAAAGCTTGAAAATGTTCGAGTCATG
CTTGTTCCTAGTCCAAGATATGTTGGTCTTCAAAATGATGAACCACCGAGATTAATGGGA
GAAGGTTTTGTGGTGATGCAGTCAAATGATGTTGACATCTACTACTACATGGATGAGCCA
GGACTTGTTCCGGAAGAAACAGAAGAAAATATTGAAGGAGAAATGAGCAGTGAGGATTGC
AAATTACAAGACTTGCCTCCATGTTGGGGACTGGATATAGTTTGTGGTAAAGGAACAGAT
TTTAATTATGGACCATGGGCCGATAGGCAGAGAGATTGTTTGTGGAAGTTTTTCTTTCCA
CCTGACTATCAAGTTCTGAAAGTTTCTGAAATTGCACAGCCTGGGAGACCAAGACAGATC
CTTGCTTTTGAATTACGAATGAATATTATTGCAGATGCTACAATTGATTTGCTGTTTACC
AAAAATAGGGAAACAAATGCTGTACATGTAAATGTAGGAGCTGGCTCATATTTAGAAATT
AATATTCCAATGACAGTTGAAGAAAATGGTTACACTCCTGCTATTAAGGACAACTCTTA
CATGTGGATGCCACTACCAGCATGCAATATCGGACCCTTTTAGAAGCAGAAATGTTAGCA
TTCCACATCAATGCCAGCTACCCCCGAATATGGAACATGCCGCAGACATGGCAGTGTGAA
TTAGAGGTTTATAAAGCCACTTACCACTTCATCTTTGCACAGAAAAACTTCTTTACAGAT
TTAATTCAAGACTGGTCTAGTGACAGTCCTCCAGACATTTTTTCATTTGTTCCATATACG
TGGAATTTTAAAATCATGTTTCATCAGTTTGAAATGATTTGGGCTGCTAATCAACACAAT
TGGATCGACTGTTCTACTAAACAACAGGAAAATGTGTATCTGGCAGCCTGTGGAGAAACA
CTAAACATTGATTTTTCTTTGCCTTTTACGGACTTTGTTCCAGCTACATGTAATACCAAG
TTCTCTTTAAGAGGAGAAGATGTTGATCTTCATTTGTTTCTACCAGACTGCCACCCTAGT
AAATATTCTTTATTTATGCTGGTAAAAAATTGCCATCCAAATAAGATGATTCATGATACT
GGTATTCCTGCTGAGTGTCAAAGTGGCCAGAAAACAGTTAAACCAAAATGGCGCAACGTT
ACTCAGGAAAAGTCTGGTTGGGTTGAATGCTGGACTGTCCCAAGTGTCATGCTTACAATT
GATTATACATGGCATCCAATTTATCCACAAAAAGCAGATGAACAGCTGAAACAATCATTA
TCAGAAATGGAAGAGACAATGCTATCTGTATTAAGGCCATCCCAGAAGACATCAGACAGA
GTTGTTTCTTCTCCCTCTACTTCTTCACGCCCACCTATTGATCCCTCAGAACTTCCACCT
GATAAACTTCATGTAGAAATGGAACTTTCTCCAGATTCTCAGATAACTCTCTATGGACCT
CTACTAAATGCCTTTTTGTGTATAAAGGAAAACTACTTTGGGGAAGATGACATGTATATG
GATTTTGAAGAGGTTATCTCAAGTCCTGTTTTGTCACTGTCAACATCATCCAGCTCTGGG
TGGACTGCTGTTGGAATGGAAAATGACAAAAAGGAAAATGAAGGTTCAGCCAAGTCAATT
CATCCACTTGCCTTGCGTCCTTGGGATATTACTGTACTTGTTAATTTGTACAAAGTTCAT
GGGCGTCTTCCTGTTCATGGAACTACTGATGGTCCTGAATGCCCTACAGCTTTCTTGGAA
AGACTATGTTTTGAAATGAAAAAAGGATTTAGGGAGACCATGCTGCAACTTATCCTGTCA
CCCCTGAATGTGTTTGTCAGTGATAACTATCAGCAGCGACCCCCTGTGGATGAAGTACTC
AGGGAAGGTCACATCAATTTGTCAGGTCTCCAGCTGAGAGCACACGCTATGTTCTCAGCA
 
GAAGGTCTTCCTTTGGGAAGCGATTCCTTAGAATACGCATGGTTAATTGATGTGCAGGCT
GGAAGTCTTACAGCTAAGGTCACAGCACCACAGCTGGCATGCCTCTTGGAGTGGGGACAG
ACATTTGTTTTTCATGTGGTATGTCGGGAGTATGAACTGGAAAGACCGAAATCAGTTATA
ATATGTCAGCATGGAATTGATCGTCGGTTCTGTGAATCCAAGTTGAGTTGTATTCCTGGG
CCTTGTCCAACTTCAGATGATTTGAAATATACTATGATTCGTTTAGCAGTAGATGGAGCC
GATATTTACATTGTTGAGCATGGTTGTGCTACAAATATAAAGATGGGTGCAATTCGAGTT
GCAAACTGTAATCTCCACAATCAATCGGTTGGGGAAGGAATCAGTGCTGCAATTCAGGAT
TTTCAAGTGAGACAGTACATTGAGCAATTAAATAATTGCAGAATTGGACTTCAGCCTGCA
GTGCTACGGAGGGCCTATTGGCTTGAAGCTGGGTCAGCCAATTTAGGACTTATTACTGTT
GATATTGCTTTAGCTGCTGACCATCATTCTAAACATGAGGCACAAAGACATTTCTTAGAA
ACTCATGATGCCAGAACTAAGAGGTTGTGGTTTTTATGGCCAGATGATATCCTGAAGAAT
AAGAGGTGTAGAAACAAATGTGGTTGTCTCGGTGGCTGCAGATTCTTTGGTGGCACAGTA
ACTGGCCTAGATTTCTTCAAACTTGAAGAGTTGACACCTTCCAGTAGCTCTGCATTTTCA
AGCACAAGTGCAGAGTCTGATATGTATTATGGACAGTCTCTGCTACAGCCTGGAGAATGG
ATAATTACTAAAGAAATTCCCAAAATTATAGATGGTAATGTGAATGGCATGAAGAGGAAA
GAATGGGAAAACAAATCAGTGGGAATAGAAGTAGAGAGAAAAACTCAGCACCTTAGTCTT
CAAGTACCATTACGATCTCATAGTTCATCCTCTTCCTCAGAAGAGAACAGTAGTTCTAGT
GCTGCACAGCCTTTGTTGGCTGGTGAAAAGGAAAGTCCTTCATCTGTTGCTGATGACCAT
TTGGTTCAAAAAGAGTTCTTGCATGGACAAAAAGAGATGATGGCCAAGCAAGTATCCCT
ACAGAAATTTCAGGAAACAGCCCTGTGTCTCCTAATACTCAGGATAAGTCAGTAGGTCAA
TCTCCTCTTAGATCTCCCTTGAAACGACAAGCCTCTGTCTGTTCCACCCGTCTTGGAAGT
ACTAAGAGTCTTACTGCTGCTTTCTATGGGGACAAGCAGCCTGTAACAGTTGGAGTCCAG
TTTAGTAGTGATGTCTCTCGAAGTGATGAGAATGTACTAGACTCACCAAAGCAGAGGAGA
AGTTTTGGTTCATTCCCATATACACCATCAGCAGACTCTAATTCATTTCATCAGTATCGA
TCAATGGATTCCAGCATGTCAATGGCTGATAGTGAAGCCTACTTTTCTGCTGCTGAGGAA
TTTGAGCCCATTAGCAGTGATGAAGGCCCTGGAACTTATCCGGGTAGAAAAAAGAAGAAA
AAGCAAACCCAGCAGATTGACTACAGTAGGGGTTCCATATATCACAGTGTAGAAGGGCCA
CTTACTGGACATGGAGAAAGCATTCAGGATTCCAGAACTCTGCCATTCAAAACTCATCCT
TCTCAGGCTTCATTTGTTTCTGCGTTAGGTGGAGAAGATGATGTTATAGAACATCTATAT
ATTGTAGAAGGTGAGAAACAGTGGAGAGTGAACAGATTACTCCGCAACAACCCGTGATG
AATTGTTATCAGACTTACCTTACTCAGTTCCAGGTAATTAATTGGTCAGTTAAGCACCCA
ACCAACAAAAGAACCTCTAAATCCTCATTGCATCGTCCCCTTGATCTGGATACACCAACC
AGTGAAGAAAGTTCATCGTCATTTGAACAGCTTTCTGTTCCAACTTTTAAGGTTATCAAG
CAGGGGCTCACAGCTAATTCTTTGCTAGACAGAGGCATGCAACTTTCAGGATCAACTTCA
AATACGCCGTATACTCCATTGGAAAAAAACTCGCTGATAACACAGATGATGAAACATTA
ACAGAAGAGTGGACCCTGGATCAACCAGTGTCCCAGACCAGGACAACAGCCATAGTTGAA
GTAAAAGGAACTGTTGATATTGTTTTGACTCCCCTGGTGGCTGAAGCTTTAGACAGATAT
ATTGAAGCAATGGTTCATTGTGCTAGTACCCGACATCCAGCTGCAATTGTAGATGATCTT
CATGCTAAAGTCCTCAGGGAAGCTGTCCAAAATAGCAAGACTACCTTTTCAGAAAATTTA
TCTTCCAAACAAGACATTAGAGGAACAAAAACTGAGCAGTCTACAATAGGAACGACTAAC
CAAGGACAAGCACAGACAAATCTTACAATGAAGCAAGATAATGTAACAATTAAAGGTCTT
CAGACTAATGTTAGCATACCAAAGGTAAACTTATGTTTGTTACAAGCCTCAGTGGAAGAA
TCTCCAACTACGGCTCCTAGTAGGAGTGTGACTCATGTTTCCCTAGTGGCATTGTGTTTT
GACAGAATTGCTACACAGGTTCGCATGAACAGAGGTGTGGTTGAAGAGACTTCAAACAAT
GCAGAACCTGGTAGAACATCAAATTTTGATAGGTATGTTCATGCCACAAAGATGCAGCCT
CAGTCATCTGGATCTCTCAGATCAAATGCTGGAGCAGAAAAAGGCAAAGAAATTGCAGCT
AAGTTAAACATTCATCGAGTTCATGGGCAACTTAGGGGTCTTGATACAACAGACATTGGG
ACCTGTGCAATCACTGCTATTCCTTTTGAGAAGTCCAAAGTTTATTTACTCTTGAAGAG
TTGGATGAATTTACTTTTGTGGATGAAACTGATCAGCAAGCTGTTCCAGATGTAACTCGA
ATAGGTCCCAGCCAAGAAAAATGGGATGGATAATGTTTGAATGTGGACTTGAAAATTTA
ACCATAAAAGGAGGAAGACAGTCTGGTGCTGTTTATATAACAGTTTTGGAATTATGGGT
AAAGCTAGTGACACAGAAAGGGGTGGAGTGCTGACATCCAATAATTCTTCTGATTCTCCA
 
ACCGGCAGTGGCTATAATACTGATGTCTCTGATGATAATCTTCCATGTGACCGGACAAGC
CCTTCCTCAGACTTAAATGGAAATTCTGTTTCAGATGAACAGGATGAAGGAGTCGAATCT
GATGATTTGAAAAAAGATCTACCTCTGATGCCTCCTCCTCCAGATTCATGTAGCATGAAG
TTGACCATTAAAGAAATTTGGTTTAGTTTTGCAGCTCCCACCAATGTGAGATCTCACACA
CATGCATTTTCTAGGCAGCTGAACCTTTTAAGCACTGCAACACCAGCTGTTGGTGCATGG
CTTGTTCCCATTGACCAACTCAAGTCATCTTTAAACAAATTGGAAACTGAGGGAACCTTG
AGAATTTGTGCTGTTATGGATGCATAATGACAGAAGCATTAGAGAATAAAAGTGTACAT
TTTCCCCTAAGAAGTAAATACAACAGGCTTACAAAAGTTGCTCGCTTTCTCCAAGAAAAT
CCTTCATGTTTACTATGTAATATACTACACCACTATCTGCACCAGGCAAATTACTCCATC
ATTGATGATGCTACAATGAGCGATGGACTTCCTGCTTTGGTAACTTTGAAGAAAGGTTTA
GTTGCACTGGCAAGGCAGTGGATGAAGTTTATTGTGGTGACACCAGCCTTTAAAGGAGTT
AGCTTACATAGACCAGCTCAGCCTCTGAAACCTCAAATAGCTATGGACCATGAACATGAA
GATGGACTTGGATTGGACAATGGGGGTGGTCTTCAAAGTGATACCAGTGCTGATGGAGCA
GAATTTGAGTTCGATGCAGCCACAGTCAGTGAACACACAATGCTATTAGAAGGAACAGCT
AACCGGCCTCCACCTGGTAGCTCTGGACCTGTAACTGGAGCTGAGATAATGAGGAAACTT
TCTAAAACTCATACCCATAGTGACTCTGCATTAAAAATAAAGGGCATTCACCCATATCAT
TCTCTGAGCTATACCAGTGGAGACACTGCCACTGATTCTCCAGTGCATGTTGGACGTGCT
GGGATGCCAGTAAAGGACAGTCCAAGGAAGGAGAGCCTACTCAGCTACCTGACTGGAAGC
TTCCCAAGCCTGCACAACCTCCTGGAAGGTACTCCTCAGAGAAGCAGTGCTGCTGTGAAA
AGTAGCTCCCTAACGAGAACAGGAAATACAGTAGCCACTGATATGTTATCTGAACATCCC
TTGCTATCTGAGCCATCATCTGTGAGTTTCTATAATTGGATGTCAAATGCTGTGGGTAAT
CGAGGAAGTGTGTTACAAGAATCTCCTGTTACAAAATCAGGACACAATAGTCTTCCCACA
GGTGTTGCACCCAATCTTCCAACAATACCCTCAGCCTCAGATTTCAACACTGTCTTGTCT
AGTGACCAAAATACTTTGGATGGGACACATTCTCAGCATAGCACCAGTCAGGATGATGTG
GCAGGTGTAGAAGAAGCAAACCAAGGGTTTCCTGCTGTTCAGCTTGCTGATGCACAGGTT
GTTTTCAAGCCTCTTCTGAGTCATACAGGGATCCAGTCACAGGATACAATGCCATTCTGC
TACCGAATGTACTTTGGAGAACACCTTTCATTTTCAGGGACTTTGGACTGCCTCAGAGCA
GATATTGTGGATTCAGACACAGCCAAAGAGAGAAAAGGCAAAAGAGCAAGAAGGCAAGGC
CATGTGAATCTTCCTCCACTTGAGTTCAAACCAGCATTAATGTTGGGAACCTTTAGCATC
AGTGCTGTTGTAATGGAAAAGTCCGTGTGCACCCCTCAGAACTCTACCAGTGCCCTTTCT
TTTCATGATCTCAGCAAGCGGTATTATAACACCTTTCACTGCAACTTTACTATTTCCTGT
CAGTCAATAAGCCAGCATGTAGATATGGCTTTGGTTCGTCTTATTCATCAGTTTAGCACA
ATGATAGATGACATCAAAGCAACTCAGACTGATATTAAACTTAGCAGATATACAGCCGGA
TCTGCTTCCCCAACACCTACCTTCAAAACCAGAAAACATCGGACTTTCGTTCATCTGAC
TTTAGCCGCAGTTCTAGAGGAAGTCTTAATGGTGGCAATAGAGTAAATAATGCAAAGAAC
AAACGGACCAACAATGAGAATAACAAAAAGGAATCTCGAAACAAGAATTCATTAGGAAGA
TCTGAAAGAAGAACATCAAAAGTGTCTAGGAAAGGTTCAAAAGATGTGGTGGATCACATG
ACTATTCATATGGATGACTCTGATTCAATTACAGTGTCAGAACAAAGTGAGCCTTCAGCT
GAGTGCTGGCAGAATATGTATAAATTGCTTAACTTCTATTCACTTATCTCCGATCCAACA
GGAATATTGGAAAAGTCTTCAGAAACATTTGGACCAGCAGGAGTTCGGAGCCCTACAGAG
CCAACATGTAAAGTTGTGTTTGAGAATGAACAAGACAACAGCAGTTTGACTAAGACTCAG
AGGAAACGTAGCTTGGTAACTTCTGAACCTCAGCATGTTACTCTAATAGTGTTTGGGATT
GGCATGGTGAACCGCACACACCTAGAGGCAGATATTGGTGGACTAACAATGGAATCAGAA
CTGAAGAGGATCCATGGCAGTTTTACTCTTAAGGAAAAAATGAAAGATGTTTTACATCAA
AAGATGACTGAGACTTGTGCTACTGCTCATATTGGTGGGGTTAATATTGTGCTGCTTGAA
GGGATTACACCAAATATACAACTGGAGGATTTTCCTACATCTCCTACAAGTACAGCCAAA
CAAGAGTTTCTAACTGTAGTGAAATGTAGTATTGCCAAGTCCCAAGCCCTCTACAGTGCC
CAAAGAGGGCTGAAGACAAACAATGCTGCTGTGTTCAAAGTAGGAGCTATCAGTATCAAC
ATTCCTCAGCACCCTGCCACCTTACATAGCATGATGGTTCGAAGTTCTCACCAACTATCT
AAACAAATCTCAGACCTAATCAGACAGCCTTCTACAGCCCCACAGCCTGTAAAAGAAGAT
ATTGCAACCCCACTACCTTCTGAAAAACCCCAACAAGTGTTAATCAAACTCCTGTTGAA
ACAAATGAATTTCCTCAGCTACCAGAAGGCTTAGAAAAGAAGCCTATTGTTCTTAAATTC
 
AGTGCCATGTTAGATGGTATAGCCATTGGAGCAGCACTTTTACCATCTCTGAAAGCAGAA
TACAAGATGGGAAGAATGAGAAGTCATGGAATGACAGGTGCACAGACAAGATTTACATTT
GAGCTGCCAAATCACAGATTGCGTTTTACTTCAAAAGTTCTGCCACAGATATGTCAACC
ATTCCTCCTTCTGCCAGTCTTAACCTTCCCCCTGTTACCATGTCAGGGAAATATATAATG
GAAGAACATGATAGTTATTCGGATCAGGTGTGGAGTATAGATGAACTGCCTTCTAAACAA
GGTTACTATTTACAGGGAAATTATCTGCGTTGTGTGGCAGAAGTTGGTTCCTTTGAACAT
AATCTTACAACTGATCTTCTAAACCACTTGGTATTTGTACAGAAAGTGTTCATGAAGGAA
GTTAATGAAGTAATACAAAAAGTTTCTGGTGGGGAGCAGCCTATTCCTCTCTGGAACGAA
CATGATGGAACAGCAGATGGAGATAAACCTAAAATTCTCCTCTATTCCCTAAACTTGCAG
TTCAAGGGTATTCAAGTAACGGCCACTACTCCATCAATGAGAGCTGTAAGATTTGAAACT
GGATTGATTGAACTGGAACTTTCTAACCGACTTCAAACCAAAGCTTCACCAGGAAGTAGC
AGCTATCTGAAACTGTTTGGCAAATGCCAGGTGGATTTAAATCTGGCATTAGGACAAATT
GTCAAACATCAGGTTTATGAGGAAGCTGGTTCTGATTTTCATCAAGTTGCCTATTTTAAA
ACCAGAATTGGATTAAGAAATGCCCTCCGAGAAGAAATCAGTGGTTCTTCAGATAGGGAA
GCTGTGCTTATTACTTTGAATAGACCAATTGTTTATGCACAGCCTGTGGCCTTTGATAGA
GCTGTGCTGTTTTGGCTGAATTATAAGGCCGCCTATGACAACTGGAATGAACAACGAATG
GCTTTACATAAGGATATTCATATGGCTACAAAGGAAGTAGTAGATATGCTACCTGGTATC
CAGCAAACATCAGCCCAGGCCTTTGGACTCTTTTTCTCCAGCTCACTGTCAATGATCTG
GGAATTTGCCTACCTATCACAAATACTGCACAGTCTAATCATACTGGAGACCTTGACACT
GGTTCTGCTTTGGTATTAACCATTGAAAGTACTCTCATCACTGCATGCTCTTCAGAGTCT
CTGGTTAGCAAAGGGCATTTCAAAAACTTTTGTATCCGTTTTGCTGATGGATTTGAGACA
TCATGGGATGACTGGAAACCAGAAATTCATGGGGATTTAGTGATGAATGCCTGTGTAGTT
CCAGATGGCACCTATGAAGTATGTTCAAGAACTACAGGACAAGCAGCAGCTGAAAGCAGT
AGTGCTGGAACCTGGACACTCAACGTATTGTGGAAAATGTGTGGATTGATGTTCACATG
GATCCTAACATTGGCAAAAGGCTTAATGCTCTGGGCAATACTCTTACAACACTGACAGGA
GAGGAAGACATAGATGACATTGCTGACTTAAATTCAGTGAACATAGCTGACCTGTCAGAT
GAGGATGAAGTTGATACTATGTCTCCCACTATCCATACTAGTTCAGATGGAAGTTCAATA
AGTGGAGATGGCCACAAACTCACCTTTGGCAGCGACTTGTAAATCACCTACTAGGCCTG
ACACCCCCAAATCAGCGCCATTCTGTTCCTGCAGAGTATCTGTGTGACCCAGAGATGTGG
GGCTCCCCTCAGTCTAGCCAGTCCCATTTGAAAGCATGCAGAGCACACTCATGGGAAAC
GAAGCCACAGATTATCGAAGACAGGCAGCATCTGCTAGCCAGCCGGGAGAACTTAGAGGA
AGAAAAATTATGAAGCGTATAGTGGATATCAGAGAACTGAATGAACAGGCCAAAGTAATA
GATGATCTGAAAAAATTAGGTGCAAGTGAAGGAACCATAAACCAGGAAATTCAACGTTAC
CAACAGTTAGAATCTGTGGCTGTGAATGACATTAGAAGAGATGTTCGTAAAAAATTACGG
AGGTCCAGTATGCGGGCTGCTTCCCTAAAGGATAAGTGGGGTTTGAGTTACAAACCAAGT
TACAGCCGATCAAAAAGCATTTCTGCTTCTGGAAGACCACCTCTTAAGCGAATGGAAAGG
GCAAGTTCTCGAGTAGGAGAAACTGAAGAGCTCCCAGAAATCCGTGTGGATGCAGCATCT
CCTGGACCTAGAGTAACTTTTAATATCCAGGATACATTGAAAAATACAGTATGGGGAAGT
ACACCACAATCCAGCTGTCCTGGGGAAGGGTATTTTCAGTTCCAGAGGAGACAGAACTG
GACCTTTTGTCAGTAACCATTGAAGGTCCATCCCATTATTCATCAAATAGTGAAGGATCA
TGTTCTGTGTTCAGTTCTCCCAAAACTCCAGGAGGCTTTTCACCAGGCATTCCTTTCCAA
ACTGAAGAGGGCCGACGGGATGACAGTTTGTCTTCTACCAGTGAAGATTCCGAGAAGGAT
GAAAAAGATGAAGACCATGAGAGGGAAAGGTTTTATATTTACAGGAAACCCTCACATACG
TCTCGTAAAAAAGCAACAGGCTTTGCTGCTGTTCATCAGCTATTTACAGAACGCTGGCCA
ACAACACCAGTCAATAGAAGTCTTAGTGGCACAGCTACAGAGAGAAATATTGACTTTGAA
CTTGATATACGGGTTGAAATTGATAGTGGAAAATGTGTACTCCACCCAACCACCCTTCTA
CAAGAACATGATGATATAAGTTTGAGAAGGAGTTATGATCGAAGTTCCAGGAGCTTAGAT
CAAGATTCACCTTCAAAAAAGAAGAAGTTTCAAACTAATTATGCTTCTACCACCCATTTA
ATGACCGGCAAGAAAGTGCCATCATCTCTACAGACAAAGCCTAGTGACTTAGAAACAACA
GTATTTTACATTCCCGGAGTTGATGTAAAGTTGCATTACAATTCCAAGACGCTAAAGACT
GAATCACCTAATGCCTCCAGGGGATCTTCCTTGCCAAGAACACTGTCCAAAGAGTCCAAG
CTGTATGGTATGAAAGATAGTGCAACATCTCCTCCTTCTCCTCCTTTACCTTCCACTGTC
 
CAGAGCAAGACTAACACCTTACTTCCTCCCCAGCCCCCACCTATTCCTGCAGCCAAAGGA
AAAGGAAGTGGAGGAGTAAAAACAGCCAAGTTATATGCCTGGGTAGCACTTCAGTCATTG
CCAGAAGAAATGGTTATTAGTCCCTGCCTATTAGACTTTCTGGAAAAAGCTCTGGAAACT
ATCCCAATTACACCAGTTGAAAGGAATTATACAGCTGTCAGCTCACAAGATGAAGATATG
GGACATTTTGAAATACCAGATCCTATGGAAGAATCAACAACATCACTAGTGTCGTCTTCA
ACATCTGCTTACTCTTCCTTCCCTGTAGATGTTGTGGTTTATGTACGAGTTCAGCCCTCA
CAGATCAAATTTAGCTGTTTACCAGTATCAAGAGTAGAATGCATGTTAAAGCTGCCATCC
CTGGATTTGGTGTTTTCTTCAAACCGAGGAGAACTGGAGACTTTAGGGACTACATATCCT
GCAGAGACTTTATCCCCTGGAGGTAATGCTACTCAGAGTGGAACAAAGACTTCTGCTAGC
AAAACTGGAATACCAGGTTCATCGGATTAGGCAGCCCTCTTGGCCGAAGTCGACATAGT
AGTAGTCAGTCAGACCTGACCAGTTCCAGCAGTAGTTCATCTGGCTTGAGCTTCACTGCA
TGCATGTCTGACTTTTCCCTTTATGTATTTCATCCATATGGAGCAGGGAAACAAAAAACT
GCTGTTTCTGGCCTCACACCTGGATCAGGAGGATTAGGGAATGTGGATGAGGAGCCCACT
TCAGTCACTGGTCGAAAAGATTCACTCAGTATAAACCTTGAGTTTGTAAAAGTGAGTTTG
TCACGGATCAGGCGTTCAGGAGGTGCCTCATTTTTTGAAAGTCAGTCTGTAAGCAAGTCT
GCAAGCAAAATGGATACTACGTTAATAAATATATCTGCTGTTTGTGATATAGGGTCTGCC
TCCTTTAAATATGATATGCGCCGACTCAGTGAAATTCTGGCATTTCCAAGAGCATGGTAT
AGAAGAAGTATTGCAAGACGTCTATTCCTTGGAGACCAAACTATAAATTTGCCAACATCT
GGCCCAGGGACACCTGATTCCATTGAAGGGTAAGCCAACACCTTTCCCCTGAATCATCA
AGAAAAGCTTACTGCAAGACCTGGGAGCAGCCAAGTCAGTCAGCCTCCTTCACCCACATG
CCTCAGTCACCTAATGTGTTCAATGAGCATATGACAAACAGCACCATGTCACCAGGGACA
GTAGGACAGAGCCTAAAATCCCCAGCTTCCATAAGATCAAGGAGTGTATCTGATTCTTCA
GTTCCTCGAAGAGATTCACTTTCAAAAACATCAACTCCTTTTAACAAATCAAACAAAGCA
GCAAGCCAACAAGGACCCCATGGAAACACTTGTCGTGTTTGCTATCAACTTGAAGCAA
TTAAACGTTCAAATGAATATGAGTAATGTAATGGAAATACAACTTGGACAACTAGTGGT
TTGAAGAGCCAGGGCCGTCTGTCAGTAGGAAGTAATCGTGATCGAGAGATCAGCATGTCT
GTTGGTCTGGGAAGATCACAATTAGATTCTAAAGGAGGAGTAGTTGGAGGGACCATAGAT
GTCAATGCTTTGGAGATGGTTGCTCATATTTCTGAACATCCAAATCAGCAACCCAGTCAC
AAAATTCAGATTACTATGGGTTCTACTGAAGCTCGTGTTGATTACATGGGCTCAAGTATC
CTCATGGGCATCTTCAGTAATGCTGATCTTAAGCTTCAGGATGAATGGAAAGTAAACTTG
TATAATACATTGGATTCAAGCATAACTGATAAAAGTGAGATTTTCGTCCATGGAGATTTG
AAGTGGGATATTTTCCAAGTAATGATATCAAGGTCAACCACACCAGATCTGATAAAAATA
GGAATGAAGCTCCAGGAATTTTTCACACAACAATTTGATACCAGCAAACGAGCTCTGTCT
ACCTGGGGACCAGTTCCTTACCTTCCGCCAAAGACAATGACTAGCAACCTAGAAAAAGT
TCACAAGAACAATTACTTGATGCAGCACATCATCGACACTGGCCTGGAGTATTGAAGGTG
GTATCAGGATGCCACATATCCTTATTTCAGATTCCATTACCAGAAGATGGAATGCAATTT
GGAGGATCAATGAGCTTACATGGAAATCATATGACACTGGCATGTTTTCATGGTCCAAAT
TTTCGTTCAAAATCTTGGGCCCTTTTTCATTTAGAAGAACCAAATATTGCTTTTTGGACT
GAAGCTCAGAAAATCTGGGAAGATGGCTCCAGTGATCATTCTACATATATTGTACAAACA
CTAGATTTTCACCTGGGTCATAATACTATGGTTACCAAACCATGTGGTGCTTTGGAAAGT
CCTATGGCAACAATAACCAAGATAACAAGGCGTCGCCATGAAAATCCACCCCATGGAGTA
GCAAGTGTGAAAGAATGGTTCAATTATGTTACAGCTACAAGGAATGAAGAGCTAAATCTG
CTTCGTAATGTTGATGCTAACAACACTGAGAATAGCACTACTGTGAAGAATTCTAGTTTG
TTGAGTGGATTCAGAGGAGGTTCTAGCTACAACCATGAAACAGAGACTATCTTTGCATTA
CCAAGGATGCAGCTTGACTTTAAATCCATTCATGTTCAAGAACCACAGGAGCCTTCATTA
CAGGATGCCAGCCTGAAGCCAAAAGTAGAATGTAGTGTGGTGACAGAGTTCACTGACCAC
ATTTGTGTGACTATGGATGCTGAGCTCATCATGTTTCTTCATGATTTAGTATCAGCTTAT
CTTAAAGAAAAAGAAAAAGCCATCTTTCCACCTCGGATTTATCTACTCGACCAGGACAA
AAAAGTCCAATTATTATACATGACGACAATTCCTCTGATAAAGATAGAGAAGATAGCATC
ACTTATACTACTGTGGACTGGAGAGATTTTATGTGCAATACATGGCATCTAGAACCTACT
CTTAGATTAATTTCTTGGACTGGAAGAAAGATTGATCCAGTAGGTGTTGATTATATTCTT
CAAAAATTGGGCTTTCATCATGCTAGGACTACTATTCCTAAATGGCTTCAAAGAGGAGTC
 
ATGGATCCACTGGACAAGGTTCTGTCAGTTCTTATCAAAAGCTCGGTACTGCACTACAG
GATGAAAAGGAAAAGAAAGGCAAAGACAAAGAAGAACACTAA
   >LRRC58-201 cds:protein coding (SEQ ID NO: 15) 
ATGGAGGAGGCCGGAGCAGCGGTGGTCACGGCCGGGGAGGCCGAACTGAACTGGTCCCGC
CTCAGCGTGTCCACCGAGACGCTGGAGTCTGAGCTGGAGGCGCGGGGGGAGGAGCGGCGC
GGGGCGCGGGAGGCGCTGCTGCGGCTGCTGCTGCCTCACAACCGTCTGGTGTCGCTGCCA
CGGGCGCTGGGCAGCGGCTTCCCGCACCTCCAGCTGCTGGACGTGAGCGGCAACGCGTTG
ACCGCGCTCGGGCCGGAGCTGCTCGCTCTGCGCGGCCTGCGCACGCTGCTGGCCAAGAAC
AACCGGCTCGGCGGGCCCAGTGCGCTGCCCAAGGGCCTGGCCCAGTCGCCGCTCTGCCGC
AGCCTCCAGGTGCTCAACCTCAGCGGCAACTGTTTCCAGGAGGTGCCTGCCTCGCTCTTA
GAGCTGCGCGCGCTGCAGACCCTGAGCCTGGGCGGCAACCAACTGCAGAGCATCCCGGCT
GAGATCGAGAACTTGCAGAGTTTAGAGTGTTTATATCTTGGAGGAAATTTCATTAAAGAA
ATCCCACCAGAATTAGGAAATCTGCCTTCTCTGAATTATTTGGTATTATGTGACAACAAA
ATCCAAAGCATACCTCCTCAACTTTCACAGTTACATTCACTTCGTTCCCTAAGTCTTCAC
AATAACTTGCTGACATATCTGCCTCGAGAGATCCTCAACCTTATTCATTTGGAAGAGTTG
AGTTTACGAGGAAATCCATTGGTTGTTCGTTTTGTTAGAGATTTAACCTATGATCCTCCA
ACTCTCCTGGAATTAGCTGCACGGACCATTAAGATTCGAAATATTTCCTACACTCCCTAT
GATCTTCCTGGAAATCTTCTTAGATACTTGGGTTCAGCCAGCAATTGCCCAAACCCAAAG
TGTGGAGGAGTCTACTTTGACTGCTGTGTCAGACAAATTAAATTTGTGGACTTCTGTGGG
AAGTATCGCCTCCCACTGATGCACTACTTGTGTTCACCAGAATGTTCTTCCCCTTGCAGT
TCTGCCTCTCACAGCTCCACTTCCCAGAGTGAATCTGACTCAGAAGATGAAGCTAGTGTT
GCTGCACGCAGAATGCAGAAAGTTCTTCTTGGTTGA
   >MYLIP-202 cds:protein_coding (SEQ ID NO: 16) 
ATGCTGTGTTATGTGACGAGGCCGGACGCGGTGCTGATGGAGGTGGAGGTGGAGGCGAAA
GCCAACGGCGAGGACTGCCTCAACCAGGTGTGCAGGCGACTGGGAATCATAGAAGTTGAC
TATTTTGGACTGCAGTTTACGGGTAGCAAAGGTGAAAGTTATGGCTAAACCTGAGAAAC
CGGATCTCCCAGCAGATGGATGGGCTAGCCCCTTACAGGCTTAAACTTAGAGTCAAGTTC
TTCGTGGAGCCTCATCTCATCTTACAGGAGCAGACTAGGCATATCTTTTTCTTGCACATC
AAGGAGGCCCTCTTGGCAGGCCACCTCTTGTGTTCCCCAGAGCAGGCAGTGGAACTCAGT
GCCCTCCTGGCCCAGACCAAGTTTGGAGACTACAACCAGAACACTGCCAAGTATAACTAT
GAGGAGCTCTGTGCCAAGGAGCTCTCCTCTGCCACCTTGAACAGCATTGTTGCAAAACAT
AAGGAGTTGGAGGGGACCAGCCAGGCTTCAGCTGAATACCAAGTTTTGCAGATTGTGTCG
GCAATGGAAAACTATGGCATAGAATGGCATTCTGTGCGGGATAGCGAAGGGCAGAAACTG
CTCATTGGGGTTGGACCTGAAGGAATCTCAATTTGTAAAGATGACTTTAGCCCAATTAAT
AGGATAGCTTATCCTGTGGTGCAGATGGCCACCCAGTCAGGAAAGAATGTATATTTGACG
GTCACCAAGGAATCTGGGAACAGCATCGTGCTCTTGTTTAAAATGATCAGCACCAGGGCG
GCCAGCGGGCTCTACCGAGCGATAACAGAGACGCACGCATTCTACAGGTGTGACACAGTG
ACCAGCGCCGTGATGATGCAGTATAGCCGTGACTTGAAGGGCCACTTGGCATCTCTGTTT
CTGAATGAAAACATTAACCTTGGCAAGAAATATGTCTTTGATATTAAAAGAACATCAAAG
GAGGTGTATGACCATGCCAGGAGGGCTCTGTACAATGCTGGCGTTGTGGACCTCGTTTCA
AGAAACAACCAGAGCCCTTCACACTCGCCTCTGAAGTCCTCAGAAAGCAGCATGAACTGC
AGCAGCTGCGAGGGCCTCAGCTGCCAGCAGACCCGGGTGCTGCAGGAGAAGCTACGCAAG
CTGAAGGAAGCCATGCTGTGCATGGTGTGCTGCGAGGAGGAGATCAACTCCACCTTCTGT
CCCTGTGGCCACACTGTGTGCTGTGAGAGCTGCGCCGCCCAGCTACAGTCATGTCCCGTC
TGCAGGTCGCGTGTGGAGCATGTCCAGCACGTCTATCTGCCAACGCACACCAGTCTTCTC
AATCTGACTGTAATCTAA
   >NDUFV3-202 cds:protein_coding (SEQ ID NO: 17) 
ATGGCTGCCCCGTGTTTGCTGCGGCAAGGACGAGCCGGGGCGCTGAAGACTATGCTCCAG
GAAGCCCAGGTGTTTCGAGGACTTGCTTCTACGGTTTCTTTGTCTGCGGAATCAGGGAAG
AGTGAAAAGGGTCAGCCACAGAATTCCAAGAAGCAAAGTCCACCAAAAAATGTAGTGGAA
CCAAAGGAGAGGGGCAAGCTCCTAGCCACCCAGACAGCAGCTGAATTGTCTAAAAACTTA
TCTTCACCCAGTTCTTACCCGCCAGCTGTGAATAAGGGCAGGAAGGTAGCTAGTCCCAGT
CCCAGTGGCAGCGTGCTATTCACAGATGAAGGGGTTCCGAAATTTTTGTCAAGAAAGACT
TTGGTAGAGTTTCCACAGAAAGTTCTGTCTCCATTCAGAAAACAGGGCTCTGATTCAGAA
GCTCGTCAGGTGGGTCGGAAAGTGACGTCGCCTTCGTCTTCATCCTCTTCCAGCTCCTCT
GATTCTGAATCTGATGATGAGGCTGACGTTTCAGAGGTCACTCCTCGAGTGGTGAGCAAA
GGCAGAGGGGGGCTTCGAAAACCAGAGGCCTCTCATTCCTTTGAAAACAGAGCCCCCCGA
GTTACAGTATCAGCAAAAGAGAAAACCTTGCTGCAGAAGCCGCATGTGGACATTACTGAT
CCAGAGAAGCCCCACCAGCCAAAGAAGAAAGGGTCCCCTGCTAAGCCATCAGAAGGCAGG
GAAAATGCGAGACCAAAAACCACAATGCCCAGATCTCAAGTAGATGAAGAGTTTTTGAAG
CAAAGTTTAAAGGAAAAACAATTGCAGAAAACATTTAGATTAAATGAAATAGATAAAGAA
AGCCAAAAGCCATTTGAAGTTAAAGGACCCTTACCTGTCCACACAAAATCAGGGTTGTCT
GCGCCACCGAAGGGCAGCCCAGCGCCTGCTGTGTTGGCAGAAGAGGCCAGAGCAGAGGGG
CAGCTGCAAGCCAGTCCTCCTGGGGCGGCAGAGGGGCATCTGGAAAAACCCGTGCCAGAG
CCCCAGCGCAAGGCGGCCCCTCCCCTGCCCAGAAAGGAAACCTCAGGGACGCAGGGAATA
GAAGGCCACCTGAAGGGTGGACAGGCAATCGTGGAAGATCAGATACCACCAAGCAATTTG
GAGACAGTTCCTGTTGAGAATAACCACGGTTTCCATGAAAAGACAGCAGCGCTGAAGCTT
GAGGCCGAGGGCGAGGCCATGGAAGATGCAGCCGCGCCAGGGGACGACCGAGGCGGCACA
CAGGAGCCAGCCCCAGTGCCTGCTGAGCCGTTTGACAACACTACCTACAAGAACCTGCAG
CATCATGACTACAGCACGTACACCTTCTTAGACCTCAACCTCGAACTCTCAAAATTCAGG
ATGCCTCAGCCCTCCTCAGGCCGGGAGTCACCTCGACACTGA
   NMRK1-201 cds:protein_coding (SEQ ID NO: 18) 
ATGAAAACATTTATCATTGGAATCAGTGGTGTGACAAACAGTGGCAAAACAACACTGGCT
AAGAATTTGCAGAAACACCTCCCAAATTGCAGTGTCATATCTCAGGATGATTTCTTCAAG
CCAGAGTCTGAGATAGAGACAGATAAAAATGGATTTTTGCAGTACGATGTGCTTGAAGCA
CTTAACATGGAAAAAATGATGTCAGCCATTTCCTGCTGGATGGAAAGCGCAAGACACTCT
GTGGTATCAACAGACCAGGAAAGTGCTGAGGAAATTCCCATTTTAATCATCGAAGGTTTT
CTTCTTTTTAATTATAAGCCCCTTGACACTATATGGAATAGAAGCTATTTCCTGACTATT
CCATATGAAGAATGTAAAAGGAGGAGGAGTACAAGGGTCTATCAGCCTCCAGACTCTCCG
GGATACTTTGATGGCCATGTGTGGCCCATGTATCTAAAGTACAGACAAGAAATGCAGGAC
ATCACATGGGAAGTTGTGTACCTGGATGGAACAAAATCTGAAGAGGACCTCTTTTTGCAA
GTATATGAAGATCTAATACAAGAACTAGCAAAGCAAAAGTGTTTGCAAGTGACAGCATAA
   PI4KA-201 cds:protein_coding (SEQ ID NO: 19) 
ATGGCGGCGGCCCCGGCCCGGGGAGGCGGAGGCGGAGGCGGAGGCGGCGGCGGCTGCTCC
GGCTCCGGCTCCAGCGCCTCGCGGGGCTTCTATTTCAACACGGTCCTGTCACTGGCCCGC
TCCCTGGCGGTGCAGAGACCAGCATCCTTGGAGAAGGTCCAAAAGCTTCTTTGCATGTGT
CCAGTGGATTTCCATGGGATCTTCCAGTTAGATGAAAGACGGAGAGATGCAGTGATTGCA
TTGGGCATTTTTCTGATTGAATCTGATCTTCAGCACAAAGATTGTGTGGTTCCTTACCTT
CTTCGACTTCTCAAAGGTCTTCCAAAAGTGTATTGGGTAGAAGAAAGCACAGCTCGGAAA
GGCAGAGGTGCCCTCCCGGTTGCAGAGAGCTTCAGCTTCTGCTTGGTAACTCTGCTGTCT
GATGTGGCCTATAGGGATCCTTCACTTAGGGATGAGATTTTAGAGGTGCTTTTGCAGGTT
TTGCATGTCCTCTTGGGGATGTGCCAGGCCTTGGAGATTCAAGACAAAGAATACCTTTGC
AAGTATGCTATCCCATGCCTGATAGGAATCTCGCGAGCATTTGGGCGTTACAGCAACATG
GAAGAGTCTCTCCTCTCAAAGCTCTTTCCCAAAATCCCTCCTCATTCCCTCCGTGTCCTG
GAAGAGCTTGAAGGTGTTCGAAGGCGTTCCTTTAATGACTTCCGCTCCATCCTCCCCAGC
 
AATCTGCTGACTGTCTGTCAGGAGGGTACCCTGAAGAGGAAAACCAGCAGTGTGTCCAGC
ATCTCTCAGGTCAGCCCTGAACGCGGCATGCCCCCTCCCAGTTCCCCTGGAGGATCTGCC
TTTCACTACTTTGAAGCCTCCTGCTTGCCCGATGGGACTGCCCTAGAGCCTGAGTACTAC
TTTTCAACCATCAGCTCCAGCTTCTCAGTCTCTCCCCTTTTCAACGGTGTCACATATAAG
GAGTTTAACATTCCATTGGAAATGCTTCGGGAACTCTTAAACCTGGTGAAGAAGATCGTT
GAGGAGGCTGTTCTCAAATCTTTGGATGCCATTGTAGCCAGTGTGATGGAGGCCAACCCC
AGTGCTGATCTCTACTACACTTCCTTCAGTGACCCTCTCTACCTGACCATGTTCAAGATG
CTGCGTGACACTCTGTACTACATGAAGGACCTCCCGACCTCTTTTGTGAAGGAGATCCAT
GATTTTGTGCTGGAGCAGTTCAACACGAGCCAGGGGGAGCTCCAGAAGATTCTACATGAC
GCAGACCGGATCCACAATGAGCTGAGCCCCCTCAAACTGCGCTGTCAGGCGAATGCTGCC
TGTGTGGACCTCATGGTGTGGGCTGTGAAGGACGAGCAGGGTGCAGAAAACCTTTGCATC
AAGCTATCTGAGAAGCTGCAGTCCAAGACGTCCAGCAAAGTCATTATTGCTCACTTGCCC
CTGCTGATCTGCTGTCTGCAGGGTTTGGGCCGCCTGTGCGAGAGGTTCCCGGTGGTGGTG
CACTCTGTGACACCGTCCTTGCGAGACTTCCTGGTCATCCCGTCCCCAGTTCTGGTGAAG
CTCTACAAGTACCACAGTCAGTACCACACAGTTGCTGGCAATGATATAAAAATCAGTGTG
ACCAATGAGCATTCCGAGTCAACCCTGAACGTCATGTCGGGTAAGAAGAGCCAGCCCTCC
ATGTACGAGCAGCTCCGAGACATCGCTATTGACAACATCTGCAGGTGCCTGAAGGCTGGA
TTGACGGTGGACCCAGTGATTGTGGAGGCGTTCTTGGCCAGCCTGTCCAACCGGCTCTAC
ATCTCTCAGGAGAGCGACAAGGACGCTCACTTGATTCCCGACCACACAATCCGAGCCTTG
GGACACATTGCGGTGGCCTTGAGGGACACCCCGAAGGTCATGGAGCCCATTCTGCAGATC
CTACAGCAGAAATTCTGCCAGCCACCCTCCCCCCTCGATGTGCTGATTATTGACCAGCTG
GGCTGCCTGGTTATCACCGGAAATCAATACATCTATCAGGAAGTGTGGAACCTCTTCCAG
CAGATCAGTGTGAAGGCCAGCTCCGTTGTATACTCAGCCACCAAAGATTACAAGGACCAC
GGCTATAGGCATTGCTCCCTGGCAGTGATTAATGCCCTGGCCAACATCGCGGCCAACATC
CAAGACGAGCACCTGGTGGATGAGCTGCTCATGAACCTGTTGGAGTTGTTTGTGCAGCTG
GGGCTGGAGGGGAAGCGAGCCAGCGAGAGGGCAAGCGAGAAGGGCCCTGCCCTAAAGGCT
TCTAGCAGTGCAGGGAACTTGGGAGTACTCATTCCTGTAATAGCTGTGCTCACCCGACGA
CTGCCACCCATCAAAGAAGCTAAGCCTCGGTTACAGAAGCTCTTCCGAGACTTCTGGCTG
TATTCCGTTCTGATGGGATTCGCTGTGGAGGGCTCAGGACTCTGGCCAGAAGAATGGTAC
GAGGGGGTCTGTGAAATAGCCACTAAGTCCCCCTTGCTCACCTTTCCCAGCAAGGAGCCA
CTGCGGTCCGTCCTCCAGTATAACTCAGCCATGAAGAATGACACGGTCACCCCCGCTGAG
CTGAGTGAGCTCCGCAGCACTATCATCAACCTGCTGGACCCCCCTCCCGAGGTGTCCGCA
CTCATCAACAAGCTGGACTTCGCCATGTCCACCTACCTCCTCTCTGTGTACCGGCTGGAG
TACATGAGGGTACTGCGTTCAACAGATCCTGATCGCTTCCAGGTAATGTTCTGCTACTTT
GAGGATAAAGCTATTCAGAAAGACAAATCTGGGATGATGCAGTGTGTGATTGCAGTCGCG
GACAAAGTATTCGATGCCTTCCTGAACATGATGGCGGATAAAGCCAAGACCAAGGAGAAC
GAGGAGGAGCTGGAGCGGCACGCTCAGTTCCTGTTGGTGAACTTCAACCACATCCACAAG
AGGATAAGGAGGGTGGCAGACAAGTATCTATCTGGTCTGGTGGATAAGTTTCCCCACTTG
CTCTGGAGCGGGACTGTGCTGAAGACCATGCTGGACATCCTGCAGACCCTGTCACTGTCA
CTGAGCGCTGATATTCACAAGGATCAGCCTTACTATGACATCCCCGACGCCCCCTACCGG
ATCACGGTTCCTGACACGTACGAAGCCCGTGAGAGCATTGTGAAGGACTTCGCTGCACGC
TGTGGGATGATCCTCCAGGAGGCCATGAAGTGGGCACCTACCGTCACCAAGTCCCACCTG
CAGGAATATCTGAACAAACATCAGAACTGGGTATCGGGACTGTCCCAGCACACGGGGCTG
GCCATGGCCACTGAGAGCATCCTTCACTTTGCTGGCTACAACAAGCAGAACACAACTCTT
GGGGCAACTCAGCTGAGCGAGCGCCCGGCCTGTGTGAAGAAAGACTACTCCAACTTCATG
GCATCCCTGAATCTGCGCAACCGCTACGCGGGCGAGGTGTATGGAATGATTCGGTTCTCA
GGCACCACAGGCCAGATGTCTGACCTGAACAAAATGATGGTCCAGGATCTACATTCAGCT
TTAGACCGCAGTCATCCTCAGCACTACACGCAGGCCATGTTCAAGCTGACCGCAATGCTC
ATTAGCAGTAAAGATTGTGACCCGCAGCTCCTTCATCATCTGTGCTGGGGTCCCCTCCGG
ATGTTCAATGAGCATGGCATGGAGACGGCCCTGGCCTGCTGGGAGTGGCTGCTGGCTGGC
AAGGATGGAGTGGAAGTGCCGTTCATGCGGGAGATGGCAGGGGCCTGGCACATGACGGTG
GAGCAGAAATTTGGCCTGTTTTCTGCTGAGATAAAGGAAGCAGACCCCCTGGCTGCCTCG
 
GAAGCAAGTCAACCCAAACCCTGTCCCCCCGAAGTGACCCCCCACTACATCTGGATCGAC
TTCCTGGTGCAGCGGTTTGAGATCGCCAAGTACTGCAGCTCTGACCAAGTGGAGATCTTC
TCCAGCCTGCTGCAGCGCTCCATGTCCCTGAACATCGGCGGGGCCAAGGGGAGCATGAAC
CGGCACGTGGCGGCCATCGGGCCCCGCTTCAAGCTGCTGACCCTGGGGCTGTCCCTCCTG
CATGCCGATGTGGTTCCAAATGCAACCATCCGCAATGTGCTTCGCGAGAAGATCTACTCC
ACTGCCTTTGACTACTTCAGCTGTCCCCCAAAGTTCCCTACTCAAGGAGAGAAGCGGCTG
CGTGAAGACATAAGCATCATGATTAAATTTTGGACCGCCATGTTCTCAGATAAGAAGTAC
CTGACCGCCAGCCAGCTTGTTCCCCCAGATAATCAGGACACCCGGAGCAACCTGGACATA
ACTGTCGGCTCTCGGCAACAAGCCACCCAAGGCTGGATCAACACATACCCCCTGTCCAGC
GGCATGTCCACCATCTCCAAGAAATCAGGCATGTCTAAGAAAACCAACCGGGGCTCCCAG
CTGCACAAATACTACATGAAGCGCAGGACGCTGCTGCTGTCCCTGCTGGCCACTGAGATC
GAGCGTCTCATCACATGGTACAACCCGCTGTCAGCCCCGGAACTGGAACTAGACCAGGCC
GGAGAGAACAGCGTGGCCAACTGGAGATCTAAGTACATCAGCCTGAGTGAGAAGCAGTGG
AAGGACAACGTGAACCTCGCCTGGAGCATCTCTCCCTACCTAGCCGTGCAGCTGCCTGCC
AGGTTTAAGAACACAGAAGCCATTGGAACGAAGTGACCCGTCTCGTTCGGTTGGACCCG
GGAGCCGTTAGTGATGTGCCTGAAGCAATCAAGTTCCTGGTCACCTGGCACACCATCGAC
GCCGATGCTCCAGAGCTCAGCCATGTGCTGTGCTGGGCGCCCACGGACCCACCCACAGGC
CTCTCCTACTTCTCCAGCATGTACCCGCCGCACCCTCTCACGGCGCAGTACGGGGTGAAA
GTCCTGCGGTCCTTCCCTCCGGACGCCATCCTCTTCTACATCCCCCAGATTGTGCAGGCC
CTCAGGTACGACAAGATGGGCTATGTGCGGGAGTATATTCTGTGGGCAGCGTCTAAATCC
CAGCTTCTGGCACACCAGTTCATCTGGAACATGAAGACTAACATTTATCTAGATGAAGAG
GGCCACCAGAAAGACCCTGACATCGGCGACCTCCTGGATCAGTTGGTAGAGGAGATCACA
GGCTCCTTGTCCGGCCCAGCGAAGGACTTTTACCAGCGGGAGTTTGATTTCTTTAACAAG
ATCACCAACGTGTCGGCTATCATCAAGCCCTACCCTAAAGGCGACGAGAGAAAGAAGGCT
TGTCTGTCGGCCCTGTCTGAAGTGAAGGTGCAGCCGGGCTGCTACCTGCCCAGCAACCCT
GAGGCCATTGTGCTGGACATCGACTACAAGTCTGGGACCCCGATGCAGAGTGCTGCAAAA
GCCCCATATCTGGCCAAGTTCAAGGTGAAGCGATGTGGAGTTAGTGAACTTGAAAAAGAA
GGTCTGCGGTGCCGCTCAGACTCCGAGGATGAGTGCAGCACGCAGGAGGCCGACGGCCAG
AAGATCTCCTGGCAGGCAGCCATCTTCAAGGTGGGAGACGACTGCCGGCAGGACATGCTG
GCCCTGCAGATCATCGACCTCTTCAAGAACATCTTCCAGCTGGTCGGCCTGGACCTCTTT
GTTTTTCCCTACCGCGTGGTGGCCACTGCCCCTGGGTGCGGGGTGATCGAGTGCATCCCC
GACTGCACCTCCCGGGACCAGCTGGGCCGCCAGACAGACTTCGGCATGTACGACTACTTC
ACACGCCAGTACGGGGATGAGTCCACTCTGGCCTTCCAGCAGGCCCGCTACAACTTCATC
CGAAGCATGGCCGCCTACAGCCTCCTGCTGTTCCTGCTGCAGATCAAGGACAGACACAAC
GGCAACATTATGCTGGACAAGAAGGGTCATATCATCCACATCGACTTTGGCTTCATGTTT
GAAAGCTCGCCGGGCGGCAATCTCGGCTGGAACCCGACATCAAGCTGACGGATGAGATG
GTGATGATCATGGGGGGCAAGATGGAGGCCACACCCTTCAAGTGGTTCATGGAGATGTGT
GTCCGAGGCTACCTGGCTGTGCGGCCCTACATGGACGCGGTCGTCTCCCTGGTCACTCTC
ATGTTGGACACGGGCCTGCCCTGTTTTCGCGGCCAGACAATCAAGCTCTTGAAGCACAGG
TTTAGCCCCAACATGACTGAGCGCGAGGCTGCAAATTTCATCATGAAGGTCATCCAGAGC
TGCTTCCTCAGCAACAGGAGCCGGACCTACGACATGATCCAGTACTATCAGAATGACATC
CCCTACTGA
   PRTFDC1-201 cds:protein_coding (SEQ ID NO: 20) 
ATGGCCGGGAGCAGCGAGGAGGCGCCAGACTACGGGCGAGGCGTCGTGATTATGGATGAT
TGGCCAGGGTATGACTTGAATTTATTCACGTACCCACAGCACTATTATGGAGACTTGGAG
TATGTCCTCATCCCTCATGGTATCATTGTGGACAGAATTGAGCGGCTGGCCAAGGATATT
ATGAAAGACATAGGATATAGTGACATCATGGTCCTGTGTGTGCTTAAAGGAGGTTACAAA
TTCTGTGCTGATCTCGTAGAACACCTTAAGAACATCAGCCGAAATTCAGATCGATTTGTC
TCAATGAAGGTTGATTTCATCAGACTAAAAAGTTACAGGAATGACCAGTCCATGGGTGAG
ATGCAGATAATCGGAGGCGATGATCTTTCAACGCTGGCTGGAAAGAATGTTCTCATTGTT
 
GAGGATGTTGTCGGAACTGGGAGGACCATGAAAGCACTACTCAGCAATATAGAGAAATAC
AAGCCCAACATGATTAAGGTAGCCAGTTTGTTGGTGAAGAGAACATCCAGAAGTGACGGC
TTTAGACCTGACTATGCTGGATTTGAGATTCCAAACTTATTTGTGGTGGGATATGCCTTA
GATTACAATGAATACTTCAGAGATCTGAATCACATATGCGTCATCAATGAGCACGGTAAA
GAAAAATATCGAGTCTAA
   PYGO2-202 cds:protein_coding (SEQ ID NO: 21) 
ATGGCCGCCTCGGCGCCGCCCCCACCGGACAAGCTGGAGGAGGTGGCGGCCCCGCACCG
CCCCCTGCGCCGCCCAGCACCGGGAGGAAGCAGGGCAAGGCCGGTCTGCAAATGAAGAGT
CCAGAAAAGAAGCGAAGGAAGTCAAATACTCAGGGCCCTGCATACTCACATCTGACGGAG
TTTGCACCACCCCCAACTCCCATGGTGGATCACCTGGTTGCATCCAACCCTTTTGAAGAT
GACTTCGGAGCCCCCAAAGTGGGGGTTGCAGCCCCTCCATTCCTTGGCAGTCCTGTGCCC
TTCGGAGGCTTCCGTGTGCAGGGGGGCATGGCGGGCCAGGTACCCCCAGGCTACAGCACT
GGAGGTGGAGGGGGCCCCCAGCCACTCCGTCGACAGCCACCCCCCTTCCCTCCCAATCCT
ATGGGCCCTGCTTTCAACATGCCCCCCCAGGGTCCTGGCTACCCACCCCCAGGCAACATG
AACTTTCCCAGCCAACCCTTCAACCAGCCTCTGGGTCAAAACTTTAGTCCTCCCAGTGGG
CAGATGATGCCGGGCCCAGTGGGGGGATTTGGTCCCATGATCTCACCCACCATGGGACAG
CCTCCCAGAGCAGAGCTGGGCCCACCTTCTCTGTCCCAACGATTTGCTCAGCCAGGGGCT
CCTTTTGGCCCTTCTCCTCTCCAGAGACCTGGTCAGGGGCTCCCCAGCCTGCCGCCTAAC
ACAAGTCCCTTTCCTGGTCCGGACCCTGGCTTTCCTGGCCCTGGTGGTGAGGATGGGGGG
AAGCCCTTGAATCCACCTGCTTCTACTGCTTTTCCCCAGGAGCCCCACTCAGGCTCCCCG
GCTGCTGCTGTTAATGGGAACCAGCCCAGTTTCCCCCCGAACAGCAGTGGGCGGGGTGGG
GGCACTCCAGATGCCAACAGCTTGGCACCCCCTGGCAAGGCAGGTGGGGGCTCCGGGCCC
CAGCCTCCCCCAGGCTTGGTGTACCCATGTGGTGCCTGTCGGAGTGAGGTGAACGATGAC
CAGGATGCCATTCTGTGTGAGGCCTCCTGCCAGAAATGGTTCCACCGTGAGTGCACAGGC
ATGACTGAGAGCGCCTATGGGCTGCTGACCACTGAAGCTTCTGCCGTCTGGGCCTGCGAT
CTCTGCCTCAAGACCAAGGAGATCCAGTCTGTCTACATCCGTGAGGGCATGGGGCAGCTG
GTGGCTGCTAACGATGGGTCA
   RNF149-201 cds:protein_coding (SEQ ID NO: 22) 
ATGGCGTGGCGGCGGCGCGAAGCCAGCGTCGGGGCTCGCGGCGTGTTGGCTCTGGCGTTG
CTCGCCCTGGCCCTGTGCGTGCCCGGGCCCGGGGCCGGGCTCTCGAGTGGTTCTCGGCC
GTGGTAAACATCGAGTACGTGGACCCGCAGACCAACCTGACGGTGTGGAGCGTCTCGGAG
AGTGGCCGCTTCGGCGACAGCTCGCCCAAGGAGGGCGCGCATGGCCTGGTGGGCGTCCCG
TGGGCGCCCGGCGGAGACCTCGAGGGCTGCGCGCCCGACACGCGCTTCTTCGTGCCCGAG
CCCGGCGGCCGAGGGGCCGCGCCCTGGGTCGCCCTGGTGGCTCGTGGGGGCTGCACCTTC
AAGGACAAGGTGCTGGTGGCGGCGCGGAGGAACGCCTCGGCCGTCGTCCTCTACAATGAG
GAGCGCTACGGGAACATCACCTTGCCCATGTCTCACGCGGGAACAGGAAATATAGTGGTC
ATTATGATTAGCTATCCAAAAGGAAGAGAAATTTTGGAGCTGGTGCAAAAAGGAATTCCA
GTAACGATGACCATAGGGGTTGGCACCCGGCATGTACAGGAGTTCATCAGCGGTCAGTCT
GTGGTGTTTGTGGCCATTGCCTTCATCACCATGATGATTATCTCGTTAGCCTGGCTAATA
TTTTACTATATACAGCGTTTCCTATATACTGGCTCTCAGATTGGAAGTCAGAGCCATAGA
AAAGAAACTAAGAAAGTTATTGGCCAGCTTCTACTTCATACTGTAAAGCATGGAGAAAAG
GGAATTGATGTTGATGCTGAAAATTGTGCAGTGTGTATTGAAAATTTCAAAGTAAAGGAT
ATTATTAGAATTCTGCCATGCAAGCATATTTTTCATAGAATATGCATTGACCCATGGCTT
TTGGATCACCGAACATGTCCAATGTGTAAACTTGATGTCATCAAAGCCCTAGGATATTGG
GGAGAGCCTGGGGATGTACAGGAGATGCCTGCTCCAGAATCTCCTCCTGGAAGGGATCCA
GCTGCAAATTTGAGTCTAGCTTTACCAGATGATGACGGAAGTGATGACAGCAGTCCACCA
TCAGCCTCCCCTGCTGAATCTGAGCCACAGTGTGATCCCAGCTTTAAAGGAGATGCAGGA
GAAAATACGGCATTGCTAGAAGCCGGCAGGAGTGACTCTCGGCATGGAGGACCCATCTCC
 TAG
   >TENT5A-201 cds:protein_coding (SEQ ID NO: 23) 
ATGGCGGAGGGTGAAGGGTACTTCGCCATGTCTGAGGACGAGCTGGCCTGCAGCCCCTAC
ATCCCCCTAGGCGGCGACTTCGGCGGCGGCGACTTCGGCGGCGGCGACTTCGGCGGCGGC
GACTTCGGCGGTGGCGGCAGCTTCGGTGGCATTGCTTGGACTATTGCGAAAGCCCTACG
GCGCACTGCAATGTGCTGAACTGGGAGCAAGTGCAGCGGCTGGACGGCATCCTGAGCGAG
ACCATTCCGATTCACGGGCGCGGCAACTTCCCCACGCTCGAGCTGCAGCCGAGCCTGATC
GTGAAGGTGGTGCGGCGGCGCCTGGCCGAGAAGCGCATTGGCGTCCGCGACGTGCGCCTC
AACGGCTCGGCAGCCAGCCATGTCCTGCACCAGGACAGCGGCCTGGGCTACAAGGACCTG
GACCTCATCTTCTGCGCCGACCTGCGCGGGAAGGGGAGTTTCAGACTGTGAAGGACGTC
GTGCTGGACTGCCTGTTGGACTTCTTACCCGAGGGGGTGAACAAAGAGAAGATCACACCA
CTCACGCTCAAGGAAGCTTATGTGCAGAAAATGGTTAAAGTGTGCAATGACTCTGACCGA
TGGAGTCTTATATCCCTGTCAAACAACAGTGGCAAAAATGTGGAACTGAAATTTGTGGAT
TCCCTCCGGAGGCAGTTTGAATTCAGTGTAGATTCTTTTCAAATCAAATTAGACTCTCTT
CTGCTCTTTTATGAATGTTCAGAGAACCCAATGACTGAGACATTTCACCCCACAATAATC
GGGGAGAGCGTCTATGGCGATTTCCAGGAAGCCTTTGATCACCTTTGTAACAAGATCATT
GCCACCAGGAACCCAGAGGAAATCCGAGGGGGAGGCCTGCTTAAGTACTGCAACCTCTTG
GTGAGGGGCTTTAGGCCCGCCTCTGATGAAATCAAGACCCTTCAAAGGTATATGTGTTCC
AGGTTTTTCATCGACTTCTCAGACATTGGAGAGCAGCAGAGAAAACTGGAGTCCTATTTG
CAGAACCACTTTGTGGGATTGGAAGACCGCAAGTATGAGTATCTCATGACCCTTCATGGA
GTGGTAAATGAGAGCACAGTGTGCCTGATGGGACATGAAAGAAGACAGACTTTAAACCTT
ATCACCATGCTGGCTATCCGGGTGTTAGCTGACCAAAATGTCATTCCTAATGTGGCTAAT
GTCACTTGCTATTACCAGCCAGCCCCCTATGTAGCAGATGCCAACTTTAGCAATTACTAC
ATTGCACAGGTTCAGCCAGTATTCACGTGCCAGCAACAGACCTACTCCACTTGGCTACCC
TGCAATTAA
   >TFIP11-204 cds:protein_coding (SEQ ID NO: 24) 
ATGTCATTGTCCCACTTATACCGGGATGGGAAGGCCGCATTGATGATGATGATGACGAG
CGGGAGAACTTTGAGATCACTGACTGGGATCTCCAGAATGAGTTCAACCCCAACCGACAG
CGCCACTGGCAGACCAAGGAAGAAGCCACCTACGGGGTGTGGCAGAGCGAGACTCGGAT
GATGAGAGGCCCAGCTTTGGAGGCAAACGGGCCCGTGACTACTCTGCGCCAGTCAACTTC
ATCAGCGCAGGGCTCAAGAAAGGGGCAGCGGAGGAGGCAGAGTTGGAAGATTCTGATGAC
GAAGAGAAACCTGTTAAGCAGGACGACTTTCCTAAGGATTTTGGACCAAGGAAGCTAAAA
ACGGGTGGCAATTTTAAGCCCAGCCAGAAAGGTTTTGCAGGAGGAACCAAATCTTTCATG
GACTTCGGCAGCTGGGAAAGACACACAAAAGGAATTGGACAGAAGCTTCTTCAGAAGATG
GGCTACGTCCCTGGACGGGGCCTCGGGAAGAATGCACAAGGTATCATTAACCCAATTGAA
GCCAAGCAGAGAAAGGGAAAAGGTGCTGTGGGGGCTTATGGATCCGAGCGCACCACTCAG
TCCATGCAAGACTTCCCTGTGGTTGACTCAGAGGAAGAAGCTGAAGAGGAGTTTCAGAAG
GAGCTGAGCCAGTGGAGGAAAGACCCAAGTGGAAGCAAGAAGAAGCCCAAATACTCTTAC
AAGACCGTGGAAGAGTTGAAGGCCAAGGGCAGGATTAGCAAGAAGCTCACTGCTCCCCAG
AAGGAACTTTCTCAAGTCAAGGTCATAGACATGACAGGCCGGGAGCAGAAGGTCTACTAC
AGCTACAGTCAGATCAGCCACAAGCACAACGTTCCCGATGATGGGCTGCCGCTACAGTCC
CAACAGCTGCCACAGTCTGGCAAAGAGGCCAAGGCCCCCGGCTTCGCGCTGCCCGAGCTG
GAGCACAACCTGCAGCTGCTCATCGACCTCACGGAGCAGGAGATCATCCAGAATGACCGG
CAGCTACAGTATGAGCGGGACATGGTGGTCAACCTCTTCCACGAGCTGGAGAAGATGACC
GAGGTCCTGGACCACGAGGAGCGGGTCATCTCGAACCTCAGCAAGGTCCTGGAGATGGTG
GAGGAGTGCGAGCGGCGGATGCAGCCCGACTGCAGCAACCCCCTCACCCTGGACGAGTGT
GCCCGCATCTTCGAAACCCTGCAGGACAAGTACTATGAGGAGTACAGGATGTCCGACCGT
GTGGACCTTGCTGTGGCCATCGTCTATCCACTCATGAAGGAGTACTTCAAGGAGTGGGAT
CCCCTCAAAGACTGCACTTATGGCACCGAGATCATCTCTAAGTGGAAAAGCCTCCTAGAG
 
AATGACCAGCTCTTGTCCCATGGCGGACAGGACCTCTCAGCAGATGCCTTTCACAGGTTG
ATATGGAAGTCTGGATGCCTTTTGTTCGAAATATTGTCACCCAGTGGCAGCCAAGGAAC
TGTGACCCGATGGTGGACTTTTTGGATAGTTGGGTGCACATTATTCCTGTGTGGATCTTA
GATAACATACTGGACCAACTCATCTTCCCCAAGCTGCAAAAGGAGGTGGAAAACTGGAAC
CCGCTCACAGACACTGTTCCCATCCACTCTTGGATCCACCCATGGCTGCCCCTTATGCAG
GCACGGCTGGAGCCACTCTATTCCCCCATCCGTAGTAAGCTGTCCAGCGCCCTGCAGAAG
TGGCACCCCAGCGACTCCTCTGCCAAGCTCATCCTCCAGCCCTGGAAGGATGTCTTCACT
CCTGGCTCCTGGGAAGCATTCATGGTCAAAAACATAGTGCCCAAGCTGGGGATGTGTCTT
GGTGAGCTAGTCATTAACCCCCACCAGCAGCACATGGATGCATTCTATTGGGTGATTGAC
TGGGAAGGGATGATCTCTGTCTCTAGCCTGGTGGGACTTCTTGAAAAGCACTTCTTCCCC
AAGTGGCTTCAGGTGCTGTGCTCTTGGCTCAGTAACAGCCCAAATTATGAGGAGATCACC
AAGTGGTACCTGGGTTGGAAGTCGATGTTCTCAGACCAAGTGCTGGCACATCCATCTGTC
AAGGACAAATTTAATGAGGCACTTGATATCATGAACCGGGCGGTGTCCTCCAACGTTGGT
GCCTACATGCAGCCAGGAGCACGGGAGAACATTGCCTATCTCACCCACACGGAGCGGAGG
AAGGACTTCCAGTACGAGGCCATGCAGGAGAGGCGGGAGGCTGAGAACATGGCTCAGAGG
GGCATTGGCGTGGCCGCTAGCTCTGTGCCCATGAACTTTAAGGACCTCATTGAGACCAAG
GCTGAGGAGCACAACATTGTCTTCATGCCCGTCATTGGGAAGCGACACGAAGGAAGCAG
CTCTACACCTTTGGCCGCATTGTGATCTACATCGACCGGGGAGTGGTCTTTGTCCAGGGC
GAGAAGACGTGGGTGCCCACCTCACTGCAGAGCCTGATCGACATGGCCAAGTGA
   >TRIM21-201 cds:protein_coding (SEQ ID NO: 25) 
ATGGCTTCAGCAGCACGCTTGACAATGATGTGGGAGGAGGTCACATGCCCTATCTGCCTG
GACCCCTTCGTGGAGCCTGTGAGCATCGAGTGTGGCCACAGCTTCTGCCAGGAATGCATC
TCTCAGGTTGGGAAAGGTGGGGGCAGCGTCTGTCCTGTGTGCCGGCAGCGCTTTCTGCTC
AAGAATCTCCGGCCCAATCGACAGCTAGCCAACATGGTGAACAACCTTAAAGAAATCAGC
CAGGAGGCCAGAGAGGGCACACAGGGGGAACGGTGTGCAGTGCATGGAGAGAGACTTCAC
CTGTTCTGTGAGAAAGATGGGAAGGCCCTTTGCTGGGTATGTGCCCAGTCTCGGAAACAC
CGTGACCACGCCATGGTCCCTCTTGAGGAGGCTGCACAGGAGTACCAGGAGAAGCTCCAG
GTGGCATTAGGGGAACTGAGAAGAAAGCAGGAGTTGGCTGAGAAGTTGGAAGTGGAAATT
GCAATAAAGAGAGCAGACTGGAAGAAAACAGTGGAAACACAGAAATCTAGGATTCACGCA
GAGTTTGTGCAGCAAAAAAACTTCCTGGTTGAAGAAGAACAGAGGCAGCTGCAGGAGCTG
GAGAAGGATGAGAGGGAGCAGCTGAGAATCCTGGGGGAGAAAGAGGCCAAGCTGGCCCAG
CAGAGCCAGGCCCTACAGGAGCTCATCTCAGAGCTAGATCGAAGGTGCCACAGCTCAGCA
CTGGAACTGCTGCAGGAGGTGATAATTGTCCTGGAAAGGAGTGAGTCCTGGAACCTGAAG
GACCTGGATATTACCTCTCCAGAACTCAGGAGTGTGTGCCATGTGCCAGGGCTGAAGAAG
ATGCTGAGGACATGTGCAGTCCACATCACTCTGGATCCAGACACAGCCAATCCGTGGCTG
ATACTTTCAGAAGATCGGAGACAAGTGAGGCTTGGAGACACCCAGCAGAGCATACCTGGA
AATGAAGAGAGATTTGATAGTTATCCTATGGTCCTGGGTGCCCAGCACTTTCACTCTGGA
AAACATTACTGGGAGGTAGATGTGACAGGAAAGGAGGCCTGGGACCTGGGTGTCTGCAGA
GACTCTGTGCGCAGGAAGGGGCACTTTTTGCTTAGTTCCAAGAGTGGCTTCTGGACAATT
TGGTTGTGGAACAAACAAAAATATGAGGCTGGCACCTACCCCCAGACTCCCCTCCACCTT
CAGGTGCCTCCATGCCAAGTTGGGATTTTCCTGGACTATGAGGCTGGCATGGTCTCCTTC
TACAACATCACTGACCATGGCTCCCTCATCTACTCCTTCTCTGAATGTGCCTTTACAGGA
CCTCTGCGGCCCTTCTTCAGTCCTGGTTTCAATGATGGAGGAAAAAACACAGCCCCTCTA
ACCCTCTGTCCACTGAATATTGGATCACAAGGATCCACTGACTATTGA
   USB1-201 cds:protein coding (SEQ ID NO: 26) 
ATGAGCGCGGCGCCCCTGGTGGGCTACAGCAGCAGCGGCTCCGAGGATGAGTCCGAGGAC
GGGATGCGGACCAGGCCGGGGGATGGAGCCACCGTCGTGGCCAGAGCCCCCTTCCCAGG
 
CAGAGATTTCCAGTACCTGACAGTGTGCTGAACATGTTCCCGGCACCGAGGAGGGGCCT
GAAGATGACAGCACAAAACACGGGGACGGTGCGCACCTTCCCCCACGAGCGAGGCAAC
TGGGCCACCCACGTCTATGTACCATATGAAGCCAAGGAGGAGTTCCTGGATCTGCTTGAT
GTGTTGCTGCCCCATGCCCAGACATATGTCCCCCGGCTGGTAAGGATGAAGGTGTTCCAC
CTCAGCCTGTCCCAGAGTGTGGTTCTGCGCCACCACTGGATCCTCCCCTTCGTGCAGGCT
CTGAAAGCCCGTATGACCTCCTTCCACAGATTCTTCTTTACTGCCAACCAGGTAAAGATT
TACACCAATCAAGAGAAAACCAGGACCTTTATTGGGCTTGAGGTCACTTCAGGGCATGCC
CAGTTCCTGGACCTGGTTTCAGAGGTGGACAGAGTCATGGAGGAATTCAACCTCACCACT
TTCTACCAGGATCCTTCTTTCCACCTCAGCCTGGCCTGGTGTGTGGGTGATGCACGTCTC
CAGCTGGAGGGGCAGTGCCTGCAGGAACTACAGGCAATCGTGGATGGGTTTGAAGATGCT
GAGGTGCTGCTGCGCGTGCACACTGAGCAAGTCCGCTGCAAGTCTGGGAACAAGTTCTTC
TCGATGCCTTTGAAGTGA
   ENST00000364542.1 Y_RNA.250-201 cdna:misc_RNA (SEQ ID NO: 27) 
GGCTGGTCTGAACATAGTGAGTTATCTCAGTTGATTGTTCAGTCAGTTATAGATCGAACT
CCTTGTTCTACTCTTTCCCCCTTCTCACTACTGCACTTGACTACTCAAAA
   ENST00000391229.2 Y_RNA.645-201 cdna:misc_RNA (SEQ ID NO: 28) 
CGCTGGTCCAAGGGCAGTGGTGTTTACAACTAATTGATCACAATCAGTTACAGATTTCTT
TGTTCCTTTTCCATTCCCACTGCTTCACCTGACGAGCCTAAA
   >ENST00000415616.1 YWHAQP5-201 cdna:processed_pseudogene (SEQ ID NO: 29)

AAGGCAGAGCTGGCCCAGCAGGCTAAGCACTGTGACAACATGGCCACCTACACGAAGGCC
ATGACCAAGTGGGGCACCCAGCTGTCCAGCAAGGAGCGCAATCTGCTCTCAGTGGCCTAC
AAGTATGGGGTCAGGGACTACAGGTCCACCTGGAAGGACATTTTGGGCACTGAGCAGGAA
ACTGACACCTCCGACATGAAGCTGCTACTGATTAAGTACCATAGGGAGAAAGTGGAGTCT
GAGCTGAGATCCACCTGTCCCATGGTCTTGGAATTTTTGGATACACCATGGTCTTGGAAT
TTTGGGATAAGTATTATATTCAATAGCCAATGCAACTAATCCAGAGAGAACCGTCTTCTA
TCTGAAAGTAAAGGGGGATTACTTCCAGTATCTTGCTGAAGTTGCATGTGGTAATGATCA
AAAACAGAAGGTAGGTCAGGTACAGTGGCTCATGCCTATAATCCTAGCACTTTGGGAGGC
CGAGGTGGGTGAATCACCTGAGGTCAGGAGTTCGAGGCCAGCCTGGCCAACATGGCAAAA
CCCCACATCTACCAAAAATACAAAAATTAGCTGGGTGTGGTGGTGCATACCTGTAATCCC
AGCTACTCAGGAGGCTAAGGTAGGAGAATCACTTGAACCCAGAAGGCGGAGGTTGAGTGA
GCTGAGATAGCACCACTGTACTCCAGCCTGGGTGACAGAGCGAGACTCCATCTCAAAATA
AAAACAAAACAAAACAAACAAACAAAAAACACAGATGGTAGATAATTCCCAGGAGCTTAC
CAAGACACATTTGACAATTTGATAAGCAAGAGAGAGATGCAACCCCCCCCTTCCGATCTG
CCTGGGGCTGGCTCTTAACTTTTCTATATTTACTGTGAGATCCTTAACAACCCTGAGCT
CACCTGCCCACTGGCTAAAATGGCTTTTTTTTTTTTTTTTTTTTTGAGACAGAGTCTTG
CTCTGTCTCCTAGGCTGGAGTGCTGTAGTGCAATCTCAGCTCACTGCAACCTCCGCCTCC
TGGGTTCAAGTGATTCTTGTGCCTCAGCCTCCCGCGTAGCTGGACTACATGTGCTCACCA
TCATGCCAGGCTAATTTTTTGTATTTTTAGTAGAGACGAGGTTTCACCATGCTGGCCTGC
CTGGTCTCTAAATCCTGGCCTCAAGTGATCCACCCGCCTTTGCCTCCCAAAGTGCTGGGA
TTACAGGCCTGAGCCACCGCACCCAGCCCTAAAACAGCCTTTGATGAAGCCACTGCAGAA
CCTGATACACTGAAGACTCAGACAGCCCCCTCATCATGCAGTTGCTGAGAGACAACCTAA
TAACACTGTGGACATCACACAGTGCAGGAAAAGAACGCAATGCAGCAGAAGGGGCTGAAC
AC

## Claims

1. A method of determining risk of preeclampsia in a pregnant subject, the method comprising:
(a) obtaining cell-free ribonucleic acid (cfRNA) from a biological sample obtained from the pregnant subject;
(b) quantifying expression levels of the cfRNA for one or more genes selected from the genes listed in Table 24, wherein the one or more genes comprise CD163 or NSRP1;
(c) determining that the expression levels of the cfRNA for the one or more genes exhibit a change in expression associated with preeclampsia relative to reference expression levels; and
(d) identifying an increased risk of preeclampsia, based at least in part on the determining in (c).

2. The method of claim 1, wherein the one or more genes comprise CD163.

3. The method of claim 1, wherein the one or more genes comprise NSRP1.

4. The method of claim 1, wherein the one or more genes comprise two or more genes selected from the genes listed in Table 24.

5. The method of claim 4, wherein the two or more genes comprise CD163 and NSRP1.

6. The method of claim 4, wherein the one or more genes comprise three or more genes selected from the genes listed in Table 24.

7. The method of claim 1, wherein the biological sample is plasma.

8. The method of claim 1, wherein the biological sample is serum.

9. The method of claim 1, wherein the biological sample is urine, saliva, or amniotic fluid.

10. The method of claim 1, wherein the quantifying in (b) further comprises performing an amplification assay, a sequencing assay, or a hybridization assay.

11. The method of claim 10, wherein the quantifying in (b) further comprises performing the amplification assay; optionally wherein the amplification assay comprises quantitative real-time polymerase chain reaction (quantitative RT-PCR) or digital PCR.

12. The method of claim 10, wherein the quantifying in (b) further comprises performing the sequencing assay; optionally wherein the sequencing assay comprises massively parallel sequencing.

13. The method of claim 10, wherein the quantifying in (b) further comprises performing the hybridization assay.

14. The method of claim 1, further comprising performing reverse transcription on the cfRNA of (a) to produce complementary deoxyribonucleic acid (cDNA), and analyzing the cDNA to quantify the expression levels.

15. The method of claim 1, wherein the preeclampsia comprises severe preeclampsia.
